(19) 

Europäisches
Patentamt

European
Patent Office

Office européen
des brevets

(11) **EP 4 417 626 A1**

(12) **EUROPEAN PATENT APPLICATION**
published in accordance with Art. 153(4) EPC

(43) Date of publication:
**21.08.2024 Bulletin 2024/34**

(21) Application number: **22879952.4**

(22) Date of filing: **03.08.2022**

(51) International Patent Classification (IPC):
*C07K 19/00* (2006.01)   *C07K 14/55* (2006.01)
*C07K 1/107* (2006.01)   *A61K 38/20* (2006.01)
*A61K 47/68* (2017.01)   *A61P 35/00* (2006.01)
*A61P 37/02* (2006.01)

(52) Cooperative Patent Classification (CPC):
**A61K 38/20; A61K 47/68; A61P 35/00;
A61P 37/02; C07K 1/107; C07K 14/55; C07K 19/00**

(86) International application number:
**PCT/CN2022/109997**

(87) International publication number:
**WO 2023/061005 (20.04.2023 Gazette 2023/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.10.2021 CN 202111196510**

(71) Applicant: **Latticon (Suzhou) Biopharmaceuticals
Co., Ltd.
Suzhou, Jiangsu 215164 (CN)**

(72) Inventor: **LI, John Yuehua
Suzhou, Jiangsu 215164 (CN)**

(74) Representative: **Cabinet Grosset-Fournier &
Demachy
54, rue Saint Lazare
75009 Paris (FR)**

Remarks:
The complete document including Reference
Table(s) and the Sequence Listing(s) can be
downloaded from the EPO website

(54) **NOVEL ANTIBODY-CYTOKINE FUSION PROTEIN, PREPARATION METHOD THEREFOR AND USE THEREOF**

(57)   Provided is a novel antibody-cytokine fusion protein. Further provided are a bispecific molecule and an immunoconjugate comprising the antibody-cytokine fusion protein, as well as a nucleic acid molecule encoding the antibody-cytokine fusion protein, and a vector and a host cell comprising the nucleic acid molecule. Also provided are a method for preparing the antibody-cytokine fusion protein, a pharmaceutical composition containing the antibody-cytokine fusion protein, and a method and a use of the antibody-cytokine fusion protein or the pharmaceutical composition thereof in treating diseases.

EP 4 417 626 A1

**Description**

**FIELD OF THE INVENTION**

The present application relates to an antibody-cytokine fusion protein and uses thereof. Specifically, the present application relates to a fusion protein comprising an interleukin-2 (IL-2) and an anti-IL-2 antibody (an IL-2/anti-IL-2 antibody fusion protein), wherein the fusion protein is incapable of binding to the α subunit of IL-2 receptor (IL2Rα, CD25) and any IL-2 receptor complex comprising the α subunit (i.e., IL2Rα/β or IL2Rα/β/γ), but can bind to the heterodimeric IL-2 receptor consisting of the β subunit and γ subunit (IE2Rβ/γ) and activate the signaling pathway mediated by it. The present application further relates to a bispecific molecule and an immunoconjugate comprising the IL-2/anti-IL-2 antibody fusion protein, as well as a nucleic acid molecule encoding the IL-2/anti-IL-2 antibody fusion protein, and an expression vector and host cell comprising the said nucleic acid molecule. The present application also provides a method of preparation of the IL-2/anti-IL-2 antibody fusion protein, a pharmaceutical composition comprising the IL-2/anti-IL-2 antibody fusion protein, and a method and use for the treatment of diseases with the IL-2/anti-IL-2 antibody fusion protein or a pharmaceutical composition thereof.

**BACKGROUND OF THE INVENTION**

Interleukin-2 (IL-2) is a globular glycoprotein with a molecular mass of approximately 15.5 kDa. It has a dual function of inducing both immunoinhibitory and immunostimulatory reactions, and thus plays a crucial role in the regulation of development, survival, expansion and homeostasis of various immune cells. The mature IL-2 protein consists of 133 amino acid residues, which fold into four antiparallel amphipathic α-helices that further fold and form a higher-order structure essential for its functionality (Smith, Science 1988, 240: 1169-1176; Bazan, Science 1992, 257: 410-413).

IL-2 exerts its immunoregulatory function *in vivo* by binding to IL-2 receptor (IL2R) expressed on the surface of immune cells and activating downstream signaling cascades (Kreig, PNAS 2010, 107: 11906-11911). There are two different forms of IL2R, including the high-affinity IL2R and the intermediate-affinity IL2R. The high-affinity IL2R is a heterotrimer consisting of the IL-2 receptor α chain (IL2Rα, CD25), the IL-2 receptor β chain (IL2Rβ, CD122) and a common γ chain (yc, CD132) and it binds to IL-2 at a binding affinity ($K_D$) of approximately $10^{-11}$ M. The intermediate-affinity IL2R is a heterodimer consisting of only IL2Rβ and yc and it binds to IL-2 at a binding affinity ($K_D$) of about $10^{-9}$ M (Wang, Science 2005, 310:1159-1163; Boyman, Nat Rev Immunol 2012, 12:180-190; Arenas-ramirez N, Trends Immunol 2015, 36:763-777). Different IL2R is expressed on the surface of different immune cells. The high-affinity IL2R is constitutively expressed on CD4[+] Foxp3[+] regulatory T cells (Tregs), and also transiently expressed on activated T cells (CD4[+] T cells and CD8[+] T cells). The intermediate-affinity IL2R is expressed at a low level on T effector cells (Teff) such as CD8[+] Teff cells at the resting (or naive) state, however it is highly expressed on antigen-stimulated CD8[+] Teff cells, the memory phenotype (MP) CD8[+] T cells, and natural killer (NK) cells (Fontenot, Nat Immunol 2005, 6: 1142-1151; Josefowicz, Annu Rev Immunol 2012, 30: 531-564; Malek & Bayer, Nat Rev Immunol 2004, 4: 665-674).

IL-2-induced signaling activation is initiated by binding and triggering the formation of IL2Rβ/γc heterodimer which further activates the downstream kinase cascades. Although the α subunit is particularly important for augmenting the binding affinity of IL-2 to IL2R, it does not play a role in the IL-2 signaling pathway in immune cells (Kreig, PNAS 2010, 107: 11906-11911).

IL-2 is mainly produced by activated T cells, especially the CD4[+] T helper (Th) cells, and acts on neighboring cells that harbor IL2R in an autocrine or paracrine manner, and therefore regulates the immune activation response and the homeostasis of defense mechanisms of the immune system. Studies have shown that IL-2 is involved in a variety of biological processes, such as promoting the proliferation and differentiation of helper T cells (Zhu, Annual Review of Immunology 2010, 28:445-489; Liao, Nat Immunol 2008, 9:1288-1296; Liao, Nat Immunol 2011, 12:551-559), maintaining the development and homeostasis of Tregs (Cheng, Immunol Rev 2011, 241:63-76), promoting the development of cytotoxic T lymphocytes (CTLs) and enhancing their cytotoxicity, inducing the differentiation of peripheral blood lymphocytes to cytotoxic cells and lymphokine-activated killer (LAK) cells (Liao, Immunity 2013, 38:13-25), augmenting the immune response of tumor infiltrating lymphocytes (TILs), promoting the proliferation and differentiation of B cells and boosting the immunoglobulin production by activated B cells, and inducing the development, proliferation and activation of NK cells (Waldmann, Nat Rev Immuno 2006, 6: 595-601; Malek, Annu Rev Immunol 2008, 26: 453-479; Liao, Immunity 2013, 38: 13-25).

IL-2 can induce the expansion of above-mentioned lymphocyte subsets *in vivo*, especially, IL-2 exerts its anti-tumor activity by stimulating and activating effector cells (including CD8[+] T cells and NK cells), and therefore IL-2 immunotherapy is one of the effective methods for the treatment of various malignant tumors. Aldesleukin (branded as Proleukin[®]), a recombinant human IL-2 protein (rhIL-2), has been approved in the 1990s for the treatment of metastatic renal cell cancer and malignant melanoma. Aldesleukin is an engineered rhIL-2. The difference between wild-type human IL-2 protein (hIL-2) and aldesleukin includes: Aldesleukin is an aglycosylated protein which lacks N-terminal alanine and has a serine substitution of its cysteine residue at the position 125. In a number of clinical trials for the treatment of metastatic renal cell cancer and malignant melanoma, aldesleukin demonstrated a relatively low complete response rate, which was about 7% (Atkins, J Clin Oncol 1999, 17:2105-2116; Fisher, Cancer J Sci Am 2000, 6 Suppl 1: S55-S57; Klapper, Cancer 2008, 113:293-301). The underlying reasons are mainly as follows: (1) Aldesleukin has a short half-life and the effector cells with antitumor activity such as CD8[+] T cells and NK cells express the intermediate-affinity IL2R. Therefore, a high dose of aldesleukin is required to effectively activate the CD8[+] T cells and NK cells. However, high-dose aldesleukin also binds to and activates the high-affinity IL2R expressed on endothelial cells and type-2 innate lymphoid cells (ILC2) and causes vascular leak syndrome (VLS) characterized by increased vascular permeability and extravasation of plasma proteins and intravascular fluid into the extravascular space and multiple organs, leading to low blood pressure, compensatory increase in heart rate, pulmonary edema and skin edema, as well as hepatocellular damage (Krieg, Proc Nat Acad Sci USA 2010, 107:11906-11911; Boyman, Nat Rev Immunol 2012, 12:180-190). Therefore, patients treated with high-dose aldesleukin often experience severe cardiovascular adverse events and toxicities in the pulmonary, hepatic, gastrointestinal, neurological and hematological systems (Proleukin[®] [aldesleukin] Package Insert, 2012). Due to the severe toxicity and side effects associated with the aldesleukin therapy, the majority of patients cannot tolerate the clinically-efficacious dose, which has limited its clinical use. (2) The low response rate of the aldesleukin therapy is also related to the dual role of IL-2 in maintaining immune cell homeostasis. IL-2 not only promotes the activation and proliferation of effector cells, but also maintains peripheral immune tolerance by regulating Tregs. Because Tregs express a high level of the high-affinity IL2R, IL-2 preferentially binds to Tregs and promotes the proliferation and activation of peripheral Tregs that dampen the antitumor immunity mediated by CD8[+] T cells and NK cells (Fontenot, Nat Immunol 2005, 6:1142-1151; D'Cruz, Nature Immunol 2005, 6:1152-1159; Maloy, Nature Immunol 2005, 97: 189-192; Boyman, Nat Rev Immunol 2012, 12: 180-190; Facciabene, Cancer Res 2012, 72: 2162-2171). Therefore, due to the dual role of IL-2 in immune regulation, the antitumor immunity of activated effector cells in the aldesleukin therapy can be inhibited by the IL-2-dependent Treg cell activation, which limits its clinical efficacy.

In recent years, various engineered IL-2 variant molecules have been developed, aiming to enhance the antitumor activity of IL-2 therapy and reduce its associated toxicity and side effects. These engineering strategies can be categorized into the following four classes: (1) The key amino acid residues in IL-2 sequence involved in the binding of a particular IL2R subunit are mutated to reduce the binding activity to IL2Rα and thus mitigate the toxicity and the activation of Tregs by the high-affinity IL2R, or to increase the binding activity to IL2Rβ and/or yc to promote the proliferation and activation of CD8[+] T cells and NK cells and thus enhance the antitumor activity. As disclosed by Gillies et al. in the patent application WO20080034473, introduction of amino acid mutations comprising R38W and F42K in IL-2 to disrupt its key binding sites for IL2Rα led to reduced interaction with IL2Rα and less activation of Tregs, and consequently reduced the suppression to antitumor immunity. Garcia et al. constructed a mutein named "IL-2 Superkine" by introducing mutations in IL-2 to enhance its affinity to IL2Rβ and reduce the dependence of IL-2 on IL2Rα expression. As a result, it induced a stronger expansion and activation of effector cells (CD8[+] T cells and NK cells) (Levin, Nature 2012, 484: 529-533). (2) IL-2 is conjugated with polyethylene glycol (PEG). For example, Bempegaldesleukin developed by Nektar was composed of aldesleukin coupled to an average of six dissociable PEG groups. This not only led to a prolonged half-life of IL-2, but also blocked the interaction of IL-2 with the high-affinity IL-2R by the PEG conjugated to the lysine residue near the IL2Rα-binding site (Deborah H, Clin Cancer Res 2016, 22: 680-690). In addition, Synthorx developed THOR-707 which used another approach to generate PEG-modified IL-2, wherein a non-natural amino acid was introduced into the IL-2 sequence and the protein was expressed by engineered bacteria, and a non-cleavable PEG molecule was then conjugated to the non-natural amino acid residue to generate the homogenous PEG-modified "not-α" IL-2. It was reported by Synthorx that THOR-707 could induce the expansion of peripheral naive T cells and NK cells, but not Tregs (Janku, European Journal of Cancer 2020, 138S2: S11). (3) Nemvaleukin alfa developed by Alkermes is constructed by fusing an IL-2 mutant to the extracellular domain of IL2Rα (CD25), which aimed to block the interaction of this drug candidate with IL2Rα on Tregs (Boni, Journal of Clinical Oncology 2021, 39:15 Suppl, 2513). In addition, Boyman et al. designed an antigen-antibody immunocomplex composed of IL-2 and an anti-IL-2 antibody to block the binding of IL-2 to IL2Rα, and thus to promote antitumor activity by biasedly activating CD8[+] T cells and NK cells which express the intermediate-affinity IL2R (Kamimura, J Immunol 2006, 177: 306-314; Boyman, Science 2006, 311: 1924-1927; Lee, Oncoimmunology 2020, 9: e1681869). (4) Trinklein et al. constructed a bispecific antibody targeting the IL2Rβ and yc and reported that this bispecific antibody could specifically activate the downstream signaling pathway mediated by the intermediate-affinity IL2R and induce the proliferation of CD8[+] T and NK cells (Harris KE, Scientific Reports 2021, 11:1-15).

However, the above approaches have their limitations. The binding domain used to block the interaction of IL-2 with IL2Rα is often in a dynamic equilibrium of association and dissociation with the IL-2 variant. Therefore, the IL-2 variant may inevitably be bound by the high-affinity IL2R in a competitive manner since it has an ultra-high binding affinity to IL-2. Moreover, some IL-2 variants comprise mutations in their sequences which may lead to augmented immunogenicity and induce the generation of neutralizing anti-drug antibodies (ADA). Such ADA not only impair the clinical use of the IL-2 product, but may also interfere with the immunoregulatory function of endogenous IL-2 and raise potential safety risks. For example, aldesleukin is an aglycosylated protein expressed by *E. coli,* which is an amphiphilic molecule and present as micro-aggregates with an average size of 27 rhIL-2 molecules, and it was found in clinical trials that 60~75% of the participating patients had ADA in their body (Proleukin (Aldesleukin) Injection information). BAY 50-479 comprises a mutation of asparagine to arginine at the position 88 in hIL-2 (i.e., N88R), and the ADA recognizing this site mutation has been detected in Phase 1 clinical study (Margolin, Clin Cancer Res 2007, 13: 3312-3319). THOR-707 comprises a non-natural amino acid residue which may be recognized as a foreign antigen by the immune system, and it may induce the production of ADA after repeated dosing (Levin, Nature 2012, 484: 529-533). Another PEG-modified IL-2 molecule, NKTR-214, exhibits a kinetically sustained release of IL-2, however, pegylation can also interfere with the binding of IL-2 to IL-2Rβ and γc and thus hinder IL-2 to activate the effector cells and promote the antitumor activity (Silva, Nature 2019, 565: 186-191). The bispecific antibody targeting IL2Rβ and γc can bind indiscriminately to the intermediate-affinity IL2R and the high-affinity IL2R and activate the downstream signaling pathway, therefore, it can indiscriminately induce the activation and expansion of Tregs and effector cells.

Hence, there exists an unmet need in the art to develop an IL-2 product that can specifically bind to the intermediate-affinity IL2R and activate the effector cells (CD8$^+$ T cells and NK cells) with no binding activity to the high-affinity IL2R and no activation of Tregs, vascular endothelial cells and ILC2. In this way, it can overcome the limitations associated with the above-mentioned IL-2 products and address unmet medical needs of the existing IL-2 therapy, including systemic toxicities and side effects, as well as tumor immunosuppression led by Treg cell activation, due to the binding of the high-affinity IL2R.

## SUMMARY OF THE INVENTION

The present invention provides a novel IL-2/anti-IL-2 antibody fusion protein that is able to selectively bind to IL2Rβ/γ receptor and activate the signaling pathway mediated by IL2Rβ/γ, but incapable of binding to IL2Rα and any IL-2 receptor complex comprising the α subunit (i.e., IL2Rα/β or IL2Rα/β, and thus to address the following technical problems and unmet medical needs:

(1) Dose limiting toxicity (DLT): Existing IL-2 products and/or products under investigation cause systemic toxicity and side effects to normal tissues and organs due to the activation of the high-affinity IL2R *in vivo.* For example, the approved drug aldesleukin and the IL-2 products currently under investigation may activate the high-affinity IL2R expressed on vascular endothelial cells and ILC2 cells, leading to systemic vascular leak syndrome (VLS).

(2) Treg cell-mediated tumor immunosuppression: Existing IL-2 products and/or IL-2 products under investigation activate Tregs and promote their proliferation by binding to the high-affinity IL2R expressed on Tregs in tumor tissues or tumor microenvironment, which may further promote the proliferation of other immunosuppressive cells in the tumor microenvironment, resulting in tumor immunosuppression and impairing the efficacy of the therapy.

(3) Immunogenicity associated with the existing IL-2 products and/or IL-2 products under investigation: The approved drug aldesleukin is an aglycosylated recombinant IL-2 protein expressed by *E.coli* and naturally exists in the form of micro-aggregates. It is found that *in vivo* administration of aldesleukin can induce ADA. The IL-2 products under investigation, such as the IL-2 mutein comprising amino acid mutations at its IL2Rα binding site, and the IL-2 mutein comprising an introduced non-natural amino acid residue and site-specific modification of PEG, etc., may provoke ADA response when administered *in vivo.* ADA recognizing IL-2 and/or IL-2 muteins not only dampens the clinical efficacy of IL-2 products, but may also interfere with the endogenous IL-2 and raise safety concerns.

(4) Short half-life and narrow therapeutic window of the existing IL-2 products: For example, the serum half-life of the approved drug aldesleukin is only 85 minutes, and effector cells with antitumor activity such as CD8$^+$ T and NK cells express the intermediate-affinity IL2R, therefore, a high-dose of aldesleukin is required to activate CD8$^+$ T cells and NK cells. The high-dose aldesleukin, however, preferentially promotes immunosuppressive response and induces systemic toxic effects. As a result, the therapeutic window of aldesleukin is extremely narrow. Although several IL-2 products under investigation are relatively selective in binding of the intermediate-affinity IL2R, they are still able to bind to the high-affinity IL2R to activate the immunosuppressive response and induce systemic toxic effects, which in turn limit the dosing level in clinic, leading to a narrow therapeutic window and a poor patient compliance with the medication.

(5) Existing IL-2 product and/or IL-2 products under investigation are structurally unstable or easy to dissociate (such as the immunocomplex comprising IL-2 and the anti-IL-2 antibody), and thus it is not easy to manufacture high quality and homogenous products.

In one aspect, the present application provides a human interleukin-2 (hIL-2) mutein. The said hIL-2 mutein is a variant of the wild-type hIL-2 (as set forth in SEQ ID No: 1) with amino acid mutations, it is capable of binding to the anti-IL-2 antibody or antigen-binding fragment thereof of the present application, wherein the mutations do not significantly affect the biological activity of the said IL-2.

In another aspect, the present application provides an isolated anti-IL-2 antibody or antigen-binding fragment thereof, wherein the anti-IL-2 antibody or antigen-binding fragment thereof can competitively bind to the hIL-2 mutein of the present application and block the binding of the said hIL-2 mutein to IL2Rα, and the anti-IL-2 antibody or antigen-binding fragment thereof has a higher binding affinity to the hIL-2 mutein of the present application and is not easy to dissociate.

In another aspect, the present application provides a novel IL-2/anti-IL-2 antibody fusion protein comprising:

(1) the wild-type human IL-2 (hIL-2) as set forth in SEQ ID NO: 1 or an hIL-2 mutein, and

(2) an anti-IL-2 antibody or antigen-binding fragment thereof capable of competing with IL2Rα for binding to hIL-2 or the hIL-2 mutein, and

(3) disulfide linkage(s) contained at the antigen-antibody binding interface of the said hIL-2 or hIL-2 mutein and the said anti-hIL-2 antibody or antigen-binding fragment thereof, to prevent the antigen-antibody complex from being dissociated.

The IL-2/anti-IL-2 antibody fusion protein provided in the present application has the following characteristics:

(i) The structure of complex formed by the said hIL-2 or mutein thereof and the anti-IL-2 antibody is super stable;

(ii) The IL2Rα-binding site of the said hIL-2 or mutein thereof is embedded inside of the said fusion protein;

(iii) The said fusion protein is incapable of binding to IL2Rα or any IL-2 receptor complex comprising the α subunit, i.e., IL2Rα/β or IL2Rα/β/γ;

(iv) The said fusion protein retains the activity in binding to and activating the dimeric receptor consisting of the IL2Rβ subunit and the γ chain (IL2Rβ/γ).

In some embodiments, the said hIL-2 is the wild-type hIL-2 as set forth in SEQ ID NO: 1. In some embodiments, the said hIL-2 mutein is a variant of the wild-type hIL-2 (as set forth in SEQ ID NO: 1) comprising at least one, or two, or more than two amino acid mutations other than the mutation to cysteine, and the hIL-2 mutein retains the activity in specifically binding to IL2Rβ/γ. In one embodiment, the amino acid mutation(s) is/are located at the antigen-antibody binding interface between the said hIL-2 or mutein thereof and the anti-IL-2 antibody or antigen-binding fragment thereof. In a particular embodiment, the amino acid mutation(s) is/are resided inside of the antigen-antibody binding interface. In one embodiment, the amino acid mutation(s) is/are resided outside of the antigen-antibody binding interface. In one embodiment, the location of amino acid mutation(s) is a combination of inside and outside of the antigen-antibody binding interface.

In some embodiments, the said anti-IL-2 antibody includes an anti-mouse IL-2 (mIL-2) antibody, or a chimeric and/or humanized antibody derived from the anti-mouse IL-2 antibody (e.g., the S4B6 antibody and/or humanized antibody thereof), an anti-IL-2 humanized antibody (e.g., the NARA1 antibody or the TCB2 antibody), or an anti-hIL-2 antibody. In one embodiment, the anti-mIL-2 antibody, or chimeric antibody or humanized antibody derived from the anti-mIL-2 antibody, or antigen-binding fragment thereof, can compete with IL2Rα for binding of the said hIL-2 mutein and can block the said hIL-2 mutein from being bound by IL2Rα or any IL-2 receptor complex comprising the α subunit (i.e., IL2Rα/β or IL2Rα/β/γ). In one embodiment, the said anti-hIL-2 antibody is able to specifically recognize the IL2Rα-binding site of the said hIL-2, and specifically bind to the said hIL-2.

In some embodiments, the antigen-antibody binding interface of the said hIL-2 or mutein thereof and the said anti-IL-2 antibody or antigen-binding fragment thereof comprises disulfide linkage, wherein the disulfide linkage is formed by introducing at least one cysteine residue in the sequence of anti-IL-2 antibody or antigen-binding fragment thereof and at least one cysteine residue in the sequence of the said hIL-2 or mutein thereof at the antigen-antibody binding interface. In one embodiment, at least one cysteine residue is introduced at the antigen-antibody binding interface by amino acid substitution, point mutation, insertion, addition, or any combination thereof.

In another aspect, the present application relates to an isolated nucleic acid molecule (also referred to as "polynucleotide") encoding the hIL-2 mutein, the anti-IL-2 antibody or antigen-binding fragment thereof, or the hIL-2/anti-IL-2 antibody fusion protein disclosed herein, as well as an expression vector comprising the said nucleic acid molecule, and a host cell comprising the said nucleic acid molecule or the expression vector. The present application also relates to a method for the preparation of the hIL-2 mutein, the anti-IL-2 antibody or antigen-binding fragment thereof, and the hIL-2/anti-IL-2 antibody fusion protein disclosed herein using the host cell.

In another aspect, the present application relates to a bispecific molecule, an immunoconjugate, a chimeric antigen receptor (CAR), an engineered T cell receptor, or an oncolytic virus that comprises the anti-IL-2 antibody or antigen-binding fragment thereof, or the hIL-2/anti-IL-2 antibody fusion protein disclosed herein.

In another aspect, the present application relates to a pharmaceutical composition comprising the anti-IL-2 antibody or antigen-binding fragment thereof, or the hIL-2/anti-IL-2 antibody fusion protein of the present application, or a bispecific molecule, an immunoconjugate, a chimeric antigen receptor, an engineered T cell receptor, or an oncolytic virus comprising the anti-IL-2 antibody or antigen-binding fragment thereof, or the hIL-2/anti-IL-2 antibody fusion protein of the present application and a pharmaceutically acceptable carrier.

In another aspect, the present application relates to a kit set comprising an effective amount of the anti-IL-2 antibody or antigen-binding fragment thereof, or the hIL-2/anti-IL-2 antibody fusion protein of the present application, or a pharmaceutical composition comprising the anti-IL-2 antibody or antigen-binding fragment thereof, or the hIL-2/anti-IL-2 antibody fusion protein of the present application, and/or another anti-cancer agent and/or immunomodulatory agent, wherein the anti-cancer agent includes but is not limited to microtubule disrupting agent, antimetabolite, topoisomerase inhibitor, DNA intercalator, alkylating agent, hormone therapeutic agent, kinase inhibitor (e.g., tyrosine kinase inhibitor, including but not limited to EGFR inhibitor, HER2 inhibitor, HER3 inhibitor, IGFR inhibitor, PI3K inhibitor and Met inhibitor), receptor antagonist, activator of tumor cell apoptosis (including but not limited to IAP inhibitor, Bcl2 inhibitor, MC11 inhibitor, TRAIL inhibitor, CHK inhibitor), or any anti-angiogenic drug; the immunomodulatory agent includes but is not limited to PD-1, PD-L1, PD-L2, TIM3, CTLA-4, LAG-3, CD47, CEACAM-1, CEACAM-5, VISTA, BTLA, TIGIT, LAIR1, CD160, 2B4, and TGFR inhibitor.

In another aspect, the present application relates to a kit comprising an effective amount of the anti-IL-2 antibody or antigen-binding fragment thereof, or the hIL-2/IL-2 antibody fusion protein of the present application, or a pharmaceutical composition comprising the anti-IL-2 antibody or antigen-binding fragment thereof, or the hIL-2/IL-2 antibody fusion protein of the present application.

In another aspect, the present application relates to use of the anti-IL-2 antibody or antigen-binding fragment thereof, or the hIL-2/anti-IL-2 antibody fusion protein disclosed herein in the manufacture of a pharmaceutical composition or drug product for the treatment of diseases, wherein the diseases include but are not limited to proliferative disease, infectious disease, and immune deficiency disease, wherein the proliferative disease includes but is not limited to cancer and other cell proliferative disorder. Alternatively, the present application relates to use of the anti-IL-2 antibody or antigen-binding fragment thereof, or the hIL-2/anti-IL-2 antibody fusion protein, or pharmaceutical composition thereof disclosed herein in the treatment of proliferative disease, infectious disease, and immune deficiency disease. Alternatively, the present application relates to a method of treating a proliferative disease, an infectious disease, or an immune deficiency disease, wherein the method includes administering to a subject in need the anti-IL-2 antibody or antigen-binding fragment thereof, or the hIL-2/anti-IL-2 antibody fusion protein herein, or pharmaceutical composition thereof disclosed herein.

In another aspect, the present application relates to a method of treating a disease, condition, or disorder by boosting the host immune response, wherein the method includes administering to a subject in need an effective amount of the anti-IL-2 antibody or antigen-binding fragment thereof, or the hIL-2/anti-IL-2 antibody fusion protein, or pharmaceutical composition thereof disclosed herein, or the kit set or kit comprising the anti-IL-2 antibody or antigen-binding fragment thereof, or the hIL-2/anti-IL-2 antibody fusion protein of the present application, to specifically activate the effector cells expressing the IL2R$\beta\gamma$ receptor (e.g., CD8$^+$ T cells and NK cells) and alleviate disease progression.

In another aspect, the present application relates to a method of stimulating immune system, wherein the method includes administering to a subject in need an effective amount of the anti-IL-2 antibody or antigen-binding fragment thereof, or the hIL-2/anti-IL-2 antibody fusion protein of the present application, or pharmaceutical composition comprising the anti-IL-2 antibody or antigen-binding fragment thereof, or the hIL-2/anti-IL-2 antibody fusion protein of the present application, or the kit set or kit comprising the anti-IL-2 antibody or antigen-binding fragment thereof, or the hIL-2/anti-IL-2 antibody fusion protein of the present application.

In another aspect, the present application relates to a method of boosting the host immune system to treat, alleviate, or prevent a disease condition (i.e., stimulation of host immune system is beneficial for alleviating the disease), wherein the method includes administering to a subject in need a therapeutically effective amount of the anti-IL-2 antibody or antigen-binding fragment thereof, or the hIL-2/anti-IL-2 antibody fusion protein of the present application, or pharmaceutical composition comprising the anti-IL-2 antibody or antigen-binding fragment thereof, or the hIL-2/anti-IL-2 antibody fusion protein of the present application, or the kit set or kit comprising the anti-IL-2 antibody or antigen-binding fragment thereof, or the hIL-2/anti-IL-2 antibody fusion protein of the present application, to alleviate the disease by enhancing the cellular immune response in host, wherein the disease comprises insufficient host immune response or immune deficiency, furthermore, the disease includes a proliferative disease or an infectious disease, wherein the proliferative disease includes cancer and immune deficiency disease.

Other features and advantages of the present application will become apparent from the following detailed description of examples and drawings. It should be understood, however, that the drawings and specific embodiments should not be construed as limiting the scope of the present application, and various changes and modifications within the spirit and scope of the present invention which will become apparent to those skilled in the art from this detailed description are included in the scope of protection of the appended claims. The contents of all references, including publications, patents and published patent applications, cited in the present application are incorporated by reference in their entirety.

**DESCRIPTION OF DRAWINGS**

FIGURE 1 shows the proliferation of CTLL-2 cells (Fig. 1A) and NK-92 cells (Fig. 1B) in response to the stimulation of the hIL-2 mutant (hIL2-DM or hIL2-TM) or the control (mIL-2 or hIL-2).

FIGURE 2 shows the binding activity of the humanized antibody of S4B6 to mIL-2-his or hIL-2-his, as measured by ELISA.

FIGURE 3 shows the binding activity of the hIL-2 mutein hIL2-DM to hIL2R$\alpha$ and hIL2R$\beta$ after binding to K2G11, as measured by Fortebio.

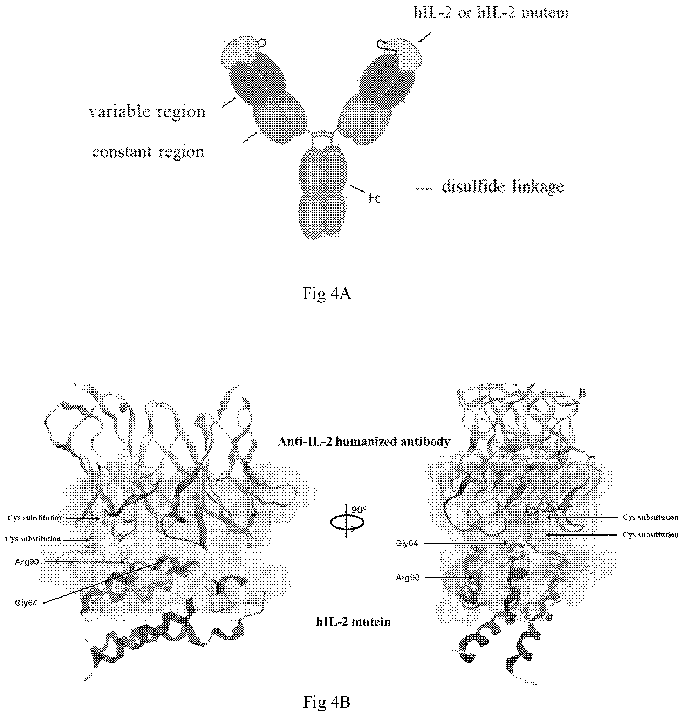

FIGURE 4 shows a schematic diagram of the structure of a representative hIL-2/anti-IL-2 antibody fusion protein of the present application, and a three-dimensional schematic diagram of the antigen-antibody binding interface of the exemplary hIL-2/anti-IL-2 antibody fusion protein. The C-terminus of the hIL-2 or hIL-2 mutein is connected to the N-terminus of the heavy chain of an anti-hIL-2 antibody through a linker, and a disulfide linkage between the hIL-2 or hIL-2 mutein and the anti-hIL-2 antibody (indicated by the dotted line) is located at the antigen-antibody binding interface (Fig. 4A). In the exemplary hIL-2/anti-IL-2 antibody fusion protein, a disulfide linkage at the binding interface of the hIL-2 mutein (with K64G and N90R mutations) and the humanized anti-IL-2 antibody is formed by introducing one cysteine mutation in the hIL-2 mutein (with K64G and N90R mutations) and another cysteine mutation in the humanized anti-IL-2 antibody (Fig. 4B).

FIGURE 5 shows the binding activity of IC-Cy08 (the hIL-2/anti-IL-2 antibody fusion protein comprising a disulfide linkage) to hCD25 (Fig. 5A) and hCD122 (Fig. 5B), respectively, as measured by ELISA, wherein hIL2-DM-Fc is served as the control.

FIGURE 6 shows the proliferation of CTLL-2 cells (Fig. 6A) and NK-92 cells (Fig. 6B) in response to the stimulation of IC-Cy08 (the hIL-2/anti-IL-2 antibody fusion protein comprising a disulfide linkage), wherein hIL-2 is served as the control.

FIGURE 7 shows STATS phosphorylation in the IL-2 signaling pathway in CTLL-2 cells in response to the stimulation of IC-Cy08 (the hIL-2/anti-IL-2 antibody fusion protein comprising a disulfide linkage) or the control (hIL-2), as measured by flow cytometry.

FIGURE 8 shows the binding activity of the IC-Cy08 variants (the hIL-2/anti-IL-2 antibody fusion protein comprising a disulfide linkage and optimized antibody light chain) to hCD25 (Fig. 8A) and hCD122 (Figure 8B), respectively, as measured by ELISA, wherein hIL2-DM-Fc and the parental molecule IC-Cy08 are served as the controls.

FIGURE 9 shows the proliferation of CTLL-2 cells (Figure 9A) and NK-92 cells (Figure 9B) in response to the stimulation of the IC-Cy08 variants (the hIL-2/anti-IL-2 antibody fusion protein comprising a disulfide linkage and optimized antibody light chain), wherein hIL-2 and the parental molecule IC-Cy08 are served as the controls.

FIGURE 10 shows the proliferation of CTLL-2 cells (Figure 10A) and NK-92 cells (Figure 10B) in response to the stimulation of the IC-Cy08 variants (the hIL-2/anti-IL-2 antibody fusion protein comprising a disulfide linkage and optimized amino acid sequence of hIL-2), wherein hIL-2 and the parental molecule IC-Cy08 are served as the controls.

FIGURE 11 shows the proliferation of CTLL-2 cells (Figure 11A) and NK-92 cells (Figure 11B) in response to the stimulation of IC-Cy08-pITM (the hIL-2/anti-IL-2 antibody fusion protein comprising pI and TM mutations in its heavy chain constant region), wherein hIL-2 and the parental molecule IC-Cy08 are served as the controls.

FIGURE 12 shows the binding activity of K2G11-Cy04, K2G11-Cy05, and K2G11-Cy06, which are hIL-2/anti-IL-2 antibody fusion proteins comprising the K2G11 antibody and a disulfide linkage, to hCD25 (Figure 12A) and hCD122 (Figure 12B), respectively, as measured by ELISA, wherein H6K3(H)-Cy08 and H6K3(H)-Cy10 are served as the controls.

FIGURE 13 shows the proliferation of CTLL-2 cells (Figure 13A) and NK-92 cells (Figure 13B) in response to the stimulation of K2G11-Cy04, K2G11-Cy05, and K2G11-Cy06, which are hIL-2/anti-IL-2 antibody fusion proteins comprising the K2G11 antibody and a disulfide linkage, wherein H6K3(H)-Cy08, H6K3(H)-Cy10, and an antigen-antibody immunocomplex formed by K2G11 and hIL2-DM ($\Delta$3, C125L) are served as the controls.

FIGURE 14 shows the binding activity of K2G11-Cy05 to hIL2R$\alpha$, hIL2R$\alpha$/$\beta$ and hIL2R$\alpha$/$\beta$/$\gamma$, as measured by Fortebio.

FIGURE 15 shows the binding activity of K2G11-Cy05 (the hIL-2/anti-IL-2 antibody fusion protein comprising a disulfide linkage) to hCD25, as determined by ELISA, wherein NARA1leukin is served as the control.

FIGURE 16 shows the IL2R$\alpha$/$\beta$/$\gamma$ and/or hIL2R$\beta$/$\gamma$ receptor activation, as determined by cell-based gene reporter assay (Figure 16A) and cell proliferation assay (including CTLL-2 cells and CD25-knockout NK-92 cells [NK-92 (CD25-KO)]) (Figure 16B), in response to the stimulation of K2G11-Cy05 (the hIL-2/anti-IL-2 antibody fusion protein comprising a disulfide linkage), wherein NARA1leukin and hIL-2 are served as the controls.

FIGURE 17 shows the binding kinetics of K2G11-pITM (the humanized anti-IL-2 antibody comprising pI and TM mutations in its heavy chain constant region) and K2G11-Cy05 (the hIL-2/anti-IL-2 antibody fusion protein comprising K2G11-pITM and a disulfide linkage) to Fc receptors, C1q and FcRn, as determined by ForteBio, wherein the K2G11 antibody is served as the control.

FIGURE 18 shows the ADCC activity of K2G11-Cy05 (the hIL-2/anti-IL-2 antibody fusion protein comprising disulfide linkage and pI and TM mutations in the antibody constant region), as determined by cell-based gene reporter assay, wherein the anti-EGFR antibody cetuximab is served as the positive control.

FIGURE 19 shows the biological activity changes of hIL-2/anti-IL-2 antibody fusion proteins (K2G11-Cy04 and K2G11-Cy05) after treated with cynomolgus monkey serum (M6, M7) or normal human serum (H2, H3) for a different period of time, as determined by cell proliferation assay with NK-92 cells (Figures 19A and 19B) and cell-based gene reporter assay (Figure 19C), wherein data labeled with K2G11-Cy04 and K2G11-Cy05 are derived from untreated samples and served as the controls.

FIGURE 20 shows the plasma drug concentration-time curves of K2G11-pITM (the anti-IL-2 antibody comprising engineered heavy chain constant region) and K2G11 (the anti-IL-2 antibody comprising wild-type heavy chain constant region) in BALB/c mice.

FIGURE 21 shows the cell count changes in CD8[+] T cells, NK cells and Tregs, and the changes in their proliferative index biomarker Ki-67 expression (i.e., the proportion of Ki67[+] cells), in the peripheral blood of cynomolgus monkeys in response to the treatment of K2G11-Cy05 (the hIL-2/anti-IL-2 antibody fusion protein comprising disulfide linkage and engineered heavy chain constant region), as determined by flow cytometry. The arrows in the figure indicate the time of dose administration.

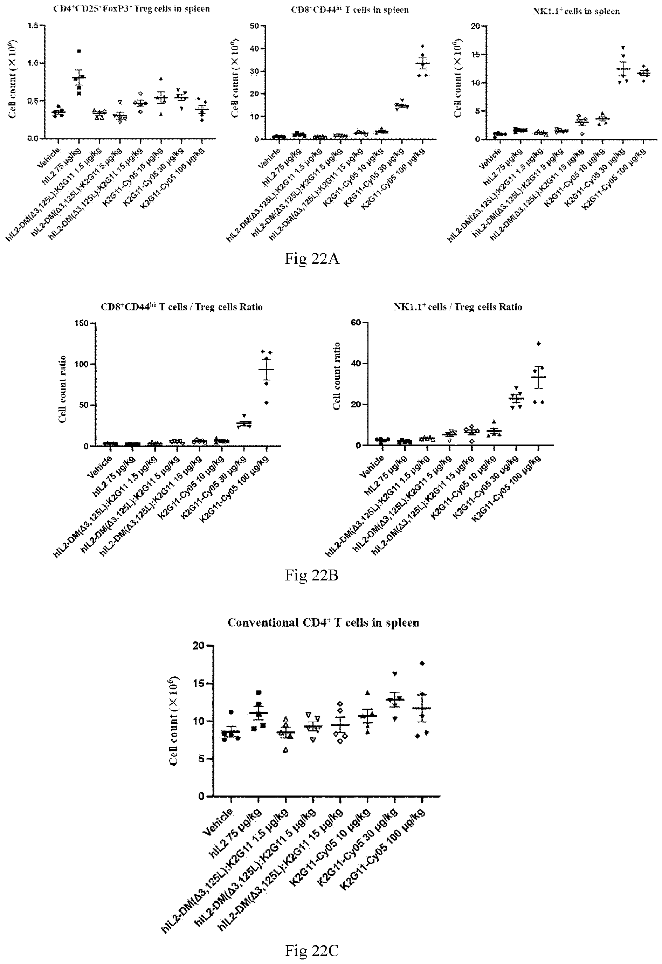

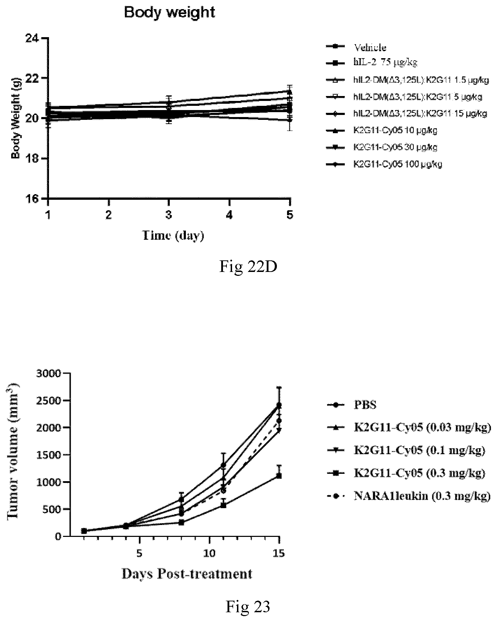

FIGURE 22 shows the cell count changes in CD4[+]CD25[+]FoxP3[+] Tregs, CD8[+]CD44[high] T cells and NK1.1[+] cells (Figure 22A), and the cell count changes in conventional CD4[+] T cells (Figure 22C), as well as the cell count ratio changes in CD8[+]CD44[high] T cells to Tregs, and NK1.1[+] cells to Tregs (Figure 22B), in individual spleens of C57BL/6 mice, as determined by flow cytometry, in response to the treatment of K2G11-Cy05 (the hIL-2/anti-IL-2 antibody fusion protein comprising disulfide linkage and engineered heavy chain constant region). The mouse body weight changes in response to treatments are shown in Figure 22D.

FIGURE 23 shows the antitumor activity of K2G11-Cy05 (the hIL-2/anti-IL-2 antibody fusion protein comprising disulfide linkage and engineered heavy chain constant region) at different dose levels in murine CT26 colon cancer model, wherein NARA1leukin is served as the control.

FIGURE 24 shows the binding activity of hIL-2/NARA1 antibody fusion proteins comprising disulfide linkage to hCD25 (Figure 24A) and hCD122 (Figure 24B), respectively, as determined by ELISA, wherein hIL2-DM-Fc is served as the control.

FIGURE 25 shows the binding activity of hIL-2/TCB2 antibody fusion proteins comprising disulfide linkage to hCD25 (Figure 25A) and hCD122 (Figure 25B), respectively, as determined by ELISA, wherein hIL2-DM-Fc is served as the control.

FIGURE 26 shows the proliferation of CTLL-2 cells (Figure 26A) and K-92 cells (Figure 26B) in response to the stimulation of hIL-2/NARA1 antibody fusion proteins comprising disulfide linkage, wherein wild-type hIL-2 is served as the control.

FIGURE 27 shows the proliferation of CTLL-2 cells (Figure 27A) and NK-92 cells (Figure 27B) in response to the stimulation of hIL-2/TCB2 antibody fusion proteins comprising disulfide linkage, wherein wild-type hIL-2 is served as the control.

## DETAILED DESCRIPTION OF THE INVENTION

*Definition:*

Unless otherwise defined, technical and scientific terms used herein have the same meaning as those commonly understood by a person skilled in the art. For the purposes of the present application, the following terms are defined below.

As used herein, "interleukin-2" or "IL-2" or "IL2" is used interchangeably and refers to any native IL-2 derived from any vertebrate, including a mammal such as a primate (e.g., human) and a rodent (e.g., mouse). This term includes unprocessed IL-2 as well as any form of IL-2 derived from cell expression. This term also includes allelic and splice variants, isotypes, homologues, and species homologues of naturally occurring IL-2. Human IL-2 (hIL-2) as used herein refers to the mature human IL-2 protein, also known as "wild-type hIL-2" or "wild-type IL-2" or "WT IL-2", having the following amino acid sequence:

APTSSSTKKTQLQLEHLLLDLQMILNGINNYKNPKLTRMLTFKFYMPKKATELKHLQ
CLEEELKPLEEVLNLAQSKNFHLRPRDLISNINVIVLELKGSETTFMCEYADETATIVEF
LNRWITFCQSIISTLT (SEQ ID NO: 1).

Unprocessed human IL-2 additionally contains an N-terminal signal peptide of 20 amino acids with the sequence as set forth in SEQ ID NO: 229, and the mature (or processed) IL-2 protein is a polypeptide from which the signal peptide has been removed.

The hIL-2 mutein (or IL-2 variant) described herein is a mutant form of the wild-type hIL-2, and the mutein can comprise one, or two, or more than two amino acid mutations in the amino acid sequence of wild-type IL-2, wherein the amino acid mutation can be amino acid substitution, deletion, insertion, or addition. In some embodiments, the preferred amino acid mutations are amino acid substitution, insertion or addition, or a combination thereof. In other embodiments, the preferred amino acid mutations are amino acid deletion. In some embodiments, the hIL-2 mutein with augmented binding affinity to the anti-IL-2 antibody or antigen-binding fragment thereof of the present application is obtained by introducing mutation(s) at its specific amino acid position(s), wherein the augmented binding affinity includes that the wild-type IL-2 which is incapable of cross-reacting with the anti-IL-2 antibody or antigen-binding fragment thereof gains cross-reactivity with the said anti-IL-2 antibody or antigen-binding fragment thereof after the mutation(s), or the said hIL-2 mutein has a moderate binding affinity (e.g., lower than $10^{-8}$ M ) or a high binding affinity (e.g., lower than $10^{-9}$ M, even lower than $10^{-10}$ M) to the said anti-IL-2 antibody or antigen-binding fragment thereof. In some embodiments, the hIL-2 mutein obtained by introducing mutation(s) at specific amino acid position(s) described in the present application is capable of forming a stable complex with the anti-IL-2 antibody or antigen-binding fragment thereof of the present application. In some embodiments, the hIL-2 mutein with eliminated glycosylation modification site(s) is obtained by introducing mutation(s) at specific amino acid position(s) described herein. In some embodiments, the hIL-2 mutein with eliminated free cysteine is obtained by introducing mutation at the specific cysteine residue position described herein.

Herein, when mentioning the amino acid position of IL-2, it is referring to the position in amino acid sequence of the wild-type hIL-2 as set forth in SEQ ID NO: 1. The corresponding amino acid positions of other IL-2 proteins or polypeptides (including the full-length sequence and truncated fragments) are defined according to the alignment of their amino acid sequences (e.g., using BLAST with default parameters available at http://blast.ncbi.nlm.nih.gov/Blast. cgi?PROGRAM=blastp&PAGE_TYPE=BlastSearch&LINK_LOC=blasthome). Thus, unless otherwise stated, an amino acid position in IL-2 protein or polypeptide described in the present application is the amino acid position numbered according to SEQ ID NO: 1. For example, when mentioning "K64", it refers to lysine residue (K) at the position 64 of the amino acid sequence as set forth in SEQ ID NO: 1, or the amino acid residue at the corresponding position in other IL-2 polypeptide sequences by alignment.

Herein, when referring to hIL-2 mutein, mutation is described as follows: an amino acid substitution is expressed as "original amino acid residue/position/substituted amino acid residue". For example, substitution of asparagine (N) at the position 90 in wild-type hIL-2 with arginine (R) is denoted as N90R.

As used herein, "IL2R" or "IL-2R" refers to the IL-2 receptor. The high-affinity IL2R consists of three subunits: IL2Rα (also known as CD25), IL2Rβ (also known as CD122), and common γ chain (i.e., yc, also known as CD132). The high-affinity IL2R can be expressed as "IL2Rαβγ" or "IL2Rα/β/γ" The term "IL-2Rβ and yc" can be used interchangeably with the term "IL-2Rβγ" or "IL-2Rβ/γ", which refers to the intermediate-affinity IL-2R that lacks the α subunit and comprises the β and γ subunits.

Herein, "IL2Rα-binding site(s)" or "IL2Rα-binding region" refers to the region where IL-2 and IL2Rα contact and interact with each other as observed in the resolved crystal structure.

The term "affinity" or "binding affinity" refers to the intrinsic binding capacity of the interaction between a molecule (e.g., a receptor or antigen) and its partner (e.g., a ligand or antibody), i.e., a total intensity of all non-covalent interactions. Unless otherwise stated, "binding affinity" as used herein is the intrinsic binding affinity which reflects a 1:1 interaction between the members of a binding pair (e.g., receptor and ligand, or antigen and antibody). The affinity of a molecule X for its binding partner Y can usually be expressed as the dissociation equilibrium constant ($K_D$), which is a ratio of the dissociation rate constant ($k_{dis}$ or $k_{off}$) and the association rate constant ($k_a$ or $k_{on}$). Affinity can be measured by conventional methods known in the art, including those used in the present application.

The term "regulatory T cells" or "Tregs" refers to a specific subset of $CD4^+$ T cells that can suppress the response of other T cells, such as effector T cells. Regulatory T cells are characterized by constitutive expression of a high level of IL2Rα and transcription factor FOXP3, and play a critical role in the induction and maintenance of peripheral self-tolerance to antigens (including tumor antigens). Regulatory T cells require IL-2 to induce activation of their suppressive activity on effector T cells.

As used herein, an "antibody" is meant to include one or more proteins encoded substantially or in part by immunoglobulin genes or their fragments. Recognized immunoglobulin genes include κ, λ, α, γ, δ, ε and μ constant region genes, as well as countless immunoglobulin variable region genes. Light chains are classified as κ or λ. Heavy chains are classified as γ, μ, α, δ or ε, each of which further define the immunoglobulin classes as IgG, IgM, IgA, IgD, and IgE. An antibody can be of any isotype/class (e.g., IgG, IgM, IgA, IgD, and IgE) or any subclass (e.g., IgG1, IgG2, IgG3, IgG4, IgA1, and IgA2). The structural unit of typical immunoglobulin (e.g., antibody) comprises a tetramer. Each tetramer consists of two identical pairs of polypeptide chains, each pair containing a "light" chain (about 25 kDa) and a "heavy" chain (about 50-70 kDa). Both light and heavy chains are composed of structural and functional homology regions. The terms "constant" and "variable" are used in terms of structure and function. The N-terminus of each chain defines a variable (V) region or domain primarily responsible for antigen recognition, having about 100 to 110 or more amino acids. As used herein, the "antibody" is used in the broadest sense and includes various antibody structures provided they exhibit the desired antigen-binding activity, including but not limited to a monoclonal antibody, polyclonal antibody, multispecific antibody (such as bispecific antibody) and antibody fragment.

An antibody exists as an intact immunoglobulin or as many well-characterized fragments produced by protease digestion. The term "antigen-binding fragment" (or abbreviated as "antibody portion" or "antibody fragment") of an antibody herein refers to a portion of an antibody comprising one or two or more CDRs, or any other antibody fragment that can bind to an antigen (such as IL-2 protein) without the entire antibody structure. An antigen-binding fragment can bind to the same antigen as the intact antibody. In some embodiments, the antigen-binding fragment may contain one or two or more CDRs from a certain human antibody, grafted to one or two or more framework regions from a different human antibody. The antigen-binding fragment includes but is not limited to Fab, Fab', F(ab)2, Fv fragment, Fd fragment, disulfide-stabilized Fv fragment (dsFv), (dsFv)2, bispecific dsFv (dsFv-dsFv'), disulfide-stabilized diabody (dsdiabody), single-chain fragment variable (scFv), scFv dimmer (bivalent diabody), bivalent single-chain antibody (BsFv), camelized single domain antibody, nanobody, domain antibody and diabody. For example, a "Fab" fragment of an antibody refers to an antibody fragment that is formed by joining a light chain (including the light chain variable region and the light chain constant region) and the variable region and CH1 of a heavy chain by disulfide linkage. A "Fab'" fragment refers to a Fab fragment that contains part of the hinge region. A "F(ab)2" refers to a dimmer of Fabs. The "Fc" fragment of an antibody is an antibody fragment formed by joining CH2 and CH3 of a heavy chain by disulfide linkage. The Fc fragment of an antibody is responsible for a variety of effector functions, such as determining the half-life of the antibody in serum in vivo, mediating immune response (such as antibody-dependent cell-mediated cytotoxicity [ADCC], complement-dependent cytotoxicity [CDC], or antibody dependent cellular phagocytosis [ADCP]), but not involved in antigen binding. An "Fv" fragment of an antibody refers to the smallest antibody fragment that contains the entire antigen-binding site. An Fv fragment consists of the variable region of a light chain and the variable region of a heavy chain. An "Fd fragment" of an antibody refers to the heavy chain portion of a Fab fragment, including VH, CH1 and part of the hinge region. "Single-chain Fv antibody (scFv)" refers to an engineered antibody composed of a light chain variable region linked to a heavy chain variable region directly or by a peptide chain (Huston JS et al., Proc Natl Acad Sci USA 1988, 85:5879-5883). A "(dsFv)2" contains three peptide chains and means that two VH groups are connected by a polypeptide linker, and then joined together with two VL groups by disulfide linkage. A "bispecific ds diabody" contains VL1-VH2 (linked by a polypeptide linker) and VH1-VL2 (also linked by a polypeptide linker), the two bound by disulfide linkage between VH1 and VL1. A "bispecific dsFv" or "dsFv-dsFv" contains three polypeptide chains: a VH1-VH2 fragment, in which the heavy chains of both are connected by a polypeptide linker (e.g., a long flexible linker) and each is linked to VL1 and VL2 fragments by disulfide linkage, and each pair of heavy and light chains connected by disulfide linkage have different antigen-binding specificities. A "scFv dimmer" is a bivalent diabody or bivalent single chain antibody (BsFv), containing two dimerized VH-VL (linked by a polypeptide linker) fragments, wherein the VH of one fragment cooperates with the VL of the other fragment to form two binding sites that can be targeted to bind to the same antigen (or antigen-binding epitope) or different antigens (or antigen-binding epitopes). In other embodiments, a "scFv dimmer" is a bispecific diabody, containing interlinked VL1-VH2 (linked by a polypeptide linker) and VH1-VL2 (linked by a polypeptide linker), wherein VH1 and VL1 cooperate, and VH2 and VL2 cooperate, and each cooperative pair has different antigen specificities. A "single-chain antibody Fv-Fc (scFv-Fc)" refers to an engineered antibody composed of scFv and an antibody Fc fragment. "Camelized single domain antibody", "heavy chain antibody" or "HCAb (Heavy-chain-only antibodies)" all refers to an antibody containing two VH domains but no light chain (Riechmann L & Muyldermans S, J Immunol Methods 1999, 231:25-38; Muyldermans S, J Biotechnol 2001, 74:277-302; Patent publication WO94/04678; Patent publication WO94/25591; US Patent No. 6005079). A heavy chain antibody was originally found in the camelidae family, which includes camels, dromedaries, and llamas. Although lack of the light chain, camelized antibodies have full antigen binding function (Hamers-Casterman C et al., Nature 1993, 363:446-448; Nguyen VK et al., Heavy-chain antibodies in Camelidae: a case of evolutionary innovation, Immunogenetics 2002, 54:39-47; Nguyen VK et al., Immunology 2003, 109:93-101). The variable region of a heavy-chain antibody (VHH domain) is the smallest antigen-binding unit produced by adaptive immunity known at present (Koch-Nolte F et al., FASEB J 2007, 21:3490-3498). A "nanobody" is an antibody fragment consisting of a VHH domain from a heavy chain antibody and two constant regions, CH2 and CH3. A "domain antibody" refers to an antibody fragment that contain only one heavy chain variable region or one light chain variable region. In certain instances, two or more VH domains are covalently bound by a polypeptide linker and form a bivalent domain antibody. The two VH domains of the bivalent domain antibody can be targeted to the same or different antigens. A "diabody" includes a small antibody fragment with two antigen-binding sites, comprising VH and VL domains (e.g., VH-VL or VL-VH) linked on the same polypeptide chain (Holliger P et al., Proc Natl Acad Sci USA 1993, 90:6444-6448; Patent EP404097; Patent publication WO93/11161). The linker between the two domains is short, which prevents the two domains on the same chain from pairing with each other, forcing them to pair with the complementary domains of the other chain, forming two antibody binding sites. The two antibody binding sites can be targeted for binding to the same or different antigens (or antigen-binding epitopes).

The anti-IL-2 antibody or the antibody domain of the hIL-2/anti-IL-2 antibody fusion protein of the present application comprises a whole full-length antibody or an antigen-binding fragment thereof (referring to the definition above), and optionally comprises all or part of the variable region of an antibody capable of competing with IL2Rα for binding to IL-2, and optionally comprises one or two or more regions encoded by V gene and/or D gene and/or J gene.

The term "immunoglobulin molecule" herein refers to a protein having the structure of a naturally occurring antibody. For example, IgG immunoglobulins are heterotetrameric glycoproteins of about 150,000 Da, composed of two light chains and two heavy chains linked by disulfide linkages. From the N-terminus to the C-terminus, each heavy chain has a variable region (VH, also known as variable heavy domain or heavy chain variable domain) followed by three constant domains (CH1, CH2 and CH3, also known as heavy chain constant region). Similarly, from the N-terminus to the C-terminus, each light chain has a variable region (VL, also known as variable light domain or light chain variable domain or light chain variable region) followed by a constant light domain (also known as light chain constant region, CL). The heavy chains of an immunoglobulin can be divided into α (IgA), δ (IgD), ε (IgE), γ (IgG) or μ (IgM), some of which can be further divided into subclasses such as γ1 (IgG1), y2 (IgG2), y3 (IgG3), y4 (IgG4), α1 (IgA1) and α2 (IgA2). Based on the amino acid sequence of their constant domains, the light chains of an immunoglobulin can be divided into kappa (κ) and lambda (λ). Immunoglobulin generally consists of two Fab molecules and an Fc domain linked by an immunoglobulin hinge region.

The terms "light chain variable region (VL)" and "heavy chain variable region (VH)" herein refer to polypeptides comprising VL or VH, respectively. The pairing of VH and VL forms a single antigen binding site. Endogenous VL is encoded by gene segments V (variable) and J (joined), and endogenous VH is encoded by V, D (diversity) and J. Both VL and VH include hypervariable regions CDRs (complementarity-determining regions) and framework regions (FRs). The term "variable region" or "V region" can be used interchangeably and refers to a heavy chain variable region or a light chain variable region comprising FR1-CDR1-FR2-CDR2-FR3-CDR3-FR4. V regions can be naturally occurring, recombinant or synthetic. Herein, the light chain variable region and/or heavy chain variable region of an antibody may sometimes be referred to as "antibody variable region" or "antibody chain". As provided and further described herein, the "antibody light chain variable region" or "antibody heavy chain variable region" and/or "antibody variable region" and/or "antibody chain" optionally comprises a polypeptide sequence introduced with cysteine residue(s).

The term "complementarity-determining region (CDR)" or "hypervariable region (HVR)" herein can be used interchangeably and refers to each region of the variable region of an antibody that is highly variable in sequence and/or forms a loop defined structurally ("Hypervariable loop"). The CDRs are the binding site of an antibody for its target protein, structurally complementary to the epitope of the target protein, and thus the binding site has the specificity for binding this target protein. Usually, a native four-chain antibody contains six CDRs, three in the VH (HCDR1, HCDR2 and HCDR3) and three in the VL (LCDR1, LCDR2 and LCDR3). The remaining VL or VH regions except for the CDRs, i.e., the framework regions (FRs), have less amino acid sequence variation (Kuby, Immunology, 4th Edition, Chapter 4, WH Freeman & Co., 2000).

The positions of CDRs and FRs can be determined using a variety of definition methods well known in the art, e.g., Kabat, Chothia, IMGT, and Contact (e.g., Kabat et al. 1991, Sequences of Proteins of Immunological Interest, Fifth Edition, U.S. Department of Health and Human Services, NIH Publication No. 91-3242; Johnson et al., Nucleic Acids Res 2001, 29:205-206; Chothia & Lesk, J Mol Biol 1987, 196:901-917; Chothia et al., Nature 1989, 342:877-883; Chothia et al., J Mol Biol 1992, 227:799-817; Al-Lazikani et al., J Mol Biol 1997, 273:927-748; Lefranc MP et al., IMGT, The International ImmunoGenetics Database, Nucleic Acids Research 1999, 27:209-212; MacCallum RM et al., J Mol Biol 1996, 262:732-745). Definitions of antigen binding sites are also described in: Ruiz et al., Nucleic Acids Res 2000, 28:219-221; Lefranc MP, Nucleic Acids Res 2001, 29:207-209; Lefranc MP, The Immunologist 1999, 7:132-136; Lefranc MP et al., Dev Comp Immunol 2003, 27:55-77; MacCallum et al., J Mol Biol 1996, 262:732-745; Martin et al., Proc Natl Acad Sci USA 1989, 86:9268-9272; Martin et al., Methods Enzymol 1991, 203:121-153; Rees et al., by Sternberg MJE (edition), Protein Structure Prediction, Oxford University Press, Oxford, 141-172 (1996). The present application includes any one of definition methods to determine the CDRs in the antibody component of the hIL-2/anti-IL-2 antibody fusion protein of the present application or the anti-IL-2 antibody or antigen-binding fragment thereof of the present application, and Table 1 shows the amino acid residues of the antibody CDRs determined by different determine methods. The exact number of amino acid residues encompassing a particular CDR varies with the CDR sequence. With the amino acid sequence of the variable region of an antibody being clarified, those skilled in the art can determine the CDR of the antibody by conventional definition methods, including but not limited to the definitions herein.

Table 1. CDR[1] identified by different definition methods

| CDRs | Kabat | Chothia | IMGT | Contact |
|---|---|---|---|---|
| HCDR1 | 31-35 | 26-32 | 27-38 | 30-35 |
| HCDR2 | 50-65 | 52-56 | 56-65 | 47-58 |
| HCDR3 | 95-102 | 95-102 | 105-117 | 93-101 |
| LCDR1 | 24-34 | 24-34 | 27-38 | 30-36 |
| LCDR2 | 50-56 | 50-56 | 56-65 | 46-55 |
| LCDR3 | 89-97 | 89-97 | 89-97 | 89-96 |

[1]: All CDR definitions in Table 1 are numbered according to the numbering system proposed by Kabat et al. (Kabat et al. 1991, Sequences of Proteins of Immunological Interest, Fifth Edition, U.S. Department of Health and Human Services).

In addition, Kabat et al. have defined a numbering system for variable region sequences, which can be applied to any antibody. A person of ordinary skill in the art would be able to unambiguously apply this "Kabat numbering" system to the variable region sequence of any antibody without relying on any experimental data other than the antibody sequence itself to determine the variable region sequence. Unless otherwise stated, the numbering of specific amino acid residue positions in the variable region of the anti-IL-2 antibody or the antibody component of the hIL-2/anti-IL-2 antibody fusion protein of the present application is determined according to the Kabat numbering system.

The term "Fc region" or "Fc domain" herein refers to the C-terminal region of an immunoglobulin heavy chain, which contains at least a portion of the constant region, such as an immunoglobulin heavy chain constant region excluding the first constant region (CH1). The Fc region as used herein includes the native Fc region and/or Fc region variants and can be a portion of the anti-IL-2 antibody or the hIL-2/anti-IL-2 antibody fusion protein of the present application. It would be understood in the art that the boundary of the Fc region may vary, and however, a human IgG heavy chain Fc region is generally defined as comprising either a cysteine residue at position 226 or a proline residue at position 230 at its carboxyl terminus, according to the EU numbering system/scheme as found in Kabat et al. (1991, NIH Publication No. 91-3242, National Technical Information Service, Springfield, VA).

The term "monoclonal antibody" herein refers to an antibody obtained from a population of antibodies that is substantially homogeneous, i.e., the individual antibodies that make up the population are identical except for natural mutations that may be present in minor amounts. A monoclonal antibody exhibits a single binding specificity and affinity for a particular epitope.

The term "chimeric antibody" herein refers to an antibody that contains sequences derived from two different antibodies (e.g., US Patent No. 4,816,567), wherein the antibody is typically derived from different species. For example, a chimeric antibody comprises human and rodent antibody fragments, usually human constant regions and mouse variable regions. Methods for producing chimeric antibodies include conventional recombinant DNA and gene transfection techniques known to those skilled in the art (e.g., Morrison SL et al., Proc Natl Acad Sci USA 1984, 81:6851-6855; US Patent No. US5,202,238 and US Patent No. US5,204,244).

The term "humanized antibody or antigen-binding fragment" herein refers to an antibody or an antigen-binding fragment comprising CDRs derived from non-human animals, FR regions derived from human, and constant regions derived from human. A humanized antibody or antigen-binding fragment has reduced immunogenicity, and thus can be used as a therapeutic agent for humans. In some embodiments, the non-human animal is a mammal such as a mouse, rat, rabbit, goat, sheep, guinea pig, or hamster. In some embodiments, the humanized antibody or antigen-binding fragment consists essentially entirely of human sequences, except for the CDR sequences which are non-human. In some embodiments, the human-derived FR region may include the same amino acid sequence as the human antibody from which it is derived, or it may include some amino acid mutations, e.g., no more than 10, 9, 8, 7, 6, 5, 4, 3, 2 or 1 amino acid modification. In some embodiments, the amino acid modification may be present only in the heavy chain FR region, only in the light chain FR region, or in both chains.

The term "corresponding human germline sequence" herein refers to an antibody variable region amino acid sequence or subsequence, which has the highest amino acid sequence identity to the amino acid sequence of the reference human germline immunoglobulin variable region, compared to the amino acid sequences of all other known human germline immunoglobulin variable region. The corresponding human germline sequence may be just a framework region, a complementarity-determining region, and other combinations of a framework region and complementarity-determining region, a variable region, and a variable region sequence or subsequence. Sequence identity can be determined by aligning two sequences using the methods described herein, e.g., using BLAST, ALIGN, or another alignment algorithm known in the art. The corresponding human germline nucleic acid or amino acid sequence may have at least about 90%, 91%, 92%, 93%, 94%, 95%, 96%, 97%, 98%, 99% or 100% identity to the amino acid sequence of reference human germline immunoglobulin variable region.

The anti-IL-2 antibody or the antibody component of an hIL-2/anti-IL-2 antibody fusion protein of the present application can be selected from any one or two or more of the following forms, including a chimeric, non-human, humanized, or fully human form, provided that the form is capable of competing with human IL2Rα for binding to hIL-2 or the mutein thereof and blocking hIL-2 or the mutein thereof from binding of human IL2Rα, while retaining the binding and functional activities of hIL-2 or the mutein thereof to IL2Rβ/γ.

The term "specifically bind/binding" or "binding specificity" or "specific for" or "binding" herein refers to a binding reaction that determines the presence of a target molecule (or protein or antigen) in a heterogeneous population of proteins and other biological agents (e.g., in biological samples, such as blood, serum, plasma or tissue samples), that is, the binding is selective for the target molecule and can distinguish from those undesired or nonspecific interactions. For example, an antibody that specifically binds to a target molecule (which may be an antigen) when binding to the target molecule has a greater affinity, binding activity, easier binding and/or longer duration than that when binding to other non-target molecules. In some embodiments, the antibody specifically binds to the target antigen with a dissociation equilibrium constant ($K_D$) value of <1 μM, <100 nM, <10 nM, <1 nM, or <0.1 nM.

The term "antigen-antibody binding interface" herein refers to the spatial structure region formed by the amino acid residues that participate in antigen-antibody interactions (e.g., through binding, steric hindrance, stabilizing/destabilizing, steric distribution) when an antibody specifically binds to an antigen, wherein the spatial region includes the antigenic epitope and the antigen-binding region of the antibody. The amino acid residues of the antigen-antibody binding interface may or may not be in contiguous sequence. For example, when the interface is three-dimensional, the amino acid residues of the interface can be separated at different positions on the linear sequence. As used herein, the term "epitope" means a protein-determining region capable of specific binding by an antibody. Epitopes typically consist of chemically active surface groups of molecules such as amino acids or sugar side chains and typically have specific three-dimensional structural characteristics, as well as specific charge characteristics. The difference between conformational epitopes and non-conformational epitopes is that in the presence of a denaturing solvent, the binding of the antibody to the former is lost, but the binding to the latter is retained.

As used herein, the term "epitope binding domain" or "antigen binding domain" or "antigen-binding region" can be used interchangeably and refers to a portion of a binding molecule (such as an antibody or an epitope-binding fragment or derivatives thereof) that specifically interacts with a binding site on a target epitope (e.g., by binding, steric hindrance, stabilizing/destabilizing, steric distribution). The "antigen-binding region" in the present application also refers to one or two or more fragments of an antibody that retains specific interaction (e.g., by binding, steric hindrance, stabilizing/destabilizing, steric distribution) with an IL-2 epitope.

The term "fusion" or "fused" herein when applied to an amino acid sequence (e.g., peptide, polypeptide, or protein) refers to the combination of two or more amino acid sequences into non-naturally occurring single amino acid sequence by for example chemical bonding or recombination. A fusion polypeptide can be produced by recombination of two polynucleotides encoding the two amino acid sequences followed by transfecting an expression construct containing the recombined polynucleotide into a host cell.

The term "antibody variant" or "antibody mutant" herein refers to an antibody polypeptide sequence that contains at least one amino acid mutation in the variable region of a reference antibody. A variant can be substantially homologous or substantially identical to the unmodified antibody. In some embodiments, amino acid mutation(s) is/are introduced in one, two, three, four, five and/or six CDRs of the anti-IL-2 antibody or the antibody component of the hIL-2/anti-IL-2 antibody fusion protein of the present application, to increase the degree of humanization of the antibody or antibody component, to enhance specificity or affinity for IL-2 muteins, and/or to form stable complexes with IL-2 or IL-2 mutein thereof. In some embodiments, amino acid mutation(s) is/are introduced in one, two, three and/or four FRs in the anti-IL-2 antibody or the antibody component of the hIL-2/anti-IL-2 antibody fusion protein of the present application, to increase the degree of humanization of the antibody or the antibody component, and/or to form stable complexes with IL-2 or IL-2 muteins thereof. In some embodiments, one or more amino acid mutations are introduced in the CDRs and FRs of the anti-IL-2 antibody or the antibody component of the IL-2/anti-IL-2 antibody fusion protein of the present application, to improve the degree of humanization of the antibody or the antibody component, to enhance specificity or affinity for IL-2 or IL-2 muteins thereof, and/or to form stable complexes with IL-2 or IL-2 muteins thereof. In some embodiments, the amino acid mutations include amino acid substitution, deletion, insertion, or any combination thereof.

Among them, amino acid substitution includes conservative amino acid substitutions and non-conservative amino acid substitutions, wherein conservative amino acid substitutions pertain to replacing with another amino acid in the same class (such as similar biochemical properties or function), and non-conservative substitutions pertain to replacing an amino acid in a class with another one in another class. A person skilled in the art can make conservative amino acid substitutions based on similarity in polarity, charge, solubility, hydrophobicity, hydrophilicity, and/or amphipathic properties of the residues involved. For example, (i) nonpolar (hydrophobic) amino acids include alanine (Ala, A), leucine (Leu, L), isoleucine (Ile, I), valine (Val, V), proline (Pro, P), phenylalanine (Phe, F), tryptophan (Trp, W) and methionine (Met, M); (ii) polar neutral amino acids include glycine (Gly, G), serine (Ser, S), threonine (Thr, T), cysteine (Cys, C), tyrosine (Tyr, Y), asparagine (Asn, N) and glutamine (Gln, Q); (iii) positively charged (basic) amino acids include arginine (Arg, R), lysine (Lys, K) and histidine (His, H); (iv) negatively charged (acidic) amino acids include aspartic acid (Asp, D) and glutamic acid (Glu, E). Non-conservative amino acid substitutions may lead to great changes in the properties or functions of biological macromolecules. Substitutions that are generally expected to result in the greatest changes in polypeptide properties include but are not limited to that: (a) hydrophilic residue(s) such as serine or threonine replaces (or is replaced with) hydrophobic residue(s) such as leucine, isoleucine, phenylalanine, valine or alanine; (b) cysteine or proline replaces (or is replaced with) any other amino acid residue; (c) residue(s) with a positively charged side chain such as lysinyl, arginyl or histidyl group replaces (or is replaced with) negatively charged residue(s) such as glutamyl or aspartyl group; or (d) residue(s) with a large side chain such as phenylalanine replaces (or is replaced with) residue(s) having no side chain such as glycine. Although the site or region where amino acid sequence mutation is introduced can be predetermined, potential changes in the properties or functions of biomacromolecules brought about by non-conservative substitutions are unpredictable. In some embodiments, the hIL-2 muteins of the present application achieve unexpected changes in function by introducing non-conservative amino acid substitutions in the wild-type hIL-2, for example, the wild-type human IL-2 that is incapable of binding with an anti-IL-2 antibody described herein gains binding activity to the said antibody after introducing mutations by non-conservative amino acid substitutions.

In some embodiments, with respect to the introduction of cysteine residues at the antigen-antibody binding interface, said ordinal numerals first and second are used only to facilitate distinguishing the location of cysteine introduction. Unless explicitly stated otherwise, the use of these terms is not intended to confer a particular order in which particular amino acid residues are substituted with cysteine residues, or in which cysteine residues are added or inserted.

The term "identical" or "identity" or "percent identity" or "percent sequence identity" herein can be used interchangeably in two or more nucleic acid or polypeptide sequences, and refers that after amino acid sequences (or nucleic acid sequences) are aligned, and if necessary, gaps are introduced to maximize the number of identical amino acids (or nucleic acids), the percentage of amino acid (or nucleic acid) residues of the candidate sequence that are identical to the reference sequence in the amino acid (or nucleic acid) residues of the candidate sequence. Conservative substitutions of such amino acid residues may or may not be considered to be the identical residues. Percent sequence identity for amino acid (or nucleic acid) sequences can be determined by aligning the sequences using the tools disclosed in the art, such as BLASTN, BLASTp (National Center for Biotechnology Information website (NCBI); Altschul SF et al., J Mol Biol 1990, 215:403-410; Stephen F et al., Nucleic Acids Res 1997, 25:3389-3402), ClustalW2 (European Bioinformatics Institute website; Higgins DG et al., Methods in Enzymology 1996, 266:383-402; Larkin MA et al., Bioinformatics 2007, 23:2947-2948), and ALIGN or Megalign (DNASTAR) software. Those skilled in the art can use the default parameters of the tools or adjust the parameters according to the alignment requirement, for example by selecting a suitable algorithm.

The term "isolated" herein when referring to a nucleic acid (or polynucleotide) or protein means that the nucleic acid or protein contains substantially no other cellular components bound to it in its natural state, preferably being in a homogeneous state, and for example, an isolated nucleic acid or protein may be removed from its natural environment. The isolated nucleic acid or protein can be lyophilized or in an aqueous solution. Generally, its purity and homogeneity can be determined using analytical chemistry techniques such as polyacrylamide gel electrophoresis or high performance liquid chromatography. As the main ingredient in the formulation, protein is basically obtained by purification. The term "purified" means that the protein produces substantially one band in an electrophoresis gel. In particular, this means that the nucleic acid or protein is at least 85% purity, more preferably at least 95% purity, and most preferably at least 99% purity. For the present application, in some embodiments, recombinant proteins expressed in host cells are considered to be isolated, and this applies to natural or recombinant proteins separated, fractionated, or partially or substantially purified by any technique well-known to those skilled in the art. In some embodiments, an "isolated antibody" refers to an antibody that is substantially free of other antibodies with different antigen specificities (e.g., an isolated antibody that specifically binds hIL-2 substantially does not contain other antibodies that specifically bind antigens other than hIL-2). However, the isolated antibody that specifically binds hIL-2 may be cross-reactive with other antigens, such as IL-2 molecules from other species, e.g., mouse IL-2. Furthermore, the isolated antibody may be substantially free of other cellular material and/or chemicals. In some embodiments, a recombinant polynucleotide encoding a polypeptide or protein of the present application (e.g., hIL-2 or mutein thereof, anti-IL-2 antibody, hIL-2/anti-IL-2 antibody fusion protein) contained in a vector is considered to be isolated. Other examples of isolated polynucleotide include recombinant polynucleotide contained in heterologous host cells or (partially or substantially) purified polynucleotide in solution. An isolated polynucleotide includes a polynucleotide molecule contained in cells that ordinarily contain the polynucleotide molecule, but the polynucleotide molecule is present extra-chromosomally or at another chromosomal location other than its natural chromosomal location. The isolated polynucleotide or nucleic acid of the present application also includes such molecules produced synthetically. Additionally, a polynucleotide or nucleic acid can be or can include a regulatory element such as promoter, ribosome binding site, or transcription terminator.

The term "engineering" herein is taken to include any manipulation of a polypeptide or protein backbone or post-translational modification of a naturally occurring or recombinant protein or polypeptide or fragment thereof. Engineering includes mutation, glycosylation patterns, or modification of individual amino acid side chain groups to amino acid sequences, and a combination of these methods.

As used herein, the term "polypeptide" refers to a polymer of amino acids and their equivalents, rather than to a specific length of a product; thus, "peptide" and "protein" are included within the definition of polypeptide. Also included within the definition of polypeptide is an "antibody" as defined herein.

Unless indicated in the context otherwise, a "derivative" is a polypeptide or fragment thereof that has one or two or more non-conservative or conservative amino acid substitutions relative to a second polypeptide (also referred to as a "variant"); or a modified polypeptide or fragment thereof by covalently linking the second molecule, e.g., by linking a heterologous polypeptide, or by glycosylation, acetylation, phosphorylation, etc. The definition of "derivative" may also include, for example, a polypeptide containing one or more amino acid analogs (e.g., unnatural amino acids, etc.), a polypeptide with unsubstituted linkage, and other (natural and non-natural) modifications known in the art.

The term "expression vector" or "vector" herein refers to a vehicle into which a polynucleotide or nucleic acid encoding a protein can be operatively inserted and the protein can be expressed. A vector can be used to transform, transduce or transfect a host cell, allowing the genetic material elements it carries to be expressed in the host cell.

The term "host cell" herein is a cell to be introduced with an exogenous polynucleotide or nucleic acid and/or vector. A host cell includes a "transformant" and "transformed cell", which includes the primary transformed cell and progeny derived therefrom, regardless of the number of passages. The progeny may not be identical in nucleic acid content to the parent cell, and may contain mutation. Included in the present application are mutant progeny screened or selected for the same function or biological activity from the originally transformed cell.

The terms "subject", "patient" or "individual" herein are used interchangeably and include, but are not limited to, a mammal, including, for example, human, a non-human primate (e.g., monkey), mouse, pig, cow, goat, rabbit, rat, guinea pig, hamster, horse, monkey, sheep, or other non-human mammal; a non-mammal, including, for example, non-mammalian vertebrates such as birds (e.g., chicken or duck) or fish; and non-mammalian invertebrates. In some embodiments, the subject and pharmaceutical composition involved in the use or method of the present application are used to treat (prophylactically and/or therapeutically) non-human animals.

"Treating" or "treatment" for a disease or symptom herein means alleviating a disease or condition, reducing the rate at which a disease or condition arises or develops, and reducing the risk of developing a disease or condition, or delaying the development of symptoms associated with a disease or condition, reducing or terminating symptoms associated with a disease or condition, producing a complete or partial reversal of a disease or condition, curing a disease or condition, or any combination of the above.

"Prevention" herein includes the inhibition of the occurrence or progression of a disease or disorder or symptoms of a particular disease or condition. In some embodiments, subjects with a family history of the disease are candidates for preventive regimens. In general, the term "prevention" refers to the administration of a drug prior to the onset of a sign or symptom, particularly in a subject at risk.

The term "therapeutically effective amount" or "effective dose" refers to a dose or concentration effective to achieve prevention or amelioration of symptoms associated with a disease or disorder and/or lessening the severity of a disease or disorder, at a dosage and for a desired period of time. A therapeutically effective amount of a preparation, antibody or antigen-binding fragment, or fusion protein, or composition thereof of the present application may vary depending on factors such as the disease state, the age, sex and weight of the individual and the ability of the antibody or antibody portion or fusion protein to elicit a desired response in the individual. A therapeutically effective amount may also be considered as any toxic or detrimental effect of the preparation, the antibody or antigen-binding fragment, or fusion protein, or composition thereof lower than the therapeutically beneficial effect. The term "effective amount" refers to an amount of an active ingredient or agent sufficient to provide a clinical benefit to a subject, including, but not limited to, amelioration, alleviation, or relief of a disease, disorder, or associated symptoms thereof, delaying or arresting disease progression.

The term "pharmaceutically acceptable" herein refers to the indicated carrier, vehicle, diluents, adjuvant and/or salt, generally chemically and/or physically compatible with the other ingredients in the formulation and physiologically compatible with the subject.

The term "about" when used in conjunction with a numerical value is meant to encompass a numerical value within a range having a lower limit of 5% less than the specified numerical value and an upper limit of 5% greater than the specified numerical value, or in one embodiment a lower limit of 10% less than the specified numerical value and an upper limit of 10% greater than the specified numerical value, or in another embodiment a lower limit of 15% less than the specified numerical value and an upper limit of 15% greater than the specified numerical value, or in another embodiment a lower limit of 20% less than the specified numerical value and an upper limit of 20% greater than the specified numerical value.

The term "and/or" should be understood to mean any one of the alternatives or a combination of any two or more of the alternatives.

As used herein, the terms "comprising" or "including" or "containing" or "having" or "involving" are used interchangeably to mean the inclusion of an element, integer or step, but not the exclusion of any other elements, integers or steps. Herein, when the terms "comprising" or "including" or "containing" or "having" or "involving" are used, unless otherwise indicated, situations consisting of the said elements, integers or steps are also encompassed.

As mentioned herein, "some embodiments", "one embodiment", "one specific embodiment", or "specific embodiments", or a combination thereof indicates that the specific features, structures or characteristics described in relation to the embodiment are included in at least one embodiment of the present application. Thus, appearances of the foregoing terms in various places throughout this specification do not necessarily refer to the same embodiment. Furthermore, the specific features, structures or characteristics may be combined in any suitable manner in one or more embodiments.

Unless the context clearly dictates otherwise, singular terms encompass plural referents and vice versa.

All patents, patent applications, and other publications are expressly incorporated herein by reference for the purposes of description and disclosure. These publications are provided solely because their disclosure predates the filing date of this application. All statements regarding the dates of these documents or representations of the contents of these documents are based on information available to the applicant and do not constitute any admission as to the correctness of the dates of these documents or the contents of these documents. Furthermore, any citation of these publications in this document does not constitute an admission that the publications form part of the common general knowledge in the field in any country.

Various aspects of the present application will be described in more details in the following sections.

_1. hIL-2 Mutein of the Present Application_

In one aspect, the present application provides a hIL-2 mutein. Compared to the wild-type hIL-2 (as set forth in SEQ ID NO: 1), the said hIL-2 mutein has gained an ability to bind to the anti-IL-2 antibody or antigen-binding fragment thereof of the present application, and the said mutations have no significant effects on the biological activity of hIL-2.

The binding affinity ($K_D$) value of the hIL-2 mutein of the present application to the anti-IL-2 antibody or antigen-binding fragment thereof disclosed herein is $<5 \times 10^{-8}$ M, preferably $<1 \times 10^{-8}$ M, $<5 \times 10^{-9}$ M, or $<1 \times 10^{-9}$ M, more preferably $<6 \times 10^{-10}$ M. The $K_D$ value of the IL-2 mutein of the present application to the anti-IL-2 antibody or antigen-binding fragment thereof disclosed herein is about $5 \times 10^{-8}$ M to $5 \times 10^{-10}$ M, or for instance, $1 \times 10^{-8}$ M to $1 \times 10^{-10}$ M, $1.5 \times 10^{-8}$

M to $1\times10^{-10}$ M, $1\times10^{-8}$ M to $10\times10^{-10}$ M, $1\times10^{-9}$ M to $1\times10^{-10}$ M.

The hIL-2 mutein of the present application also contains the following functional characteristics: (i) the said hIL-2 mutein retains the binding activity and biological function of hIL-2 to hIL2R $\beta/\gamma$; (ii) the anti-IL-2 antibody or antigen-binding fragment thereof of the present application is capable of competing with IL2R$\alpha$ for binding to the hIL-2 mutein of the present application; (iii) the anti-IL-2 antibody or antigen-binding fragment thereof of the present application can block the binding of the hIL-2 mutein to IL-2R$\alpha$.

The hIL-2 mutein of the present application also has other improved properties, for example, the mutation sites therein are located inside of the antigen-antibody binding interface, unexposed to the outside of the antigen-antibody binding interface, which significantly reduces the risk of inducing potential ADA (anti-drug antibody).

In some embodiments, the hIL-2 mutein of the present application has 90% or more amino acid sequence identity, preferably 93% or more amino acid sequence identity, but less than 100% sequence identity to the wild-type hIL-2 (as set forth in SEQ ID NO: 1). In some embodiments, the hIL-2 mutein of the present application comprises at least one or two or more amino acid mutations relative to the wild-type hIL-2. In some embodiments, the hIL-2 mutein of the present application comprises at least one or two or more amino acid substitutions and/or deletions relative to the wild-type hIL-2. In some embodiments, the hIL-2 mutein of the present application comprises at least one or two or more amino acid substitutions other than the substitution to cysteine residue, relative to the wild-type hIL-2. In one embodiment, the said amino acid mutations are located at the antigen-antibody binding interface.

In a specific embodiment, the said amino acid mutations are located at the antigen-antibody binding interface. In a specific embodiment, the mutations in the hIL-2 mutein of the present application are embedded inside of the binding interface of the hIL-2 mutein and the anti-IL-2 antibody or antigen-binding fragment thereof, and the part of the hIL-2 mutein exposed outside of the binding interface retains the same amino acid sequence as the wild-type hIL-2. Therefore, while the biological activity of hIL-2 is not significantly affected, the immunogenicity of the said hIL-2 mutein is significantly reduced, i.e., the potential ADA risk of the said hIL-2 mutein is significantly reduced after it forms the antigen-antibody complex with the anti-IL-2 antibody or antigen-binding fragment thereof. The said hIL-2 mutein comprises one or two or more amino acid mutations at positions 64, 90 and/or 104 relative to the amino acid sequence of wild-type hIL-2 as set forth in SEQ ID NO: 1. In some embodiments, the said hIL-2 mutein comprises one, two, or three amino acid mutations at positions selected from K64, N90 and M104 corresponding to the wild-type hIL-2. In some embodiments, relative to the wild-type hIL-2 as set forth in SEQ ID NO: 1, the said hIL-2 mutein comprises at least one of the following mutations: (1) K64 corresponding to the wild-type hIL-2 as set forth in SEQ ID NO: 1 is mutated to G, S, T, C, Y, N or Q; (2) N90 corresponding to the wild-type hIL-2 as set forth in SEQ ID NO: 1 is mutated to R, K or H; (3) M104 corresponding to the wild-type hIL-2 as set forth in SEQ ID NO: 1 is mutated to E or D.

In a specific embodiment, relative to the wild-type hIL-2 as set forth in SEQ ID NO: 1, the said hIL-2 mutein comprises one or two or more of the following mutations: (a) K64G at the position 64 corresponding to the wild-type hIL-2 as set forth in SEQ ID NO: 1, (b) N90R at the position 90 corresponding to the wild-type hIL-2 as set forth in SEQ ID NO: 1, or (c) M104E at the position 104 corresponding to the wild-type hIL-2 as set forth in SEQ ID NO: 1; preferably, the amino acid mutations are K64G and/or N90R, or K64G and N90R and M104E, and more preferably the amino acid mutations are K64G and N90R.

In one embodiment, the said hIL-2 mutein comprises amino acid mutations located outside of the antigen-antibody binding interface to mitigate the liability associated with the chemistry, manufacturing and controls (CMC) of hIL-2 mutein products. In a specific embodiment, in order to solve the problem of glycosylated isomers in the manufacturing of hIL-2 mutein products, the wild-type hIL-2 is mutated to remove its glycosylation modification site, wherein the said hIL-2 mutein comprises a deletion of the first three or five amino acid residues at the N-terminus corresponding to the wild-type hIL-2 as set forth in SEQ ID NO: 1, or a point mutation at the position 3 corresponding to the wild-type hIL-2 as set forth in SEQ ID NO: 1; and the preferred amino acid mutation is the deletion of the first three amino acid residues at the N-terminus of wild-type hIL-2. In a specific embodiment, in order to solve the problem of possible cysteine-mismatch in the manufacturing of hIL-2 mutein products, the wild-type hIL-2 is mutated to eliminate the unpaired cysteine residue, wherein the said hIL-2 mutein comprises a point mutation at the position 125 corresponding to the wild-type hIL-2 as set forth in SEQ ID NO: 1, and the preferred amino acid mutation is C125A, C125L, C125S, C125Q or C125V.

In one embodiment, the sites of said amino acid mutations include both inside and outside of the antigen-antibody binding interface. In a specific embodiment, the said hIL-2 mutein comprises one or more amino acid mutations at the first 3 or 5 residues at the N-terminus, or the 3rd position, or the 64th position, or the 90th position, or the 104th position, or the 125th position corresponding to the wild-type hIL-2 as set forth in SEQ ID NO: 1. In some embodiments, the said hIL-2 mutein comprises a deletion of the first 3 residues at the N-terminus, substitution mutations at one or more positions selected from K64, N90 and M104, and substitution mutation at the position C125; the preferred amino acid mutations are a combination of K64G and/or N90R, deletion of the first 3 residues at the N-terminus, and any one mutation selected from C125A, C125L, C125S, C125Q and C125V; or a combination of K64G, N90R, M104E, deletion of the first 3 residues at the N-terminus, and any one mutation selected from C125A, C125L, C125S, C125Q and C125V; the more preferred mutations are a combination of K64G, N90R, deletion of the first 3 residues at the N-terminus, and C125L.

*2. Anti-IL-2 Antibody or Antigen-binding Fragment thereof of the Present Application*

In one aspect, the present application provides an anti-IL-2 antibody or antigen-binding fragment thereof capable of competing with IL2Rα for binding to the hIL-2 mutein of the present application and blocking the IL-2 mutein from binding to IL2Rα or any IL-2 receptor complex comprising the α subunit (i.e., IL2Rα/β or IL2Rα/β/γ), and having a high binding affinity to the hIL-2 mutein of the present application, not easy to dissociate.

The binding affinity ($K_D$) of the anti-IL-2 antibody or antigen-binding fragment thereof of the present application to the said hIL-2 mutein is $<5 \times 10^{-8}$ M, preferably $<1 \times 10^{-8}$ M, $<5 \times 10^{-9}$ M, or $<1 \times 10^{-9}$ M, more preferably $<6 \times 10^{-10}$ M. The anti-IL-2 antibody or antigen-binding fragment thereof of the present application and the said hIL-2 mutein have a $K_D$ value of about $5 \times 10^{-8}$ M to $5 \times 10^{-10}$ M, or for instance, $1 \times 10^{-8}$ M to $1 \times 10^{-10}$ M, $1.5 \times 10^{-8}$ M to $1 \times 10^{-10}$ M, $1 \times 10^{-8}$ M to $10 \times 10^{-10}$ M, $1 \times 10^{-9}$ M to $1 \times 10^{-10}$ M. The dissociation rate constant ($k_{dis}$) between the anti-IL-2 antibody or antigen-binding fragment thereof of the present application and the said hIL-2 mutein is $<5 \times 10^{-3}$ s$^{-1}$, $<1 \times 10^{-3}$ s$^{-1}$, $<5 \times 10^{-4}$ s$^{-1}$, or $<3 \times 10^{-4}$ s$^{-1}$.

The antibody of the present application can compete with IL2Rα for binding of hIL-2 or hIL-2 mutein. In a specific embodiment, the anti-IL-2 antibody or antigen-binding fragment thereof can specifically recognize the IL2Rα-binding site of IL-2 or IL-2 mutein. The antibody of the present application may also optionally include F(ab)2, F(ab')2, Fab, Fab', scFv, single domain antibody, and the like. The antibody of the present application can be a chimeric antibody, a humanized antibody, a fully human antibody, and can be a monoclonal antibody, a polyclonal antibody, a bispecific antibody, a multispecific antibody, or an antibody fragment, as long as the antibody can specifically recognize the IL2Rα-binding site of hIL-2 or hIL-2 mutein, and can prevent hIL-2 or hIL-2 mutein from binding to IL2Rα. In some embodiments, the anti-IL-2 antibody is a humanized antibody. In some embodiments, the anti-IL-2 antibody is selected from an anti-mouse IL-2 antibody and a humanized antibody thereof. In a specific embodiment, the anti-mouse IL-2 antibody is selected from an antibody that specifically recognize the epitope of mouse IL-2 (mIL-2) involved in binding of mouse IL2Rα (mIL2Rα), and the anti-mIL-2 antibody is capable of cross-reacting with the said hIL-2 mutein of the present application.

In another aspect, the anti-IL-2 antibody of the present application comprises VH and VL. In some embodiments, the antibody of the present application is derived from an anti-IL-2 antibody comprising the VH amino acid sequence as set forth in SEQ ID NO: 5 and the VL amino acid sequence as set forth in SEQ ID NO: 6, and is capable of specifically binding to the said IL-2 mutein and blocking the IL-2 mutein from binding of IL2Rα.

In some embodiments, the anti-IL-2 antibody of the present application is derived from an antibody comprising the VH amino acid sequence as set forth in SEQ ID NO: 5 as shown below:

EVQLQESGAELVRPGTSVKLSCKVSGDTITAYYLHFVRQRPGQGLEWIGRIDPEDDS
TKYAENFKNKATFTADASSNTAYLRLSSLTSEDTATYFCTTVTFYYSRELRWFAYW
GQGTLVTVSS (SEQ ID NO: 5)

In some embodiments, the anti-IL-2 antibody of the present application is derived from an antibody comprising the VL amino acid sequence as set forth in SEQ ID NO: 6 as shown below:

DIQVTQSPASLSASLEEIVTITCQASQDIGNYLSWYQQKLGKSPQLLIHSATSLADGVP
SRFSGSRSGTQYSLKINRLQVEDTGIYYCLQHYSTPYTFGAGTKLELK (SEQ ID NO: 6)

In some embodiments, the anti-IL-2 antibody of the present application comprises one or two or more CDRs of the VH amino acid sequence as set forth in SEQ ID NO: 5, or amino acid sequences as set forth as SEQ ID NOs: 7, 8 and 9, or a variant thereof, wherein the variant thereof includes a humanized antibody or any other variant described in the present application. In some embodiments, the anti-IL-2 antibody comprises HCDR1 (as set forth in SEQ ID NO: 7), HCDR2 (as set forth in SEQ ID NO: 8) and HCDR3 (as set forth in SEQ ID NO: 9) of the VH amino acid sequence as set forth in SEQ ID NO: 5.

In some embodiments, the anti-IL-2 antibody of the present application comprises one or two or more CDRs of the VL amino acid sequence as set forth in SEQ ID NO: 6, or amino acid sequences as set forth in SEQ ID NOs: 10, 11 and 12, or a variant thereof wherein the variant thereof includes a humanized antibody or any other variant described in the present application. In some embodiments, the anti-IL-2 antibody comprises LCDR1 (as set forth in SEQ ID NO: 10), LCDR2 (as set forth in SEQ ID NO: 11), and LCDR3 (as set forth in SEQ ID NO: 12) of the VL amino acid sequence as set forth in SEQ ID NO: 6.

In some embodiments, the anti-IL-2 antibody of the present application comprises one or two or more CDRs of the VH amino acid sequence as set forth in SEQ ID NO: 5, or amino acid sequences as set forth in SEQ ID NOs: 7, 8 and 9, or a variant thereof; and one or two or more CDRs of the VL amino acid sequence as set forth in SEQ ID NO: 6, or amino acid sequences as set forth in SEQ ID NOs: 10, 11 and 12, or a variant thereof, wherein the variant thereof includes a humanized antibody or any other variant described in the present application. In some embodiments, the anti-IL-2 antibody comprises HCDR1 (as set forth in SEQ ID NO: 7), HCDR2 (as set forth in SEQ ID NO: 8) and HCDR3 (as set forth in SEQ ID NO: 9) of the VH amino acid sequence as set forth in SEQ ID NO: 5, and LCDR1 (as set forth in SEQ ID NO: 10), LCDR2 (as set forth in SEQ ID NO: 11) and LCDR3 (as set forth in SEQ ID NO: 12) of the VL amino acid sequence as set forth in SEQ ID NO: 6. In some embodiments, the anti-IL-2 antibody comprises HCDR1 as set forth in SEQ ID NO: 7, HCDR2 as set forth in SEQ ID NO: 8, and HCDR3 as set forth in SEQ ID NO: 9, and LCDR1 as set forth in SEQ ID NO: 10, LCDR2 as set forth in SEQ ID NO: 11, and LCDR3 as set forth in SEQ ID NO: 12.

In another aspect, the VH and/or VL of the anti-IL-2 antibodies of the present application can be used as starting materials for engineering to produce the antibody described herein for use in humans. The antibody can be engineered by mutating one or two or more amino acid residues within one or both of the variable regions (i.e., VH and/or VL), e.g., mutating one or two or more amino acid residues within one or more CDR regions and/or one or two or more framework regions.

In some embodiments, the variable regions of the anti-IL-2 antibody of the present application are engineered by CDR grafting. Antibodies interact with target antigens primarily through the amino acid residues of the six CDRs of the heavy and light chains. Therefore, amino acid sequences within the CDR regions among individual antibodies are more diverse than sequences outside the CDR regions such as FRs. Because the amino acid sequences of the CDR regions are responsible for most interactions of antibodies with antigens, recombinant antibodies can be expressed to mimic the properties of a particular naturally occurring antibody by constructing expression vectors comprising the CDR sequences from the particular naturally occurring antibody grafting onto the FR sequences of another antibody with different properties (e.g., Riechmann et al., Nature 1998, 332:323-327; Jones et al., Nature 1986, 321:522-525; Queen et al., Proc Natl Acad Sci USA 1989, 86:10029-10033; US Patent Nos.: US5225539, US5530101, US5585089, US5693762, and US6180370).

In some embodiments, the present application relates to an isolated monoclonal antibody or an antigen-binding fragment thereof, comprising a heavy chain variable region and/or a light chain variable region, wherein the heavy chain variable region comprises the amino acid sequences of the HCDR1, HCDR2 and HCDR3 regions described in the present application, and the light chain variable region comprises the amino acid sequences of the LCDR1, LCDR2 and LCDR3 regions described in the present application. Although the antibody comprises the VH and VL CDR region sequences of the monoclonal antibody of the present application, it may also comprise different framework region sequences.

Such framework region sequences can be obtained from public DNA databases or published references relating to germline antibody gene sequences. For example, the germline DNA sequences of human heavy and light chain variable region genes can be found in the "VBase" human germline sequence database (available on the Internet at www.mrc-cpe.cam.ac.uk/vbase), and also in published references as follows: Kabat et al., 1991, Ibid; Tomlinson et al., J Mol Biol 1992, 227:776-798; and Cox et al., Eur J Immunol 1994, 24:827-836; and the content of each is explicitly incorporated herein by reference. As another example, the germline DNA sequences of human heavy and light chain variable region genes can be found in the IMGT database. For example, the following germline heavy chain sequences found in human immunoglobulins can be obtained by IMGT accession numbers: 4-59*02 (M29812), 1-69-2*01 (KF698734, IMGT000035 or Z12305). As another example, the following germline light chain sequences found in human immunoglobulins can be obtained by IMGT accession numbers: 3-20*01 (X12686, M15038, M15039, X56593 or X93639), 3-11*01 (X01668, L37726 or X92342), 1-39*01 (X59315 or X93627).

Amino acid sequences of antibodies can be aligned based on translated protein sequences using one of the sequence similarity search methods, e.g., Gapped BLAST, known to a person skilled in the art (Altschul et al., 1997, vide supra).

The amino acid sequence ID numbers of the heavy chain CDR regions, light chain CDR regions, heavy chain variable regions and light chain variable regions of the anti-IL-2 antibody of the present application are summarized in Table 2, wherein the heavy chain variable region CDRs and light chain variable region CDRs are defined by the Kabat numbering system. However, as is known in the art, the CDR regions can also be defined based on other numbering systems/methods for the heavy/light chain variable region such as Chothia and IMGT, AbM or Contact, and the CDR regions defined by different numbering systems/methods are slightly different, but a person skilled in the art can determine them according to conventional methods. The CDR regions defined by other numbering systems/methods and the CDR regions defined by Kabat adopted in the present application are all within the protection scope of the present application.

Table 2. Amino acid sequence ID numbers of CDRs of the heavy and light chain variable regions defined by the Kabat numbering system

| Description | Antibody ID | SEQ ID NO: | | | | | | | |
|---|---|---|---|---|---|---|---|---|---|
| | | HCDR1 | HCDR2 | HCDR3 | $V_H$ | LCDR1 | LCDR2 | LCDR3 | $V_L$ |
| Parental Antibody | S4B6 | 7 | 8 | 9 | 5 | 10 | 11 | 12 | 6 |
| Humanized Antibody | hS4B6-H1K1 | 7 | 8 | 9 | 45 | 10 | 11 | 12 | 46 |
| | hS4B6-H3K3 | 7 | 8 | 9 | 47 | 10 | 11 | 12 | 48 |
| | **hS4B6-H6K3** | 7 | 8 | 9 | 49 | 10 | 11 | 12 | 48 |
| | hS4B6-H6K7 | 7 | 8 | 9 | 49 | 10 | 11 | 12 | 50 |

(continued)

| Description | Antibody ID | SEQ ID NO: | | | | | | | |
|---|---|---|---|---|---|---|---|---|---|
| | | HCDR1 | HCDR2 | HCDR3 | $V_H$ | LCDR1 | LCDR2 | LCDR3 | $V_L$ |
| Optimally Humanized Antibody | hS4B6-H6K3-hu01 | 7 | 8 | 9 | 51 | 10 | 11 | 12 | 48 |
| | hS4B6-H6K3-hu02 | 7 | 8 | 9 | 52 | 10 | 11 | 12 | 48 |
| | hS4B6-H6K3-hu03 | 13 (X1=D, X2=L) | 8 | 9 | 53 | 10 | 11 | 12 | 48 |
| | hS4B6-H6K3-hu04 | 14 (XI=A, X2=M) | 8 | 9 | 54 | 10 | 11 | 12 | 48 |
| | hS4B6-H6K3-hu05 | 7 | 8 | 9 | 55 | 10 | 11 | 12 | 48 |
| | hS4B6-H6K3-hu06 | 7 | 16 (Z1=E, X3=S) | 9 | 56 | 10 | 11 | 12 | 48 |
| | hS4B6-H6K3-hu07 | 7 | 15 (Z1=E, X3=G) | 9 | 57 | 10 | 11 | 12 | 48 |
| | hS4B6-H6K3-hu08 | 7 | 8 | 9 | 58 | 10 | 11 | 12 | 48 |
| | hS4B6-H6K3-hu09 | 7 | 8 | 9 | 49 | 10 | 17 (Z7=S, Z8=T, X4=S) | 12 | 59 |
| | hS4B6-H6K3-hul0 | 7 | 8 | 9 | 49 | 10 | 18 (Z7=S, Z8=T, X4=T) | 12 | 60 |
| | hS4B6-H6K3-hul1 | 7 | 8 | 9 | 49 | 10 | 11 | 12 | 61 |
| | hS4B6-H6K3-hul2 | 7 | 8 | 9 | 49 | 10 | 11 | 12 | 62 |
| Optimally Humanized Antibody Lead | **hS4B6-H6K3(H)** | 14 (XI=A, X2=M) | 15 (Z1=E, X3=G) | 9 | 63 | 10 | 17 (Z7=S, Z8=T, X4=S) | 12 | 64 |
| | K1G1 | 14 | 15 | 9 | 63 | 26 (Z5=Y, Z6=S) | 28 (Z7=Q, Z8=T, X4=S) | 12 | 76 |

(continued)

| Description | Antibody ID | SEQ ID NO: | | | | | | | |
|---|---|---|---|---|---|---|---|---|---|
| | | HCDR1 | HCDR2 | HCDR3 | $V_H$ | LCDR1 | LCDR2 | LCDR3 | $V_L$ |
| | K1G2 | 14 | 15 | 20 (Z2=I, Z3=Y, Z4=S) | 65 | 26 (Z5=Y, Z6=S) | 28 (Z7=Q, Z8=T, X4=S) | 12 | 76 |
| | K1G3 | 14 | 19 (Z1=D, X3=G) | 9 | 66 | 26 | 28 | 12 | 76 |
| | K1G4 | 14 | 15 | 21 (Z2=V, Z3=L, Z4=S) | 67 | 26 | 28 | 12 | 76 |
| | K1G5 | 14 | 15 | 22 (Z2=V, Z3=Y, Z4=T) | 68 | 26 | 28 | 12 | 76 |
| | K1G6 | 14 | 19 (Z1=D, X3=G) | 20 (Z2=I, Z3=Y, Z4=S) | 69 | 26 | 28 | 12 | 76 |
| | K1G7 | 14 | 15 | 23 (Z2=I, Z3=Y, Z4=T) | 70 | 26 | 28 | 12 | 76 |
| | K1G8 | 14 | 15 | 24 (Z2=I, Z3=L, Z4=S) | 71 | 26 | 28 | 12 | 76 |
| | K1G9 | 14 | 15 | 25 (Z2=I, Z3=L, Z4=T) | 72 | 26 | 28 | 12 | 76 |
| | K1G10 | 14 | 19 (Z1=D, X3=G) | 23 (Z2=I, Z3=Y, Z4=T) | 73 | 26 | 28 | 12 | 76 |
| | K1G11 | 14 | 19 (Z1=D, X3=G) | 24 (Z2=I, Z3=L, Z4=S) | 74 | 26 | 28 | 12 | 76 |
| Affinity-matured hS4B6-H6K3(H) Antibody | K1G12 | 14 | 19 (Z1=D, X3=G) | 25 (Z2=I, Z3=L, Z4=T) | 75 | 26 | 28 | 12 | 76 |
| | K2G1 | 14 | 15 | 9 | 63 | 26 (Z5=Y, Z6=S) | 17 | 12 | 77 |

(continued)

| Description | Antibody ID | SEQ ID NO: | | | | | | | |
|---|---|---|---|---|---|---|---|---|---|
| | | HCDR1 | HCDR2 | HCDR3 | $V_H$ | LCDR1 | LCDR2 | LCDR3 | $V_L$ |
| | K2G2 | 14 | 15 | 20 (Z2=I, Z3=Y, Z4=S) | 65 | 26 | 17 | 12 | 77 |
| | K2G3 | 14 | 19 (Z1=D, X3=G) | 9 | 66 | 26 | 17 | 12 | 77 |
| | K2G4 | 14 | 15 | 21 (Z2=V, Z3=L, Z4=S) | 67 | 26 | 17 | 12 | 77 |
| | K2G5 | 14 | 15 | 22 (Z2=V, Z3=Y, Z4=T) | 68 | 26 | 17 | 12 | 77 |
| | K2G6 | 14 | 19 | 20 (Z2=I, Z3=Y, Z4=S) | 69 | 26 | 17 | 12 | 77 |
| | K2G7 | 14 | 15 | 23 (Z2=I, Z3=Y, Z4=T) | 70 | 26 | 17 | 12 | 77 |
| | K2G8 | 14 | 15 | 24 (Z2=I, Z3=L, Z4=S) | 71 | 26 | 17 | 12 | 77 |
| | K2G9 | 14 | 15 | 25 (Z2=I, Z3=L, Z4=T) | 72 | 26 | 17 | 12 | 77 |
| | K2G10 | 14 | 19 (Z1=D, X3=G) | 23 (Z2=I, Z3=Y, Z4=T) | 73 | 26 | 17 | 12 | 77 |
| | K2G11 | 14 | 19 (Z1=D, X3=G) | 24 (Z2=I, Z3=L, Z4=S) | 74 | 26 | 17 | 12 | 77 |
| | K2G12 | 14 | 19 (Z1=D, X3=G) | 25 (Z2=I, Z3=L, Z4=T) | 75 | 26 | 17 | 12 | 77 |
| | K3G1 | 14 | 15 | 9 | 63 | 27 (Z5=Y, Z6=G) | 28 (Z7=Q, Z8=T, X4=S) | 12 | 78 |

(continued)

| Description | Antibody ID | SEQ ID NO: | | | | | | | |
|---|---|---|---|---|---|---|---|---|---|
| | | HCDR1 | HCDR2 | HCDR3 | $V_H$ | LCDR1 | LCDR2 | LCDR3 | $V_L$ |
| | K3G2 | 14 | 15 | 20 (Z2=I, Z3=Y, Z4=S) | 65 | 27 | 28 | 12 | 78 |
| | K3G3 | 14 | 19 (Z1=D, X3=G) | 9 | 66 | 27 | 28 | 12 | 78 |
| | K3G4 | 14 | 15 | 21 (Z2=V, Z3=L, Z4=S) | 67 | 27 | 28 | 12 | 78 |
| | K3G5 | 14 | 15 | 22 (Z2=V, Z3=Y, Z4=T) | 68 | 27 | 28 | 12 | 78 |
| | K3G6 | 14 | 19 (Z1=D, X3=G) | 20 (Z2=I, Z3=Y, Z4=S) | 69 | 27 | 28 | 12 | 78 |
| | K3G7 | 14 | 15 | 23 (Z2=I, Z3=Y, Z4=T) | 70 | 27 | 28 | 12 | 78 |
| | K3G8 | 14 | 15 | 24 (Z2=I, Z3=L, Z4=S) | 71 | 27 | 28 | 12 | 78 |
| | K3G9 | 14 | 15 | 25 (Z2=I, Z3=L, Z4=T) | 72 | 27 | 28 | 12 | 78 |
| | K3G10 | 14 | 19 (Z1=D, X3=G) | 23 (Z2=I, Z3=Y, Z4=T) | 73 | 27 | 28 | 12 | 78 |
| | K3G11 | 14 | 19 (Z1=D, X3=G) | 24 (Z2=I, Z3=L, Z4=S) | 74 | 27 | 28 | 12 | 78 |
| | K3G12 | 14 | 19 (Z1=D, X3=G) | 25 (Z2=I, Z3=L, Z4=T) | 75 | 27 | 28 | 12 | 78 |
| | K4G1 | 14 | 15 | 9 | 63 | 27 | 17 | 12 | 79 |

(continued)

| Description | Antibody ID | SEQ ID NO: | | | | | | | |
|---|---|---|---|---|---|---|---|---|---|
| | | HCDR1 | HCDR2 | HCDR3 | $V_H$ | LCDR1 | LCDR2 | LCDR3 | $V_L$ |
| | K4G2 | 14 | 15 | 20 (Z2=I, Z3=Y, Z4=S) | 65 | 27 | 17 | 12 | 79 |
| | K4G3 | 14 | 19 (Z1=D, X3=G) | 9 | 66 | 27 | 17 | 12 | 79 |
| | K4G4 | 14 | 15 | 21 (Z2=V, Z3=L, Z4=S) | 67 | 27 | 17 | 12 | 79 |
| | K4G5 | 14 | 15 | 22 (Z2=V, Z3=Y, Z4=T) | 68 | 27 | 17 | 12 | 79 |
| | K4G6 | 14 | 19 | 20 (Z2=I, Z3=Y, Z4=S) | 69 | 27 | 17 | 12 | 79 |
| | K4G7 | 14 | 15 | 23 (Z2=I, Z3=Y, Z4=T) | 70 | 27 | 17 | 12 | 79 |
| | K4G8 | 14 | 15 | 24 (Z2=I, Z3=L, Z4=S) | 71 | 27 | 17 | 12 | 79 |
| | K4G9 | 14 | 15 | 25 (Z2=I, Z3=L, Z4=T) | 72 | 27 | 17 | 12 | 79 |
| | K4G10 | 14 | 19 (Z1=D, X3=G) | 23 (Z2=I, Z3=Y, Z4=T) | 73 | 27 | 17 | 12 | 79 |
| | K4G11 | 14 | 19 (Z1=D, X3=G) | 24 (Z2=I, Z3=L, Z4=S) | 74 | 27 | 17 | 12 | 79 |
| | K4G12 | 14 | 19 (Z1=D, X3=G) | 25 (Z2=I, Z3=L, Z4=T) | 75 | 27 | 17 | 12 | 79 |

The preferred framework sequence used in the anti-IL-2 antibody of the present application is the acceptor framework region that is structurally similar (or highly homologous) to the framework sequence of the parental antibody as described in the present application. In some embodiments, CDR1, CDR2, and CDR3 region sequences of the VH or VL can be grafted into the acceptor framework region respectively, wherein the acceptor framework region has an identical or the highest homologous sequence with immunoglobulin genes in its germline. In a specific embodiment, the present application selects and grafts the CDR regions of the VH as set forth in SEQ ID NO: 5 and the VL as set forth in SEQ ID NO: 6 into human-derived IgG FR regions, to give a humanized antibody, wherein the humanized antibody is capable of retaining an antigen-binding activity similar to that of the parental antibody comprising the VH as set forth in SEQ ID NO: 5 and the VL amino acid sequence as set forth in SEQ ID NO: 6.

Additionally, VH and VL sequences (or CDR sequences) of other anti-IL-2 antibodies that bind to hIL-2 can also be "mixed and matched" with the VH and VL sequences (or CDR sequences) of the anti-IL-2 antibody of the present application. Preferably, when mixing and matching VH and VL chains (or CDRs within these chains), the VH sequence from a particular VH/VL pair is replaced with a structurally similar VH sequence. Likewise, preferably, the VL sequence from a particular VH/VL pair is replaced with a structurally similar VL sequence.

Thus, in one embodiment, the anti-IL-2 antibody or antigen-binding fragment thereof of the present application comprises:

(a) a heavy chain variable region comprising the amino acid sequences listed in Table 2; and

(b) a light chain variable region comprising the amino acid sequences listed in Table 2, or the VL of another anti-IL-2 antibody, wherein the said another anti-IL-2 antibody specifically binds to the hIL-2 mutein.

In a specific embodiment, the humanized antibody of the present application comprises a VH amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 45, SEQ ID NO: 47 or SEQ ID NO: 49.

In a specific embodiment, the humanized antibody of the present application comprises a VL amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 46, SEQ ID NO: 48 or SEQ ID NO: 50.

In a specific embodiment, the humanized antibody of the present application comprises a VH amino acid sequence which is at least 85%, 86%, 87%, 88%, 89%, 90%, 91%, 92%, 93%, 94%, 95%, 96%, 97%, 98%, 99%, or 100% identical to a sequence as set forth in SEQ ID NO: 45, SEQ ID NO: 47 or SEQ ID NO: 49, and a VL amino acid sequence which is at least 85%, 86%, 87%, 88%, 89%, 90%, 91%, 92%, 93% , 94%, 95%, 96%, 97%, 98%, 99% or 100% identical to a sequence as set forth in SEQ ID NO: 46, SEQ ID NO: 48 or SEQ ID NO: 50.

In a more specific embodiment, the humanized antibody of the present application comprises a VH amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 45, and a VL amino acid sequence which is at least 85%, 86% , 87%, 88%, 89%, 90%, 91%, 92%, 93%, 94%, 95%, 96%, 97%, 98%, 99% or 100% identical to a sequence as set forth in SEQ ID NO: 46; or a VH amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 47, and a VL amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 48; or a VH amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 49, and a VL amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 48; or a VH amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 49, and a VL amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 50. Preferred humanized antibody comprises a VH amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 45, and a VL amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 46; or a VH amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 49, and a VL amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 48 or 50. More preferred humanized antibody comprises a VH amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 49, and a VL amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 48.

In some embodiments, CDR sequences can be grafted into a framework region that contains one or two or more mutations compared to the germline sequence. For example, it has been found that mutating amino acid residue(s) within a framework region can maintain or enhance the antigen-binding ability of an antibody (e.g., US Patent Nos.: US5530101, US5585089, US5693762, and US6180370). In some embodiments, grafting the CDRs of the parental antibody described in the present application into acceptor framework regions with one or two or more mutations compared to the germline sequence can increase humanization of the anti-IL-2 antibody of the present application. In a specific embodiment, one or two or more amino acids are mutated in the FR regions of the VH as set forth in SEQ ID NO: 49, and further one or two or more amino acid mutations can be made in FR1, FR2, or FR3, and one or two or more amino acid mutations can be further carried out in FR1, FR2 or FR3, respectively, to improve humanization of the anti-IL-2 antibody of the present application. Preferably, the aspartic acid residue at position 27 of FR1, the isoleucine residue at position 29 of FR1, the isoleucine residue at position 13 of FR2 (corresponding to position 48 of the VH amino acid sequence as set forth in SEQ ID NO: 49), and/or the asparagine residue at position 11 of FR3 (corresponding to position 76 of the VH amino acid sequence as set forth in SEQ ID NO: 49) are mutated. In a specific embodiment, one or two or more amino acids are mutated in the FR region of the VL as set forth in SEQ ID NO: 48, and further one or two or more amino acids can be selected to mutate in FR3, to improve humanization of the anti-IL-2 antibody of the present application. Preferably, the arginine residue at position 10 of FR3 (corresponding to position 66 of the VL amino acid sequence as set forth in SEQ ID NO: 48), and/or the tyrosine residue at position 15 of FR3 (corresponding to position 71 of the VL amino acid sequence as set forth in SEQ ID NO: 48) are mutated.

In some embodiments, amino acid mutation(s) can also be made in the VH and/or VL CDR regions of the humanized antibody to improve one or more properties of the antibody of interest (e.g., to increase the degree of humanization, to increase the expression, to reduce the risk of isomerization, to improve binding properties). Site-directed mutagenesis or PCR-induced mutagenesis can be performed, and the effect of mutation on binding capacity or other functional properties of an antibody can be assessed by *in vitro* or *in vivo* assays known in the art. Mutation can be amino acid substitution, addition or deletion, preferably amino acid substitution. Specifically, no more than 1, 2, 3, 4 or 5 amino acid residues within the CDR regions are mutated. In a specific embodiment, one or two or more amino acid mutations are made in the CDR regions of the VH as set forth in SEQ ID NO: 49, and further, one or more amino acid mutations can be made in HCDR1 and/or HCDR2; preferably, one or more amino acid mutations can be made at position 1 the alanine residue of HCDR1 (corresponding to position 31 of the VH as set forth in SEQ ID NO: 49), the leucine residue at position 4 (corresponding to position 34 of the VH as set forth in SEQ ID NO: 49) and/or the asparagine residue at position 17 of HCDR2 (corresponding to position 65 of the VH as set forth in SEQ ID NO:49).

In other embodiments, the present application also relates to engineering the antibody to reduce its isomerization risk, since isomerization can cause changes in antibody conformation and surface charge of the antibody, resulting in antibody charge heterogeneity. In preferred embodiments, the antibody of the present invention does not contain aspartate isomeric sites. Aspartate isomerization can occur on D-G sequence, resulting in isoaspartic acid residue, which reduces the stability of the polypeptide chain due to the introduction of a linkage into the polypeptide chain by this amino acid residue (also known as isoaspartate effect). In one embodiment, one or two or more amino acid mutations are made in the CDR regions of the VL as set forth in SEQ ID NO: 48, and further, one or two or more amino acids can be mutated in LCDR2; preferably, amino acid mutation can be carried out at position 7 the aspartic acid residue of LCDR2 (corresponding to position 56 of the VL as set forth in SEQ ID NO: 48) to reduce the risk of aspartate isomerization.

In some embodiments, combinatorial amino acid mutations in CDR and framework regions of the humanized antibody variable regions can also be carried out, to improve one or more properties of the antibody of interest (e.g., to improve degree of humanization, to improved binding properties, to reduce risk of isomerization, and to increase expression) to a greater extent. In a specific embodiment, two CDR regions and one FR region of the VH as set forth in SEQ ID NO: 49, and one CDR region and one FR region of the VL as set forth in SEQ ID NO: 48 of the above-mentioned humanized antibody are subjected to multiple amino acid mutations. Preferably, combinatorial amino acid mutations can be performed on the leucine residue at position 4 of HCDR1 (corresponding to position 34 of the VH as set forth in the SEQ ID NO: 49, for example, the mutated amino acid sequence can be the HCDR1 as set forth in SEQ ID NO: 14), the asparagine residue at position 17 of HCDR2 (corresponding to position 65 of the VH as set forth in SEQ ID NO: 49, for example, the mutated amino acid sequence can be the HCDR2 as set forth in SEQ ID NO: 15 or 16), and the asparagine residue at position 11 of FR3 (corresponding to position 76 of the VH as set forth in SEQ ID NO: 49, for example, the mutated amino acid sequence can be the VH as shown in SEQ ID NO: 58) in the VH as set forth in SEQ ID NO: 49, and the aspartic acid residue at position 7 of LCDR2 (corresponding to position 56 of the VL as set forth in SEQ ID NO: 48, for example, the mutated amino acid sequence can be the LCDR2 as set forth in SEQ ID NO: 17 or 18), the tyrosine residue at position 15 of FR3 (corresponding to position 71 of the VL as set forth in SEQ ID NO: 48, for example, the mutated amino acid sequence can be the VL as set forth in SEQ ID NO: 62) in the VL as set forth in SEQ ID NO: 48, which not only enables the humanized antibody of the present application to have a higher level of humanization, but also retains the binding affinity of the hIL-2 mutein of the present application ($K_D$ value of about $10^{-8}$ M), and reduces the risk of aspartate isomerization.

In other embodiments, the present application provides an isolated anti-IL-2 monoclonal antibody or antigen-binding fragment thereof, comprising a heavy chain variable region and a light chain variable region, wherein the heavy chain variable region comprises: (a) a VH CDR1 region, comprising the sequence of the present application or an amino acid sequence with 1, 2, 3, 4 or 5 amino acid mutations; (b) a VH CDR2 region, comprising the sequence of the present application or an amino acid sequence with 1, 2, 3, 4 or 5 amino acid mutations; (c) a VH CDR3 region, comprising the sequence of the present application or an amino acid sequence with 1, 2, 3, 4 or 5 amino acid mutations; (d) a VL CDR1 region, comprising the sequence of the present application or an amino acid sequence with 1, 2, 3, 4 or 5 amino acid mutations; (e) a VL CDR2 region, comprising the sequence of the present application or an amino acid sequence with 1, 2, 3, 4 or 5 amino acid mutations; and (f) a VL CDR3 region, comprising the sequence of the present application or an amino acid sequence with 1, 2, 3, 4 or 5 amino acid mutations.

In some specific embodiments, in order to further improve the binding affinity of the anti-IL-2 monoclonal antibody to hIL-2 mutein of the present application, for example, of $<5\times10^{-9}$ M, or $<1\times10^{-9}$ M, more preferably, $<6\times10^{-10}$ M, amino acid mutations are performed at specific sites on the heavy chain CDRs and light chain CDRs of the anti-IL-2 antibody. Since CDR3 (especially HCDR3) domain is independent of CDR1 domain and/or CDR2 domain, it is important on the binding specificity to antigen and has greater diversity relative to the other two CDR domains. In a specific embodiment, one, two and/or three amino acid mutations of HCDR3 comprising a sequence as set forth in SEQ ID NO: 9 are carried out in the present application, wherein the amino acid mutation(s) is/are located at the valine residue of position 1, the tyrosine residue of position 4, and/or the serine residue of position 6 of HCDR3 as set forth in SEQ ID NO: 9, and preferably, the amino acid mutations include V1R, V1I, V1T, Y4H, Y4L, Y4W, or S6A, S6R, S6N, S6D, S6Q, S6E, S6H, S6I, S6L, S6K, S6F, S6P, S6T, S6W, S6Y, or S6V. In a specific embodiment, one or two or more amino acid mutations (preferably one amino acid mutation) of HCDR2 comprising a sequence as set forth in SEQ ID NO: 15 are carried out in the present application, wherein the amino acid mutation is located at the glutamic acid residue of position 5 of HCDR2 as set forth in SEQ ID NO: 15, and further the preferred amino acid mutation is E5D. In a specific embodiment, one, two and/or three amino acid mutations on HCDR3 as set forth in SEQ ID NO: 9 are further combined with amino acid mutation on HCDR2 as set forth in SEQ ID NO: 15; and preferably, the amino acid mutations include the combination of one, two and/or three amino acid mutation(s) on HCDR3 as set forth in SEQ ID NO: 9 including V1I, Y4L and S6T with the amino acid mutation E5D on HCDR2 as set forth in SEQ ID NO: 15. In a specific embodiment, LCDR1 as set forth in SEQ ID NO: 10 and/or LCDR2 as set forth in SEQ ID NO: 17 are subjected to one or two or more amino acid mutations in the present application, wherein the amino acid mutations include those on the aspartic acid residue at position 5 or the glycine residue at position 7 of LCDR1 as set forth in SEQ ID NO: 10, and/or the serine residue at position 1 or the threonine residue at position 3 of LCDR2 as set forth in SEQ ID NO: 17, and further the amino acid mutations include D5N, D5H, D5I, D5L, D5F, D5Y and/or G7A, G7R, G7N, G7Q, G7I, G7L, G7S, G7T, G7D on LCDR1 as set forth in SEQ ID NO: 10, and/or S1R, S1Q, S1T, S1W, S1Y or T3G on LCDR2 as set forth in SEQ ID NO: 17, and preferred amino acid mutations include D5N, D5H, D5L, D5Y and/or G7I, G7L, G7S, G7T on LCDR1 as set forth in SEQ ID NO: 10, and/or S1Q or T3G on LCDR2 as set forth in SEQ ID NO: 17.

In a specific embodiment, any one or two or more of the heavy chain CDR region mutations described above, and/or any one or two or more of the light chain CDR region mutations can be selected. When selecting combination of any one of the heavy chain CDR region mutations and any one of the light chain CDR region mutations, it can retain the binding affinity of the humanized anti-IL-2 antibody comprising the VH amino acid sequence as set forth in SEQ ID NO: 63 and the VL amino acid sequence as set forth in SEQ ID NO: 64 of the present application to hIL-2 mutein of the present application. When selecting combination of two or more of the heavy chain CDR region mutations and/or any one or two or more of the light chain CDR region mutations, the binding affinity of the humanized anti-IL-2 antibody to the hIL-2 mutein can be significantly increased to $<10^{-9}$ M, and even $<6\times10^{-10}$ M.

In one embodiment, one of the light chain CDR region mutations can be selected or be combined with any one, two, three or four mutations in the heavy chain CDR regions, wherein the amino acid mutation in the light chain CDR regions includes D28Y (located at the aspartic acid of position 28 of the VL amino acid sequence as set forth in SEQ ID NO: 64, corresponding to position 5 of LCDR1 as set forth in SEQ ID NO: 10), and the amino acid mutations in the heavy chain CDR regions include V95I (located at the valine residue of position 1 of HCDR3 as set forth in SEQ ID NO: 9, corresponding to position 95 of the VH amino acid sequence as set forth in SEQ ID NO: 63), E53D (located at the glutamic acid residue of position 5 of HCDR2 as set forth in SEQ ID NO: 15, corresponding to position 53 of the VH amino acid sequence as set forth in SEQ ID NO: 63), S 100T (located at the serine residue of position 6 of HCDR3 as set forth in SEQ ID NO: 9, corresponding to position 100 of the VH amino acid sequence as set forth in SEQ ID NO: 63), and Y98L (located at the tyrosine residue of position 4 of HCDR3 as set forth in SEQ ID NO: 9, corresponding to position 98 of the VH amino acid sequence as set forth in SEQ ID NO: 63); further, the two amino acid mutations in the heavy chain CDR regions include V95I and any one selected from E53D, S 100T or Y98L, and the three amino acid mutations in the heavy chain CDR regions include V95I and any two selected from E53D, S 100T or Y98L, and the four amino acid mutations in the heavy chain CDR regions include V95I, E53D, S 100T and Y98L.

In another embodiment, two amino acid mutations in the light chain CDR regions are selected or be combined with any one, two, three or four amino acid mutations in the heavy chain CDR regions, wherein the amino acid mutations in the light chain CDR regions include D28Y and G30S (located at the glycine residue of position 30 of the VL amino acid sequence as set forth in SEQ ID NO: 64, corresponding to position 7 of the LCDR1 as set forth in SEQ ID NO: 10) or S50Q (located at the serine residue of position 50 of the VL amino acid sequence as set forth in SEQ ID NO: 64, corresponding to position 1 of the LCDR2 as set forth in SEQ ID NO: 17), and the one amino acid mutation in the heavy chain CDR regions includes V95I, E53D, Y98L or S 100T, the two amino acid mutations in the heavy chain CDR regions include V95I and any one selected from E53D, S 100T or Y98L, the three amino acid mutations in the heavy chain CDR regions include V95I and any two selected from E53D, S 100T or Y98L, and the four amino acid mutations in the heavy chain CDR regions include V95I, E53D, S 100T and Y98L.

In another embodiment, three mutations of the light chain CDR regions are selected or be combined with any one, two, three or four mutations in the heavy chain CDR regions, wherein the mutations of the light chain CDR regions include D28Y, G30S and S50Q, and the one amino acid mutation in the heavy chain CDR regions includes V95I, E53D, Y98L or S 100T, and the two mutations in the heavy chain CDR regions include V95I and any one selected from E53D, S 100T or Y98L, and the three mutations in the heavy chain CDR regions include V95I and any two selected from E53D, S 100T or Y98L, and the four amino acid mutations in the heavy chain CDR regions include V95I, E53D, S 100T and Y98L.

In another embodiment, the combinatorial amino acid mutations in the heavy chain CDR regions and amino acid mutations in the light chain CDR regions comprise a combination of four amino acid mutations in the heavy chain CDR regions and the amino acid mutation D28Y in the light chain CDR region; or a combination of three or four amino acid mutations in the heavy chain CDR regions and two amino acid mutations D28Y and S50Q or G30S in the light chain CDR regions, wherein the three amino acid mutations in the heavy chain CDR regions are V95I and any two selected from E53D, S100T or Y98L; or a combination of two or three or four amino acid mutations in the heavy chain CDR regions and three amino acid mutations D28Y, G30S and S50Q in the light chain CDR regions, wherein the two amino acid mutations in the heavy chain CDR regions are V95I and Y98L, and the three amino acid mutations in the heavy chain CDR regions are V95I and any two selected from E53D, S100T or Y98L. Specifically, the combinatorial amino acid mutations include amino acid mutations V95I, S100T, Y98L and E53D in the heavy chain CDR regions and amino acid mutation D28Y in the light chain CDR region, amino acid mutations V95I, E53D and Y98L in the heavy chain CDR regions and amino acid mutations D28Y and S50Q in the light chain CDR regions, amino acid mutations V95I and any two selected from E53D, S100T or Y98L in the heavy chain CDR regions and amino acid mutations D28Y and G30S in the light chain CDR regions, amino acid mutations V95I, S100T, Y98L and E53D in the heavy chain CDR regions and amino acid mutations D28Y and G30S in the light chain CDR regions, amino acid mutations V95I and Y98L in the heavy chain CDR regions and amino acid mutations D28Y, G30S and S50Q in the light chain CDR regions, amino acid mutations V95I and any two selected from E53D, S100T or Y98L in the heavy chain CDR regions and amino acid mutations D28Y, G30S and S50Q in the light chain CDR regions, or mutations V95I, S100T, Y98L and E53D in the heavy chain CDR regions and mutations D28Y, G30S and S50Q in the light chain CDR regions. The combinatorial mutations can significantly improve the binding affinity of the humanized anti-IL-2 antibody comprising the VH amino acid sequence as set forth in SEQ ID NO: 63 and the VL amino acid sequence as set forth in SEQ ID NO: 64 of the present application to the hIL-2 mutein of the present application to a maximum of about $5 \times 10^{-10}$ M, and reduce the dissociation rate constant ($k_{dis}$) of the said humanized antibody and the hIL-2 mutein to about $10^{-3}$ s$^{-1}$ or lower, even $<3 \times 10^{-4}$ s$^{-1}$, or reduce the dissociation equilibrium constant ($K_D$) of the said humanized antibody and the hIL-2 mutein by two orders of magnitude or more, even three orders of magnitude or more.

In one embodiment, the antibody of the present application comprises a heavy chain variable region comprising a CDR1 sequence, a CDR2 sequence and a CDR3 sequence and/or a light chain variable region comprising a CDR1 sequence, a CDR2 sequence and a CDR3 sequence, wherein:

(a) the CDR1 sequence in the heavy chain variable region comprises a sequence listed in Table 2 and/or conservative modification thereof; and/or

(b) the CDR2 sequence in the heavy chain variable region comprises a sequence listed in Table 2 and/or conservative modification thereof; and/or

(c) the CDR3 sequence in the heavy chain variable region comprises a sequence listed in Table 2 and/or conservative modification thereof; and/or

(d) the CDR1 sequence and/or CDR2 sequence and/or CDR3 sequence in the light chain variable region comprises a sequence listed in Table 2 and/or conservative modification thereof; and

(e) the antibody competitively binds to the hIL-2 mutein of the present application and blocks the hIL-2 mutein from binding of IL2Rα.

In some embodiments, the antibody or antigen-binding fragment thereof of the present application comprises: HCDR1 as set forth in SEQ ID NO: 7, SEQ ID NO: 13 or SEQ ID NO: 14; HCDR2 as set forth in SEQ ID NO: 8, SEQ ID NO: 15, SEQ ID NO: 16 or SEQ ID NO: 19; HCDR3 sequence as set forth in SEQ ID NO: 9, SEQ ID NO: 20, SEQ ID NO: 21, SEQ ID NO: 22, SEQ ID NO: 23, SEQ ID NO: 24, SEQ ID NO: 25, SEQ ID NO: 29 or SEQ ID NO: 34; and LCDR1 as set forth in SEQ ID NO: 10, SEQ ID NO: 26, SEQ ID NO: 27, SEQ ID NO: 30, SEQ ID NO: 31, SEQ ID NO: 35, SEQ ID NO: 36, SEQ ID NO: 37, SEQ ID NO: 39, SEQ ID NO: 40, SEQ ID NO: 41, SEQ ID NO: 42, SEQ ID NO: 43 or SEQ ID NO: 44; LCDR2 as set forth in SEQ ID NO: 11, SEQ ID NO: 17, SEQ ID NO: 18, SEQ ID NO: 28, SEQ ID NO: 32 or SEQ ID NO: 33; LCDR3 as set forth in SEQ ID NO: 12 or SEQ ID NO: 38.

In some specific embodiments, the antibody or antigen-binding fragment thereof of the present application comprises: HCDR1 as set forth in SEQ ID NO: 7 or 14, HCDR2 as set forth in SEQ ID NOs: 8, 15, 16 or 19, HCDR3 as set forth in SEQ ID NOs: 9, 20, 21, 22, 23, 24 or 25; and

LCDR1 as set forth in SEQ ID NOs: 10, 26 or 27, LCDR2 as set forth in SEQ ID NOs: 11, 17, 18 or 28, LCDR3 as set forth in SEQ ID NO: 12.

In some embodiments, the antibody or antigen-binding fragment thereof of the present application comprises: a heavy chain variable region VH having at least 85% (e.g., at least 88%, at least 90%, at least 95%, at least 96%, at least 97%, at least 98%, at least 99%) identity to a sequence as set forth in SEQ ID NOs: 5, 45, 47, 49, 51, 52, 53, 54, 55, 56, 57, 58, 63, 65, 66, 67, 68, 69, 70, 71, 72, 73, 74, 75, 86 or 112; and a light chain variable region VL having at least 85% (e.g., at least 88%, at least 90%, at least 95%, at least 96%, at least 97%, at least 98%, at least 99%) identity to a sequence as set forth in SEQ ID NOs: 6, 46, 48, 50, 59, 60, 61, 62, 64, 76, 77, 78, 79, 80, 81, 82, 83, 84, 85, 87, 88, 89, 90, 91, 92, 93, 104, 105, 106, 107, 108, 109, 110 or 111.

The humanized anti-IL-2 antibody of the present application also has one or more of the following functional characteristics: for example, the binding affinity to the hIL-2 mutein of the present application is optimized to about $5 \times 10^{-10}$ M, and the dissociation rate constant of the humanized antibody to the hIL-2 mutein is lowered to about $3 \times 10^{-4}$ s$^{-1}$, making their binding less likely to dissociate, which significantly reduces the probability of exposure of the IL2R $\alpha$ -binding site of hIL-2 due to their dissociation; the degree of humanization of the anti-IL-2 antibody of the present application is further improved, and the amino acid mutations in the hIL-2 mutein of the present application are hidden in the antigen-antibody binding interface, which reduces the risk of inducing ADA to a great extent.

### 3. hIL-2/Anti-IL-2 Antibody Fusion Protein of the Present Application

In one aspect, the present application also provides an hIL-2/anti-IL-2 antibody fusion protein. The said fusion protein comprises a purposely designed disulfide linkage introduced at the antigen-antibody binding interface of the hIL-2 or hIL-2 mutein and the anti-IL-2 antibody or antigen-binding fragment thereof of the present application, making the two components form an ultra-stable covalently-bound structure, and ensuring that the $\alpha$ subunit binding site of hIL-2 or the mutein thereof is permanently hidden inside of the constructed fusion protein without being exposed due to the dissociation of the antigen-antibody complex. In this way, the IL-2 component in the said fusion protein will never be competitively bound by the high-affinity IL2R when its binding affinity to IL-2 is higher than that of the antibody component in the fusion protein.

The hIL-2/anti-IL-2 antibody fusion protein of the present application comprises:

(1) the wild-type human interleukin-2 (hIL-2) as set forth in SEQ ID NO: 1 or an hIL-2 mutein of the present application, and

(2) an anti-IL-2 antibody or antigen-binding fragment thereof which competes with IL2R$\alpha$ for binding to hIL-2 or hIL-2 mutein of the present application, and

(3) disulfide linkage(s) located at the antigen-antibody binding interface of the hIL-2 or hIL-2 mutein and the anti-IL-2 antibody or antigen-binding fragment thereof of the present application.

The hIL-2/anti-IL-2 antibody fusion protein of the present application is unable to bind to the $\alpha$ subunit, or the $\alpha/\beta$ dimer, or the $\alpha/\beta/\gamma$ trimer of IL2R; however, it retains binding and functional activity to the dimeric receptor consisting of IL2R$\beta$ and yc (IL2R$\beta/\gamma$).

Thus, the IL-2/anti-IL-2 antibody fusion protein of the present application has the following functional characteristics:

(i) The hIL-2/anti-IL-2 antibody fusion protein of the present application does not bind to the high-affinity IL2R expressed on Tregs and thus does not induce tumor immune suppression mediated by Tregs;

(ii) The hIL-2/anti-IL-2 antibody fusion protein of the present application does not bind to the high-affinity IL2R expressed on normal tissues (e.g., vascular endothelium, pulmonary capillary endothelium, etc.) or type-2 innate lymphoid cells (ILC2), therefore, the toxicity and side effects (e.g., VLS, pulmonary edema, hypotension, etc.) as observed *in vivo* with the existing IL-2 products are significantly reduced;

(iii) The hIL-2/anti-IL-2 antibody fusion protein of the present application is structurally stable. Unlike the currently-available hIL-2/anti-hIL-2 antibody complex, the IL-2 component and the antibody component in the said fusion protein do not dissociate and therefore it can effectively avoid the possible exposure of the IL2R$\alpha$-binding site of hIL-2 and prevent it from being competitively bound by the high-affinity IL2R, and thus avoid potential toxicities mediated by the high-affinity IL2R and narrow therapeutic window led by the Treg cell activation;

(iv) The hIL-2/anti-IL-2 antibody fusion protein of the present application can specifically bind to and activate the IL-2 signaling pathway in effector T (Teff) cells (such as memory CD8$^+$ T cells) and/or NK cells to promote the expansion of Teff cells and/or NK cells as well as their antitumor activity. The said fusion protein is unable to activate the IL-2 signaling pathway in Tregs and promote the cellular activity. In addition, the said fusion protein has no adverse effect on the conventional CD4$^+$ T cells (Tconv);

(v) The hIL-2/anti-IL-2 antibody fusion protein of the present application has a low risk of immunogenicity. All mutations are embedded inside of the antigen-antibody binding interface, which significantly reduces the potential ADA risk;

(vi) The hIL-2/anti-IL-2 antibody fusion protein of the present application has a good stability, and it is easy to manufacture and has a high expression level which may lead to a lower production cost;

(vii) The hIL-2/anti-IL-2 antibody fusion protein of the present application has a prolonged serum half-life and can induce effective and lasting immune activation and antitumor activity *in vivo.* In addition, it does not activate or induce tumor immunosuppression *in vivo.*

There is/are disulfide linkage(s) between the hIL-2 or hIL-2 mutein and the anti-IL-2 antibody or antigen-binding fragment thereof of the present application. The said disulfide linkage is formed by introducing at least one cysteine mutation in the anti-IL-2 antibody or antigen-binding fragment thereof at its antigen-antibody binding interface, and at least one cysteine mutation in the hIL-2 or hIL-2 mutein at its antigen-antibody binding interface.

The anti-IL-2 antibody or antigen-binding fragment thereof of the present application is capable of competing with IL2R$\alpha$ for binding to hIL-2 or hIL-2 mutein and blocking the said hIL-2 or hIL-2 mutein from binding with IL2R$\alpha$ or any IL-2 receptor complex comprising the $\alpha$ subunit (i.e., IL2R$\alpha/\beta$ or IL2R$\alpha/\beta/\gamma$). In some embodiments, the said anti-IL-2 antibody is able to specifically bind to the IL2R$\alpha$-binding site of the hIL-2 or hIL-2 mutein. In some embodiments, the binding affinity ($K_D$) of the said anti-IL-2 antibody or antigen-binding fragment thereof to the hIL-2 mutein is $<5\times10^{-8}$ M, preferably, $<1\times10^{-8}$ M, $<5\times10^{-9}$ M, or $<1\times10^{-9}$ M, more preferably, $<6\times10^{-10}$ M. In some embodiments, the dissociation rate constant ($k_{dis}$) between the said anti-IL-2 antibody or antigen-binding fragment thereof and the hIL-2 mutein is $<5\times10^{-3}$ s$^{-1}$, $<1\times10^{-3}$ s$^{-1}$, $<5\times10^{-4}$ s$^{-1}$, or $<3\times10^{-4}$ s$^{-1}$.

The antigen-antibody binding interface of hIL-2 or hIL-2 mutein and the anti-IL-2 antibody or antigen-binding fragment thereof of the present application encompasses or overlaps with the binding interface of hIL-2 or hIL-2 mutein to IL2R$\alpha$. In some embodiments, the antigen-antibody binding interface of the said hIL-2 or hIL-2 mutein and the said anti-IL-2 antibody or antigen-binding fragment thereof is located in AB-loop, B-helix, C-helix, BC-loop and/or CD-loop of the hIL-2 or hIL-2 mutein, preferably in AB-loop, B-helix, C-helix and CD-loop of the hIL-2 or hIL-2 mutein; the IL2R$\alpha$-binding interface of the hIL-2 or hIL-2 mutein is located in the AB-loop and B-helix of the hIL-2 or hIL-2 mutein. In a specific embodiment, the binding interface of the said hIL-2 or hIL-2 mutein and the said anti-IL-2 antibody or antigen-binding fragment thereof is located at the amino acid position 30-45 or their adjacent residues, the amino acid position 57-77 or their adjacent residues, and/or the amino acid position 90-111 or their adjacent residues of the hIL-2 or hIL-2 mutein. In some embodiments, the binding interface of the said hIL-2 or hIL-2 mutein and the said anti-IL-2 antibody or antigen-binding fragment thereof is located in the variable region of the anti-IL-2 antibody or antigen-binding fragment thereof.

In some embodiments, the hIL-2 component of the hIL-2/anti-IL-2 antibody fusion protein of the present application comprises the hIL-2 mutein of the present application, which comprises one or more of the following amino acid mutations relative to the wild type hIL-2 as set forth in SEQ ID NO: 1: (a) amino acid mutation K64G at the position 64 corresponding to the wild-type hIL-2 as set forth in SEQ ID NO: 1; (2) amino acid mutation N90R at the position 90 corresponding to wild-type hIL-2 as set forth in SEQ ID NO: 1; (3) amino acid mutation M104E at the position 104 corresponding to the wild-type hIL-2 as set forth in SEQ ID NO: 1. Preferably, the amino acid mutations are K64G and/or N90R, or K64G, N90R and M104E; more preferably the amino acid mutations are K64G and N90R. In specific embodiments, the hIL-2 mutein comprises the amino acid sequence as set forth in SEQ ID NO: 2 or 3.

In some embodiments, the anti-IL-2 antibody or antigen-binding fragment thereof of the hIL-2/anti-IL-2 antibody fusion protein of the present application comprises the anti-IL-2 antibody or antigen-binding fragment thereof of the present application, or the other humanized anti-IL-2 antibody or antigen-binding fragment thereof capable of specifically recognizing the IL2Rα-binding site of hIL-2, and preferably the other humanized anti-IL-2 antibody is selected from NARA1 as described in PCT Published Patent Application WO2016005950 and TCB2 as described in PCT Published Patent Application WO2018217058.

In a specific embodiment, the anti-IL-2 antibody or antigen-binding fragment thereof of the hIL-2/anti-IL-2 antibody fusion protein of the present application comprises HCDR1 amino acid sequence as set forth in SEQ ID NO: 7, HCDR2 amino acid sequence as set forth in ID NO: 8, HCDR3 amino acid sequence as set forth in SEQ ID NO: 9, LCDR1 amino acid sequence as set forth in SEQ ID NO: 10, LCDR2 amino acid sequence as set forth in SEQ ID NO: 11, LCDR3 amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively.

In a specific embodiment, the anti-IL-2 antibody or antigen-binding fragment thereof of the hIL-2/anti-IL-2 antibody fusion protein of the present application comprises: (1) HCDR1 having an amino acid sequence of X1YYX2H (wherein X1=A or D; X2=L or M), (2) HCDR2 having an amino acid sequence of RIDPZ1DDSTKYAENFKX3 (wherein Z1=E or D; X3=N, S or G) as set forth in SEQ ID NO: 230, (3) HCDR3 having an amino acid sequence of Z2TFZ3YZ4RELRWFAY (wherein Z2=V, I, R or T; Z3=Y, L, H or W; Z4=S, T, A, R, N, D, Q, E, H, I, L, K, F, P, W, Y or V) as set forth in SEQ ID NO: 231, preferably Z2=V or I; Z3=Y or L; Z4=S or T, (4) LCDR1 having an amino acid sequence of QASQZ5IZ6NYLS (wherein Z5=D, Y, N, H, I, L or F; Z6=G, S, A, R, N, Q, I, L, T or D) as set forth in SEQ ID NO: 232, preferably, Z5=D or Y; Z6=G or S, (5) LCDR2 having an amino acid sequence of Z7AZ8SLAX4 (wherein Z7=S, Q, R, T, W or Y; Z8=T or G; X4=D, S or T) as set forth in SEQ ID NO: 233, preferably Z7=S or Q; Z8=T; X4=S, and (6) LCDR3 having an amino acid sequence of LQHYSTPYT as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively.

In some embodiments, the above-mentioned antibody or antigen-binding fragment thereof of the present invention comprises: HCDR1 as set forth in SEQ ID NO: 7, SEQ ID NO: 13 or SEQ ID NO: 14; HCDR2 as set forth in SEQ ID NO: 8, SEQ ID NO: 15, SEQ ID NO: 16 or SEQ ID NO: 19; HCDR3 as set forth in SEQ ID NO: 9, SEQ ID NO: 20, SEQ ID NO: 21, SEQ ID NO: 22, SEQ ID NO: 23, SEQ ID NO: 24, SEQ ID NO: 25, SEQ ID NO: 29 or SEQ ID NO: 34; and LCDR1 as set forth in SEQ ID NO: 10, SEQ ID NO: 26, SEQ ID NO: 27, SEQ ID NO: 30, SEQ ID NO: 31, SEQ ID NO: 35, SEQ ID NO: 36, SEQ ID NO: 37, SEQ ID NO: 39, SEQ ID NO: 40, SEQ ID NO: 41, SEQ ID NO: 42, SEQ ID NO: 43 or SEQ ID NO: 44; LCDR2 as set forth in SEQ ID NO: 11, SEQ ID NO: 17, SEQ ID NO: 18, SEQ ID NO: 28, SEQ ID NO: 32 or SEQ ID NO: 33; LCDR3 as set forth in SEQ ID NO: 12 or SEQ ID NO: 38.

In some embodiments, the above-mentioned antibody or antigen-binding fragment thereof of the present invention comprises: HCDR1 as set forth in SEQ ID NO: 7 or 14, HCDR2 as set forth in SEQ ID NO: 8, 15, 16 or 19, HCDR3 as set forth in SEQ ID NO: 9, 20, 21, 22, 23, 24, 25, 29 or 34; and LCDR1 as set forth in SEQ ID NO: 10, 26, 27, 30, 31, 35, 36 or 37, LCDR2 as set forth in SEQ ID NO: 11, 17, 18, 28, 32 or 33, LCDR3 as set forth in SEQ ID NO: 12 or 38.

In a specific embodiment, the anti-IL-2 antibody or antigen-binding fragment thereof of the hIL-2/anti-IL-2 antibody fusion protein of the present application comprises HCDR1, HCDR2, HCDR3, LCDR1, LCDR2, and LCDR3 as follows:

(1) HCDR1 having an amino acid sequence as set forth in SEQ ID NO: 13 (wherein X1=D; X2=L) or 14 (wherein X1=A; X2=M), HCDR2 having an amino acid sequence as set forth in SEQ ID NO: 8 (wherein Z1=E; X3=N), HCDR3 having an amino acid sequence as set forth in SEQ ID NO: 9 (wherein Z2=V; Z3=Y; Z4=S), LCDR1 having an amino acid sequence as set forth in SEQ ID NO: 10 (wherein Z5=D; Z6=G), LCDR2 having an amino acid sequence as set forth in SEQ ID NO: 11 (wherein Z7=S; Z8=T; X4=D), and LCDR3 having an amino acid sequence as set forth in ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively; or

(2) HCDR1 having an amino acid sequence as set forth in SEQ ID NO: 7 (wherein X1=A; X2=L), HCDR2 having an amino acid sequence as set forth in SEQ ID NO: 15 (wherein Z1=E; X3=G) or 16 (wherein Z1=E; X3=S), HCDR3 having an amino acid sequence as set forth in SEQ ID NO: 9 (wherein Z2=V; Z3=Y; Z4=S), LCDR1 having an amino acid sequence as set forth in SEQ ID NO: 10 (wherein Z5=D; Z6=G), LCDR2 having an amino acid sequence as set forth in SEQ ID NO: 11 (wherein Z7=S; Z8=T; X4=D), and LCDR3 having an amino acid sequence as set forth in SEQ ID NO: 12, or amino acid sequences which are at least 85%, 86%, 87%, 88%, 89%, 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively; or

(3) HCDR1 having an amino acid sequence as set forth in SEQ ID NO:7 (wherein X1=A; X2=L), HCDR2 having an amino acid sequence as set forth in SEQ ID NO: 8 (wherein Z1=E; X3=N), HCDR3 having an amino acid sequence as set forth in SEQ ID NO: 9 (wherein Z2=V; Z3=Y; Z4=S), LCDR1 amino acid sequence as set forth in SEQ ID NO: 10 (wherein Z5=D; Z6=G), LCDR2 having an amino acid sequence as set forth in SEQ ID NO: 17 (wherein, Z7=S; Z8=T; X4=S) or 18 (wherein Z7=S; Z8=T; X4=T), and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively; or

(4) HCDR1 having an amino acid sequence as set forth in SEQ ID NO: 14 (wherein X1=A; X2=M), HCDR2 having an amino acid sequence as set forth in SEQ ID NO: 15 (wherein Z 1=E; X3=G), HCDR3 having an amino acid sequence as set forth in SEQ ID NO: 9 (wherein Z2=V; Z3=Y; Z4=S), LCDR1 having an amino acid sequence as set forth in SEQ ID NO: 10 (wherein Z5=D; Z6=G), LCDR2 having an amino acid sequence as set forth in SEQ ID NO: 17 (wherein Z7=S; Z8=T; X4=S), and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively.

In one embodiment, the anti-IL-2 antibody or antigen-binding fragment thereof of the hIL-2/anti-IL-2 antibody fusion protein of the present application comprises HCDR1, HCDR2, HCDR3, LCDR1, LCDR2, and LCDR3 as follows: HCDR1 having an amino acid sequence as set forth in SEQ ID NO: 14 (wherein X1=A; X2=M), HCDR2 having an amino acid sequence as set forth in SEQ ID NO: 15 (wherein Z1=E; X3=G), HCDR3 having an amino acid sequence as set forth in SEQ ID NO: 9 (wherein Z2=V; Z3=Y; Z4=S), LCDR1 having an amino acid sequence as set forth in SEQ ID NO: 10 (wherein Z5=D; Z6=G), LCDR2 having an amino acid sequence as set forth in SEQ ID NO: 17 (wherein Z7=S; Z8=T; X4=S), and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively.

In a specific embodiment, the anti-IL-2 antibody or antigen-binding fragment thereof of the hIL-2/anti-IL-2 antibody fusion protein of the present application comprises HCDR1, HCDR2, HCDR3, LCDR1, LCDR2, and LCDR3 as follows: (1) HCDR1 having an amino acid sequence of X1YYX2H (wherein X1=A; X2=M; i.e., the sequence as set forth in SEQ ID NO: 14), (2) HCDR2 having an amino acid sequence of RIDPZ1DDSTKYAENFKX3 (Z1=E or D; X3=G; i.e., the sequence as set forth in SEQ ID NO: 15 or 19), (3) HCDR3 having an amino acid sequence of Z2TFZ3YZ4RELRWFAY (Z2=V, I, R or T; Z3=Y, L, H or W; Z4=S, T, A, R, N, D, Q, E, H, I, L, K, F, P, W, Y or V; SEQ ID NO: 231), preferably, Z2=V or I; Z3=Y or L; Z4=S or T, (4) LCDR1 having an amino acid sequence of QASQZSIZ6NYLS (Z5=D, Y, N, H, I, L or F; Z6=G, S, A, R, N, Q, I, L, T or D; SEQ ID NO: 232), preferably, Z5=D or Y; Z6=G or S, (5) LCDR2 having an amino acid sequence of Z7AZ8SLAX4 (Z7=S, Q, R, T, W or Y; Z8=T or G; X4=D, S or T; SEQ ID NO: 233), preferably Z7=S or Q; Z8=T; X4=S, and (6) LCDR3 having an amino acid sequence of LQHYSTPYT (as set forth in SEQ ID NO: 12), or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively.

In a specific embodiment, the anti-IL-2 antibody or antigen-binding fragment thereof of the hIL-2/anti-IL-2 antibody fusion protein of the present application comprises HCDR1, HCDR2, HCDR3, LCDR1, LCDR2, and LCDR3 as follows:

(1) HCDR1 having an amino acid sequence as set forth in SEQ ID NO: 14 (wherein X1=A; X2=M), HCDR2 having an amino acid sequence as set forth in SEQ ID NO: 15 (wherein Z 1=E; X3=G), HCDR3 having an amino acid sequence as set forth SEQ ID NO: 9 (wherein Z2=V; Z3=Y; Z4=S), LCDR1 having an amino acid sequence as set forth in SEQ ID NO: 26 (wherein Z5=Y; Z6=S) or 27 (wherein Z5=Y; Z6=G), LCDR2 having an amino acid sequence as set forth in SEQ ID NO: 17 (wherein Z7=S; Z8=T; X4=S) or 28 (wherein Z7=Q; Z8=T; X4=S), and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively; or

(2) HCDR1 having an amino acid sequence as set forth in SEQ ID NO: 14 (wherein X1=A; X2=M), HCDR2 having an amino acid sequence as set forth in SEQ ID NO: 15 (wherein Z 1=E; X3=G), HCDR3 having an amino acid sequence as set forth in SEQ ID NO: 20 (wherein Z2=I, Z3=Y, Z4=S), 21 (wherein Z2=V, Z3=L, Z4=S), 22 (wherein Z2=V, Z3=Y, Z4=T), 23 (wherein Z2=I, Z3=Y, Z4=T), 24 (wherein Z2=I, Z3=L, Z4=S), or 25 (wherein Z2=I, Z3=L, Z4=T), LCDR1 having an amino acid sequence as set forth in SEQ ID NO: 26 (wherein Z5=Y; Z6=S) or 27 (wherein Z5=Y; Z6=G), LCDR2 having an amino acid sequence as set forth in SEQ ID NO: 17 (wherein Z7=S, Z8=T; X4=S) or 28 (wherein Z7=Q; Z8=T; X4=S), and LCDR3 having an amino acid sequence as set forth in SEQ ID NO : 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively; or

(3) HCDR1 having an amino acid sequence as set forth in SEQ ID NO: 14 (wherein X1=A; X2=M), HCDR2 having an amino acid sequence as set forth in SEQ ID NO: 19 (wherein Z1=D; X3=G), HCDR3 having an amino acid sequence as set forth in SEQ ID NO: 9 (wherein Z2=V; Z3=Y; Z4=S), 20 (wherein Z2=I, Z3=Y, Z4=S), 23 (wherein Z2=I, Z3=Y, Z4=T), 24 (wherein Z2=I, Z3=L, Z4=S) or 25 (wherein Z2=I, Z3=L, Z4=T), LCDR1 having an amino acid sequence as set forth in SEQ ID NO:26 (wherein Z5=Y; Z6=S) or 27 (wherein Z5=Y; Z6=G), LCDR2 having an amino acid sequence as set forth in SEQ ID NO: 17 (wherein Z7=S, Z8=T, X4=S) or 28 (wherein Z7=Q; Z8=T; X4=S), and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively.

In one embodiment, the anti-IL-2 antibody or antigen-binding fragment thereof of the hIL-2/anti-IL-2 antibody fusion protein of the present application comprises HCDR1, HCDR2, HCDR3, LCDR1, LCDR2, and LCDR3 as follows:

(1) HCDR1 having an amino acid sequence as set forth in SEQ ID NO: 14 (wherein X1=A; X2=M), HCDR2 having an amino acid sequence as set forth in SEQ ID NO: 15 (wherein Z 1=E; X3=G), HCDR3 having an amino acid sequence as set forth in SEQ ID NO: 24 (wherein Z2=I, Z3=L, Z4=S) or 25 (wherein Z2=I, Z3=L, Z4=T), LCDR1 having an amino acid sequence as set forth in SEQ ID NO:26 (wherein Z5=Y; Z6=S) or 27 (wherein Z5=Y; Z6=G), LCDR2 having an amino acid sequence as set forth in SEQ ID NO: 17 (wherein Z7=S, Z8=T, X4=S) or 28 (wherein Z7=Q; Z8=T; X4=S), and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97% %, 98%, 99% or 100% identical to the HCDR1, HCDR2, HCDR3, LCDR1, LCDR2, and LCDR3, respectively; or

(2) HCDR1 having an amino acid sequence as set forth in SEQ ID NO: 14 (wherein X1=A; X2=M), HCDR2 having an amino acid sequence as set forth in SEQ ID NO: 19 (wherein Z1=D; X3=G), HCDR3 having an amino acid sequence as set forth in SEQ ID NO: 24 (wherein Z2=I, Z3=L, Z4=S) or 25 (wherein Z2=I, Z3=L, Z4=T), LCDR1 having an amino acid sequence as set forth in SEQ ID NO: 26 (wherein Z5=Y; Z6=S) or 27 (wherein Z5=Y; Z6=G), LCDR2 having an amino acid sequence as set forth in SEQ ID NO: 17 (wherein Z7=S, Z8=T, X4=S) or 28 (wherein Z7=Q; Z8=T; X4=S), and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively.

More preferably, the anti-IL-2 antibody or antigen-binding fragment thereof of the present invention comprises HCDR1, HCDR2, HCDR3, LCDR1, LCDR2, and LCDR3 as follows: HCDR1 having an amino acid sequence as set forth in SEQ ID NO:14, HCDR2 having an amino acid sequence as set forth in SEQ ID NO:19 (wherein Z1=D; X3=G), HCDR3 having an amino acid sequence as set forth in SEQ ID NO: 24 (wherein Z2=I, Z3=L, Z4=S), LCDR1 having an amino acid sequence as set forth in SEQ ID NO: 26 (wherein Z5=Y; Z6=S), LCDR2 having an amino acid sequence as set forth in SEQ ID NO: 17 (wherein Z7=S, Z8=T, X4=S), and LCDR3 having an amino acid as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively.

In some embodiments, the anti-IL-2 antibody or antigen-binding fragment thereof of the hIL-2/anti-IL-2 antibody fusion protein of the present application further comprises a heavy chain variable region having the HCDR1, HCDR2 and HCDR3 and a light chain variable region having the LCDR1, LCDR2 and LCDR3.

In some embodiments, the antibody or antigen-binding fragment thereof of the present application comprises: a heavy chain variable region VH having at least 80% (e.g., at least 85%, at least 90%, at least 95%, at least 96%, at least 97%, at least 98%, at least 99%) identity to the sequence as set forth in SEQ ID NOs: 5, 45, 47, 49, 51, 52, 53, 54, 55, 56, 57, 58, 63, 65, 66, 67, 68, 69, 70, 71, 72, 73, 74, 75, 86 or 112; and a light chain variable region VL having at least 80% (e.g., at least 85%, at least 90%, at least 95%, at least 96%, at least 97%, at least 98%, at least 99%) identity to the sequence as set forth in SEQ ID NOs: 6, 46, 48, 50, 59, 60, 61, 62, 64, 76, 77, 78, 79, 80, 81, 82, 83, 84, 85, 87, 88, 89, 90, 91, 92, 93, 104, 105, 106, 107, 108, 109, 110 or 111.

In a specific embodiment, the anti-IL-2 antibody or antigen-binding fragment thereof of the hIL-2/anti-IL-2 antibody fusion protein of the present application comprises a heavy chain variable region having the HCDR1, HCDR2 and HCDR3, and a light chain variable region having the LCDR1, LCDR2 and LCDR3, including amino acid sequences which are at least 85%, 86%, 87%, 88%, 89%, 90%, 91%, 92%, 93%, 94%, 95%, 96%, 97%, 98%, 99% or 100% identical to VH as set forth in SEQ ID NOs: 45, 47 or 49, and VL as set forth in at SEQ ID NOs: 46, 48 or 50 respectively, preferably, amino acid sequences which are at least 85%, 86%, 87%, 88%, 89%, 90%, 91%, 92%, 93%, 94%, 95%, 96%, 97%,

98%, 99% or 100% identical to VH as set forth in SEQ ID NO: 49 and VL as set forth in SEQ ID NO: 48 or 50, respectively.

In a specific embodiment, the anti-IL-2 antibody or antigen-binding fragment thereof of the hIL-2/anti-IL-2 antibody fusion protein of the present application comprises a heavy chain variable region having the HCDR1, HCDR2 and HCDR3, and a light chain variable region having the LCDR1, LCDR2 and LCDR3, comprising amino acid sequences which are at least 85%, 86%, 87%, 88%, 89%, 90%, 91%, 92%, 93%, 94%, 95%, 96%, 97%, 98%, 99% or 100% identical to VH as set forth in SEQ ID NOs: 54, 55, 56, 57 or 58, and VL as set forth in SEQ ID NO: 48 respectively; or comprising amino acid sequences which are at least 85%, 86%, 87%, 88%, 89%, 90%, 91%, 92%, 93%, 94%, 95%, 96%, 97%, 98%, 99% or 100% identical to VH as set forth in SEQ ID NO: 49 and VL as set forth in SEQ ID NO: 59, 60 or 62, respectively.

In one embodiment, the anti-IL-2 antibody or antigen-binding fragment thereof of the hIL-2/anti-IL-2 antibody fusion protein of the present application comprises a heavy chain variable having the HCDR1, HCDR2 and HCDR3 and a light chain variable region having the LCDR1, LCDR2 and LCDR3, comprising amino acid sequences which are at least 85%, 86%, 87%, 88%, 89%, 90%, 91%, 92%, 93%, 94%, 95%, 96%, 97%, 98%, 99% or 100% identical to VH as set forth in SEQ ID NO: 63 and VL as set forth in SEQ ID NO:64, respectively.

In a specific embodiment, the anti-IL-2 antibody or antigen-binding fragment thereof of the hIL-2/anti-IL-2 antibody fusion protein of the present application comprises a heavy chain variable region having the HCDR1, HCDR2 and HCDR3 and a light chain variable region having the LCDR1, LCDR2 and LCDR3, comprising amino acid sequences which are at least 85%, 86%, 87%, 88%, 89%, 90%, 91%, 92%, 93%, 94%, 95%, 96%, 97%, 98%, 99% or 100% identical to VH as set forth in any of SEQ ID NOs: 63, 65-75, and VL as set forth in any of SEQ ID NOs: 76-79, respectively.

In one embodiment, the anti-IL-2 antibody or antigen-binding fragment thereof of the hIL-2/anti-IL-2 antibody fusion protein of the present application comprises a heavy chain variable region having the HCDR1, HCDR2 and HCDR3 and a light chain variable region having the LCDR1, LCDR2 and LCDR3, comprising amino acid sequences which are at least 85%, 86%, 87%, 88%, 89%, 90%, 91%, 92%, 93%, 94%, 95%, 96%, 97%, 98%, 99% or 100% identical to VH as set forth in SEQ ID NO: 71 and VL as set forth in SEQ ID NO: 76, respectively.

In one embodiment, the anti-IL-2 antibody or antigen-binding fragment thereof of the hIL-2/anti-IL-2 antibody fusion protein of the present application comprises a heavy chain variable region having the HCDR1, HCDR2 and HCDR3 and a light chain variable region having the LCDR1, LCDR2 and LCDR3, comprising amino acid sequences which are at least 85%, 86%, 87%, 88%, 89%, 90%, 91%, 92%, 93%, 94%, 95%, 96%, 97%, 98%, 99% or 100% identical to VH as set forth in SEQ ID NO:72 and VL as set forth in SEQ ID NO: 77, respectively.

In one embodiment, the anti-IL-2 antibody or antigen-binding fragment thereof of the hIL-2/anti-IL-2 antibody fusion protein of the present application comprises a heavy chain variable region having the HCDR1, HCDR2 and HCDR3 and a light chain variable region having the LCDR1, LCDR2 and LCDR3, comprising amino acid sequences which are at least 85%, 86%, 87%, 88%, 89%, 90%, 91%, 92%, 93%, 94%, 95%, 96%, 97%, 98%, 99% or 100% identical to VH as set forth in SEQ ID NO: 72 or 74 and VL as set forth in SEQ ID NO: 76 or 79, respectively.

In one embodiment, the anti-IL-2 antibody or antigen-binding fragment thereof of the hIL-2/anti-IL-2 antibody fusion protein of the present application comprises a heavy chain variable region having the HCDR1, HCDR2 and HCDR3 and a light chain variable region having the LCDR1, LCDR2 and LCDR3, comprising amino acid sequences which are at least 85%, 86%, 87%, 88%, 89%, 90%, 91%, 92%, 93%, 94%, 95%, 96%, 97%, 98%, 99% or 100% identical to VH as set forth in SEQ ID NO: 74 or 75 and VL as set forth in SEQ ID NO: 77 or 78, respectively.

In some embodiments, the antigen-antibody binding interface between the hIL-2 mutein and the anti-IL-2 antibody or antigen-binding fragment thereof in the hIL-2/anti-IL-2 antibody fusion protein of the present application comprises disulfide linkage, wherein the antigen-antibody binding interface overlaps with the binding interface between the hIL-2 mutein and IL2Rα. In a specific embodiment, the antigen-antibody binding interface of the hIL-2 mutein and the anti-IL-2 antibody or antigen-binding fragment thereof is located in AB-loop, B-helix, C-helix and CD-loop of the said hIL-2 mutein, and preferably located at amino acid position 41-45 or their adjacent residues, amino acid position 57-68 or their adjacent residues, and amino acid position 90-107 or their adjacent residues of the said hIL-2 mutein.

In some embodiments, the antigen-antibody binding interface between the hIL-2 mutein and the anti-IL-2 antibody or antigen-binding fragment thereof in the hIL-2/anti-IL-2 antibody fusion protein of the present application comprises disulfide linkage, wherein the disulfide linkage is formed by at least one cysteine residue introduced in the anti-IL-2 antibody or antigen-binding fragment thereof at the antigen-antibody binding interface, and at least one cysteine residue introduced in the hIL-2 mutein at the antigen-antibody binding interface.

"Introducing" or "introduced" as used in the present application refers to the placement of mutation(s) to the corresponding native or natural amino acid residues in the parental polypeptide (e.g., the wild-type hIL-2 or mutein thereof, the anti-IL-2 antibody or antigen-binding fragment thereof of the present application), including substitutions, insertions or additions. For example, if an amino acid residue at a particular site in the parental polypeptide (e.g., the wild-type hIL-2 or mutein thereof, the anti-IL-2 antibody or antigen-binding fragment thereof of the present application) is referred to as the "native" or "natural" amino acid residue, the correspondingly introduced cysteine residue which is different from the native amino acid residue is placed at the same position in the parental polypeptide. In some embodiments, an introduced cysteine residue replaces the native residue at the same position. In some embodiments, the introduced cysteine residue is an insertion or addition of a cysteine residue or a polypeptide comprising a cysteine residue at the same position or the adjacent position.

In one embodiment, the antigen-antibody binding interface between the hIL-2 mutein and the anti-IL-2 antibody or antigen-binding fragment thereof in the hIL-2/anti-IL-2 antibody fusion protein comprises two or more introduced cysteine residues to form the said disulfide linkage. In a specific embodiment, the hIL-2 mutein has one or two or more amino acid mutations at the amino acid positions 42, 60, 61, 64, 65, 102, and 104 relative to the wild-type hIL-2 as set forth in SEQ ID NO: 1 to introduce a first cysteine residue. In one embodiment, the anti-IL-2 antibody is selected from the humanized anti-IL-2 antibody or antigen-binding fragment thereof of the present application, which comprises: (1) HCDR1 having an amino acid sequence of X1YYX2H (wherein X1=A; X2=M; i.e., the sequence as set forth in SEQ ID NO: 14), (2) HCDR2 having an amino acid sequence of RIDPZ1DDSTKYAENFKX3 (wherein Z1=E or D; X3=G; i.e., the sequence as set forth in SEQ ID NO: 15 or 19), (3) HCDR3 having an amino acid sequence of Z2TFZ3YZ4RELRWFAY (wherein Z2=V or I; Z3=Y or L; Z4=S or T; e.g., including the sequence as set forth in SEQ ID NO: 9, 20, 21, 22, 23, 24 or 25), (4) LCDR1 having an amino acid sequence of QASQZSIZ6NYLS (wherein Z5=D or Y; Z6=G or S; e.g., including the sequence as set forth in SEQ ID NO: 10, 26 or 27), (5) LCDR2 having an amino acid sequence of Z7AZ8SLAX4 (wherein Z7=S or Q; Z8=T; X4=S; i.e., the sequence as set forth in SEQ ID NO: 17 or 28), and (6) LCDR3 having an amino acid sequence of LQHYSTPYT as set forth in SEQ ID NO: 12, and the said antibody or antigen-binding fragment thereof has one or two or more amino acid mutations at position 3 of HCDR3, or at position 7, 8, 9 of LCDR1, or at position 1, 3 of LCDR2, or at position 4 of LCDR3, to introduce a second cysteine residue. In a specific embodiment, the anti-IL-2 antibody is selected from the humanized anti-IL-2 antibody or antigen-binding fragment thereof of the present application comprising a heavy chain variable region having the HCDR1, HCDR2 and HCDR3 and/or a light chain variable region having the LCDR1, LCDR2 and LCDR3, comprising amino acid sequences which are at least 85%, 86%, 87%, 88%, 89%, 90%, 91%, 92%, 93%, 94%, 95%, 96%, 97%, 98%, 99%, or 100% identical to VH as set forth in SEQ ID NO: 63 and/or VL as set forth in SEQ ID NO: 64, respectively; or comprising amino acid sequences which are at least 85%, 86%, 87%, 88%, 89%, 90%, 91%, 92%, 93%, 94%, 95%, 96%, 97%, 98%, 99%, or 100% identical to VH as set forth in any of ID NOs: 63, 65-75 and/or VL as set forth in any of SEQ ID NOs: 76-79, respectively, and the antibody or antigen-binding fragment thereof has one or two or more amino acid mutations in its light chain variable framework region to introduce a second cysteine residue, wherein the amino acid mutation is located at position 67 or 68 of the light chain variable region.

In order to introduce the cysteine mutation at specific sites in the hIL-2 mutein and/or the anti-IL-2 antibody or antigen-binding fragment thereof, the coding sequence of the hIL-2 mutein and/or the anti-IL-2 antibody or antigen-binding fragment thereof can be manipulated, e.g., substituting codons encoding natural residues with those encoding cysteine residues, or inserting or adding codons encoding cysteine residues at positions encoding natural residues or their adjacent positions.

In a specific embodiment, the first cysteine residue is introduced into the hIL-2 mutein at the antigen-antibody binding interface of the hIL-2/anti-IL-2 antibody fusion protein of the present application by amino acid mutation, and the second cysteine residue is introduced into the humanized anti-IL-2 antibody or antigen-binding fragment thereof at the antigen-antibody binding interface by amino acid mutation. Preferably, the amino acid mutation is amino acid substitution, insertion or addition; for example, a non-cysteine residue may be substituted with a cysteine residue, or a cysteine residue or a polypeptide fragment containing a cysteine residue may be inserted or added to the same or adjacent position of the non-cysteine residue, wherein the positions for substitution, insertion or addition of cysteine residue are those where the disulfide linkage can be formed between the said hIL-2 mutein and the said humanized anti-IL-2 antibody or antigen binding fragment thereof at the antigen-antibody binding interface of the hIL-2/anti-IL-2 antibody fusion protein of the present application, after the non-cysteine residue is replaced with a cysteine residue or inserted or added with a cysteine residue or a polypeptide fragment containing a cysteine residue. To this end, a number of factors may be considered, including that, for example, the cysteine residues forming disulfide linkage are sufficiently close to each other in space, and/or the substitution or insertion or addition will not disrupt the tertiary structure of the hIL-2 mutein, or the anti-IL-2 antibody or antigen-binding fragment thereof, or the hIL-2/anti-IL-2 antibody fusion protein per se, and the formed disulfide linkage should be located at the antigen-antibody binding interface.

The person skilled in the art can use suitable methods well known in the art, including but not limited to distance mapping by photoelectric detection, computer modeling, NMR spectroscopy, or X-ray crystal diffraction, to determine the distance and angle between two amino acid residues to be mutated. In one embodiment, the protein crystal structure of the related protein (e.g., the antigen-antibody complex formed by IL-2 and anti-IL-2 antibody) can be obtained from a public database (such as the PDB database), or elucidated using methods such as X ray crystal diffraction; a computer software can also be used to determine distances and angles between amino acid residues based on protein crystal structure data. Further, it is necessary to ensure that the site for introducing a first cysteine residue in the hIL-2 mutein and the site for introducing a second cysteine residue in the humanized anti-IL-2 antibody or antigen-binding fragment can be paired correctly so that a stable disulfide linkage can be formed at the antigen-antibody binding interface between the said hIL-2 mutein and the said humanized anti-IL-2 antibody or antigen-binding fragment thereof, and thus reduces the risk of dissociation *in vivo* as well as cysteine-mismatch event in the manufacturing process. In one embodiment, the sites able to pair correctly are located at the antigen-antibody binding interface between the hIL-2 mutein and the humanized anti-IL-2 antibody or antigen-binding fragment thereof, and the paired amino acid residues are sufficiently close to each other in space, e.g., the spatial distance between the paired amino acid residues is less than 10 angstroms, and preferably less than 5 angstroms.

From a public database containing the crystal structure of antigen-antibody complex formed by IL-2 and anti-IL-2 antibody, the crystal structure of mIL-2/S4B6 antigen-antibody complex (as set forth in PDB ID: 4YUE) and the distance between amino acid residues in mIL-2 and S4B6 antibody can be obtained. These data are used to select suitable sites for the introduction of the first and second cysteine residues in the hIL-2 mutein and the humanized anti-IL-2 antibody or antigen binding fragment thereof to make them pair correctly. After the pairing sites for introducing cysteine mutations or the pairing amino acid residues to be mutated are confirmed, the person skilled in the art can readily mutate the corresponding codons into cysteine codon using methods well known in the art (e.g., PCR mutagenesis).

The formation of disulfide linkage can be verified using suitable methods well known in the art, for example, being determined by mass spectrometry.

The said disulfide linkage can stabilize the hIL-2/anti-IL-2 antibody fusion protein, which is reflected in a number of aspects. For example, the said disulfide linkage can enable the said hIL-2/antiIL-2 antibody fusion protein to be expressed mainly in a monomeric form, and/or can increase the protein thermal stability and serum stability, and/or turns the construct into a stable fusion protein with a unique biological activity (e.g., able to more effectively prevent the hIL-2 mutein from being bound by the $\alpha$ subunit of IL2R, or the $\alpha/\beta$ heterodimer, or the $\alpha/\beta/\gamma$ heterotrimer, but retain the binding and functional activity to IL2R$\beta/\gamma$), and/or facilitates the expression and folding of the fusion protein into one having specific biological activity and stability.

In a specific embodiment, a pair of the first cysteine residue and the second cysteine residue for forming the disulfide linkage at the antigen-antibody binding interface of the hIL-2 mutein and the humanized anti-IL-2 antibody or antigen-binding fragment thereof in the said hIL-2/anti-IL-2 antibody fusion protein is selected from one, or two, or more of the following paired amino acid mutations:

(1) the first cysteine residue is introduced by a mutation of F42C at the position 42, or a mutation of K64C at the position 64, or a mutation of P65C at the position 65 of the hIL-2 mutein corresponding to the wild-type hIL-2 as set forth in SEQ ID NO: 1; and the second cysteine residue is introduced by a mutation of F3C in HCDR3 having the amino acid sequence Z2TFZ3YZ4RELRWFAY (wherein Z2=V or I; Z3=Y or L; Z4=S or T; e.g., including the sequence as set forth in SEQ ID NOs: 9, 20, 21, 22, 23, 24 or 25) in the humanized anti-IL-2 antibody or antigen-binding fragment thereof; and/or

(2) the first cysteine residue is introduced by a mutation of E60C at the position 60 of the hIL-2 mutein corresponding to the wild-type hIL-2 as set forth in SEQ ID NO: 1, and the second cysteine residue is introduced by a mutation of G7C or S7C in LCDR1 having the amino acid sequence QASQZ5IZ6NYLS (wherein Z5=D or Y; Z6=G or S; e.g., including the sequence as set forth in SEQ ID NOs: 10, 26 or 27) in the humanized anti-IL-2 antibody or antigen-binding fragment thereof; and/or

(3) the first cysteine residue is introduced by a mutation of E61C at the position 61 of the hIL-2 mutein corresponding to the wild-type hIL-2 as set forth in SEQ ID NO: 1, and the second cysteine residue is introduced by a mutation of G7C or S7C in LCDR1 having the amino acid sequence of QASQZ5IZ6NYLS (wherein Z5=D or Y; Z6=G or S; e.g., including the sequence as set forth in SEQ ID NOs: 10, 26 or 27) in the humanized anti-IL-2 antibody or antigen-binding fragment thereof, or

the second cysteine residue is introduced by a mutation of N8C in LCDR1 having the amino acid sequence of QASQZ5IZ6NYLS (wherein Z5=D or Y; Z6=G or S; e.g., including the sequence as set forth in SEQ ID NOs: 10, 26 or 27) in the humanized anti-IL-2 antibody or antigen-binding fragment thereof, or

the second cysteine residue is introduced by a mutation of S1C or Q1C in LCDR2 having the amino acid sequence Z7AZ8SLAX4 (wherein Z7=S or Q; Z8=T; X4=S; i.e., the sequence as set forth in SEQ ID NO: 17 or 28) in the humanized anti-IL-2 antibody or antigen-binding fragment thereof; and/or

(4) the first cysteine residue is introduced by a mutation of M104C at the position 104 of the hIL-2 mutein corresponding to the wild-type hIL-2 as set forth in SEQ ID NO: 1, and the second cysteine residue is introduced by a mutation of T3C in LCDR2 having the amino acid sequence of Z7AZ8SLAX4 (wherein Z7=S or Q; Z8=T; X4=S; i.e., the sequence as set forth in SEQ ID NO: 17 or 28) in the humanized anti-IL-2 antibody or antigen-binding fragment thereof; and/or

(5) the first cysteine residue is introduced by a mutation of T102C at the position 102 of the hIL-2 mutein corresponding to the wild-type hIL-2 as set forth in SEQ ID NO: 1, and the second cysteine residue is introduced by a mutation of S67C in the light chain variable region of the humanized anti-IL-2 antibody or antigen-binding fragment thereof, or the second cysteine residue is introduced by a mutation of G68C in the light chain variable region of the humanized anti-IL-2 antibody or antigen-binding fragment thereof; and/or

(6) the first cysteine residue is introduced by a mutation of K64C at the position 64 of the hIL-2 mutein corresponding to the wild-type hIL-2 as set forth in SEQ ID NO: 1, and

the second cysteine residue is introduced by a mutation of Y9C in LCDR1 having the amino acid sequence of QASQZ5IZ6NYLS (wherein Z5=D or Y; Z6=G or S; e.g., including the sequence as set forth in SEQ ID NOs: 10, 26 or 27) in the humanized anti-IL-2 antibody or antigen-binding fragment thereof, or

the second cysteine residue is introduced by a mutation of Y4C in LCDR3 having the amino acid sequence of LQHYSTPYT (SEQ ID NO: 12) in the humanized anti-IL-2 antibody or antigen-binding fragment thereof.

Preferably, a pair of the first cysteine residue and the second cysteine residue for forming disulfide linkage at the antigen-antibody binding interface of the anti-IL-2 humanized antibody or antigen-binding fragment thereof and the hIL-2 mutein in the said hIL-2/anti-IL-2 antibody fusion protein is selected from of one, or two, or more of the following paired amino acid mutations:

(1) the first cysteine residue is introduced by a mutation of M104C at the position 104 of the hIL-2 mutein corresponding to the wild-type hIL-2 as set forth in SEQ ID NO: 1, and the second cysteine residue is introduced by a mutation of T3C in LCDR2 in the humanized anti-IL-2 antibody or antigen-binding fragment thereof, wherein the humanized anti-IL-2 antibody or antigen-binding fragment thereof comprises HCDR1 having the amino acid sequence as set forth in SEQ ID NO: 14 (wherein X1=A; X2=M), HCDR2 having the amino acid sequence as set forth in SEQ ID NO: 19 (wherein Z 1=D; X3=G), HCDR3 having the amino acid sequence as set forth in SEQ ID NO: 24 (wherein Z2=I, Z3=L, Z4=S), LCDR1 having the amino acid sequence as set forth in SEQ ID NO: 26 (wherein Z5=Y; Z6=S), LCDR2 having the amino acid sequence as set forth in SEQ ID NO: 17 (wherein Z7=S; Z8=T; X4=S), and LCDR3 having the amino acid sequence as set forth in SEQ ID NO: 12; and/or

(2) the first cysteine residue is introduced by a mutation of T102C at the position 102 of the hIL-2 mutein corresponding to the wild-type hIL-2 as set forth in SEQ ID NO: 1, and the second cysteine residue is introduced by a mutation of S67C in the light chain variable region of the humanized anti-IL-2 antibody or antigen-binding fragment thereof, wherein the humanized anti-IL-2 antibody or binding fragment thereof comprises an amino acid sequence which is 85%, 86%, 87%, 88%, 89%, 90%, 91%, 92%, 93%, 94%, 95%, 96%, 97%, 98%, 99% or 100% identical to the VL as set forth in SEQ ID NO: 77, or
the second cysteine residue is introduced by a mutation of G68C in the light chain variable region of the humanized anti-IL-2 antibody or antigen-binding fragment thereof, wherein the humanized anti-IL-2 antibody or binding fragment thereof comprises an amino acid sequence which is 85%, 86%, 87%, 88%, 89%, 90%, 91%, 92%, 93%, 94%, 95%, 96%, 97%, 98%, 99% or 100% identical to the VL as set forth in SEQ ID NO: 77; and/or

(3) the first cysteine residue is introduced by a mutation of F42C at the position 42, or a mutation of K64C at the position 64, or a mutation of P65C at the position 65 of the hIL-2 mutein corresponding to the wild-type hIL-2 as set forth in SEQ ID NO: 1, and the second cysteine residue is introduced by a mutation of F3C in HCDR3 having the amino acid sequence of Z2TFZ3YZ4RELRWFAY (wherein Z2=V or I; Z3=Y or L; Z4=S or T; e.g., including the sequence as set forth in SEQ ID NO: 9, 20, 21, 22, 23, 24 or 25) in the humanized anti-IL-2 antibody or antigen-binding fragment thereof, wherein the humanized anti-IL-2 antibody or antigen-binding fragment thereof comprises HCDR1 having the amino acid sequence as set forth in SEQ ID NO: 14 (wherein X1=A; X2=M), HCDR2 having the amino acid sequence as set forth in SEQ ID NO: 15 (wherein Z 1=E; X3=G), HCDR3 having the amino acid sequence as set forth in SEQ ID NO: 9 (wherein Z2=V, Z3=Y, Z4=S), LCDR1 having the amino acid sequence as set forth in SEQ ID NO: 10 (wherein Z5=D; Z6=G), LCDR2 having the amino acid sequence as set forth in SEQ ID NO: 17 (wherein Z7=S; Z8=T; X4=S), and LCDR3 having the amino acid sequence as set forth in SEQ ID NO: 12; and/or more preferably, a pair of the first cysteine residue and the second cysteine residue for forming disulfide linkage at the antigen-antibody binding interface of the humanized anti-IL-2 antibody or antigen-binding fragment thereof and the hIL-2 mutein in the said hIL-2/anti-IL-2 antibody fusion protein is selected from one, or two, or more of the following paired amino acid mutations:

(1) the first cysteine residue is introduced by a mutation of M104C at the position 104 of the hIL-2 mutein corresponding to the wild-type hIL-2 as set forth in SEQ ID NO: 1, and the second cysteine residue is introduced by a mutation of T3C in LCDR2 having the amino acid sequence of Z7AZ8SLAX4 (wherein Z7=S or Q; Z8=T; X4=S; i.e., the sequence as set forth in SEQ ID NO: 17 or 28) in the humanized anti-IL-2 antibody or antigen-binding fragment thereof, wherein the humanized anti-IL-2 antibody or antigen-binding fragment thereof comprises HCDR1 having the amino acid sequence as set forth in SEQ ID NO: 14 (wherein X1=A; X2=M), HCDR2 having the amino acid sequence as set forth in SEQ ID NO: 19 (wherein Z 1=D; X3=G), HCDR3 having the amino acid sequence as set forth in SEQ ID NO: 24 (wherein Z2=I, Z3=L, Z4=S), LCDR1 having the amino acid sequence as set forth in SEQ ID NO: 26 (wherein Z5=Y; Z6=S), LCDR2 having the amino acid sequence as set forth in SEQ ID NO: 17 (wherein Z7=S; Z8=T; X4=S), and LCDR3 having the amino acid sequence as set forth in SEQ ID NO: 12; and/or

(2) the first cysteine residue is introduced by a mutation of T102C at the position 102 of the hIL-2 mutein corresponding to the wild-type hIL-2 as set forth in SEQ ID NO: 1, and the second cysteine residue is introduced by a mutation of S67C in the light chain variable region of the humanized anti-IL-2 antibody or antigen-binding fragment thereof, wherein the humanized anti-IL-2 antibody or antigen-binding fragment thereof comprises an amino acid sequence which is 85%, 86%, 87%, 88%, 89%, 90%, 91%, 92%, 93%, 94%, 95%, 96%, 97%, 98%, 99% or 100% identical to the VL as set forth in SEQ ID NO: 77, or
the second cysteine residue is introduced by a mutation of G68C in the light chain variable region of the humanized anti-IL-2 antibody or antigen-binding fragment thereof, wherein the humanized anti-IL-2 antibody or antigen-binding fragment thereof comprises an amino acid sequence which is 85%, 86%, 87%, 88%, 89%, 90%, 91%, 92%, 93%, 94%, 95%, 96%, 97%, 98%, 99% or 100% identical to the VL as set forth in SEQ ID NO: 77; and/or

(3) the first cysteine residue is introduced by a mutation of P65C at the position 65 of the hIL-2 mutein corresponding to in the wild-type hIL-2 as set forth in SEQ ID NO: 1, and the second cysteine residue is introduced by a mutation of F3C in HCDR3 having the amino acid sequence of Z2TFZ3YZ4RELRWFAY (wherein Z2=V or I; Z3=Y or L; Z4=S or T; e.g., including the sequence as set forth in SEQ ID Nos: 9, 20, 21, 22, 23, 24 or 25) in the humanized anti-IL-2 antibody or antigen-binding fragment thereof, wherein the humanized anti-IL-2 antibody or antigen-binding fragment thereof comprises HCDR1 having the amino acid sequence as set forth in SEQ ID NO: 14 (wherein X1=A; X2=M), HCDR2 having the amino acid sequence as set forth in SEQ ID NO: 15 (wherein Z1=E; X3=G), HCDR3 having the amino acid sequence as set forth in SEQ ID NO: 9 (wherein Z2=V, Z3=Y, Z4=S), LCDR1 having the amino acid sequence as set forth in SEQ ID NO: 10 (wherein Z5=D; Z6=G), LCDR2 having the amino acid sequence as set forth in SEQ ID NO: 17 (wherein Z7=S; Z8=T; X4=S), and LCDR3 having the amino acid sequence as set forth in SEQ ID NO: 12; and/or

(4) the first cysteine residue is introduced by a mutation of F42C at the position 42 of the hIL-2 mutein corresponding to the wild-type hIL-2 as set forth in SEQ ID NO: 1, and the second cysteine residue is introduced by a mutation of F3C in HCDR3 having the amino acid sequence of Z2TFZ3YZ4RELRWFAY (wherein Z2=V or I; Z3=Y or L; Z4=S or T; e.g., including the sequence as set forth in SEQ ID NOs: 9, 20, 21, 22, 23, 24 or 25) in the humanized anti-IL-2 antibody or antigen-binding fragment thereof, wherein the humanized anti-IL-2 antibody or antigen-binding fragment thereof comprises HCDR1 having the amino acid sequence as set forth in SEQ ID NO: 14 (wherein X1=A; X2=M), HCDR2 having the amino acid sequence as set forth in SEQ ID NO: 15 (wherein Z 1=E; X3=G), HCDR3 having the amino acid sequence as set forth in SEQ ID NO: 9 (wherein Z2=V, Z3=Y, Z4=S), LCDR1 having the amino acid sequence as set forth in SEQ ID NO: 10 (wherein Z5=D; Z6=G), LCDR2 having the amino acid sequence as set forth in SEQ ID NO: 17 (wherein Z7=S; Z8=T; X4=S), and LCDR3 having the amino acid sequence as set forth in SEQ ID NO: 12.

The above paired amino acid mutations can ensure the designed disulfide linkage to be formed at the antigen-antibody binding interface between the humanized anti-IL-2 antibody or antigen-binding fragment thereof and the hIL-2 mutein in the said hIL-2/anti-IL-2 antibody fusion protein, and consequently the $\alpha$ subunit binding site of the hIL-2 mutein is entirely embedded inside of the IL-2/anti-IL-2 antibody fusion protein (i.e., the antigen-antibody binding interface). When used as a therapy *in vivo,* the said fusion protein cannot be competitively bound by the high-affinity IL2R, since the antigen-antibody complex will never dissociate to expose the $\alpha$ subunit binding site of the hIL-2 mutein, in this way, it significantly reduces the risks of inducing systemic toxicity mediated by the high-affinity IL2R as well as the immunosuppression mediated by Tregs.

The said hIL-2/anti-IL-2 antibody fusion protein is different from the antigen-antibody complex formed by IL-2 and anti-IL- 2 antibody. In fact, the antigen-antibody complex formed by IL-2 and anti-IL-2 antibody can easily dissociate and its IL-2 component can be competitively bound by the high-affinity IL2R, as a result, it can induce the expansion of Tregs which express a high level of CD25 and promote the immunosuppressive response *in vitro* and *in vivo*.

In some embodiments, the hIL-2/anti-IL-2 antibody fusion protein of the present application comprises HCDR1, HCDR2, HCDR3 of the heavy chain variable region and LCDR1, LCDR2, LCDR3 of the light chain variable region, wherein the HCDR3, LCDR1, LCDR2 and/or LCDR3 comprise one or two or more cysteine mutations, and the HCDR1, HCDR2, HCDR3 of the heavy chain variable region and the LCDR1, LCDR2 and LCDR3 of the light chain variable region comprise: (1) HCDR1 having an amino acid sequence of X1YYX2H (wherein X1=A; X2=M; i.e., the sequence as set forth in SEQ ID NO: 14); (2) HCDR2 having an amino acid sequence of RIDPZ1DDSTKYAENFKX3 (Z1=E or D; X3=G; i.e., the sequence as set forth in SEQ ID NO: 15 or 19); (3) HCDR3 having an amino acid sequence of Z2TXnZ3YZ4RELRWFAY (wherein Z2=V or I; $X_{II}$=F or C; Z3=Y or L; Z4=S or T) as set forth in SEQ ID NO: 234; (4) LCDR1 having an amino acid sequence of QASQZ5IZ6X$_I$X$_{IV}$LS (wherein Z5=D or Y; Z6=G, S or C; $X_I$=N or C; $X_{IV}$=Y or C) as set forth in SEQ ID NO: 235; (5) LCDR2 having an amino acid sequence of Z7AZ8SLAX4 (wherein Z7=S, Q or C; Z8=T or C; X4=S) as set forth in SEQ ID NO: 236; and (6) LCDR3 having an amino acid sequence of LQHX$_{III}$STPYT (wherein X$_{III}$=Y or C) as set forth in SEQ ID NO: 237.

In a specific embodiment, the hIL-2/anti-IL-2 antibody fusion protein comprises HCDR1, HCDR2, HCDR3 of the heavy chain variable region and LCDR1, LCDR2, LCDR3 of the light chain variable region, wherein the HCDR3, LCDR1, LCDR2 and/or LCDR3 comprise one or two or more cysteine mutations, and HCDR1, HCDR2, HCDR3 of the heavy chain variable region and LCDR1, LCDR2, LCDR3 of the light chain variable region comprise: (1) HCDR1 having an amino acid sequence as set forth in SEQ ID NO: 14, HCDR2 having an amino acid sequence as set forth in SEQ ID NO: 15 (wherein Z1=E; X3=G), HCDR3 having an amino acid sequence as set forth in SEQ ID NO: 9 (wherein Z2=V; $X_{II}$=F; Z3=Y; Z4=S), LCDR1 having an amino acid sequence as set forth in SEQ ID NO: 30 (wherein Z5=D; Z6=C; $X_I$=N; $X_{IV}$=Y) or 31 (wherein Z5=D; Z6=G; $X_I$=C; $X_{IV}$=Y), LCDR2 having an amino acid sequence as set forth in SEQ ID NO: 17 (Z7=S, Z8=T, X4=S), and LCDR3 having an amino acid sequence as set forth in SEQ ID NO: 12 (wherein X$_{III}$=Y); and/or (2) HCDR1 having an amino acid sequence as set forth in SEQ ID NO: 14, HCDR2 having an amino acid sequence as set forth in SEQ ID NO: 15 (wherein Z1=E; X3=G), HCDR3 having an amino acid sequence as set forth in SEQ ID NO: 9 (wherein Z2=V; $X_{II}$=F; Z3=Y; Z4=S); LCDR1 having an amino acid sequence as set forth in SEQ ID NO: 10 (wherein Z5=D; Z6=G; $X_I$=N; $X_{IV}$=Y), LCDR2 having an amino acid sequence as set forth in SEQ ID NO: 17 (wherein Z7=S, Z8=T, X4=S), 32 (wherein Z7=C, Z8=T, X4=S) or 33 (wherein Z7=S, Z8=C, X4=S), and LCDR3 having an amino acid sequence as set forth in SEQ ID NO: 12 (wherein Xm=Y); and/or (3) HCDR1 having an amino acid sequence as set forth in SEQ ID NO: 14, HCDR2 having an amino acid sequence as set forth in SEQ ID NO: 15 (wherein Z1=E; X3=G), HCDR3 having an amino acid sequence as set forth in SEQ ID NO: 29 (wherein Z2=V; $X_{II}$=C; Z3=Y; Z4=S), LCDR1 having an amino acid sequence as set forth in SEQ ID NO: 10 (wherein Z5=D; Z6=G; $X_I$=N; $X_{IV}$=Y), LCDR2 having an amino acid sequence as set forth in SEQ ID NO: 17 (wherein Z7=S, Z8=T, X4=S), and LCDR3 having an amino acid sequence as set forth in SEQ ID NO: 12 (Xm=Y); and/or (4) HCDR1 having an amino acid sequence as set forth in SEQ ID NO: 14, HCDR2 having an amino acid sequence as set forth in SEQ ID NO: 19 (wherein Z 1=D; X3=G), HCDR3 having an amino acid sequence as set forth in SEQ ID NO: 24 (wherein Z2=I; $X_{II}$=F; Z3=L; Z4=S), LCDR1 having an amino acid sequence as set forth in SEQ ID NO: 35 (wherein Z5=Y; Z6=C; $X_I$=N; $X_{IV}$=Y) or 36 (wherein Z5=Y; Z6=S; $X_I$=C; $X_{IV}$=Y), LCDR2 having an amino acid sequence as set forth in SEQ ID NO: 17 (wherein Z7=S, Z8=T, X4=S), and LCDR3 having an amino acid sequence as set forth in SEQ ID NO: 12 (wherein X$_{III}$=Y); and/or (5) HCDR1 having an amino acid sequence as set forth in SEQ ID NO: 14, HCDR2 having an amino acid sequence as set forth in SEQ ID NO: 19 (wherein Z1=D; X3=G), HCDR3 having an amino acid sequence as set forth in SEQ ID NO: 24 (wherein Z2=I; $X_{II}$=F; Z3=L; Z4=S), LCDR1 having an amino acid sequence as set forth in SEQ ID NO: 26 (wherein Z5=Y; Z6=S; $X_I$=N; $X_{IV}$=Y), LCDR2 having an amino acid sequence as set forth in SEQ ID NO: 17 (wherein Z7=S, Z8=T, X4=S), 32 (wherein Z7=C, Z8=T, X4=S) or 33 (wherein Z7=S, Z8=C, X4=S), and LCDR3 having an amino acid sequence as set forth in SEQ ID NO: 12 (wherein X$_{III}$=Y); and/or (6) HCDR1 having an amino acid sequence as set forth in SEQ ID NO: 14, HCDR2 having an amino acid sequence as set forth in SEQ ID NO: 19 (wherein Z 1=D; X3=G), HCDR3 having an amino acid sequence as set forth in SEQ ID NO: 34 (wherein Z2=I; $X_{II}$=C; Z3=L; Z4=S), LCDR1 having an amino acid sequence as set forth in SEQ ID NO: 26 (wherein Z5=Y; Z6=S; $X_I$=N; $X_{IV}$=Y), LCDR2 having an amino acid sequence as set forth in SEQ ID NO: 17 (wherein Z7=S, Z8=T, X4=S), and LCDR3 having an amino acid sequence as set forth in SEQ ID NO: 12 (wherein Xm=Y); and/or (7) HCDR1 having an amino acid sequence as set forth in SEQ ID NO: 14, HCDR2 having an amino acid sequence as set forth in SEQ ID NO: 19 (wherein Z1=D; X3=G), HCDR3 having an the amino acid sequence as set forth in SEQ ID NO: 24 (wherein Z2=I; $X_{II}$=F; Z3=L; Z4=S), LCDR1 having an amino acid sequence as set forth in SEQ ID NO: 26 (wherein Z5=Y; Z6=S; $X_I$=N; $X_{IV}$=Y), LCDR2 having an amino acid sequence as set forth in SEQ ID NO: 17 (wherein Z7=S, Z8=T, X4=S), and LCDR3 having an amino acid sequence as set forth in SEQ ID NO: 38 (wherein X$_{III}$=C); and/or (8) HCDR1 having an amino acid sequence as set forth in SEQ ID NO: 14, HCDR2 having an amino acid sequence as set forth in SEQ ID NO: 19 (wherein Z1=D; X3=G), HCDR3 having an amino acid sequence as set forth in SEQ ID NO: 24 (wherein Z2=I; $X_{II}$=F; Z3=L; Z4=S), LCDR1 having an amino acid sequence as set forth in SEQ ID NO: 37 (wherein Z5=Y; Z6=S; $X_I$=N; $X_{IV}$=C), LCDR2 having an amino acid sequence as set forth in SEQ ID NO: 17 (wherein Z7=S, Z8=T, X4=S), and LCDR3 having an amino acid sequence as set forth in SEQ ID NO: 12 (wherein $X_{III}$=Y).

Preferably, the hIL-2/anti-IL-2 antibody fusion protein comprises HCDR1, HCDR2, HCDR3 of the heavy chain variable region and LCDR1, LCDR2, LCDR3 of the light chain variable region as follows:

(1) HCDR1 having an amino acid sequence as set forth in SEQ ID NO: 14, HCDR2 having an amino acid sequence as set forth in SEQ ID NO: 15 (wherein Z1=E; X3=G), HCDR3 having an amino acid sequence as set forth in SEQ ID NO: 29 (wherein Z2=V; $X_{II}$=C; Z3=Y; Z4=S), LCDR1 having an amino acid sequence as set forth in SEQ ID NO: 10 (wherein Z5=D; Z6=G; $X_I$=N; $X_{IV}$=Y), LCDR2 having an amino acid sequence as set forth in SEQ ID NO: 17 (wherein Z7=S, Z8=T, X4=S), and LCDR3 having an amino acid sequence as set forth SEQ ID NO: 12 (wherein $X_{III}$=Y); and/or

(2) HCDR1 having an amino acid sequence as set forth in SEQ ID NO: 14, HCDR2 having an amino acid sequence as set forth in SEQ ID NO: 19 (wherein Z1=D; X3=G), HCDR3 having an amino acid sequence as set forth in SEQ ID NO: 24 (wherein Z2=I; $X_{II}$=F; Z3=L; Z4=S), LCDR1 having an amino acid sequence as set forth in SEQ ID NO: 26 (wherein Z5=Y; Z6=S; $X_I$=N; $X_{IV}$=Y), LCDR2 having an amino acid as set forth in SEQ ID NO: 17 (wherein Z7=S, Z8=T, X4=S) or 33 (Z7=S, Z8=C, X4=S), and LCDR3 having an amino acid sequence as set forth in SEQ ID NO: 12 (wherein $X_{III}$=Y).

In a specific embodiment, the hIL-2/anti-IL-2 antibody fusion protein comprises a heavy chain variable region having the HCDR1, HCDR2 and HCDR3, and/or a light chain variable region having the LCDR1, LCDR2 and LCDR3, wherein the HCDR3, LCDR1, LCDR2 and/or LCDR3 comprise one or two or more cysteine mutations, and the heavy chain variable region comprises an amino acid sequence which is at least 85%, 86%, 87%, 88%, 89%, 90%, 91%, 92%, 93%, 94%, 95%, 96%, 97%, 98%, 99% or 100% identical to the sequence as set forth in SEQ ID NO: 63, 86, 74 or 112, respectively, and/or the light chain variable region comprises an amino acid sequence which is at least 85%, 86%, 87%, 88%, 89%, 90%, 91%, 92%, 93%, 94%, 95%, 96%, 97%, 98%, 99% or 100% identical to the sequence as set forth in SEQ ID NO: 64, 77, 80, 81, 82, 83, 84, 85, 104, 105, 106, 107, 108, 109, 110, or 111, respectively.

Preferably, the heavy chain variable region comprises an amino acid sequence which is at least 85%, 86%, 87%, 88%, 89%, 90%, 91%, 92%, 93%, 94%, 95%, 96%, 97%, 98%, 99% or 100% identical to the sequence as set forth in SEQ ID NO: 86 or 74, respectively, and/or the light chain variable regions comprises an amino acid sequence which is at least 85%, 86%, 87%, 88%, 89%, 90%, 91%, 92%, 93%, 94%, 95%, 96%, 97%, 98%, 99% or 100% identical to the sequence as set forth in SEQ ID NO: 64, 87, 88, 89, 90, 91, 107, 108 or 109, respectively.

More preferably, the heavy chain variable region and the light chain variable region comprise: amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 86, and the light chain variable region as set forth in SEQ ID NO: 64, 87, 88, 89, 90 or 91, respectively; or amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 74 and the light chain variable region as set forth in SEQ ID NO: 107, 108 or 109, respectively.

Disulfide linkage is introduced at the binding interface of the hIL-2 mutein of the present application and the anti-IL-2 humanized antibody or antigen-binding fragment thereof of the present application. The paired amino acid residues with a spatial distance of less than 10 angstroms (preferably less than 5 angstroms) at the antigen-antibody binding interface of the hIL-2 mutein and the humanized anti-IL-2 antibody or antigen-binding fragment thereof are selected and mutated to cysteine residues, consequently, the disulfide linkage formed between the hIL-2 mutein and the humanized anti-IL-2 antibody further stabilizes the molecular structure disclosed in the present application, making ensure that the IL2Rα-binding site of the hIL-2 mutein of the present application is permanently embedded inside of the hIL-2/anti-IL-2 antibody fusion protein (i.e., the antigen-antibody binding interface) and never dissociated or exposed when given *in vivo* as a therapy, and thus it cannot be competitively bound by the high-affinity IL2R. Based on these characteristics, when used *in vivo,* the hIL-2/anti-IL-2 antibody fusion protein of the present application can significantly promote the expansion of Teff cells and/or NK cells and activate the IL-2 signaling pathway in Teff cells and/or NK cells to promote their antitumor activity. Furthermore, since the hIL-2/anti-IL-2 antibody fusion protein of the present application does not bind with the α subunit, or α/β heterodimer, or α/β/γ heterotrimer of IL2R, it cannot bind to the high-affinity IL2R expressed on Tregs to activate Tregs and induce tumor immunosuppression, and it also cannot bind to cells or tissues that express a high level of CD25 or the high-affinity IL2R, therefore significantly reduces the toxicities and side effects (e.g., VLS, pulmonary edema, hypotension, etc.) associated with the existing IL-2 products. In addition, no adverse effects have been observed on the conventional CD4$^+$T cells (Tconv) after the administration of the hIL-2/anti-IL-2 antibody fusion protein of the present application.

In some embodiments, the present application also discloses a disulfide linkage introduced at the binding interface of antigen-antibody complex formed by the wild-type hIL-2 and the other humanized anti-IL-2 antibody which specifically recognizes the IL2Rα-binding site of hIL-2, wherein the antigen-antibody complex is selected from the hIl,-2:NARA1 antigen-antibody complex and the hIL-2:TCB2 antigen-antibody complex. Each amino acid pair in the antigen-antibody binding interface of the said complex with a spatial distance from each other of less than 10 angstroms (preferably less than 5 angstroms) is mutated to cysteine residue pair so as to form the disulfide linkage. Similarly, it can ensure the hIL2Rα-binding site of IL-2 to be more stably embedded inside of the antigen-antibody binding interface, and thus further reduces or eliminates the competitive binding of the antigen-antibody complex by IL2Rα or the high-affinity IL2R, while retains its binding activity to IL2Rβ/γ.

In some embodiments, the binding interface of the hIl,-2:NARA1 antigen-antibody complex encompasses the binding interface between hIL-2 and IL2Rα, wherein the binding interface of the hIl,-2:NARA1 antigen-antibody complex is located in AB loop, B helix and BC loop of hIL-2. In a specific embodiment, the binding interface of hIl,-2:NARA1 antigen-antibody complex is located at amino acid positions 30-43 or their adjacent residues in hIL-2, as well as amino acid positions 71-77 or their adjacent residues in hIL-2.

In one embodiment, the binding interface of hIl,-2:NARA1 antigen-antibody complex comprises two or more cysteine mutations so as to form the disulfide linkage. In a specific embodiment, the hIL-2 has one or two or more cysteine mutations at amino acid positions 34, 37, 41, 42, 68, 73, 75, 77, and 111 relative to the wild-type hIL-2 as set forth in SEQ ID NO: 1, to introduce the first cysteine residue. In a specific embodiment, the NARA1 antibody or antigen-binding fragment thereof has one or two or more cysteine mutations at specific amino acid positions in the CDR regions, including the position 1 of HCDR1, and/or the position 2, 6 and/or 8 of HCDR2, and/or the position 3 and/or 8 of HCDR3, and/or the position 9 of LCDR1, and/or the position 3, 4 and/or 6 of LCDR3, to introduce the second cysteine residue.

In one embodiment, a pair of the first cysteine residue and the second cysteine residue for forming the disulfide linkage at the binding interface of hIl,-2:NARA1 antigen-antibody complex is selected from one, or two, or more of the following paired amino acid mutations:

(1) the first cysteine residue is introduced by a mutation of P34C at the position 34 of the hIL-2, and the second cysteine residue is introduced by a mutation of A8C at the position 8 in HCDR3 of the NARA1 antibody or antigen-binding fragment thereof, or the second cysteine residue is introduced by a mutation of S3C or N4C at the position 3 or 4 in LCDR3 of the NARA1 antibody or antigen-binding fragment thereof; and/or

(2) the first cysteine residue is introduced by a mutation of T37C at the position 37 of the hIL-2, and the second cysteine residue is introduced by a mutation of D6C at the position 6 in LCDR3 of the NARA1 antibody or antigen-binding fragment thereof; and/or

(3) the first cysteine residue is introduced by a mutation of T41C at the position 41 of the hIL-2, and the second cysteine residue is introduced by a mutation of I2C at the position 2 in HCDR2 of the NARA1 antibody or antigen-binding fragment thereof; and/or

(4) the first cysteine residue is introduced by a mutation of F42C at the position 42 of the hIL-2, and the second cysteine residue is introduced by a mutation of G8C at the position 8 in HCDR2 of the NARA1 antibody or antigen-binding fragment thereof; and/or

(5) the first cysteine residue is introduced by a mutation of E68C at the position 68 of the hIL-2, and the second cysteine residue is introduced by a mutation of N1C at the position 1 in HCDR1 of the NARA1 antibody or antigen-binding fragment thereof; and/or

(6) the first cysteine residue is introduced by a mutation of A73C at the position 73 of the hIL-2, and the second cysteine residue is introduced by a mutation of G3C at the position 3 in HCDR3 of the NARA1 antibody or antigen-binding fragment thereof; and/or

(7) the first cysteine residue is introduced by a mutation of S75C or N77C at the position 75 or 77 of the hIL-2, and the second cysteine residue is introduced by a mutation of D9C at the position 9 in LCDR1 of the NARA1 antibody or antigen-binding fragment thereof; and/or

(8) the first cysteine residue is introduced by a mutation of T111C at the position 111 of the hIL-2, and the second cysteine residue is introduced by a mutation of S6C at the position 6 in HCDR2 of the NARA1 antibody or antigen-binding fragment thereof.

Preferably, a pair of the first cysteine residue and the second cysteine residue for forming the disulfide linkage at the binding interface of hII,-2:NARA1 antigen-antibody complex is selected from one, or two, or more of the following paired amino acid mutations:

(1) the first cysteine residue is introduced by a mutation of T37C at the position 37 of the hIL-2, and the second cysteine residue is introduced by a mutation of D6C at the position 6 in LCDR3 of the NARA1 antibody or antigen-binding fragment thereof; or

(2) the first cysteine residue is introduced by a mutation of E68C at the position 68 of the hIL-2, and the second cysteine residue is introduced by a mutation of N1C at the position 1 in HCDR1 of the NARA1 antibody or antigen-binding fragment thereof; and/or

(3) the first cysteine residue is introduced by a mutation of S75C or N77C at the position 75 or 77 of the hIL-2, and the second cysteine residue is introduced by a mutation of D9C at the position 9 in LCDR1 of the NARA1 antibody or antigen-binding fragment thereof; and/or

(4) the first cysteine residue is introduced by a mutation of T111C at the position 111 of the hIL-2, and the second cysteine residue is introduced by a mutation of S6C at the position 6 in HCDR2 of the NARA1 antibody or antigen-binding fragment thereof.

In some embodiments, the hIL-2/NARA1 antibody fusion protein comprises HCDR1, HCDR2, HCDR3 of the heavy chain variable region and LCDR1, LCDR2, LCDR3 of the light chain variable region, wherein the HCDR1, HCDR2, HCDR3, LCDR1, and/or LCDR3 comprise one or two or more cysteine substitution mutations, and the HCDR1, HCDR2, HCDR3 of the heavy chain variable region and LCDR1, LCDR2, LCDR3 of the light chain variable region comprise: (1) HCDR1 having an amino acid sequence as set forth in SEQ ID NO: 142 or 148, (2) HCDR2 having an amino acid sequence as set forth in SEQ ID NO: 143, 149, 150 or 151, (3) HCDR3 having an amino acid sequence as set forth in SEQ ID NO: 144, 152 or 153, (4) LCDR1 having an amino acid sequence as set forth in SEQ ID NO: 145 or 154, (5) LCDR2 having an amino acid sequence as set forth in SEQ ID NO: 146, and (6) LCDR3 having an amino acid sequence as set forth in SEQ ID NO: 147, 155, 156 or 157,
further, the HCDR1, HCDR2, HCDR3 of the heavy chain variable region and LCDR1, LCDR2, LCDR3 of the light chain variable region comprise:

(1) HCDR1 having an amino acid sequence as set forth in SEQ ID NO: 142, HCDR2 having an amino acid sequence as set forth in SEQ ID NO: 149, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147; and/or

(2) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NO: 152 or 153, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147; and/or

(3) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 154, LCDR2 having an amino acid sequence as set forth in SEQ ID NO: 146, and LCDR3 having an amino acid sequence as set forth in SEQ ID NO: 147; and/or

(4) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 155, 156 or 157; and/or

(5) HCDR1 having an amino acid sequence as set forth in SEQ ID NO: 148, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147; and/or

(6) HCDR1 having an amino acid sequence as set forth in SEQ ID NO: 142, HCDR2 having an amino acid sequence as set forth in SEQ ID NO: 150 or 151, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147.

Preferably, the HCDR1, HCDR2, HCDR3 of the heavy chain variable region and LCDR1, LCDR2, LCDR3 of the light chain variable region comprise:

(1) HCDR1 having an amino acid sequence as set forth in SEQ ID NO: 142, HCDR2 having an amino acid sequence as set forth in SEQ ID NO: 149, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147; and/or

(2) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 154, LCDR2 having an amino acid sequence as set forth in SEQ ID NO: 146, and LCDR3 having an amino acid sequence as set forth in SEQ ID NO: 147; and/or

(3) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 157; and/or

(4) HCDR1 having an amino acid sequence as set forth in SEQ ID NO: 148, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147.

In some embodiments, the hIL-2/NARA1 antibody fusion protein comprises a heavy chain variable region having the HCDR1, HCDR2 and HCDR3 and/or a light chain variable region having the LCDR1, LCDR2 and LCDR3, wherein the HCDR1, HCDR2, HCDR3, LCDR1, and/or LCDR3 comprise one or two or more cysteine mutations, the heavy chain variable region comprising an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 140, 158, 159, 160, 161, 162 or 163, respectively, and/or the light chain variable region comprising an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NOs: 141, 164, 165, 166 or 167, respectively;
further, the hIL-2/NARA1 antibody fusion protein comprises a heavy chain variable region and a light chain variable region as follows:

(1) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 158, 159, 160, 161, 162 or 163 and the light chain variable region as set forth in SEQ ID NO: 141, respectively; or

(2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 140 and the light chain variable region as set forth in SEQ ID NO: 164, 165, 166 or 167, respectively.

Preferably, the hIL-2/NARA1 antibody fusion protein comprises a heavy chain variable region and a light chain variable region as follows:

(1) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 158 or 161 and the light chain variable region as set forth in SEQ ID NO: 141, respectively; or

(2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 140 and the light chain variable region as set forth in SEQ ID NO: 164 or 167, respectively.

The hIL-2/NARA1 antibody fusion protein disclosed in the present application is unable to bind with IL2R$\alpha$, but

retains the binding activity to IL2Rβγ.

In some embodiments, the binding interface of the hIL-2:TCB2 antigen-antibody complex encompasses the binding interface between hIL-2 and IL2Rα, wherein the binding interface of hIL-2:TCB2 antigen-antibody complex is located in AB loop, B helix and CD loop of the hIL-2. In a specific embodiment, the binding interface of hIL-2:TCB2 antigen-antibody complex is located at amino acid positions 34-45 or their adjacent residues, amino acid positions 62-76 or their adjacent residues, and the amino acid position 111 or its adjacent residues in hIL-2.

In one embodiment, the binding interface of the hIL-2:TCB2 antigen-antibody complex comprises two or more cysteine mutations so as to form the disulfide linkage. In a specific embodiment, the hIL-2 has one or two or more cysteine mutations at amino acid positions 34, 36, 37, 38, 42, 68, and 109 relative to the wild-type hIL-2 as set forth in SEQ ID NO: 1, to introduce the first cysteine residue. In a specific embodiment, the TCB2 antibody or antigen-binding fragment thereof has one or two or more cysteine mutations at specific amino acid positions in the CDR region, to introduce the second cysteine residue, wherein the cysteine mutations are located at the amino acid position 6 of HCDR2, and/or the amino acid position 3, 5, 6 and/or 7 of HCDR3, and/or the amino acid position 4 of LCDR1, and/or the amino acid position 4, 5 and/or 6 of LCDR3.

In one embodiment, a pair of the first cysteine residue and the second cysteine residue for forming the disulfide linkage at the binding interface of hIL-2:TCB2 antigen-antibody complex is selected from one, or two, or more of the following paired amino acid mutations:

(1) the first cysteine residue is introduced by a mutation of P34C at the position 34 of the hIL-2, and the second cysteine residue is introduced by a mutation of T4C at the position 4 in LCDR1 of the TCB2 antibody or antigen-binding fragment thereof, or the second cysteine is introduced by a mutation of D4C or N5C at the position 4 or 5 in LCDR3 of the TCB2 antibody or antigen-binding fragment thereof; and/or

(2) the first cysteine residue is introduced by a mutation of L36C at the position 36 of the hIL-2, and the second cysteine residue is introduced by a mutation of N5C at the position 5 in LCDR3 of the TCB2 antibody or antigen-binding fragment thereof; and/or

(3) the first cysteine residue is introduced by a mutation of T37C at the position 37 of the hIL-2, and the second cysteine residue is introduced by a mutation of N5C or L6C at the position 5 or 6 in LCDR3 of the TCB2 antibody or antigen-binding fragment thereof; and/or

(4) the first cysteine residue is introduced by a mutation of R38C at the position 38 of the hIL-2, and the second cysteine residue is introduced by a mutation of F7C at the position 7 in HCDR3 of the TCB2 antibody or antigen-binding fragment thereof; and/or

(5) the first cysteine residue is introduced by a mutation of F42C at the position 42 of the hIL-2, and the second cysteine residue is introduced by a mutation of R5C at the position 5 in HCDR3 of the TCB2 antibody or antigen-binding fragment thereof; and/or

(6) the first cysteine residue is introduced by a mutation of E68C at the position 68 of the hIL-2, and the second cysteine residue is introduced by a mutation of A3C or G6C at the position 3 or 6 in HCDR3 of the TCB2 antibody or antigen-binding fragment thereof; and/or

(7) the first cysteine residue is introduced by a mutation of D109C at the position 109 of the hIL-2, and the second cysteine residue is introduced by a mutation of D6C at the position 6 in HCDR2 of the TCB2 antibody or antigen-binding fragment thereof.

Preferably, a pair of the first cysteine residue and the second cysteine residue for forming the disulfide linkage at the binding interface of hIL-2:TCB2 antigen-antibody complex is selected from one, or two, or more of the following paired amino acid mutations:

(1) the first cysteine residue is introduced by a mutation of P34C at the position 34 of the hIL-2, and the second cysteine is introduced by a mutation of D4C or N5C at the position 4 or 5 in LCDR3 of the TCB2 antibody or antigen-binding fragment thereof; and/or

(2) the first cysteine residue is introduced by a mutation of L36C at the position 36 of the hIL-2, and the second cysteine residue is introduced by a mutation of N5C at the position 5 in LCDR3 of the TCB2 antibody or antigen-binding fragment thereof; and/or

(3) the first cysteine residue is introduced by a mutation of T37C at the position 37 of the hIL-2, and the second cysteine residue is introduced by a mutation of L6C at the position 6 in LCDR3 of the TCB2 antibody or antigen-binding fragment thereof; and/or

(4) the first cysteine residue is introduced by a mutation of F42C at the position 42 of the hIL-2, and the second cysteine residue is introduced by a mutation of R5C at the position 5 in HCDR3 of the TCB2 antibody or antigen-binding fragment thereof; and/or

(5) the first cysteine residue is introduced by a mutation of E68C at the position 68 of the hIL-2, and the second cysteine residue is introduced by a mutation of A3C at the position 3 in HCDR3 of the TCB2 antibody or antigen-binding fragment thereof.

In some embodiments, the hIL-2/TCB2 antibody fusion protein comprises HCDR1, HCDR2, HCDR3 of the heavy chain variable region and LCDR1, LCDR2, LCDR3 of the light chain variable region, wherein the HCDR2, HCDR3, LCDR1, and/or LCDR3 comprise one or two or more cysteine substitution mutations. The HCDR1, HCDR2, HCDR3 of the heavy chain variable region and the LCDR1, LCDR2, LCDR3 of the light chain variable region comprise: (1) HCDR1 having an amino acid sequence as set forth in SEQ ID NO: 170; (2) HCDR2 having an amino acid sequence as set forth in SEQ ID NO: 171 or 176; (3) HCDR3 having an amino acid sequence as set forth in SEQ ID NO: 172, 177, 178, 179 or 180; (4) LCDR1 having an amino acid sequence as set forth in SEQ ID NO : 173 or 181; (5) LCDR2 having an amino acid sequence as set forth in SEQ ID NO: 174; (6) LCDR3 having an amino acid sequence as set forth in SEQ ID NO: 175, 182, 183 or 184.

In one embodiment, the hIL-2/TCB2 antibody fusion protein comprises HCDR1, HCDR2, HCDR3 of the heavy chain variable region and LCDR1, LCDR2, LCDR3 of the light chain variable region as follows:

(1) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NO: 177, 178, 179 or 180, LCDR1 having an amino acid sequence as set forth in SEQ ID NO: 173, LCDR2 having an amino acid sequence as set forth in SEQ ID NO: 174, and LCDR3 having an amino acid sequence as set forth in SEQ ID NO: 175; and/or

(2) HCDR1 having an amino acid sequence as set forth in SEQ ID NO: 170, HCDR2 having an amino acid sequence as set forth in SEQ ID NO: 176, HCDR3 having an amino acid sequence as set forth in SEQ ID NO: 172, LCDR1 having an amino acid sequence as set forth in SEQ ID NO: 173, LCDR2 having an amino acid sequence as set forth in SEQ ID NO: 174, and LCDR3 having an amino acid sequence as set forth in SEQ ID NO: 175; and/or

(3) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NO: 172, LCDR1 having an amino acid sequence as set forth in SEQ ID NO: 181, LCDR2 having an amino acid sequence as set forth in SEQ ID NO: 174, and LCDR3 having an amino acid sequence as set forth in SEQ ID NO: 175; and/or

(4) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NO: 172, LCDR1 having an amino acid sequence as set forth in SEQ ID NO: 173, LCDR2 having an amino acid sequence as set forth in SEQ ID NO: 174, and LCDR3 having an amino acid sequence as set forth in SEQ ID NO: 182, 183 or 184.

In a specific embodiment, the hIL-2/TCB2 antibody fusion protein comprises HCDR1, HCDR2, HCDR3 of the heavy chain variable region and LCDR1, LCDR2, LCDR3 of the light chain variable region as follows:

(1) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NO: 177 or 178, LCDR1 having an amino acid sequence as set forth in SEQ ID NO: 173, LCDR2 having an amino acid sequence as set forth in SEQ ID NO: 174, and LCDR3 having an amino acid sequence as set forth in SEQ ID NO: 175; and/or

(2) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NO: 172, LCDR1 having an amino acid sequence as set forth in SEQ ID NO: 173, LCDR2 having an amino acid sequence as set forth in SEQ ID NO: 174, and LCDR3 having an amino acid sequence as set forth in SEQ ID NO: 182, 183 or 184.

In some embodiments, the hIL-2/TCB2 antibody fusion protein comprises a heavy chain variable region having HCDR1, HCDR2 and HCDR3 and/or a light chain variable region having LCDR1, LCDR2 and LCDR3, wherein the HCDR2, HCDR3, LCDR1, and/or LCDR3 comprise one, or two, or more than two cysteine mutations; the heavy chain variable region comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 168, 185, 186, 187, 188 or 189, respectively; and/or the light chain variable region comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 169, 190, 191, 192 or 193, respectively.

In one embodiment, the hIL-2/TCB2 antibody fusion protein comprises a heavy chain variable region and a light chain variable region as follows:

(1) amino acid sequences which are at least at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 185, 186, 187, 188 or 189 and the light chain variable region as set forth in SEQ ID NO: 169, respectively; or

(2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 168 and the light chain variable region as set forth in SEQ ID NO: 190, 191, 192 or 193, respectively.

In a specific embodiment, the hIL-2/TCB2 antibody fusion protein comprises a heavy chain variable region and a light chain variable region as follows:

(1) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 185 or 186 and the light chain variable region as set forth in SEQ ID NO: 169, respectively; or

(2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 168 and the light chain variable region as set forth in SEQ ID NO: 191, 192 or 193, respectively.

The hIL-2/TCB2 antibody fusion protein disclosed in the present application is unable to bind to IL2Rα, but has a retained or augmented binding activity to IL2Rβ/γ.

The numbering of the heavy/light chain variable region and the CDR regions thereof (defined by Kabat numbering system) in the antibody or antigen-binding fragment thereof of the exemplary hIL-2/anti-IL-2 antibody fusion protein of the present application are shown in Table 3.

Table 3. Numbering of the heavy/light chain variable regions and CDR regions thereof (defined by the Kabat numbering system) in the antibody or antigen-binding fragment thereof of the exemplary hIL-2/anti-IL-2 antibody fusion protein

| hIL-2/Anti-IL-2 Antibody Fusion Protein ID | SEQ ID NO: | | | | | | | |
| --- | --- | --- | --- | --- | --- | --- | --- | --- |
| | HCDR1 | HCDR2 | HCDR3 | VH | LCDR1 | LCDR2 | LCDR3 | VL |
| H6K3(H)-Cy-01 | 14 | 15 | 9 | 63 | 30 (Z5=D, Z6=C, Xi=N, Xiv=Y) | 17 | 12 | 80 |
| H6K3(H)-Cy-02 | 14 | 15 | 9 | 63 | 31 (Z5=D, Z6=G, Xi=C, Xiv=Y) | 17 | 12 | 81 |
| H6K3(H)-Cy-03 | 14 | 15 | 9 | 63 | 10 | 32(Z7=C, Z8=T, X4=S) | 12 | 82 |
| H6K3(H)-Cy-04 | 14 | 15 | 9 | 63 | 10 | 33(Z7=S, Z8=C, X4=S) | 12 | 83 |
| H6K3(H)-Cy-05 | 14 | 15 | 9 | 63 | 10 | 17 | 12 | 84 |

(continued)

| hIL-2/Anti-IL-2 Antibody Fusion Protein ID | SEQ ID NO: | | | | | | | |
|---|---|---|---|---|---|---|---|---|
| | HCDR1 | HCDR2 | HCDR3 | VH | LCDR1 | LCDR2 | LCDR3 | VL |
| H6K3(H)-Cy-06 | 14 | 15 | 9 | 63 | 10 | 17 | 12 | 85 |
| H6K3(H)-Cy-07/ Cy-08/Cy-10 | 14 | 15 | 29 (Z2=V, $X_{II}$=C, Z3=Y, Z4=S) | 86 | 10 | 17 | 12 | 64 |
| H6K3(H)-Cy-08-DE | 14 | 15 | 29 | 86 | 10 | 17 | 12 | 87 |
| H6K3(H)-Cy-08-QD | 14 | 15 | 29 | 86 | 39 | 17 | 12 | 88 |
| H6K3(H)-Cy-08-QE | 14 | 15 | 29 | 86 | 40 | 17 | 12 | 89 |
| H6K3(H)-Cy-08-QS | 14 | 15 | 29 | 86 | 41 | 17 | 12 | 90 |
| H6K3(H)-Cy-08-QG | 14 | 15 | 29 | 86 | 42 | 17 | 12 | 91 |
| H6K3(H)-Cy-08-QK | 14 | 15 | 29 | 86 | 43 | 17 | 12 | 92 |
| H6K3(H)-Cy-08-QR | 14 | 15 | 29 | 86 | 44 | 17 | 12 | 93 |
| K2G11-Cy-01 | 14 | 19 | 24 | 74 | 35 (Z5=Y, Z6=C, Xi=N, Xiv=Y) | 17 | 12 | 104 |
| K2G11-Cy-02 | 14 | 19 | 24 | 74 | 36 (Z5=Y, Z6=S, Xi=C, Xiv=Y) | 17 | 12 | 105 |
| K2G11-Cy-03 | 14 | 19 | 24 | 74 | 26 | 32(Z7=C, Z8=T, X4=S) | 12 | 106 |
| K2G11-Cy-04 | 14 | 19 | 24 | 74 | 26 | 33(Z7=S, Z8=C, X4=S) | 12 | 107 |
| K2G11-Cy-05 | 14 | 19 | 24 | 74 | 26 | 17 | 12 | 108 |
| K2G11-Cy-06 | 14 | 19 | 24 | 74 | 26 | 17 | 12 | 109 |
| K2G11-Cy-07 | 14 | 19 | 34 (Z2=I, $X_{II}$=C, Z3=L, Z4=S) | 112 | 26 | 17 | 12 | 77 |
| K2G11-Cy-08 | 14 | 19 | 34 (Z2=I, $X_{II}$=C, Z3=L, Z4=S) | 112 | 26 | 17 | 12 | 77 |
| K2G11-Cy-09 | 14 | 19 | 24 | 74 | 26 | 17 | 38 ($X_{III}$=C) | 110 |
| K2G11-Cy-10 | 14 | 19 | 34 (Z2=I, $X_{II}$=C, Z3=L, Z4=S) | 112 | 26 | 17 | 12 | 77 |

(continued)

| hIL-2/Anti-IL-2 Antibody Fusion Protein ID | SEQ ID NO: | | | | | | | |
|---|---|---|---|---|---|---|---|---|
| | HCDR1 | HCDR2 | HCDR3 | VH | LCDR1 | LCDR2 | LCDR3 | VL |
| K2G11-Cy-11 | 14 | 19 | 24 | 74 | 35 | 17 | 12 | 104 |
| K2G11-Cy-12 | 14 | 19 | 24 | 74 | 37 (Z5=Y, Z6=S, Xi=N, Xiv=C) | 17 | 12 | 111 |
| NARA1-Cy01 | 142 | 149 | 144 | 158 | 145 | 146 | 147 | 141 |
| NARA1-Cy02 | 142 | 143 | 152 | 159 | 145 | 146 | 147 | 141 |
| NARA1-Cy03 | 142 | 143 | 153 | 160 | 145 | 146 | 147 | 141 |
| NARA1-Cy04 | 142 | 143 | 144 | 140 | 154 | 146 | 147 | 164 |
| NARA1-Cy05 | 142 | 143 | 144 | 140 | 154 | 146 | 147 | 164 |
| NARA1-Cy06 | 142 | 143 | 144 | 140 | 145 | 146 | 155 | 165 |
| NARA1-Cy07 | 142 | 143 | 144 | 140 | 145 | 146 | 156 | 166 |
| NARA1-Cy08 | 142 | 143 | 144 | 140 | 145 | 146 | 157 | 167 |
| NARA1-Cy09 | 148 | 143 | 144 | 161 | 145 | 146 | 147 | 141 |
| NARA1-Cy10 | 142 | 150 | 144 | 162 | 145 | 146 | 147 | 141 |
| NARA1-Cy11 | 142 | 151 | 144 | 163 | 145 | 146 | 147 | 141 |
| TCB2-Cy01 | 170 | 171 | 177 | 185 | 173 | 174 | 175 | 169 |
| TCB2-Cy02 | 170 | 171 | 178 | 186 | 173 | 174 | 175 | 169 |
| TCB2-Cy03 | 170 | 171 | 179 | 187 | 173 | 174 | 175 | 169 |
| TCB2-Cy04 | 170 | 171 | 180 | 188 | 173 | 174 | 175 | 169 |
| TCB2-Cy05 | 170 | 176 | 172 | 189 | 173 | 174 | 175 | 169 |
| TCB2-Cy06 | 170 | 171 | 172 | 168 | 181 | 174 | 175 | 190 |
| TCB2-Cy07 | 170 | 171 | 172 | 168 | 173 | 174 | 182 | 191 |
| TCB2-Cy08 | 170 | 171 | 172 | 168 | 173 | 174 | 183 | 192 |
| TCB2-Cy09 | 170 | 171 | 172 | 168 | 173 | 174 | 183 | 192 |
| TCB2-Cy10 | 170 | 171 | 172 | 168 | 173 | 174 | 183 | 192 |
| TCB2-Cy11 | 170 | 171 | 172 | 168 | 173 | 174 | 184 | 193 |

The hIL-2/anti-IL-2 antibody fusion protein of the present application, in one aspect, ensures the IL2Rα-binding site of hIL-2 or hIL-2 mutein to be permanently embedded inside of the fusion protein as described in the present application (i.e., the antigen-antibody binding interface), while the IL2Rβ/γ-binding site of IL-2 or IL-2 mutein is kept exposed to the outside of the molecule, thereby retaining its binding affinity to IL2Rβ/γ as well as its biological activity of specifically activating IL2Rβ/γ (including promoting the proliferation of Teff cells and/or NK cells, activating the antitumor activity of Teff cells and/or NK cells), and eliminating the binding to the high-affinity IL2R and the associated toxicity (including VLS, pulmonary edema, hypotension, etc. caused by the binding of hIL-2 or hIL-2 mutein to the high-affinity IL2R expressed on normal tissues and/or ILC2 cells) and agonistic effects on the clinical efficacy (including the tumor immunosuppression mediated by Tregs which express the high-affinity IL2R). In another aspect, the hIL-2/anti-IL-2 antibody fusion protein of the present application has a stable structure, which never dissociate as it may happen in the hIL-2/anti-IL-2 antibody immunocomplex, and its hIL-2 or hIL-2 mutein component cannot be competitively bound by the high-affinity IL2R, and thus significantly reduces the potential clinical risks which are associated with the hIL-2/anti-IL-2 antibody immunocomplex. In a third aspect, the constructed protein has a low immunogenicity which greatly reduces the risk of ADA. In a fourth aspect, the constructed protein is easy to manufacture, and the generated drug product contains a good homogeneity, which not only facilitates the quality control, but also maintains a high stability and is not easy to aggregate, thereby significantly reducing potential adverse effects caused by product quality.

*Linker*

In a specific embodiment, the hIL-2/anti-IL-2 antibody fusion protein of the present application also includes the hIL-2 or hIL-2 mutein fused to or operatively linked through a linker to the heavy chain variable region or the light chain variable region of the anti-IL-2 antibody or antigen-binding fragment thereof of the present application. The present application provides the evaluation of the location where the hIL-2 or IL-2 mutein is connected to the anti-IL-2 antibody or antigen-binding fragment thereof, and the type and length of the linker, and whether the linker will affect the binding activity of the hIL-2 or mutein thereof to IL2Rβ/γ. In addition, the selected linker should have a low immunogenicity. A flexible peptide may be selected as the linker herein. In some embodiments, the linker sequence comprises G4S or GAF, and one or two or more copies of the linker sequence can be used. In other embodiments, 2, 3, 4 or 5 copies of the G4S or GAF linker sequence or any other linker sequences known in the art and suitable for the fusion protein of the present application may be used herein. Preferably, the linker includes $(G4S)_4$, $(G4S)_3$, $(GAF)_2$, which can ensure the stability of the internal structure of the constructed IL-2/anti-IL-2 antibody fusion protein; and more preferably, the linker is $(G4S)_4$.

As used herein, the term "linker" refers to a molecule (including, but not limited to, an unmodified or modified amino acid or amino acid sequence) that connects two compounds, such as two polypeptides. A linker may consist of one or two or more linking molecules, or may comprise a linking molecule and at least one spacer molecule designed to separate the linking molecule and the compound by a specified distance.

In some embodiments, "operatively linked" refers to the linking of amino acid sequences, peptides or proteins with different functional properties, such as the linking of the hIL-2 or IL-2 mutein to the anti-IL-2 antibody or antigen-binding fragment thereof via a linker sequence described herein.

In some embodiments, the hIL-2 mutein of the present application is linked to the heavy chain variable region or light chain variable region of the humanized anti-IL-2 antibody of the present application through a linker. In a specific embodiment, the linker is connected to the C-terminus of the hIL-2 mutein and the N-terminus of the heavy chain of the anti-IL-2 antibody, which can ensure no negative impact on the binding activity of the hIL-2 mutein component to the IL2Rβ/γ.

In some embodiments, hIL-2 is linked to the heavy chain variable region or light chain variable region of the other humanized anti-IL-2 antibody that specifically recognizes the IL2Rα-binding site of hIL-2, through a linker. In a specific embodiment, the linker is connected to the C-terminus of hIL-2 and the N-terminus of the light chain variable region of the NARA1 antibody, which can ensure no negative impact on the binding activity of the hIL-2 component to IL2Rβ/γ. In a specific embodiment, 4 or more copies of the linker sequence can be selected as a linker to connect the C-terminus of hIL-2 and the N-terminus of the heavy chain variable region of the NARA1 antibody, which does not affect the binding activity of the hIL-2 component to IL2Rβ/γ. In a specific embodiment, the linker is connected to the C-terminus of hIL-2 and the N-terminus of the light chain variable region of the TCB2 antibody, which can ensure no negative impact on the binding activity of the hIL-2 component to IL2Rβ/γ. In a specific embodiment, 4 or more copies of the linker sequence can be selected as a linker to connect the C-terminus of hIL-2 and the N-terminus of the heavy chain variable region of the TCB2 antibody, which does not affect the binding activity of the hIL-2 component to IL2Rβ/γ.

The linker selected in the present application is compatible to the structural stability, manufacturing, and generation of homogeneous product of the hIL-2/anti-IL-2 antibody fusion protein of the present application, and the selected linker also has a low immunogenicity. *Further Optimization of hIL-2/Anti-IL-2 Antibody Fusion Protein of the Present Application*

The present application also provides a further optimization of the above-mentioned hIL-2/anti-IL-2 antibody fusion protein, including optimization of the anti-IL-2 antibody or antigen-binding fragment thereof and the hIL-2 mutein in the fusion protein molecule.

In some embodiments, the first aspartic acid or glutamate at the position 27 (located in the light chain CDR1) of the light chain variable region of the anti-IL-2 antibody (including the anti-mIL-2 antibody and the humanized antibody derived therefrom) is mutated, wherein the amino acid mutation includes D1E, Q27D, Q27E, Q27S, Q27G, Q27K or Q27R; and preferably the amino acid mutation includes D1E, Q27D, Q27E, Q27S or Q27G, and these mutations lead to a significant increase in the expression level of the hIL-2/anti-IL-2 antibody fusion protein of the present application.

In some specific embodiments, the hIL-2/anti-IL-2 antibody fusion protein comprises the following HCDR1, HCDR2, HCDR3, and LCDR1, LCDR2, LCDR3: HCDR1 of an amino acid sequence as set forth in SEQ ID NO: 14, HCDR2 of an amino acid sequence as set forth in SEQ ID NO: 15, HCDR3 of an amino acid sequence as set forth in SEQ ID NO: 29, LCDR1 of an amino acid sequence as set forth in any of SEQ ID NOs: 39-44, LCDR2 of an amino acid sequence as set forth in SEQ ID NO: 17, and LCDR3 of an amino acid sequence as set forth in SEQ ID NO: 12.

In a specific embodiment, the hIL-2/anti-IL-2 antibody fusion protein comprises the following HCDR1, HCDR2, HCDR3, and LCDR1, LCDR2, LCDR3: HCDR1 of an amino acid sequence as set forth in SEQ ID NO: 14, HCDR2 of an amino acid sequence as set forth in SEQ ID NO: 15, HCDR3 of an amino acid sequence as set forth in SEQ ID NO: 29, LCDR1 of an amino acid sequence as set forth in any of SEQ ID NOs: 39-42, LCDR2 of an amino acid sequence as set forth in SEQ ID NO: 17, and LCDR3 of an amino acid sequence as set forth in SEQ ID NO: 12.

In some specific embodiments, the hIL-2/anti-IL-2 antibody fusion protein comprises the following heavy chain variable region and/or light chain variable region, wherein the heavy chain variable region comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 86, and/or the light chain variable region comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 87-93.

In a specific embodiment, the hIL-2/anti-IL-2 antibody fusion protein comprises the following heavy chain variable region and/or light chain variable region, wherein the heavy chain variable region comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NO: 86, and/or the light chain variable region comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 87-91.

In some embodiments, to facilitate the manufacturing of the hIL-2/anti-IL-2 antibody fusion protein of the present application, increase the expression level, facilitate the quality control and the generation of homogenous product, and reduce potential ADA risks, a further optimization of the hIL-2 mutein is performed, including:

(i) Elimination of glycosylation modification sites to avoid the generation of glycosylated isomers in the manufacturing process:

In some embodiments, the glycosylation site present in the hIL-2 mutein of the IL-2/anti-IL-2 antibody fusion protein of the present application is mutated (e.g., deleted) or remain unchanged. The term "glycosylation site" in the present application, as used in polypeptide sequences, refers to an amino acid residue whose side chain can be attached to a carbohydrate moiety (e.g., an oligosaccharide structure), and it can be mutated so as to eliminate the native glycosylation modifications.

In some specific embodiments, at least one native glycosylation site is absent or present in the hIL-2 mutein of the hIL-2/anti-IL-2 antibody fusion protein of the present application. In one embodiment, the glycosylation site in the hIL-2 mutein of the hIL-2/anti-IL-2 antibody fusion protein of the present application is removed by a deletion of the first 3 or 5 amino acid residues at the N-terminus of the IL-2 mutein, or by a mutation of threonine residue at the position 3 of the hIL-2 mutein, preferably by a deletion of the first 3 amino acid residues at the N-terminus of the hIL-2 mutein (e.g., as shown in SEQ ID NO: 134); and/or

(ii) Elimination of free cysteine residues to avoid the formation of mismatched disulfide linkages: the cysteine residue at the position 125 in the hIL-2 mutein is mutated to alanine, leucine, serine, glutamine or valine.

*First and Second Polypeptide of the hIL-2/Anti-IL-2 Antibody Fusion Protein of the Present Application*

The hIL-2/anti-IL-2 antibody fusion protein of the present application comprises a first polypeptide and a second polypeptide, wherein the first polypeptide comprises the hIL-2 or hIL-2 mutein with a cysteine mutation, which is operatively linked to a variable region of the anti-IL-2 antibody or antigen-binding fragment thereof, and the second polypeptide comprises another variable region of the anti-IL-2 antibody or antigen-binding fragment thereof. Further, the first polypeptide comprises the hIL-2 or hIL-2 mutein having a cysteine mutation, a linker and a variable region of the anti-IL-2 antibody or antigen-binding fragment thereof. Another cysteine mutation can be placed in one variable region of the anti-IL-2 antibody or antigen-binding fragment thereof in the first polypeptide and/or in the other variable region of the anti-IL-2 antibody or antigen-binding fragment thereof in the second polypeptide, whereby a correctly paired and stable disulfide linkage can be formed with the cysteine residue in the hIL-2 or hIL-2 mutein. The said disulfide linkage is located at the antigen-antibody binding interface of the said hIL-2 or hIL-2 mutein and the said anti-IL-2 antibody or antigen-binding fragment thereof.

In some embodiments, the said hIL-2/anti-IL-2 antibody fusion protein comprises a first polypeptide and a second polypeptide, wherein the first polypeptide comprises the hIL-2 mutein with a cysteine mutation, a linker, and a heavy chain variable region of the anti-IL-2 antibody or antigen-binding fragment thereof, and the second polypeptide comprises a light chain variable region of the anti-IL-2 antibody or antigen-binding fragment thereof.

Further, the first polypeptide comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 95-103, 113-120, or 133-138, and/or the second polypeptide comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 64, 77, 80-85, 87-93, or 104-111.

In a specific embodiment, the said hIL-2/anti-IL-2 antibody fusion protein comprises a first polypeptide and a second polypeptide as follows:

(1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 95 and the second polypeptide as set forth in SEQ ID NO: 80, respectively; or

(2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 96 and the second polypeptide as set forth in SEQ ID NO: 81, respectively; or

(3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 97 and the second polypeptide as set forth in SEQ ID NO: 82, respectively; or

(4) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 98 and the second polypeptide as set forth in SEQ ID NO: 83, respectively; or

(5) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 99 and the second polypeptide as set forth in SEQ ID NO: 84, respectively; or

(6) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 100 and the second polypeptide as set forth in SEQ ID NO: 85, respectively; or

(7) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in any of SEQ ID NOs: 101, 102, 103, and 133-138 and the second polypeptide as set forth in SEQ ID NO: 64, respectively; or

(8) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 102 and the second polypeptide as set forth in any of SEQ ID NOs: 87-93, respectively.

Preferably, the said hIL-2/anti-IL-2 antibody fusion protein comprises a first polypeptide and a second polypeptide as follows:

(1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in any of SEQ ID NOs: 101, 102, 103, and 133-138 and the second polypeptide as set forth in SEQ ID NO: 64, respectively; or

(2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 102 and the second polypeptide as set forth in any of SEQ ID NOs: 87-91, respectively.

In a specific embodiment, the said hIL-2/anti-IL-2 antibody fusion protein comprises a first polypeptide and a second polypeptide as follows:

(1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 113 and the second polypeptide as set forth in SEQ ID NOs: 104, 105 or 106, respectively; or

(2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 114 and the second polypeptide as set forth in SEQ ID NO: 107, respectively; or

(3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 115 and the second polypeptide as set forth in SEQ ID NO: 108 or 109, respectively; or

(4) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NOs: 116, 117 or 119 and the second polypeptide as set forth in SEQ ID NO: 77, respectively; or

(5) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 118 and the second polypeptide as set forth in SEQ ID NO: 110, respectively; or

(6) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 120 and the second polypeptide as set forth in SEQ ID NO: 104, respectively; or

(7) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 118 and the second polypeptide as set forth in SEQ ID NO: 111, respectively.

Preferably, the said hIL-2/anti-IL-2 antibody fusion protein comprises a first polypeptide and a second polypeptide as follows:

(1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 114 and the second polypeptide as set forth in SEQ ID NO: 107, respectively; or

(2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 115 and the second polypeptide as set forth in SEQ ID NO: 108 or 109, respectively.

In some embodiments, the said hIL-2/anti-IL-2 antibody fusion protein comprises a first polypeptide and a second polypeptide, wherein the first polypeptide comprises the hIL-2 with a cysteine mutation, a linker, and the light chain variable region of the anti-IL-2 antibody or antigen-binding fragment thereof, and the second polypeptide comprises the heavy chain variable region of the anti-IL-2 antibody or antigen-binding fragment thereof.

Further, the first polypeptide comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 206-226, and/or the second polypeptide comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 140, 158-163, 168, or 185-189.

In a specific embodiment, the said hIL-2/anti-IL-2 antibody fusion protein comprises a first polypeptide and a second polypeptide as follows:

(1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 206 and the second polypeptide as set forth in SEQ ID NO: 158, respectively; or

(2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 207 and the second polypeptide as set forth in SEQ ID NO: 159, respectively; or

(3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 208 and the second polypeptide as set forth in SEQ ID NO: 160, respectively; or

(4) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in any of SEQ ID NOs: 209-213 and the second polypeptide as set forth in SEQ ID NO: 140, respectively; or

(5) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 214 and the second polypeptide as set forth in SEQ ID NO: 161, respectively; or

(6) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 215 and the second polypeptide as set forth in SEQ ID NO: 162, respectively; or

(7) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 216 and the second polypeptide as set forth in SEQ ID NO: 163, respectively.

Preferably, the said hIL-2/anti-IL-2 antibody fusion protein comprises a first polypeptide and a second polypeptide as follows:

(1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 206 and the second polypeptide as set forth in SEQ ID NO: 158, respectively; or

(2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID Nos: 209, 210 or 213 and the second polypeptide as set forth in SEQ ID NO: 140, respectively; or

(3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 214 and the second polypeptide as set forth in SEQ ID NO: 161, respectively.

In a specific embodiment, the said hIL-2/anti-IL-2 antibody fusion protein comprises a first polypeptide and a second polypeptide as follows:

(1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 217 and the second polypeptide as set forth in SEQ ID NO: 185 or 187, respectively; or

(2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 218 and the second polypeptide as set forth in SEQ ID NO: 186, respectively; or

(3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 219 and the second polypeptide as set forth in SEQ ID NO: 188, respectively; or

(4) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 220 and the second polypeptide as set forth in SEQ ID NO: 189, respectively; or

(5) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in any of SEQ ID NOs: 221-226 and the second polypeptide as set forth in SEQ ID NO: 168 respectively.

Preferably, the said hIL-2/anti-IL-2 antibody fusion protein comprises a first polypeptide and a second polypeptide as follows:

(1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 217 and the second polypeptide as set forth in SEQ ID NO: 185, respectively; or

(2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 218 and the second polypeptide as set forth in SEQ ID NO: 186, respectively; or

(3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NOs: 222, 223, 224 or 226 and the second polypeptide as set forth in SEQ ID NO: 168, respectively.

## 4. Constant Region

The anti-IL-2 antibody or antigen-binding fragment thereof and/or the hIL-2/anti-IL-2 antibody fusion protein of the present application may further comprise an antibody constant region including a heavy chain constant region and a light chain constant region. The heavy chain constant region of the present application includes native and mutant forms of the Fc region of the human IgG heavy chain constant region, and also includes truncated forms of polypeptides containing a hinge region that facilitate the dimerization. In certain embodiments, the Fc region comprises CH2 and CH3 domains of an antibody. A fusion protein comprising a Fc moiety (and an oligomer formed therefrom) offers the advantage of easy purification by the protein A or protein G affinity chromatography, as well as increased serum half life. Preferably, the Fc region is derived from human IgGs, including IgG1, IgG2, IgG3 and IgG4. Herein, the positions of specific amino acid residues in the Fc region are determined according to the EU numbering system.

One function of the Fc region of an antibody is to produce "effector functions" with the immune system when the antibody binds to its target, including production of antibody-dependent cellular cytotoxicity (ADCC), antibody-dependent cellular phagocytosis (ADCP), and/or complement-dependent cytotoxicity (CDC). ADCC and ADCP are mediated by binding of Fc to Fc receptors (FcRs) on the surface of immune cells (Raghavan et al., Annu Rev Cell Dev Biol 1996, 12:181-220; Ghetie et al., Annu Rev Immunol 2000, 18:739-766; Ravetch et al., Annu Rev Immunol 2001, 19:275-290), wherein the immune cells include monocytes, macrophages, neutrophils, dendritic cells, eosinophils, mast cells, platelets, B cells, large granular lymphocytes, Langerhans cells, NK cells, and T cells. CDC is mediated by the binding of Fc to proteins of the complement system such as C1q (Ward et al., Ther Immunol 1995, 2:77-94).

In some embodiments, the anti-IL-2 antibody of the present application and/or the hIL-2/anti-IL-2 antibody fusion protein of the present application comprise an engineered IgG Fc region, thereby reducing the effector functions mediated by it. An exemplary Fc molecule with reduced effector functions includes an Fc molecule with the following amino acid substitution mutations:

N297A or N297Q (IgG1);

L234A/L235A (IgG1);

V234A/G237A (IgG2);

L235A/G237A/E318A (IgG4);

H268Q/V309L/A330S/A331S (IgG2);

C220S/C226S/C229S/P238S (IgG1);

C226S/C229S/E233P/L234V/L235A (IgG1);

L234F/L235E/P331S (IgG1).

Preferably, the engineered Fc region is human IgG1 Fc with amino acid substitution mutations of L234F/L235E/P331S (EU numbering system), which has reduced binding activities to one or more FcγRs and C1q (Oganesyan V et al., Acta Crystallogr D Biol Crystallogr 2008, 64:700-704; US Patent Nos.: US5624821, US6194551). The FcγR family includes FcγRI (also known as CD64), including the isoforms FcγRIa, FcγRIb, and FcγRIc; FcγRII (also known as CD32), including the isoforms FcγRIIa, FcγRIIb, and FcγRIIc; and FcγRIII (also known as CD16), including isoforms FcγRIIIa and FcγRIIIb (Jefferis et al., Immunol Lett 2002, 82:57-65). Among the FcγRs, FcγRI, FcγRIIa, FcγRIIc and FcγRIIIa can induce ADCC, endocytosis, phagocytosis and/or cytokine release. Binding activities include, but are not limited to, binding specificity, binding affinity constant ($K_D$), dissociation and association rate ($k_{dis}$ and $k_a$, respectively), from any one or more of which those skilled in the art can analyze whether the engineered Fc region has altered ADCC and/or CDC activity.

In some embodiments, the anti-IL-2 antibody and/or the hIL-2/anti-IL-2 antibody fusion protein of the present application comprising the mutations L234F/L235E/P331S in their heavy chain constant region have a reduced binding affinity to one or more of FcγRs including FcγRI, FcγRIIa, FcγRIIb, FcγRIIIa (158F), and/or FcγRIIIa (158V). In one embodiment, the anti-IL-2 antibody and/or hIL-2/anti-IL-2 antibody fusion protein of the present application comprising the mutations L234F/L235E/P331S in their heavy chain constant region essentially do not bind to FcγRI, FcγRIIa, FcγRIIb, FcγRIIIa (158F), and/or FcγRIIIa (158V). In one embodiment, the anti-IL-2 antibody and/or hIL-2/anti-IL-2 antibody fusion protein of the present application comprising the mutations L234F/L235E/P331S in their heavy chain constant region essentially do not bind to C1q. In one embodiment, introduction of the mutations L234F/L235E/P331S in the heavy chain constant region of the anti-IL-2 antibody and/or hIL-2/anti-IL-2 antibody fusion protein of the present application does not affect their binding affinity to FcRn.

Preferably, an engineered Fc region is a human IgG1 Fc with amino acid substitution mutations L234FAL235E/P331S (EU numbering system). In some embodiments, introduction of the mutations L234F/L235E/P331S in the heavy chain constant region can significantly reduce the ADCC activity of the anti-IL-2 antibody and/or hIL-2/anti-IL-2 antibody fusion protein of the present application. In one embodiment, the anti-IL-2 antibody and/or hIL-2/anti-IL-2 antibody fusion protein of the present application have a significantly attenuated or eliminated ADCC activity as compared to the antibody without the amino acid mutations in its Fc region. In one embodiment, the ADCC activity of the anti-IL-2 antibody and/or hIL-2/anti-IL-2 antibody fusion protein of the present application is decreased to undetectable. Such attenuation or elimination of the ADCC activity is likely caused by the significant reduction in affinity of the antibody and/or the fusion protein of the present application to FcγR.

In some embodiments, the anti-IL-2 antibody of the present application and/or the hIL-2/anti-IL-2 antibody fusion protein of the present application comprise an engineered IgG1 constant region which results in a reduction in the isoelectric point (pI) of the said antibody and/or fusion protein and leads to a prolonged serum half-life. Shifts in the isoelectric point of an antibody and/or fusion protein can change their tissue distribution and pharmacokinetic (PK) properties. Typically, a decrease in pI can lead to reduced nonspecific tissue absorption and metabolism and degradation, and leads to a prolonged serum half-life (Boswell CA et al., Bioconjug Chem 2010, 21:2153-2163; Li et al., MAbs 2014, 6:1255-1264). In some embodiments, the pI of the anti-IL-2 antibody and/or hIL-2/anti-IL-2 antibody fusion protein of the present application can be reduced by mutating amino acids at specific sites in their heavy chain constant region, for example, by substituting a basic amino acid (such as lysine or arginine) or neutral amino acid (such as asparagine or glutamine) with an acidic amino acid (such as aspartic acid or glutamic acid). The selection of specific sites for mutation depends on a number of factors, including the domain location in the antibody and/or fusion protein, effects on the function, and potential immunogenicity risk, etc.. The mutation should have no significant effect on the biophysical properties of domains of the antibody and/or fusion protein. In one embodiment, in order to minimize the risk of immunogenicity caused by mutations, a specific approach is to make substitutions according to amino acid sequence differences among native isoforms of human proteins. As to the selection of mutation sites in the heavy chain constant region of the anti-IL-2 antibody and/or hIL-2/anti-IL-2 antibody fusion protein of the present application, isoform differences across the human IgG subclasses (i.e., IgG1, IgG2, IgG3, and IgG4) can provide mutations with a low risk of immunogenicity. Specifically, the said mutations can be identified by aligning the amino acid sequences of the constant regions of IgG1, IgG2, IgG3 and IgG4, and the selected non-acidic or basic amino acid residues in the constant region of IgG1 are mutated to acidic or non-basic amino acid residues present in the other IgG subclasses at the corresponding positions. For example, as shown in Table 4, the mutations can be selected from one or more of N203D, K274Q and Q419E to reduce the pI value of IgG1 protein. In one embodiment, the anti-IL-2 antibody and/or hIL-2/anti-IL-2 antibody fusion protein of the present application comprise a heavy chain constant region with mutations comprising N203D/K274Q/Q419E (EU numbering system), wherein the pI value of the anti-IL-2 antibody and/or hIL-2/anti-IL-2 antibody fusion protein of the present application is reduced by about 1-2 pH units as compared to the antibody and/or fusion protein without the mutations in their heavy chain constant region. The pI value can be theoretically calculated or experimentally determined. In one embodiment, the anti-IL-2 antibody and/or hIL-2/anti-IL-2 antibody fusion protein with a lowered pI disclosed in the present application exhibit significantly improved pharmacokinetics, including that, as compared to the antibody and/or fusion protein without the mutations, the elimination half-life ($t_{1/2}$) of the anti-IL-2 antibody and/or hIL-2/anti-IL-2 antibody fusion protein of the present application is prolonged by at least 2-3 times, and/or the area under the curve (AUC) is increased by about 30% or more.

Table 4. pI mutation in the constant region of hIgG1 heavy chain of the antibody

| pI Mutation Site (EU Numbering) | hIgG1 | hIgG2 | hIgG3 | hIgG4 |
| --- | --- | --- | --- | --- |
| Position 203 | N | D | N | D |
| Position 274 | K | Q | Q | Q |
| Position 419 | Q | Q | Q | E |

*5. Polynucleotide, Vector and Host Cell*

In one aspect, the present application provides a nucleic acid encoding a hIL-2 mutein, an anti-IL-2 antibody or antigen-binding fragment thereof, or a hIL-2/anti-IL-2 antibody fusion protein. The present application also includes a polynucleotide variant encoding the amino acid sequence described herein.

Nucleotide sequences corresponding to the amino acid sequences described in the present application, of probes or primers for use of nucleic acid isolation, or queryable in the databases can be obtained by back-translation of the amino acid sequences. Polymerase chain reaction (PCR) procedures can be used to isolate and amplify DNA sequences encoding the hIL-2 muteins, the anti-IL-2 antibodies or antigen-binding fragments thereof, and the hIL-2/anti-IL-2 antibody fusion proteins of the present application. Oligonucleotides that define the desired ends of the DNA fragment assembly are used as 5' and 3' primers. The oligonucleotides may additionally contain recognition sites for restriction endonucleases to facilitate insertion of the amplified DNA fragments in combination into an expression vector. PCR techniques are described in many publications, e.g., Saiki et al., Science 1988, 239:487-491; Recombinant DNA Methodology, Wu et al., (eds), Academic Press, Inc., San Diego (1989), pp. 189-196; PCR Protocols: A Guide to Methods and Applications, Innis et al., (eds). Academic Press, Inc. (1990).

The nucleic acids of the present application include single- and double-stranded forms of DNA and RNA, and corresponding complementary sequences, including isolated nucleic acids, preferably DNA or RNA derived from at least one isolation in substantially pure form and in amounts or concentrations that can be used for identification, manipulation and recovery of the nucleotide sequences in the constituent through standard biochemical methods (e.g., as described in Sambrook et al., Molecular Cloning: A Laboratory Manual, 2nd Edition, Cold Spring Harbor Laboratory, Cold Spring Harbor, NY [1989]). Preferably, such sequences include those that are provided and/or constructed in the form of internal untranslated sequences or intron interrupted open reading frames not commonly found in eukaryotic genes. Sequences of untranslated DNA may be present at 5' or 3' of the open reading frames, wherein the sequences do not interfere with manipulation or expression of the coding regions.

The hIL-2 mutein, the anti-IL-2 antibody or antigen-binding fragment thereof, and the hIL-2/anti-IL-2 antibody fusion protein of the present application are prepared by the following steps: performing site-specific mutagenesis to the nucleotide in the DNA encoding the hIL-2 mutein, the anti-IL-2 antibody or antigen-binding fragment thereof, or the hIL-2/anti-IL-2 antibody fusion protein using PCR mutagenesis or other techniques known by those of ordinary skill in the art, and then allowing expression of the recombinant DNA in cell culture as outlined herein. In addition, hIL-2 muteins, anti-IL-2 antibodies or antigen-binding fragments thereof, and hIL-2/anti-IL-2 antibody fusion proteins can also be prepared by *in vitro* synthesis using established techniques.

As known to those skilled in the art, due to the degeneracy of the genetic codes, hIL-2 muteins, anti-IL-2 antibodies or antigen-binding fragments thereof, or hIL-2/anti-IL-2 antibody fusion proteins of the present application are encoded by an extremely large number of nucleic acids, each of which is within the scope of the present application and can be made using standard techniques. Thus, upon identification of a specific amino acid sequence, those skilled in the art can prepared many different nucleic acids by simply making one or two or more codon modifications to their respective coding sequences, without altering the amino acid sequences of the hIL-2 mutein, the anti-IL-2 antibody or antigen-binding fragment thereof, or the hIL-2/anti-IL-2 antibody fusion protein of the present application.

In another aspect, the present application also provides an expression vector for the nucleic acid encoding the hIL-2 mutein, the anti-IL-2 antibody or antigen-binding fragment thereof, or the hIL-2/anti-IL-2 antibody fusion protein herein.

The nucleic acid encoding the hIL-2 mutein, the anti-IL-2 antibody or antigen-binding fragment thereof, or the hIL-2/anti-IL-2 antibody fusion protein of the present application can be constructed in a suitable vector, for introduction into a host cell to express the protein of interest. The components of a vector typically include, but are not limited to, one or more of the following: a signal sequence, origin of replication, one or more marker genes, an enhancer element, a promoter, and a transcription termination sequence. The nucleic acid encoding the protein of interest in the vector is operatively linked to the promoter.

As used herein, the term "operatively linked" refers to the functional linkage between a nucleic acid expression control sequence (e.g., a promoter, a signal sequence, or an array of transcriptional regulator binding sites) and another nucleic acid sequence, and the control sequence thus controls the transcription and/or translation of the other nucleic acid sequence.

Suitable vectors include plasmids, phagemids, cosmids, artificial chromosomes such as yeast artificial chromosomes (YACs), bacterial artificial chromosomes (BACs) or P1-derived artificial chromosomes (PACs), bacteriophages such as λ phage or M13 phage, and animal virus, etc. Animal virus species used as vectors include retroviruses (including lentiviruses), adenoviruses, adeno-associated viruses, herpesviruses (e.g., herpes simplex virus), poxviruses, baculoviruses, papillomaviruses, papovaviruses (e.g., SV40). Vectors may contain a variety of elements for expression control, including promoter sequences, transcription initiation sequences, enhancer sequences, selectable elements, and reporter genes. Additionally, the vector may also contain an origin of replication. The vector may also include components to facilitate its entry into cells, including but not limited to viral particles, liposomes, or protein coats.

On the other hand, the present application also provides a host cell comprising a nucleic acid or expression vector comprising a hIL-2 mutein, an anti-IL-2 antibody or antigen-binding fragment thereof, or a hIL-2/anti-IL-2 antibody fusion protein of the present application.

The cell can be eukaryotic cells, e.g., mammalian host cells, including, but not limited to, SV40 transformed monkey kidney cell line CV1 (COS-7, ATCC, CRL-1651), human embryonic kidney cell line (293 or suspension cultured 293 cell subclone, Graham et al., J Gen Virol 1977, 36:59-74), baby hamster kidney cells (BHK-21, ATCC, CCL-10), Chinese hamster ovary cells/-DHFR (CHO, Urlaub et al., Proc Natl Acad Sci USA 1980, 77:4216-4220), mouse Sertoli cells (TM4, Mather, Biol Reprod 1980, 23:243-251), monkey kidney cells (CV1, ATCC, CCL-70), African Green monkey kidney cells (VERO-76, ATCC, CRL-1587), human cervical cancer cells (HELA, ATCC, CCL-2), canine kidney cells (MDCK, ATCC, CCL-34), Buffalo rat hepatocytes (BRL 3A, ATCC, CRL-1442), human lung cells (W138, ATCC, CCL-75), human hepatoma cell lines (HepG2, ATCC, HB-8065), mouse mammary tumor (MMT060562, ATCC, CCL-51), TRI cells (Mather et al., Ann NY Acad Sci 1982, 383:44-68), MRC5 cells, or FS4 cells.

## 6. Method of Preparation

The present application provides a method for preparing the hIL-2 mutein, the anti-IL-2 antibody or antigen-binding fragment thereof, and the hIL-2/anti-IL-2 antibody fusion protein of the present application using the host cell.

The method comprises transfecting a nucleic acid or expression vector encoding the hIL-2 mutein, the anti-IL-2 antibody or antigen-binding fragment thereof, or the hIL-2/anti-IL-2 antibody fusion protein of the present application into a host cell, and culturing the host cell in a culture medium for a period of time to express the hIL-2 mutein, the anti-IL-2 antibody or antigen-binding fragment thereof, or the hIL-2/anti-IL-2 antibody fusion protein of the present application. Without limitation, commercially available media can be used as the culture media.

Preferably, the hIL-2 mutein, the anti-IL-2 antibody or antigen-binding fragment thereof, or the hIL-2/anti-IL-2 antibody fusion protein is expressed and secreted into the medium in which the host cells are cultured. Antibodies are recovered from the culture medium using standard protein purification methods, such as centrifugation, ultrafiltration, and affinity chromatography to remove impurities; other purification techniques can also be used, such as anion or cation exchange chromatography, hydrophobic interaction chromatography, and hydroxyapatite chromatography.

## 7. Bispecific Molecule

The present application also provides a bispecific antibody comprising the anti-IL-2 antibody or antigen-binding fragment thereof, or the hIL-2/anti-IL-2 antibody fusion protein of the present application, one of the two arms of the said bispecific antibody comprises the anti-IL-2 antibody or antigen-binding fragment thereof, or the hIL-2/anti-IL-2 antibody fusion protein of the present application, and the other arm comprises an antibody specific for an antigen other than hIL-2 (preferably, a tumor-associated antigen or an immune checkpoint protein antigen) or an antigen associated with immune effector cells. The said tumor-associated antigen may be selected from HER2, EGFR, VEGF, VEGFR, CD19, CD20, CD30, CD33, CD52, BCMA, SLAMF7, MUC1, MUC16, EphB2, E-selectin, EpCam, CEA, PSMA, PSA, ERBb3, c-MET, ILT3, ILT7, 5T4, GPC-3, DLL3, etc., and the said immune checkpoint protein antigen may be selected from PD-1, PD-L1, PD-L2, TIM3, CTLA-4, LAG-3, CD47, CEACAM-1, CEACAM-5, VISTA, BTLA, TIGIT, LAIR1, CD40, CD40L, OX40, CD160, 2B4 or TGFR.

The bispecific molecule of the present application can have many different forms, for example, a bispecific molecule has the traditional antibody format, except for those having two binding arms with the same specificity. The bispecific molecule has two binding arms with different specificities, each of which may be formed by two single-chain antibody fragments (scFv) linked by a peptidyl linker, i.e., the so-called Bs(scFv)2 structure. A bispecific molecule may also include two different F(ab) fragments linked by a peptidyl linker. These and other forms of bispecific molecules can be prepared by methods of genetic engineering, somatic hybridization or chemical synthesis (Kufer et al., vide supra; Cao and Suresh, Bioconjugate Chemistry 1998, 9:635-644; Van Spriel et al., Immunology Today 2000, 21:391-397; and references cited therein).

## 8. Immunoconjugate, Chimeric Antigen Receptor, Engineered T Cell Receptor, or Oncolytic Virus

In one aspect, the present application provides an immunoconjugate comprising the anti-IL-2 antibody or antigen-binding fragment thereof, or the hIL-2/anti-IL-2 antibody fusion protein disclosed herein.

The anti-IL-2 antibody or antigen-binding fragment thereof, or the hIL-2/anti-IL-2 antibody fusion protein of the present application can be conjugated with a therapeutic agent to form an immunoconjugate, such as an antibody-drug conjugate (ADC). Suitable therapeutic agents include cytotoxins, alkylating agents, DNA minor groove binders, DNA intercalators, DNA cross-linking agents, histone deacetylase inhibitors, nuclear export inhibitors, proteasome inhibitors, topoisomerase I or II inhibitors, heat shock protein inhibitors, tyrosine kinase inhibitors, antibiotics and antimitotics. In the ADC, the linker for coupling the antibody and the therapeutic agent can be a cleavable linker, such as a peptidyl linker, disulfide linkage or a hydrazone linker, and can be a non-cleavable linker. ADC can be prepared using the method disclosed in US Patent Nos. 7087600, 6989452, and 7129261; PCT publication WO 02/096910, WO 07/038658, WO 07/051081, WO 07/059404, WO 08/083312, and WO 08/103693; US patent application Nos.: 20060024317, 20060004081, and 20060247295; the disclosures of which are incorporated herein by reference.

In another aspect, the present application also provides a chimeric antigen receptor, an engineered T cell receptor, or an oncolytic virus comprising the anti-IL-2 antibody or antigen-binding fragment thereof, or the hIL-2/anti-IL-2 antibody fusion protein disclosed herein.

### 9. Pharmaceutical Composition

The present application provides a pharmaceutical composition comprising the anti-IL-2 antibody or antigen-binding fragment thereof, or the hIL-2/anti-IL-2 antibody fusion protein of the present application (or an immunoconjugate, or a bispecific molecule, or a chimeric antigen receptor, or an engineered T cell receptor, or an oncolytic virus), and a pharmaceutically acceptable carrier.

The pharmaceutical composition can contain any kind of excipients. Excipients that can be used include carriers, surfactants, thickening or emulsifying agents, solid binders, dispersing or suspending auxiliaries, solubilizers, coloring agents, flavoring agents, coating agents, disintegrating agents, lubricants, sweeteners, preservatives, isotonic agents, or combinations thereof. The selection and use of suitable excipients is taught in Gennaro ed., Remington: The Science and Practice of Pharmacy, 20th Ed. (Lippincott Williams & Wilkins 2003), the disclosure of which is incorporated herein by reference.

Preferably, the pharmaceutical composition is suitable for intravenous, intramuscular, subcutaneous, parenteral, spinal or epidermal administration (e.g., by injection or infusion). In view of the different routes of administration, the active ingredient can be encapsulated in a material to protect it from the action of acids and other natural conditions that may inactivate it. As used herein, the term "parenteral administration" refers to non-enteral and topical modes of administration including, but not limited to, intravenous, intramuscular, intraarterial, intrathecal, intracapsular, intraorbital, intracardiac, intradermal, intraperitoneal, transtracheal, subcutaneous, subepidermal, intraarticular, subcapsular, subarachnoid, intraspinal, epidural, and intrasternal injection and infusion. Alternatively, the antibody of the present application can be administered by non-parenteral routes (e.g., routes of topical, epidermal or mucosal administration), e.g., intranasal, oral, vaginal, rectal, sublingual, or topical administration.

The pharmaceutical composition can be a sterile aqueous solution or dispersion. They are also capable of forming microemulsions, liposomes, or other ordered structural combinations suitable for high drug concentrations.

The amount of an active ingredient that can be combined with a carrier material to form a single dosage form will be determined by a subject and the particular mode of administration, and will generally be that amount of the composition which will produce a therapeutic effect. In general, the composition formed with a pharmaceutically acceptable carrier contains from about 0.01% to about 99% of the active ingredient, preferably from about 0.1% to about 70%, most preferably from about 1% to about 30% of the active ingredient.

Dosage regimens are adjusted to provide the best expected response (e.g., therapeutic response). For example, a single administration may be administered, several divided administrations may be administered, or the dose may be proportionally reduced or increased as indicated by the therapeutic situation. It is especially advantageous to formulate compositions for parenteral administration in dosage unit for ease of administration and uniformity of dosage. As used herein, dosage unit refers to physically discrete units suitable as unitary dosages for treatment of subjects; each unit dosage contains a predetermined quantity of the active ingredient obtained by calculation of the active ingredient co-administered with the required pharmaceutical carrier to produce the desired therapeutic effect. In addition, the anti-IL-2 antibody, or the hIL-2/anti-IL-2 antibody fusion protein of the present application can also be administered as a sustained-release formulation, which can reduce the frequency of administration.

The anti-IL-2 antibody, or the hIL-2/anti-IL-2 antibody fusion protein, or the composition comprising the anti-IL-2 antibody or the hIL-2/anti-IL-2 antibody fusion protein can be administered in a dosage range of about 0.0001 mg/kg to 100 mg/kg body weight, typically 0.001 mg/kg to 50 mg/kg body weight.

Preferably, a "therapeutically effective dose" of the anti-IL-2 antibody or antigen-binding fragment thereof, or the hIL-2/anti-IL-2 antibody fusion protein, or a bispecific molecule, or an immunoconjugate, or a chimeric antigen receptor, or an engineered T cell receptor, or an oncolytic virus according to the present application can cause a reduction in the severity of disease symptoms, an increase in the frequency and duration of progression-free period of disease symptoms, or prevention of physical damage or disability caused by disease affliction. For example, relative to untreated subjects, a "therapeutically effective dose" for tumor-bearing subjects preferably inhibits tumor growth by at least about 20%, more preferably by at least about 40%, even more preferably by at least about 60%, still more preferably by at least about 80%. A therapeutically effective amount of a therapeutic antibody can reduce the size of a tumor, or ameliorate the symptoms of a subject, wherein the subject is typically a human or other mammal. A "therapeutically effective dose" can also be determined differently depending on various factors including, but not limited to, a method of formulation, a method of administration, age, body, body weight, gender or pathological conditions of the patient, diet, administration time, dosing interval, route of administration, excretion rate and response sensitivity.

The pharmaceutical composition may be selected in a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid (Sustained and Controlled Release Drug Delivery Systems, J.R. Robinson, ed., Marcel Dekker, Inc., New York, 1978).

A therapeutic pharmaceutical composition can be delivered by a medical device selected from the group consisting of: (1) needle-free hypodermic injection devices (e.g., US Patent Nos.: 5399163, 5383851, 5312335, 5064413, 4941880, 4790824, and 4596556); (2) miniature infusion pump (US Patent No. 4487603); (3) transdermal device (US Patent No. 4486194); (4) infusion set (US Patent Nos. 4447233 and 4447224); and (5) osmotic device (US Patent Nos. 4439196 and 4475196); and the disclosures of these are incorporated herein by reference.

In certain embodiments, the anti-IL-2 antibody or antigen-binding fragment thereof, or the hIL-2/anti-IL-2 antibody fusion protein of the present application can be formulated to ensure biodistribution in the body. For example, to ensure that the therapeutic antibody or antibody fusion protein of the present application can cross the blood-brain barrier, they can be formulated into liposomes, which can additionally contain targeting moieties to enhance selective delivery to specific cells or organs (U.S. Patent Nos.: 4522811, 5374548, 5416016, and 5399331; Ranade VV, J Clin Pharmacol 1989, 29:685-694; Umezawa et al., Biochem Biophys Res Commun 1988, 153:1038-1044; Bloeman et al., FEBS Lett 1995, 357:140-144; Owais M et al., Antimicrob Agents Chemother 1995, 39:180-184; Briscoe et al., Am J Physiol 1995, 268:L374-L380; Schreier et al., J Biol Chem 1994, 269:9090-9098; Keinanen and Laukkanen, FEBS Lett 1994, 346:123-126; and Killion and Fidler, Immunomethods 1994, 4:273-279).

*10. Kit Set/Combined Product, Kit*

In one aspect, the present application provides a kit set comprising a pharmaceutical composition of the anti-IL-2 antibody or antigen-binding fragment or the hIL-2/anti-IL-2 antibody fusion protein of the present application, and another anti-cancer agent and/or immunomodulatory agent, wherein the anti-cancer agent includes but is not limited to microtubule disrupting agents, antimetabolites, topoisomerase inhibitors, DNA intercalators, alkylating agents, hormone therapeutic agent, kinase inhibitors (e.g., tyrosine kinase inhibitors, including but not limited to EGFR inhibitors, HER2 inhibitors, HER3 inhibitors, IGFR inhibitors and Met inhibitors), receptor antagonists, tumor cell apoptosis activators (including but not limited to IAP inhibitors, Bcl2 inhibitors, MC11 inhibitors, TRAIL inhibitors, CHK inhibitors), or any anti-angiogenic drugs; the immunomodulatory agent includes but is not limited to PD-1, PD-L1, PD-L2, TIM3, CTLA-4, LAG-3, CD47, CEACAM-1, CEACAM-5, VISTA, BTLA, TIGIT, LAIR1, CD160, 2B4 or TGFR inhibitor.

In another aspect, the present application relates to a kit comprising an effective amount of the anti-IL-2 antibody or antigen-binding fragment thereof, or the hIL-2/anti-IL-2 antibody fusion protein of the present application, or comprising a pharmaceutical composition of the anti-IL-2 antibody or antigen-binding fragment thereof, or the hIL-2/anti-IL-2 antibody fusion protein of the present application.

*11. Treatment Methods and Uses*

In one aspect, the present application relates to use of the anti-IL-2 antibody or antigen-binding fragment thereof, or the hIL-2/anti-IL-2 antibody fusion protein herein in the preparation of a pharmaceutical composition or preparation for the treatment of a disease, wherein the disease includes, but is not limited to, proliferative disease, infectious disease, and immunodeficiency disease, wherein the proliferative disease includes, but is not limited to, cancer and other cell proliferative disorder.

Alternatively, the present application relates to the anti-IL-2 antibody or antigen-binding fragment thereof herein, or the hIL-2/anti-IL-2 antibody fusion protein, or a pharmaceutical combination comprising the same for use in the treatment of proliferative disease, or infectious disease, or immunodeficiency disease.

In some embodiments, the cancer includes, but is not limited to, melanoma, lung cancer, colorectal cancer, prostate cancer, breast cancer, ovarian cancer, cervical cancer, kidney cancer, liver cancer, brain cancer, esophageal cancer, gallbladder cancer, pancreatic cancer, gastric cancer, thyroid cancer, bladder cancer, and lymphoma.

In some embodiments, the other cell proliferative disorder includes, but is not limited to, hypergammaglobulinemia, lymphoproliferative disorder, paraproteinemias, purpura, sarcoidosis, sezary syndrome, Walderström macroglobulinemia, histiocytosis, and any cell proliferative disorder located outside of neoplasia in, for example, abdomen, bone, breast, digestive system, liver, pancreas, peritoneum, endocrine glands (e.g., adrenal gland, parathyroid, pituitary, testis, ovary, thymus, thyroid), eyes, head and neck, nervous system, lymphatic system, pelvis, skin, soft tissues, spleen, and genitourinary system.

In some embodiments, the immunodeficiency disease includes, but is not limited to, HIV positivity, immunosuppressive disease, and chronic infection.

In another aspect, the present application relates to a method of treating a disease, condition or disorder by boosting host immune response, wherein the method comprises administering to a subject an effective amount of the anti-IL-2 antibody or antigen-binding fragment thereof, or hIL-2/anti-IL-2 antibody fusion protein of the present application, or a pharmaceutical composition comprising the anti-IL-2 antibody or antigen-binding fragment thereof, or the hIL-2/anti-IL-2 antibody fusion protein of the present application, or a kit or kit set comprising the anti-IL-2 antibody or antigen-binding fragment thereof, or the hIL-2/anti-IL-2 antibody fusion protein of the present application, to specifically activate effector cells expressing the IL2Rβ/γ (e.g., CD8$^+$ T cells and NK cells), thereby improving disease status.

In another aspect, the present application relates to a method to stimulate the immune system, wherein the method comprises administering to a subject an effective amount of the anti-IL-2 antibody or antigen-binding fragment thereof, or hIL-2/anti-IL-2 antibody fusion protein of the present application, or a pharmaceutical composition comprising the anti-IL-2 antibody or antigen-binding fragment thereof, or hIL-2/anti-IL-2 antibody fusion protein of the present application, or a kit set or kit comprising the anti-IL-2 antibody or antigen-binding fragment thereof, or hIL-2/anti-IL-2 antibody fusion protein of the present application, to stimulate the immune system, wherein the immune system stimulation includes a general increase in immunity, an increase in T cell function, an increase in B cell function, a recovery of lymphocyte function, an increase in IL2Rβ/γ expression, an increase in T cell responsiveness, an increase in natural killer cell activity or lymphokine-activated killer cell activity, an increase in the ratio of CD8$^+$ T cells to CD4$^+$ T cells in the subject, etc..

In another aspect, the present application relates to a method of treating, alleviating or preventing a disease condition in which stimulation of the host immune system is beneficial, wherein the method comprises administering to a subject a therapeutically effective amount of the anti-IL-2 antibody or antigen-binding fragment thereof, or the hIL-2/anti-IL-2 antibody fusion protein of the present application, or a pharmaceutical composition comprising the anti-IL-2 antibody or antigen-binding fragment thereof, or the hIL-2/anti-IL-2 antibody fusion protein of the present application, or a kit or kit set comprising the anti-IL-2 antibody or antigen-binding fragment thereof, or the hIL-2/anti-IL-2 antibody fusion protein of the present application, to improve a disease condition by enhancing cellular immune responses in the subject, wherein the disease condition includes insufficient immune response or immunodeficiency in the host, and further the disease condition is a proliferative disease, or an infectious disease, or an immunodeficiency disease, wherein the proliferative disease includes cancer and other cell proliferative disorders.

In some embodiments, the cancer is selected from squamous cell carcinoma (e.g., epithelial squamous cell carcinoma), lung cancer (including small cell lung cancer, non-small cell lung cancer, adenocarcinoma of the lung, and squamous cell carcinoma of the lung), peritoneal cancer, hepatocellular carcinoma, gastric cancer (including gastrointestinal cancer and gastrointestinal stromal cancer), pancreatic cancer, glioblastoma, cervical cancer, ovarian cancer, liver cancer, bladder cancer, urethra cancer, liver tumor, breast cancer, colon cancer, rectal cancer, colorectal cancer, endometrial or uterine cancer, salivary gland cancer, kidney cancer, prostate cancer, vulvar cancer, thyroid cancer, anal cancer, penile cancer, melanoma, superficial spreading melanoma, lentigo maligna melanoma, acral melanoma, nodular melanoma, multiple myeloma and B-cell lymphoma, Non-Hodgkin Lymphoma (NHL), chronic lymphocytic leukemia (CLL), acute lymphoblastic leukemia (ALL), hairy cell leukemia, chronic myeloblastic leukemia, and post-transplant lymphoproliferative disorder (PTLD), as well as abnormal vascular proliferation associated with phakomatoses, edema (e.g., associated with brain tumor) and Meigs syndrome, brain tumor and brain cancer, and head and neck cancer.

In some embodiments, the method of treating, alleviating or preventing a disease condition in which stimulation of the host immune system is beneficial further comprises administering an immune checkpoint inhibitor, an antigen-specific immunotherapeutic agent, a chemotherapeutic agent, an epigenetic modifier, a cytokine, a growth factor, an inhibitor, an antibody targeting tumor antigen, a tumor vaccine, and/or other cancer therapy. In one embodiment, the immune checkpoint includes, but is not limited to, PD-1, PD-L1, PD-L2, TIM3, CTLA-4, LAG-3, CEACAM-1, CEACAM-5, VISTA, BTLA, TIGIT, LAIR1, CD160, 2B4, or TGFR. In one embodiment, the antigen-specific immunotherapeutic agent includes chimeric antigen receptor T cell (CAR-T cell), chimeric antigen receptor NK cell (CAR-NK cell), and/or tumor infiltrating lymphocyte (TIL). In one embodiment, the other cancer therapy includes, but is not limited to, surgery, chemotherapy, cytotoxic agent, photodynamic therapy, immunomodulation or radiation therapy.

In one embodiment, the anti-IL-2 antibody or antigen-binding fragment thereof, or the hIL-2/anti-IL-2 antibody fusion protein of the present application, or a pharmaceutical composition comprising the anti-IL-2 antibody or antigen-binding fragment thereof, or the hIL-2/anti-IL-2 antibody fusion protein of the present application can be combined with other therapeutic agents (e.g., immune checkpoint inhibitors, antigen-specific immunotherapeutic agents, chemotherapeutic agents, epigenetic modifiers, cytokines, growth factors, inhibitors, antibodies targeting tumor antigen, and/or tumor vaccines) and can be administered simultaneously, sequentially, or in reverse order.

The present application is further illustrated by the following examples, which should not be construed as further limiting. The contents of all drawings and all references, patents and patent applications cited throughout the present application are expressly incorporated herein by reference.

**EXAMPLES**

*Example 1: Modification of Human IL-2 (hIL-2)*

1.1 Screening, Construction and Expression of IL-2 Muteins

Analysis using MOE (Molecular Operating Environment) software showed that the tertiary structure of mIL-2 and hIL-2 was almost identical; there were only two significant differences at key interaction sites with the rat anti-mouse IL-2 antibody S4B6 (S4B6), which were lysine at the position 64 in hIL-2 (hIL-2/K64) corresponding to glycine at the position 78 in mIL-2 (mIL-2/G78) and asparagine at the position 90 in hIL-2 (hIL-2/N90) corresponding to arginine at the position 105 in mIL-2 (mIL-2/R105). The differences at these two sites may hinder the binding of hIL-2 to the S4B6 antibody, of which the site hIL-2/N90 is particularly important as its corresponding mIL-2/R105 can form multiple interaction bonds with S4B6, such as salt bond and hydrogen bond, and hIL-2/K64 can form a significant hindrance/clash with the light chain CDR3 of S4B6. In addition, compared with glutamic acid at the position 119 in mIL-2 (mIL-2/E119), the corresponding methionine residue at the position 104 in hIL-2 (hIL-2/M104) has a relatively long side chain which may interfere its binding with the antibody.

Based on above analysis results, a hIL-2 mutein containing two point mutations K64G and N90R (with a his-tag, named hIL2-DM-his) and a hIL-2 mutein containing three point mutations K64G, N90R and M104E (with a his-tag, named hIL2-TM-his) were constructed and expressed, and the wild-type mouse IL-2 (with a his-tag, named mIL2-his) was used as the control. The method of construction, expression and purification of the hIL-2 muteins was described as follows: the gene sequence encoding wild-type hIL-2 was obtained through gene synthesis, and the hIL-2 was then subjected to site-directed mutagenesis at K64G, N90R and/or M104E using PCR; DNA fragments encoding hIL-2 containing different site mutations were inserted into the pcDNA3.1 expression vector, transiently transfected into ExpiCHO-S cells (Thermo, A29127) and expressed by culturing cells in serum-free media, and the hIL-2 mutein proteins were purified by nickel-column affinity chromatography with AKTA purification system.

1.2 Determination of Binding Kinetics of hIL-2 Muteins with chS4B6

The binding kinetics of above three IL-2 antigens to S4B6 chimeric antibody (chS4B6, consisting of the variable region of S4B6 antibody and the constant region of human IgG1) was determined using a molecular interaction analyzer (ForteBio, Model R8). The specific method was as follows: the chS4B6 antibody was diluted to 5 μg/mL in 1×PBS and added to a 96-well black-wall plate at 200 μL per well; the antigen to be tested was serially diluted in 1×PBS at 1:2 dilution starting from 100 nM, and added to the 96-well black-wall plate at 200 μL per well. The 96-well black-wall plate containing testing samples was uploaded onto the analyzer and detected by a Protein A sensor placed in the analyzer with a program inputted to allow the Protein A sensor to bind to the chS4B6 antibody and then the testing antigens to be detected by an association-dissociation cycle. After each cycle of association and dissociation steps, the sensor was regenerated by 10 mM glycine buffer (pH 1.5) and then stated with the next cycle of detection. After the detection, the association rate constant and dissociation rate constant between the antibody and antigen were fitted and extrapolated using the analytical software, and the value of affinity constant ($K_D$) was calculated.

The detection results are shown in Table 5, indicating that hIL-2 muteins with the designed mutations can bind to chS4B6.

Table 5. Results of binding kinetics of chS4B6 antibody to antigens

| Antibody:Antigen | $k_{off}$(1/s) | $k_{on}$(1/Ms) | $K_D$(M) | Full $R^2$ |
|---|---|---|---|---|
| chS4B6:hIL2-DM-his | $1.060\times10^{-2}$ | $2.691\times10^5$ | $3.939\times10^{-8}$ | 0.9968 |
| chS4B6:hIL2-TM-his | $4761\times10^{-3}$ | $2.104\times10^5$5 | $2.263\times10^{-8}$ | 0.9955 |
| chS4B6:mIL2-his | $1.823\times10^{-4}$ | $1.829\times10^5$ | $9.964\times10^{-10}$ | 0.9995 |
| (Full $R^2$ represents the goodness-of-fit between the fitted curve and the measured curve) | | | | |

1.3 Detection of Biological Activity of hIL-2 Muteins

CTLL-2 cells (obtained from ATCC) and NK-92 cells (obtained from ATCC) were used to detect the biological activity of hIL-2 muteins, and the detection method was as follows: CTLL-2 cells or NK-92 cells grown at the exponential phase were collected by centrifugation at 1000 rpm and then resuspended in complete media without the hIL-2 supplement; cells were seeded in a 96-well white-wall cell culture plate at $1\times10^4$ cells per well. The testing samples (hIL-2 muteins, including hIL2-DM-his and hIL2-TM-his) and the control samples mIL2-his and hIL-2 (Novoprotein) were diluted in media to 500 nM (for CTLL-2 cells) or 50 nM (for NK-92 cells), and then subjected to 1:5 serial dilution; the diluted samples were added to the 96-well white-wall cell culture plate containing the cells at 20 µL per well, and mixed by tapping gently. The cell culture plate was incubated at 37°C/5% $CO_2$ for 3 days and then removed from the incubator to allow cooling to room temperature. A detection reagent, Cell Counting-Lite 2.0 (Nanjing Vazyme), was added to each well. After shaking and incubating for 10 mins, the cell culture plate was detected by a multimode microplate reader (Thermo, Varioskan). The chemiluminescence value of each well was read, and the ratio of the sample well to the control well was calculated.

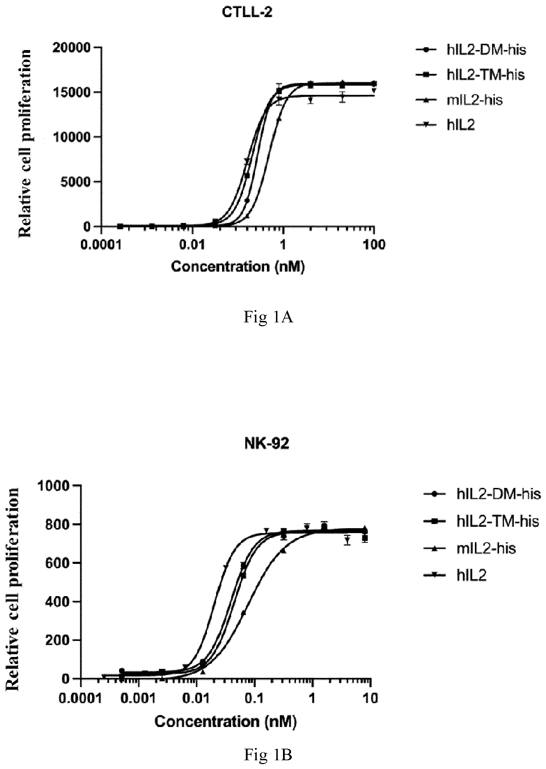

As shown in Figure 1 and Table 6, both hIL2-DM-his and hIL2-TM-his can promote the proliferation of CTLL-2 cells and NK-92 cells, and their activities (represented by EC50) are comparable to the hIL-2 control, indicating that the hIL-2 muteins of the present application retain the biological activity of hIL-2.

Table 6. Biological activity of hIL-2 muteins determined by CTLL-2 and NK-92 cell proliferation

| Sample ID | CTLL-2 Cell Proliferation Activity (EC50, nM) | NK-92 Cell Proliferation Activity (EC50, nM) |
|---|---|---|
| hIL2-DM-his | 0.2739 | 0.03904 |
| hIL2-TM-his | 0.209 | 0.04434 |
| mIL2-his | 0.4878 | 0.07761 |
| hIL-2 | 0.1615 | 0.02033 |
| (mIL-2-his and hIL-2 are control samples) | | |

Overall, by site-directed mutagenesis of key amino acid residues in the wild-type hIL-2 which are involved in the binding of the IL-2 antigen to the anti-IL-2 antibody, the present application has generated hIL-2 muteins that can bind to the anti-mIL-2 antibody S4B6 and retain the biological activity of hIL-2.

*Example 2: Humanization of the Rat Anti-mouse IL-2 Antibody S4B6*

To minimize the immunogenic reactions of host to the rat anti-mouse IL-2 antibody S4B6, humanization of S4B6 was performed to make it suitable for use in the human body.

2.1 CDR Grafting

The S4B6 antibody was humanized by CDR grafting. Briefly, based on the amino acid sequence of S4B6, CDR grafting was performed according to the germlines recommended by MOE software, wherein blast alignment was performed on the light and heavy chain variable regions of S4B6 with those in the human immunoglobulin gene database, and human germlines of IGVH and IGVK with the highest homology to S4B6 antibody were selected and served as acceptor frameworks for humanization. The CDRs of the heavy and light chain variable regions of the S4B6 antibody were grafted into the corresponding human germline framework regions, and the amino acid sequences of four humanized hS4B6 antibodies were obtained and shown in Table 7.

Table 7. Humanization of anti-mouse IL-2 antibody S4B6 and analysis of their sequences

| Antibody ID | Heavy Chain ID | Heavy Chain Germline | Heavy Chain Homology | Light Chain ID | Light Chain Germline | Light Chain Homology |
|---|---|---|---|---|---|---|
| S4B6 | S4B6 | IGHV1-69-2*01 | 62% | S4B6 | IGKV1-39*01 | 66% |
| H1K1 | H1 | IGHV4-59*02 | 98% | K1 | IGKV3-20*01 | 98% |
| H3K3 | H3 | IGHV2-5*02 | 96% | K3 | IGKV3-11*01 | 98% |
| H6K3 | H6 | IGHV1-69-2*01 | 96% | K3 | IGKV3-11*01 | 98% |
| H6K7 | H6 | IGHV1-69-2*01 | 96% | K7 | IGKV1-39*01 | 98% |

Gene sequences encoding the humanized antibodies described in Table 7 were synthesized. Each of the coding sequences was inserted into the pcDNA3.1 expression vector, and transiently transfected into the ExpiCHO-S cells for expression by culturing the cells in serum-free media, and the humanized antibodies were purified by Protein A affinity chromatography with AKTA purification system. After the humanized antibodies listed in Table 7 were obtained, their binding activity and binding affinity to the antigen mIL2-his or hIL2-his were detected by ELISA and ForteBio kinetic analysis, respectively.

2.1.1 Analysis of Binding Activity of hS4B6 Humanized Antibodies to Antigen mIL2-his or hIL2-his by ELISA

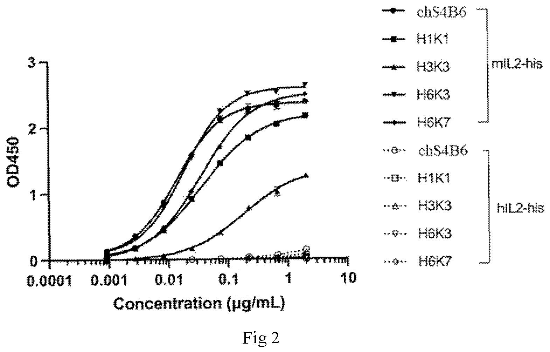

ELISA was performed as follows: a goat anti-human IgG (Jackson Immunoresearch) was diluted to 1 $\mu$g/mL in 1×PBS and added to an ELISA plate at 100 $\mu$L/well, and then incubated at 4°C overnight. The plate was washed with PBS and then blocked at room temperature for 1 hour after adding the blocking solution (PBST containing 1% BSA) to each well. The plate was washed with PBST, and the testing samples (including the humanized antibodies of hS4B6 and the control chimeric antibody chS4B6) were added to each well at a final concentration of 13.3 nM and incubated at room temperature for 2 hours. The plate was washed with PBST, and blocking solution containing different concentrations of IL-2 antigen (mIL2-his or hIL2-his) prepared by serial dilution was added to each well at 100 $\mu$L and incubated at room temperature for 2 hours. The plate was washed with PBST; the HRP-labeled anti-6×His-tag secondary antibody (Proteintech) diluted at a ratio of 1:10,000 in blocking solution was then added to each well and incubated at room temperature for 1 hour. The plate was washed with PBST; the TMB chromogenic solution (Thermo) was added to each well and incubated in dark for 3-10 minutes at room temperature. The stop solution (2 M HCl) was added to each well, and OD values at a wavelength of 450-570 nm were read using a multimode microplate reader (Varioskan, Thermo).
As shown in Figure 2 and Table 8, among the humanized anti-IL-2 antibodies, the binding activity of the humanized antibody H6K3 to mIL-2 is comparable to that of chS4B6, and the binding activities of H1K1 and H6K7 to mIL-2 are slightly lower than that of chS4B6. These data suggest that the three humanized antibodies retain the binding activity of the murine antibody S4B6 to mIL-2, of which the binding activity of H6K3 to mIL-2 is the highest and H1K1 and H6K7 come as the second.

Table 8. Binding activity of S4B6 humanized antibodies (hS4B6) to antigen mIL2-his detected by ELISA

|  | chS4B6 | hS4B6 | | | |
| --- | --- | --- | --- | --- | --- |
|  |  | H1K1 | H3K3 | H6K3 | H6K7 |
| EC50 ($\mu$g/mL) | 0.01388 | 0.03569 | 0.1851 | 0.01832 | 0.03565 |

2.1.2 Determination of Binding Kinetics of the hS4B6 Humanized Antibodies to Antigen mIL2-his

The binding kinetics of the hS4B6 humanized antibodies and the control chimeric antibody chS4B6 to the antigen mIL2-his were determined using the method described in Section 1.2 of Example 1. The results are shown in Table 9. Among the humanized anti-IL-2 antibodies, H6K3 and H6K7 can bind to mIL-2 at a relatively high affinity, and H1K1 and H3K3 bind to mIL-2 at a moderate affinity, indicating that all humanized hS4B6 antibodies can specifically bind to mIL-2.

Table 9. Results of binding kinetics of hS4B6 humanized antibodies to antigen

| Antibody : Antigen | $k_{off}$ (1/s) | $k_{on}$ (1/Ms) | $K_D$ (M) | Full $R^2$ |
| --- | --- | --- | --- | --- |
| chS4B6:mIL2-his | $1.823 \times 10^{-4}$ | $1.829 \times 10^5$ | $9.964 \times 10^{-10}$ | 0.9995 |
| H1K1:mIL2-his | $7.186 \times 10^{-4}$ | $2.769 \times 10^4$ | $2.595 \times 10^{-8}$ | 0.9993 |
| H3K3:mIL2-his | $2.646 \times 10^{-3}$ | $2.005 \times 10^5$ | $1.320 \times 10^{-8}$ | 0.8133 |
| H6K3:mIL2-his | $1.799 \times 10^{-4}$ | $1.792 \times 10^5$ | $1.004 \times 10^{-9}$ | 0.9993 |
| H6K7:mIL2-his | $1.212 \times 10^{-3}$ | $8.638 \times 10^5$ | $1.403 \times 10^{-9}$ | 0.9984 |
| (Full $R^2$ represent the goodness-of-fit between the fitted curve and the measured curve) | | | | |

2.1.3 Determination of Binding Affinity of the Humanized Antibody H6K3 to IL-2 Muteins

The binding kinetics of humanized antibody H6K3 to hIL-2 muteins (hIL2-DM-his or hIL2-TM-his) were determined using the method described in Section 1.2 of Example 1. As shown in Table 10, the humanized antibody H6K3 exhibits decent binding kinetics to both hIL-2 muteins, and there is no significant difference in the binding affinity of H6K3 to hII,2-DM-his and hIL2-TM-his. As shown in Figure 2, the humanized antibody H6K3 does not bind to hIL-2, demonstrating that the antigen hIL-2 muteins acquire the ability to bind to the anti-IL-2 antibody or antigen-binding fragment thereof.

Table 10. Binding kinetics of humanized antibody H6K3 to IL-2 muteins

| Antibody : Antigen | $k_{off}$(1/s) | kon (1 /Ms) | $K_D$(M) | Full $R^2$ |
|---|---|---|---|---|
| H6K3:hIL2-DM-his | $1.005 \times 10^{-2}$ | $2.220 \times 10^5$ | $4.529 \times 10^{-8}$ | 0.9976 |
| H6K3:hIL2-TM-his | $5.688 \times 10^{-3}$ | $2.372 \times 10^4$ | $2.398 \times 10^{-8}$ | 0.9963 |
| (Full $R^2$ represents the goodness-of-fit between the fitted curve and the measured curve) | | | | |

2.2 Humanization of hS4B6-H6K3 Antibody

To further improve the humanness of the antibody described above, amino acid residues in the light and heavy chain CDR sequences of H6K3 with relatively low-frequency in human antibodies and the murine-derived amino acid residues in FR regions were identified using the online tool abYsis (http://abysis.org/abysis/index.html) and MOE software, and these amino acid residues were optimized for further humanization. The mutation sites for further humanization are listed in Table 11.

Table 11. Mutations selected for improving the humanness of H6K3 antibody

| Further Humanized Antibody ID | Amino Acid Position | Mutation |
|---|---|---|
| H6K3-hu01 | H27 | D27Y |
| H6K3-hu02 | H29 | I29F |
| H6K3-hu03 | H31 | A31D |
| H6K3-hu04 | H34 | L34M |
| H6K3-hu05 | H48 | I48M |
| H6K3-hu06 | H65 | N65S |
| H6K3-hu07 | H65 | N65G |
| H6K3-hu08 | H76 | N76D |
| H6K3-hu09 | L56 | D56S |
| H6K3-hu10 | L56 | D56T |
| H6K3-hu11 | L66 | R66G |
| H6K3-hu12 | L71 | Y71F |

Twelve further-humanized antibodies listed in Table 11 were constructed and expressed according to the method described in Section 2.1 of Example 2, and all of these antibodies demonstrated a high level of expression. These 12 further-humanized antibodies were purified, and their binding kinetics to the antigen hIL2-DM-his were determined using the method described in Section 1.2 of Example 1. As shown in Table 12, the further-humanized antibodies H6K3-hu04, H6K3-hu06, H6K3-hu07, H6K3-hu08, H6K3-hu09, H6K3-hu10 and H6K3-hu12 can bind to hIL2-DM-his at a relatively high binding affinity, indicating that these further-humanized antibodies can specifically bind to the hIL-2 mutein hIL2-DM.

Table 12. Results of binding kinetics of hS4B6-H6K3 further-humanized antibodies to antigen hIL2-DM-his

| Antibody : Antigen | $k_{off}$ (1/s) | $k_{on}$ (1/Ms) | $K_D$ (M) | Binding Response (nm) |
|---|---|---|---|---|
| H6K3-hu01:hIL2-DM-his | / | / | / | No binding |
| H6K3-hu02:hIL2-DM-his | / | / | / | No binding |
| H6K3-hu03 :hIL2-DM-his | / | / | / | No binding |
| H6K3-hu04:hIL2-DM-his | $1.486\times10^{-2}$ | $4.298\times10^{5}$ | $3.457E\times10^{-8}$ | 0.1429 |
| H6K3-hu05 :hIL2-DM-his | $1.525\times10^{-2}$ | $3.874\times10^{5}$ | $3.937\times10^{-8}$ | 0.1387 |
| H6K3-hu06:hIL2-DM-his | $1.917\times10^{-2}$ | $3.647\times10^{5}$ | $5.255\times10^{-8}$ | 0.1345 |
| H6K3-hu07:hIL2-DM-his | $1.811\times10^{-2}$ | $4.001\times10^{5}$ | $4.528\times10^{-8}$ | 0.1296 |
| H6K3-hu08:hIL2-DM-his | $2.068\times10^{-2}$ | $3.559\times10^{5}$ | $5.810\times10^{-8}$ | 0.1269 |
| H6K3-hu09:hIL2-DM-his | $8.673\times10^{-3}$ | $3.669\times10^{5}$ | $2.364\times10^{-8}$ | 0.1804 |
| H6K3-hu10:hIL2-DM-his | $1.055\times10^{-2}$ | $3.725\times10^{5}$ | $2.833\times10^{-8}$ | 0.1716 |
| H6K3-hu11:hIL2-DM-his | / | / | / | No binding |
| H6K3-hu12:hIL2-DM-his | $1.736\times10^{-2}$ | $5.368\times10^{5}$ | $3.233\times10^{-8}$ | 0.1517 |
| H6K3:hIL2-DM-his | $1.935\times10^{-2}$ | $4.130\times10^{5}$ | $4.686\times10^{-8}$ | 0.1330 |

Based on the results shown in Table 12, combinatorial mutations of H6K3-hu04 (the mutation L34M), H6K3-hu07 (the mutation N65G) and H6K3-hu08 (the mutation N76D) were introduced into the heavy chain variable region of the further-humanized antibody, while the combinatorial mutations of H6K3-hu09 (the mutation D56S) and H6K3-hu12 (the mutation Y71F) were introduced into the light chain variable region. The humanized antibody comprising these combinatorial mutations was named as hS4B6-H6K3(H), and the risk of Asp isomerization in this humanized antibody was also significantly reduced.

The hS4B6-H6K3(H) antibody was constructed, expressed and purified according to the method described in Section 2.1 of Example 2, and the binding kinetics of hS4B6-H6K3(H) to the antigen hIL2-DM-his, hIL2-TM-his, or the control mIL2-his were determined using the method described in Section 1.2 of Example 1. As shown in Table 13, the hS4B6-H6K3(H) antibody not only has an increased humanness score, but also retains the high binding specificity to the hIL-2 muteins.

Table 13. Results of the antigen-binding kinetics of hS4B6-H6K3(H) antibody

| Antibody : Antigen | $k_{off}$(1/s) | $k_{on}$(1/Ms) | $K_D$(M) | Full $R^2$ |
|---|---|---|---|---|
| hS4B6-H6K3(H):mIL2-his | $2.359\times10^{-4}$ | $9.277\times10^{5}$ | $2.543\times10^{-10}$ | 0.9998 |
| hS4B6-H6K3(H):hIL2-DM-his | $3.843\times10^{-3}$ | $2.421\times10^{4}$ | $1.587\times10^{-8}$ | 0.9971 |
| hS4B6-H6K3(H):hIL2-TM-his | $2.702\times10^{-3}$ | $2.735\times10^{5}$ | $9.879\times10^{-9}$ | 0.9963 |
| (Full $R^2$ represents the goodness-of-fit between the fitted curve and the measured curve.) | | | | |

*Example 3: Affinity Maturation of hS4B6-H6K3(H) Antibody*

To enhance the binding affinity of hS4B6-H6K3(H) antibody to the hIL-2 mutein, site-directed mutagenesis in the heavy chain CDR regions and light chain CDR regions of hS4B6-H6K3(H) antibody was designed based on the computational simulation performed using the MOE software and the resolved crystal structure data of the S4B6:mIL-2 antibody-antigen complex. The hS4B6-H6K3(H) antibody mutants with single-site mutations in its heavy chain CDRs or its light chain CDRs were constructed, expressed and purified according to the method described in Section 2.1 of Example 2. The binding kinetics of hS4B6-H6K3(H) antibody mutants with single-site mutations to the antigen hIL2-DM-his were determined using the method described in Section 1.2 of Example 1. It was found that the hS4B6-H6K3(H) antibody mutants comprising the following single-site mutations exhibited an enhanced binding affinity to the hIL-2 mutein as compared to their parental antibody hS4B6-H6K3(H): E53D, V95I, Y98L, or S100T in the heavy chain, and D28N, D28H, D28L, D28Y, G30I, G30S, G30T, S50Q, or T52G in the light chain.

The selected single-site mutations in the light and heavy chain CDR regions were combined to generate affinity-matured humanized antibodies, which were constructed and expressed according to the method described in Section 2.1 of Example 2. The binding kinetics of hS4B6-H6K3(H) and/or its affinity-matured antibodies to the antigen hIL2-DM-his were determined using the method described in Section 1.2 of Example 1, and results are presented in Table 14. By selecting the following mutation(s): D28Y, or D28Y/S50Q, or D28Y/G30S, or D28Y/G30S/S50Q in the light chain CDR regions, or its combination with various mutations in the heavy chain CDR regions of the parental antibody hS4B6-H6K3(H), the obtained affinity-matured antibodies exhibited significantly enhanced binding affinity to the hIL-2 mutein. The binding affinity (dissociation equilibrium constant, $K_D$) of the affinity-matured hS4B6-H6K3(H) antibodies to the hIL-2 mutein showed reaching $10^{-9}$ M, even $10^{-10}$ M.

Table 14. Results of binding kinetics of hS4B6-H6K3(H) affinity-matured antibodies to antigen hIL2-DM-his

| hS4B6-H6K3(H) Affinity-matured Antibody | | | $K_D$ (M) | $k_a$ (1/Ms) | $k_{dis}$ (1/s) | Full $R^2$ |
|---|---|---|---|---|---|---|
| Antibody ID | Heavy Chain CDR Mutation | Light Chain CDR Mutation | | | | |
| K1G1 | None | D28Y+G30S+S50Q | $1.433 \times 10^{-9}$ | $3.427 \times 10^5$ | $4.911 \times 10^{-4}$ | 0.9134 |
| K1G2 | V95I | | $1.537 \times 10^{-9}$ | $3.372 \times 10^5$ | $5.181 \times 10^{-4}$ | 0.9092 |
| K1G3 | E53D | | $1.353 \times 10^{-9}$ | $3.978 \times 10^5$ | $5.381 \times 10^{-4}$ | 0.8953 |
| K1G4 | Y98L | | $1.403 \times 10^{-9}$ | $3.637 \times 10^5$ | $5.102 \times 10^{-4}$ | 0.9161 |
| K1G5 | S100T | | $1.472 \times 10^{-9}$ | $4.253 \times 10^5$ | $6.262 \times 10^{-4}$ | 0.8891 |
| K1G6 | V95I+E53D | | $1.076 \times 10^{-9}$ | $3.897 \times 10^5$ | $4.193 \times 10^{-4}$ | 0.8981 |
| K1G7 | V95I+S100T | | $1.375 \times 10^{-9}$ | $4.527 \times 10^5$ | $6.225 \times 10^{-4}$ | 0.8598 |
| K1G8 | V95I+Y98L | | $7.782 \times 10^{-10}$ | $3.737 \times 10^5$ | $2.909 \times 10^{-4}$ | 0.9157 |
| K1G9 | V95I+S100T+Y98L | | $8.564 \times 10^{-10}$ | $3.331 \times 10^5$ | $2.852 \times 10^{-4}$ | 0.9272 |
| K1G10 | V95I+E53D+S100T | | $9.571 \times 10^{-10}$ | $3.887 \times 10^5$ | $3.720 \times 10^{-4}$ | 0.8895 |
| K1G11 | V95I+E53D+Y98L | | $9.284 \times 10^{-10}$ | $4.012 \times 10^5$ | $3.725 \times 10^{-4}$ | 0.9037 |
| K1G12 | V95I+S100T+Y98L+E53D | | $9.267 \times 10^{-10}$ | $4.278 \times 10^5$ | $3.964 \times 10^{-4}$ | 0.8976 |

(continued)

| hS4B6-H6K3(H) Affinity-matured Antibody | | | $K_D$ (M) | $k_a$ (1/Ms) | $k_{dis}$ (1/s) | Full $R^2$ |
|---|---|---|---|---|---|---|
| Antibody ID | Heavy Chain CDR Mutation | Light Chain CDR Mutation | | | | |
| K2G1 | None | D28Y+G30S | $1.534 \times 10^{-9}$ | $4.413 \times 10^5$ | $6.769 \times 10^{-4}$ | 0.9167 |
| K2G2 | V95I | | $1.326 \times 10^{-9}$ | $4.238 \times 10^5$ | $5.620 \times 10^{-4}$ | 0.9141 |
| K2G3 | E53D | | $1.318 \times 10^{-9}$ | $3.886 \times 10^5$ | $5.122 \times 10^{-4}$ | 0.9038 |
| K2G4 | Y98L | | $1.081 \times 10^{-9}$ | $4.206 \times 10^5$ | $4.547 \times 10^{-4}$ | 0.917 |
| K2G5 | S100T | | $1.396 \times 10^{-9}$ | $3.963 \times 10^5$ | $5.531 \times 10^{-4}$ | 0.9138 |
| K2G6 | V95I+E53D | | $1.331 \times 10^{-9}$ | $4.456 \times 10^5$ | $5.932 \times 10^{-4}$ | 0.8858 |
| K2G7 | V95I+S100T | | $1.355 \times 10^{-9}$ | $3.610 \times 10^5$ | $4.892 \times 10^{-4}$ | 0.9085 |
| K2G8 | V95I+Y98L | | $1.133 \times 10^{-9}$ | $4.476 \times 10^5$ | $5.069 \times 10^{-4}$ | 0.9043 |
| K2G9 | V95I+S100T+Y98L | | $9.241 \times 10^{-10}$ | $4.621 \times 10^5$ | $4.270 \times 10^{-4}$ | 0.8997 |
| K2G10 | V95I+E53D+S100T | | $9.734 \times 10^{-10}$ | $4.293 \times 10^5$ | $4.179 \times 10^{-4}$ | 0.8973 |
| K2G11 | V95I+E53D+Y98L | | $5.442 \times 10^{-10}$ | $4.586 \times 10^5$ | $2.496 \times 10^{-4}$ | 0.919 |
| K2G12 | V95I+S100T+Y98L+E53D | | $7.437 \times 10^{-10}$ | $4.647 \times 10^5$ | $3.456 \times 10^{-4}$ | 0.9004 |

(continued)

| | hS4B6-H6K3(H) Affinity-matured Antibody | | | | | |
| Antibody ID | Heavy Chain CDR Mutation | Light Chain CDR Mutation | $K_D$ (M) | $k_a$ (1/Ms) | $k_{dis}$ (1/s) | Full $R^2$ |
|---|---|---|---|---|---|---|
| K3G1 | None | D28Y+S50Q | $4.401 \times 10^{-9}$ | $4.237 \times 10^{5}$ | $1.865 \times 10^{-3}$ | 0.9138 |
| K3G2 | V95I | | $2.912 \times 10^{-9}$ | $4.067 \times 10^{5}$ | $1.184 \times 10^{-3}$ | 0.9023 |
| K3G3 | E53D | | $3.337 \times 10^{-9}$ | $4.002 \times 10^{5}$ | $1.335 \times 10^{-3}$ | 0.9084 |
| K3G4 | Y98L | | $2.999 \times 10^{-9}$ | $4.080 \times 10^{5}$ | $1.224 \times 10^{-3}$ | 0.9206 |
| K3G5 | S100T | | $3.722 \times 10^{-9}$ | $3.335 \times 10^{5}$ | $1.241 \times 10^{-3}$ | 0.9173 |
| K3G6 | V95I+E53D | | $2.129 \times 10^{-9}$ | $3.966 \times 10^{5}$ | $8.442 \times 10^{-4}$ | 0.8889 |
| K3G7 | V95I+S100T | | $1.956 \times 10^{-9}$ | $3.356 \times 10^{5}$ | $6.563 \times 10^{-4}$ | 0.9154 |
| K3G8 | V95I+Y98L | | $1.502 \times 10^{-9}$ | $2.969 \times 10^{5}$ | $4.460 \times 10^{-4}$ | 0.9339 |
| K3G9 | V95I+S100T+Y98L | | $1.486 \times 10^{-9}$ | $3.559 \times 10^{5}$ | $5.288 \times 10^{-4}$ | 0.9297 |
| K3G10 | V95I+E53D+S100T | | $1.455 \times 10^{-9}$ | $4.018 \times 10^{5}$ | $5.848 \times 10^{-4}$ | 0.903 |
| K3G11 | V95I+E53D+Y98L | | $6.145 \times 10^{-10}$ | $3.911 \times 10^{5}$ | $2.403 \times 10^{-4}$ | 0.9318 |
| K3G12 | V95I+S100T+Y98L+E53D | | $1.102 \times 10^{-9}$ | $3.441 \times 10^{5}$ | $3.793 \times 10^{-4}$ | 0.9077 |

(continued)

| hS4B6-H6K3(H) Affinity-matured Antibody | | | | | | |
|---|---|---|---|---|---|---|
| Antibody ID | Heavy Chain CDR Mutation | Light Chain CDR Mutation | $K_D$ (M) | $k_a$ (1/Ms) | $k_{dis}$ (1/s) | Full $R^2$ |
| K4G1 | None | D28Y | $4.277 \times 10^{-9}$ | $3.521 \times 10^{5}$ | $1.506 \times 10^{-3}$ | 0.9424 |
| K4G2 | V95I | | $3.718 \times 10^{-9}$ | $3.835 \times 10^{5}$ | $1.426 \times 10^{-3}$ | 0.9326 |
| K4G3 | E53D | | $3.084 \times 10^{-9}$ | $3.511 \times 10^{5}$ | $1.083 \times 10^{-3}$ | 0.9373 |
| K4G4 | Y98L | | $2.251 \times 10^{-9}$ | $3.644 \times 10^{5}$ | $8.201 \times 10^{-4}$ | 0.9602 |
| K4G5 | S100T | | $2.699 \times 10^{-9}$ | $4.184 \times 10^{5}$ | $1.129 \times 10^{-3}$ | 0.949 |
| K4G6 | V95I+E53D | | $2.332 \times 10^{-9}$ | $4.264 \times 10^{5}$ | $9.943 \times 10^{-4}$ | 0.9287 |
| K4G7 | V95I+S100T | | $3.203 \times 10^{-9}$ | $3.958 \times 10^{5}$ | $1.268 \times 10^{-3}$ | 0.9128 |
| K4G8 | V95I+Y98L | | $2.490 \times 10^{-9}$ | $3.436 \times 10^{5}$ | $8.557 \times 10^{-4}$ | 0.9366 |
| K4G9 | V95I+S100T+Y98L | | $2.216 \times 10^{-9}$ | $3.916 \times 10^{5}$ | $8.679 \times 10^{-4}$ | 0.9157 |
| K4G10 | V95I+E53D+S100T | | $2.472 \times 10^{-9}$ | $3.669 \times 10^{5}$ | $9.071 \times 10^{-4}$ | 0.9118 |
| K4G11 | V95I+E53D+Y98L | | $1.564 \times 10^{-9}$ | $4.125 \times 10^{5}$ | $6.451 \times 10^{-4}$ | 0.9278 |
| K4G12 | V95I+S100T+Y98L+E53D | | $6.858 \times 10^{-10}$ | $4.759 \times 10^{5}$ | $3.264 \times 10^{-4}$ | 0.9269 |

(Full $R^2$ represents the goodness-of-fit between the fitted curve and the measured curve.)

The binding activity of a protein complex formed by hIL2-DM and the antibody K2G11 to IL2Rα and IL2Rβ was determined by a molecular interaction analyzer (ForteBio, Model Qke). The specific method was described as follows: the antibody K2G11 was diluted in 1×PBS to 5 μg/mL and added to a 96-well black-wall plate at 200 μL per well. The antigen hIL2-DM was diluted to 100 nM in 1×PBS and then added to the 96-well black-wall plate at 200 μL per well. The IL2Rα and IL2Rβ proteins were diluted to 1000 nM in 1×PBS and then subjected to 1:2 serial dilution, and then added to the 96-well black-wall plate at 200 μL per well. The 96-well black-wall plate containing the testing samples and a Protein A sensor were uploaded onto the analyzer, and a program was inputted to allow the Protein A sensor to immerse in the antibody K2G11 and then bind with the antigen hIL2-DM to saturation, followed by association-dissociation cycle with the CD25 and CD122 proteins. After each association-dissociation cycle, the sensor was regenerated by 10 mM glycine buffer (pH 1.5) and then started with the next cycle of detection.

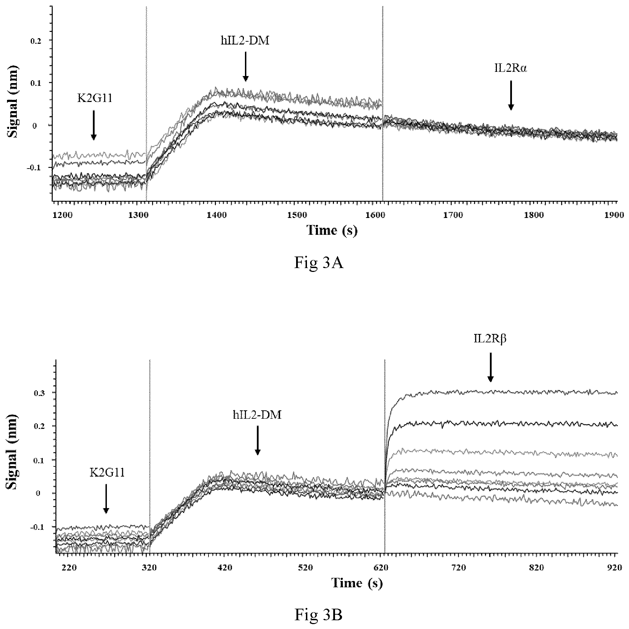

As shown in Figure 3, data demonstrated that the antibody K2G11 could block hIL2-DM from binding of hIL2Rα (Fig. 3A), whereas the interaction of hIL2-DM with IL2Rβ was not affected (Fig. 3B).

*Example 4: Construction and Characterization of IL-2/Anti-IL-2 Antibody Fusion Protein*

4.1 Screening of Linker

A computational simulation analysis was conducted using the MOE software to determine the position of linking hIL2-DM to the antibody hS4B6-H6K3(H), the type and length of linker, and to simulate whether the linker would affect the binding activity of hIL2-DM to hIL2Rβ/γ receptor. The results suggested that $(G4S)_4$, $(G4S)_3$, $(GAF)_2$ sequences could be selected as the linker, which could ensure the stability of internal structure of the fusion protein. In addition, when the linker connects the C-terminus of hIL2-DM and the N-terminus of the antibody heavy chain, it has little to no interference for hIL2-DM to interact with hIL2Rβ. However, when the linker connects the C-terminus of hIL2-DM and the N-terminus of the antibody light chain, it has significant interference for hIL2-DM to interact with hIL2Rβ and the production of fusion protein may also be affected. Furthermore, the $(G4S)_4$ linker has a low immunogenicity risk. Therefore, the linker with four tandem G4S sequences is selected to construct the IL-2/anti-IL-2 antibody fusion protein, wherein hIL2-DM is fused to the N-terminus of the antibody heavy chain through the linker.

4.2 Screening of Disulfide Linkage

To stabilize the interaction and avoid the dissociation of the antigen-antibody complex in the fusion protein, and thus prevent the IL-2 component in the fusion protein from being competitively bound by the high-affinity IL2R, the distance between amino acid residues in the crystal structure of S4B6/mIL-2 antigen-antibody complex was calculated using the MOE software and amino acid pairs with a spatial distance of 10 angstroms or less were identified. These amino acid pairs were mutated to cysteines so as to form disulfide linkage to further stabilize the interaction between the hIL-2 mutein component and the hS4B6-H6K3(H) antibody component in the fusion protein. Table 15 shows the amino acid pairs and their positions where the disulfide linkage may form between the hIL-2 mutein and the hS4B6-H6K3(H) antibody. The structure of designed fusion protein containing a disulfide linkage is schematically illustrated in Figure 4.

Table 15. Amino acid mutation sites where disulfide linkage may form between hIL-2 mutein and hS4B6-H6K3(H) antibody

| ID | Position of Amino Acid Pair | Amino Acid Residue in hIL2 | Amino Acid Residue in hS4B6-H6K3(H) Antibody | Location of Introducing Cysteine Residue Mutation in hS4B6-H6K3 (H) |
|---|---|---|---|---|
| IC-Cy01 | IL:61-L:30 | **E** 61 | **G** 30 | LCDR1 |
| IC-Cy02 | IL:61-L:31 | **E** 61 | **N** 31 | LCDR1 |
| IC-Cy03 | IL:61-L:50 | **E** 61 | **S** 50 | LCDR2 |
| IC-Cy04 | IL:104-L:52 | **M** 104 | **T** 52 | LCDR2 |
| IC-Cy05 | IL:102-L:67 | **T** 102 | **Su** 67 | LFR3 |
| IC-Cy06 | IL:102-L:68 | **T** 102 | **G** 68 | LFR3 |
| IC-Cy07 | IL:64-H:97 | **K** 64 | **F** 97 | HCDR3 |

(continued)

| ID | Position of Amino Acid Pair | Amino Acid Residue in hIL2 | Amino Acid Residue in hS4B6-H6K3(H) Antibody | Location of Introducing Cysteine Residue Mutation in hS4B6-H6K3 (H) |
|---|---|---|---|---|
| IC-Cy08 | IL:65-H:97 | P 65 | F 97 | HCDR3 |
| (IL represents IL-2, L represents the antibody light chain, H represents the antibody heavy chain, and the number after IL or L/H represents the corresponding position of amino acid mutation.) | | | | |

The constructs shown in Table 15 were constructed, expressed and purified according to the method described in Section 2.1 of Example 2, and obtained fusion proteins were analyzed by SEC-HPLC. Results showed that the hIL-2/anti-IL-2 antibody fusion proteins IC-Cy07 (i.e., H6K3(H)-Cy07) and IC-Cy08 (i.e., H6K3(H)-Cy08) were expressed as stable monomer, and they were selected for further analysis.

4.2.1 Determination of Binding Activity of IC-Cy07 and IC-Cy08 to hIL2Rα (hCD25) and IL2Rβ (hCD25) by ELISA

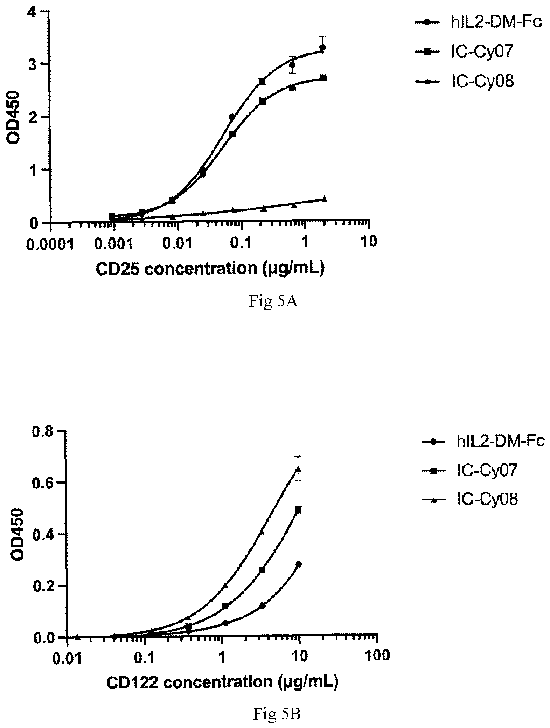

The binding activity of IC-Cy07 and IC-Cy08 to hCD25 and hCD122 was determined by ELISA according to the method described in Section 2.1.1 of Example 2, wherein the antigens were replaced with hCD25 (Aero) and hCD122 (Aero), respectively, and hIL2-DM-Fc protein was used as the control. The binding activity of the fusion proteins to hCD25 is shown in Figure 5A. Compared to the control hIL2-DM-Fc, IC-Cy08 barely binds to hCD25, whereas IC-Cy07 binds to hCD25 at a slightly reduced activity. The binding activity of the fusion proteins to hCD122 is shown in Figure 5B. Compared to the control hIL2-DM-Fc, the binding activity of both IC-Cy08 and IC-Cy07 to hCD122 is somewhat enhanced, which is consistent with the published reports suggesting that the C-helix of mIL-2 will have an allosteric shift after bound by the S4B6 antibody, resulting in an augmented binding to mCD122. In addition, the binding activity of IC-Cy08 to hCD122 is significantly higher than that of IC-Cy07 and hIL2-DM-Fc.

Overall, both IC-Cy08 and IC-Cy07 have an enhanced binding activity to hCD122, and the binding activity of IC-Cy08 to hCD25 is nearly completely lost, which further indicates that the $\alpha$ subunit binding site of hIL-2 mutein is fully embedded in the antibody fusion protein IC-Cy08, which cannot be readily dissociated and thus prevent the hIL-2 mutein component from binding by hCD25.

4.2.2 Analysis of Biological Activity of IC-Cy07 and IC-Cy08

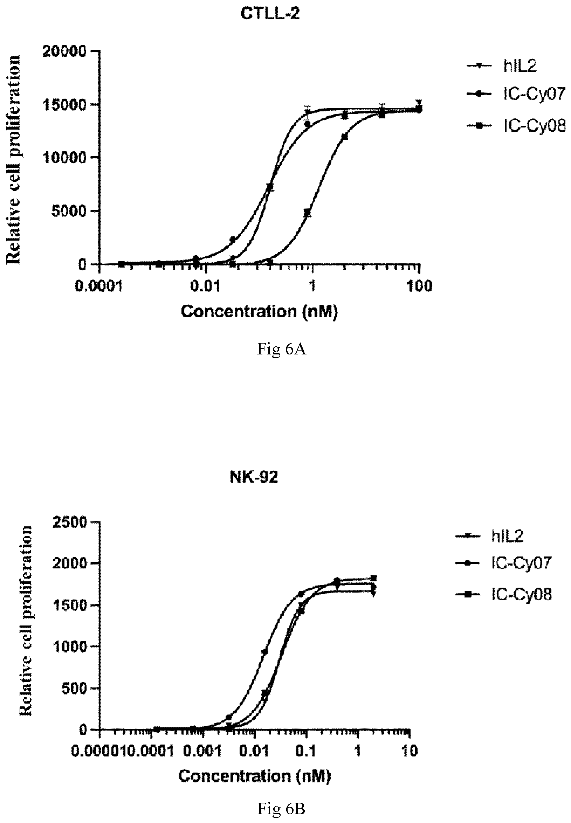

The biological activity of IC-Cy07 and IC-Cy08 was analyzed by cell proliferation assay with CTLL-2 cells and NK-92 cells according to the method described in Section 1.3 of Example 1, wherein wild-type hIL-2 was used as the control. As shown in Figure 6, the activity (EC50 value) of IC-Cy08 in promoting the proliferation of CTLL-2 cells which express a high level of CD25 is significantly lower than that of the hIL-2 control (Fig. 6A and Table 16), and the activity of IC-Cy08 in promoting the proliferation of NK-92 cells which express a minimal level of CD25 is comparable to that of the hIL-2 control (Fig. 6B and Table 16). The activity of IC-Cy07 in promoting the CTLL-2 cell proliferation is comparable to that of the hIL-2 control (Fig. 6A and Table 16), and the activity of IC-Cy07 in promoting NK-92 cell proliferation is slightly higher than that of the hIL-2 control (Fig. 6B and Table 16).

Table 16. Biological activity of IC-Cy07 and IC-Cy08 (hIL-2/anti-IL-2 antibody fusion proteins containing a disulfide linkage) determined by cell proliferation assay with CTLL-2 and NK-92 cells

| Sample ID | Proliferative Activity in CTLL-2 Cells (EC50, nM) | Proliferative Activity in NK-92 Cells (EC50, nM) |
|---|---|---|
| hIL-2 | 0.1615 | 0.03056 |
| IC-Cy07 | 0.1494 | 0.01481 |
| IC-Cy08 | 1.316 | 0.03399 |
| (hIL-2 is served as the control.) | | |

4.2.3 Effect of IC-Cy08 on IL-2 Signaling Pathway in CTLL-2 Cells Detected by Flow Cytometry

Binding of IL-2 to IL2R can lead to the activation of intracellular JAK kinase, which in turn phosphorylates and activates signal transduction and activator of transcription 5 (STATS) to regulate the expression of a variety of genes involved in cellular processes (e.g., proliferation, survival and apoptosis). The effect of IC-Cy08, an IL-2/anti-IL-2 antibody fusion protein containing a disulfide linkage, on the IL-2-activated signaling pathway was determined by evaluating the phosphorylation of STATS in CTLL2 cells which express the high-affinity IL2R. The specific method was described as follows: CTLL-2 cells were collected by centrifugation and resuspended in the complete medium and incubated at 37°C/5% $CO_2$ under starvation conditions for 4 hours. The starved cells were collected by centrifugation and resuspended in the complete medium, and then seeded in a 96-well U-bottom cell culture plate at $5 \times 10^5$ cells per well. The testing samples (IC-Cy08 and the wild-type hIL-2 control) were serially diluted in the complete medium at a ratio of 1:5 and added to the cells at 50 µL per well. The cell culture plate was incubated at 37°C/5% $CO_2$ for 15-30 minutes. The fixation buffer (Biolegend) pre-heated at 37°C was added to each well and the plate was incubated at 37°C for 15-30 minutes. The cells were collected by centrifugation and washed twice with FACS buffer (1×PBS containing 1% BSA). True-Phos Perm buffer (Biolegend) pre-cooled at -20°C was added to each well and the plate was incubated at -20°C for 1 hour. Cells were collected by centrifugation and washed twice with FACS buffer, and then resuspended in 100 µL/well of anti-pSTAT5 (Tyr694)-PE antibody (Thermo) diluted at 1:50 in FACS buffer and incubated on ice for 40 minutes. Cells were collected by centrifugation, washed twice with FACS buffer, resuspended in FACS buffer, and analyzed using a flow cytometer.

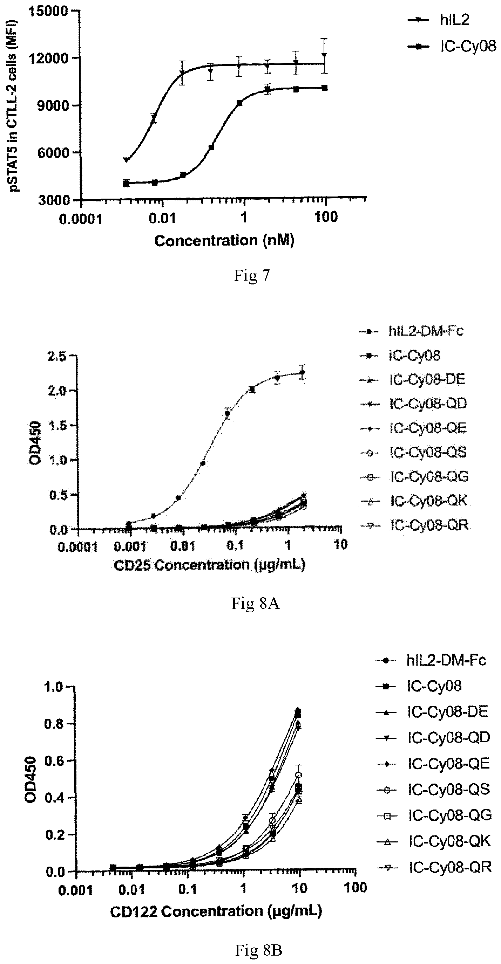

As shown in Figure 7 and Table 17, compared to the hIL-2 control, the activity of IC-Cy08 in inducing STATS phosphorylation in CTLL-2 cells is significantly attenuated, confirming that the α subunit binding site of hIL-2 mutein component in the IC-Cy08 construct containing disulfide linkage is stably masked by the antibody component and thus unexposed to the surface of molecule, consequently, that effectively blocks the hIL-2 mutein component from being bound by the high-affinity IL2R expressed on the surface of CTLL-2 cells, resulting in a down-regulated activation of intracellular signaling pathway induced by IL-2.

Table 17. Analysis of STAT5 phosphorylation in CTLL-2 cells in response to the treatment of IC-Cy08 (an hIL-2/anti-IL-2 antibody fusion protein containing a disulfide linkage) or the hIL-2 control by flow cytometry

|  | hIL-2 | IC-Cy08 |
|---|---|---|
| EC50 (nM) | 0.006492 | 0.2335 |

*Example 5: Further Optimization of the hIL-2/Anti-IL-2 Antibody Fusion Protein*

5.1 Analysis of Steric Hindrance between S4B6 Antibody and CD122

By analyzing the crystal structure of the S4B6/mIL-2 antigen-antibody complex (PDB: 4YUE), it was found that Gln at the position 27 of antibody light chain (located in LCDR1) may form a steric hindrance against Arg at the position 43 of CD122, thereby interfering the binding of the hIL-2 component in the hIL-2/anti-IL-2 antibody fusion protein of the present application to hCD122. To confirm whether this hypothetical steric hindrance dose exist and affect the biological activity of IC-Cy08, a list of mutants with site mutations on the light chain of IC-Cy08 were designed, constructed and expressed. In addition, Asp at the position 1 of antibody light chain was also mutated (as shown in Table 18). The effect of these mutations on the binding activity to hCD122 as well as the biological activity was evaluated.

Table 18. Optimized IC-Cy08 mutants with mutation in the light chain of hS4B6-H6K3(H)

| Mutant ID | Site of Mutation and Related Amino Acid | Amino Acid Residue before Mutation | Amino Acid Residue after Mutation |
|---|---|---|---|
| IC-Cy08-DE | L1: **D** | D | E |
| IC-Cy08-QD | L27 : **Q** | Q | D |
| IC-Cy08-QE | | | E |
| IC-Cy08-QS | | | S |
| IC-Cy08-QG | | | G |
| IC-Cy08-QK | | | K |
| IC-Cy08-QR | | | R |
| (L represents the light chain, and the number after L represents the site of mutation.) | | | |

5.1.1 Analysis of Binding Activity of IC-Cy08 Mutants to hIL2Rα (hCD25) and hIL2Rβ (hCD122) by ELISA

The binding activity of IC-Cy08 mutants listed in Table 18 to hIL2Rα (hCD25) and hIL2Rβ (hCD122) was determined by ELISA according to the method described in Section 2.1.1 of Example 2, wherein the antigens were replaced by hCD25 (Aero) and hCD122 (Aero), respectively, and hIL2-DM-Fc was used as the control. As shown in Figure 8A, there is no significant change in the binding activity of IC-Cy08 mutants to hCD25 as compared to their parental molecule IC-Cy08. However, compared to the hIL2-DM-Fc control, the binding activity of these IC-Cy08 mutants to hCD25 is significantly reduced. As to the binding activity to hCD122, the mutants including IC-Cy08-QS, IC-Cy08-QG, IC-Cy08-QK, and IC-Cy08-QR exhibit somewhat attenuated activity as compared to their parental molecule IC-Cy08, which is comparable to that of the hIL2-DM-Fc control. The binding activity to hCD122 of the other three mutants including IC-Cy08-DE, IC-Cy08-QD and IC-Cy08-QE is similar to their parental molecule IC-Cy08, which is augmented as compared to the hIL2-DM-Fc control (Figure 8B).

5.1.2 Analysis of Biological Activity of IC-Cy08 Mutants

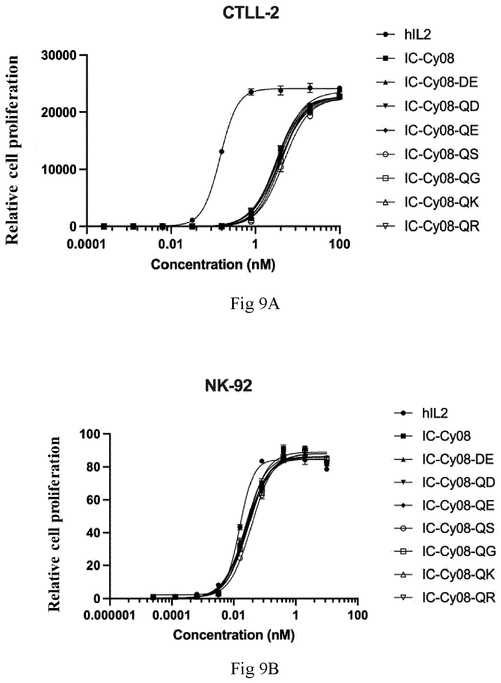

The biological activity of IC-Cy08 mutants was determined by cell proliferation assay with CTLL-2 cells and NK-92 cells according to the method described in Section 1.3 of Example 1, wherein wild-type hIL-2 was used as the control. As shown in Figure 9, all of the seven IC-Cy08 mutants have the proliferative activity similar to their parental molecule IC-Cy08 in CTLL-2 cells (Figure 9A and Table 19) and NK-92 cells (Figure 9B and Table 19). Compared to the hIL-2 control, all of the seven IC-Cy08 mutants and their parental molecule IC-Cy08 have significantly reduced activity in promoting CTLL-2 cell proliferation (Figure 9A and Table 19), and comparable activity in promoting NK-92 cell proliferation (Figure 9B and Table 19).

Table 19. Biological activity of the IL-2/anti-IL-2 antibody fusion protein IC-Cy08 mutants containing disulfide linkage and optimized antibody light chain determined by the cell proliferation assay in CTLL-2 cells and NK-92 cells

| Sample ID | Activity in Promoting CTLL-2 Cell Proliferation (EC50, nM) | Activity in Promoting NK-92 Cell Proliferation (EC50, nM) |
|---|---|---|
| hIL-2 | 0.1469 | 0.01572 |
| IC-Cy08 | 3.352 | 0.02635 |
| IC-Cy08-DE | 3.36 | 0.02332 |
| IC-Cy08-QD | 3.621 | 0.02743 |
| IC-Cy08-QE | 3.847 | 0.02612 |
| IC-Cy08-QS | 4.64 | 0.03701 |
| IC-Cy08-QG | 3.991 | 0.0301 |
| IC-Cy08-QK | 3.518 | 0.02691 |
| IC-Cy08-QR | 3.061 | 0.0247 |
| (hIL-2 and the parental molecule IC-Cy08 are used as the control.) | | |

Although IC-Cy08-QS, IC-Cy08-QG, IC-Cy08-QK and IC-Cy08-QR exhibit attenuated binding activity to hCD122 as compared to their parental molecule IC-Cy08, their activities in promoting CTLL-2 and NK cell proliferation are comparable to that of IC-Cy08, indicating that their proliferative activities in CTLL-2 cells and NK cells are not attenuated. Overall, these data suggest that Gin at the position 27 of antibody light chain in the IC-Cy08 molecule does not significantly affect the binding activity of the hIL2 component to hCD122 and its functional activity.

In addition, during the production of above-mentioned IC-Cy08 mutants, it was found that the expression level of IC-Cy08-QD, IC-Cy08-QE, IC-Cy08-QS and IC-Cy08-QG was increased by multiple fold as compared to their parental molecule IC-Cy08 (Table 20).

Table 20. Expression levels of IC-Cy08 mutants

| IC-Cy08 Mutant Sample ID | Expression Level (mg/L) |
|---|---|
| IC-Cy08 | 40 |
| IC-Cy08-DE | 93 |

(continued)

| IC-Cy08 Mutant Sample ID | Expression Level (mg/L) |
|---|---|
| IC-Cy08-QD | 158 |
| IC-Cy08-QE | 140 |
| IC-Cy08-QS | 113 |
| IC-Cy08-QG | 154 |
| IC-Cy08-QK | 28 |
| IC-Cy08-QR | 47 |

5.2 Optimization of hIL-2 Amino Acid Sequence

Analysis of the amino acid sequence of hIL-2 revealed that the threonine residue at position 3 would be glycosylated during expression in eukaryotic cells. In addition, there is a free cysteine residue at the position 125, which may lead to formation of mismatched disulfide bond and aggregation in the downstream manufacturing, thereby compromising the quality control of the product. Therefore, the first three amino acid residues at the N-terminus of hIL-2 were deleted, and the cysteine residue at position 125 was mutated to eliminate the possible liabilities caused by these sites. As shown in Table 21, a list of IC-Cy08 mutants comprising the deletion of the first 3 amino acid residues at the N-terminus of hIL-2-DM and different substitution mutations of the cysteine at position 125 were designed for analysis.

Table 21. IC-Cy08 mutants comprising optimized hIL2-DM amino acid sequence

| Mutant ID | APT, the First Three Amino Acid Residues at the N-Terminus of hIL-2 | Amino Acid Residue at Position 125 of hIL-2 |
|---|---|---|
| IC-Cy08 (Δ3,WT) | APT deleted | C |
| IC-Cy08 (Δ3, C125A ) | APT deleted | A |
| IC-Cy08 (Δ3, C125L ) | APT deleted | L |
| IC-Cy08 (Δ3, C125S ) | APT deleted | S |
| IC-Cy08 (Δ3, C125Q ) | APT deleted | Q |
| IC-Cy08 (Δ 3, C125V ) | APT deleted | V |

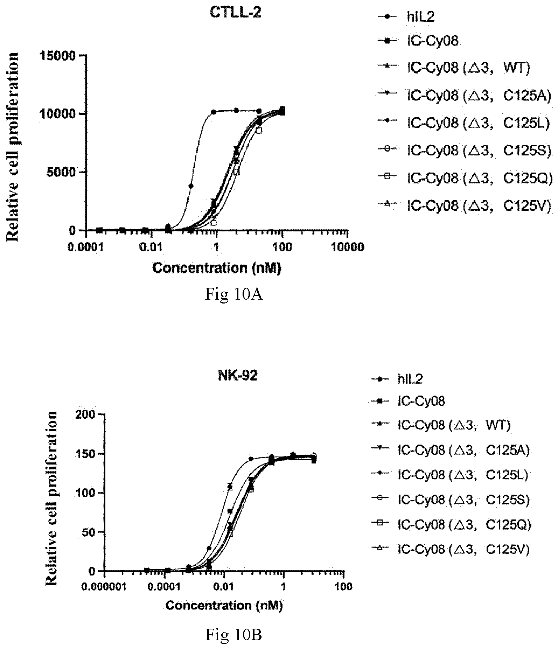

IC-Cy08 mutants listed in Table 21 were expressed and purified according to the method described in Section 2.1 of Example 2, and their activities in promoting CTLL-2 and NK-92 cell proliferation were determined according to the method described in Section1.3 of Example 1. As shown in Figure 10, the activity of each IC-Cy08 mutant in promoting the proliferation of CTLL-2 cells (Figure 10A and Table 22) and NK-92 cells (Figure 10B and Table 22) was comparable to the parental molecule IC-Cy08. Compared to the hIL-2 control, the mutants have significantly reduced activity in promoting CTLL-2 cell proliferation (Fig. 10A and Table 22) and slightly reduced activity in promoting NK-92 cell proliferation (Fig. 10B and Table 22). Therefore, mitigation of potential manufacturing liabilities associated with this fusion molecule through deletion of the first three amino acid residues at the N-terminus of hIL-2 and mutation of the cysteine at position 125 did not significantly affect its biological activity.

Table 22. Biological activity of the hIL-2/anti-IL-2 antibody fusion protein IC-Cy08 mutants comprising disulfide linkage and optimized hIL-2 amino acid sequence determined by cell proliferation assay in CTLL-2 and NK-92 cells

| Sample ID | Activity in Promoting CTLL-2 Cell Proliferation (EC50, nM) | Activity in Promoting NK-92 Cell Proliferation (EC50, nM) |
|---|---|---|
| hIL-2 | 0.1956 | 0.008233 |

(continued)

| Sample ID | Activity in Promoting CTLL-2 Cell Proliferation (EC50, nM) | Activity in Promoting NK-92 Cell Proliferation (EC50, nM) |
|---|---|---|
| IC-Cy08 | 2.332 | 0.01564 |
| IC-Cy08 (Δ3,WT) | 2.140 | 0.02636 |
| IC-Cy08 (Δ3, C125A) | 2.270 | 0.02469 |
| IC-Cy08 (Δ3, C125L) | 3.158 | 0.02812 |
| IC-Cy08 (Δ3, C125S) | 3.260 | 0.02886 |
| IC-Cy08 (Δ3, C125Q) | 4.425 | 0.03354 |
| IC-Cy08 (Δ3, C125V) | 2.407 | 0.02878 |
| (hIL-2 and the parental molecule IC-Cy08 are used as the control.) | | |

5.3 Modification of the Antibody Heavy Chain Constant Regions of the hIL-2/Anti-IL-2 Antibody Fusion Protein

To prolong the *in vivo* half-life of hIL-2/anti-IL-2 antibody fusion protein of the present application and to prevent the effector function mediated by the Fc region of the antibody component from killing target or non-target cells, the antibody heavy chain constant region of the fusion protein was modified by engineering.

5.3.1 Engineering of Isoelectric Point (pI) of the Antibody Heavy Chain Constant Region

To prolong the *in vivo* half-life of the fusion protein of the present application, a combinatorial mutation of N203D/K274Q/Q419E (EU numbering; named "pI mutation") was introduced into the heavy chain constant region of the antibody component of IC-Cy08.

5.3.2 Antibody Fc Fragment Engineering

To prevent the effector function mediated by the antibody Fc region from killing the target or non-target cells, a combinatorial mutation of L234F/L235E/P331S (EU numbering; named "TM mutation") was introduced into the Fc region to reduce the binding activity of Fc region to FcyRs and C1q, thereby eliminating the Fc-mediated effector function.

5.3.3 Analysis of Biological Activity of the IL-2/Anti-IL-2 Antibody Fusion Protein Comprising pI and TM mutations in the Heavy Chain Constant Region of the Antibody Component

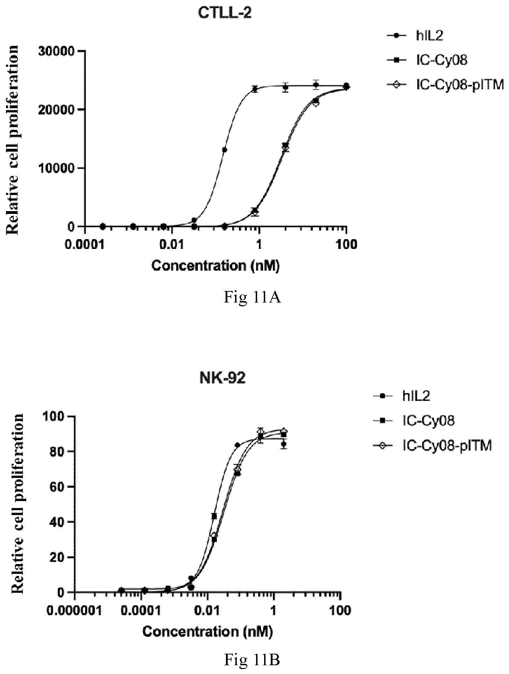

An IL-2/anti-IL-2 antibody fusion protein IC-Cy08-pITM comprising the pI and TM mutations in the heavy chain constant region of the antibody component was expressed and purified according to the method described in Section 2.1 of Example 2, and its activity in promoting CTLL-2 and NK-92 cell proliferation was determined according to the method described in Section 1.3 of Example 1. As shown in Figure 11, IC-Cy08-pITM has an activity in promoting the proliferation of CTLL-2 cells (Figure 11A and Table 23) and NK-92 cells (Figure 11B and Table 23) similar to its parental molecule IC-Cy08.

Table 23. Biological activity of the hIL-2/anti-IL-2 antibody fusion protein IC-Cy08-pITM comprising pI and TM mutations in the heavy chain constant region of its antibody component determined by cell proliferation assay in CTLL-2 and NK-92 cells

| Sample ID | Activity in Promoting CTLL-2 Cell Proliferation (EC50, nM) | Activity in Promoting NK-92 Cell Proliferation (EC50, nM) |
|---|---|---|
| hIL-2 | 0.1469 | 0.01626 |
| IC-Cy08 | 3.159 | 0.03117 |
| IC-Cy08-pITM | 3.352 | 0.02929 |

*Example 6: Construction and Biological Activity Analysis of hIL-2/Anti-IL-2 Antibody Fusion Protein Comprising the hS4B6-H6K3(H) Affinity-Matured Antibody*

6.1 Disulfide Linkage Screening

K2G11 is an antibody derived from hS4B6-H6K3(H) through affinity maturation and has demonstrated significantly enhanced binding activity to hIL2-DM. To construct an antigen-antibody fusion molecule with more stable structure, the C-terminus of hIL2-DM (Δ3, C125L) mutein was connected to the N-terminus of the heavy chain of the K2G11 antibody via the (G4S)$_4$ linker, using the method described in Example 4. The paired amino acid mutations designed for the disulfide linkage formation between hIL2-DM (Δ3, C125L) mutein and the K2G11 antibody are shown in Table 24, and whether the stable disulfide linkage can be formed is further evaluated. The structure of the hIL-2/anti-IL-2 antibody fusion protein containing a disulfide linkage is schematically illustrated in Figure 4.

Table 24. Paired amino acid mutations and positions for disulfide linkage formation between the hIL-2 mutein and the affinity-matured antibody K2G11

| Sample ID | Position of Amino Acid Pair | Amino Acid Position in hIL2 | Amino Acid Position in K2G11 | Position of Introduced Cysteine Mutation in K2G11 |
|---|---|---|---|---|
| K2G11-Cy01 | IL:61-L:30 | **E** 61 | **S** 30 | LCDR1 |
| K2G11-Cy02 | IL:61-L:31 | **E** 61 | **N** 31 | LCDR1 |
| K2G11-Cy03 | IL:61-L:50 | **E** 61 | **S** 50 | LCDR2 |
| K2G11-Cy04 | IL:104-L:52 | **M** 104 | **T** 52 | LCDR2 |
| K2G11-Cy05 | IL:102-L:67 | **T** 102 | **S** 67 | LFR3 |
| K2G11-Cy06 | IL:102-L:68 | **T** 102 | **G** 68 | LFR3 |
| K2G11-Cy07 | IL:64-H:97 | **K** 64 | **F** 97 | HCDR3 |
| K2G11-Cy08 | IL:65-H:97 | **P** 65 | **F** 97 | HCDR3 |
| K2G11-Cy09 | IL:64-L:92 | **K** 64 | **Y** 92 | LCDR3 |
| K2G11-Cy10 | IL:42-H:97 | **F** 42 | **F** 97 | HCDR3 |
| K2G11-Cy11 | IL:60-L:30 | **E** 60 | **S** 30 | LCDR1 |
| K2G11-Cy12 | IL:64-L:32 | **K** 64 | **Y** 32 | LCDR1 |
| H6K3(H)-Cy10 | IL:42-H:97 | **F** 42 | **F** 97 | HCDR3 |
| (IL represents IL-2, L represents the antibody light chain, H represents the antibody heavy chain, and the number after IL or L/H represents the position of amino acid mutation.) | | | | |

The fusion protein constructs listed in Table 24 were constructed, expressed and purified according to the method described in Section 2.1 of Example 2, and then analyzed by SEC-HPLC. The hIL-2/anti-IL-2 antibody fusion proteins K2G11-Cy04, K2G11-Cy05 and K2G11-Cy06 are expressed mainly as stable monomer and selected for further evaluation. The hIL-2/anti-IL-2 antibody fusion proteins IC-Cy08 (H6K3(H)-Cy08) and H6K3(H)-Cy10 comprising the parental antibody hS4B6-H6K3(H) were constructed and used as controls.

6.2 Analysis of Binding Activity of the hIL-2/Anti-IL-2 Antibody Fusion Protein Comprising K2G11 to hIL2Rα (hCD25) and hIL2Rβ (hCD122) by ELISA

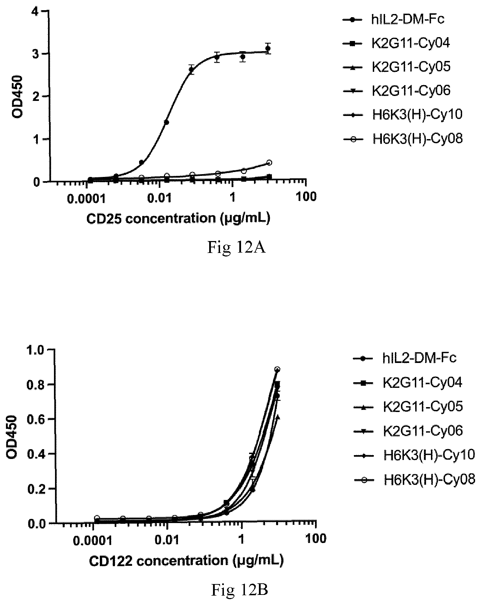

The binding activity of K2G11-Cy04, K2G11-Cy05 and K2G11-Cy06 to hCD25 and hCD122 was determined by ELISA according to the method described in Section 4.2.1 of Example 4, wherein hIL2-DM-Fc, H6K3(H)-Cy08 and H6K3(H)-Cy10 were used as controls. As shown in Figure 12A, K2G11-Cy04, K2G11-Cy05, K2G11-Cy06 and H6K3(H)-Cy10 barely bind to hCD25, while H6K3(H)-Cy08 shows a weak binding activity to hCD25 at high concentrations as compared to the hIL2-DM-Fc control. As shown in Figure 12B, the binding activity to CD122 showed no significant difference among the fusion proteins being tested.

In summary, compared to H6K3(H)-Cy08, K2G11-Cy04, K2G11-Cy05, K2G11-Cy06 and H6K3(H)-Cy10 can completely inhibit the binding of hIL-2 mutein to hCD25, even at high concentrations, further suggesting that the $\alpha$ subunit binding site of the IL-2 component is more stably masked by these antibody fusion proteins and cannot be competitively bound by hCD25.

6.3 Analysis of Biological Activity of the hIL-2/Anti-IL-2 Antibody Fusion Proteins Comprising K2G11

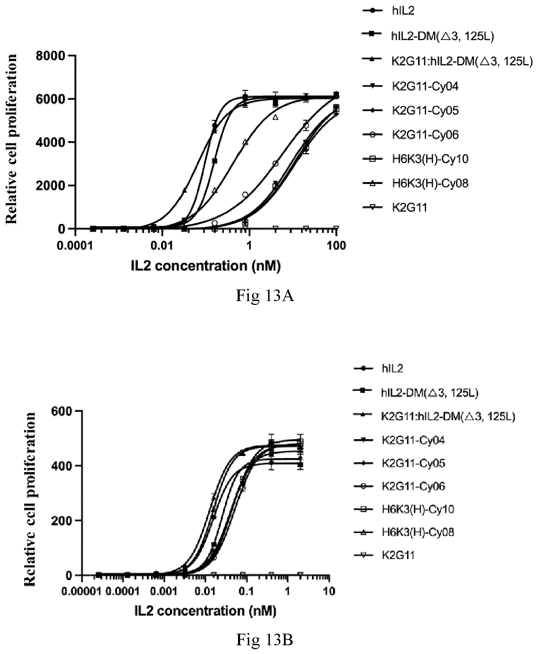

The biological activity of the hIL2/anti-IL2 antibody fusion protein comprising the K2G11 antibody component and the hIL2-DM ($\Delta$3, 125L) component was determined by cell proliferation assay with CTLL-2 and NK-92 cells according to the method described in Section 1.3 of Example 1. As shown in Figure 13, compared to the IL-2 controls including hIL-2 (purchased from Novoprotein), hIL2-DM ($\Delta$3, C125L) mutein, and an antigen-antibody immunocomplex formed by pre-incubation of the K2G11 antibody and hIL2-DM ($\Delta$3, 125L) mutein, K2G11-Cy04, K2G11-Cy05, K2G11-Cy06 and H6K3(H)-Cy10 exhibit a significant attenuated activity in promoting the proliferation of CTLL-2 cells which express a high level of CD25, and the level of attenuation is significantly greater than that of H6K3(H)-Cy08 (Figure 13A, Table 25). In NK-92 cells which express a low level of CD25, K2G11-Cy04, K2G11-Cy05, K2G11-Cy06, H6K3-Cy10 and the above-mentioned IL-2 controls show no significant difference in activity of promoting cell proliferation (Figure 13B, Table 25). These results indicate that the IL2$\alpha$ subunit binding site of IL-2 component is more stably embedded inside of the fusion proteins K2G11-Cy04, K2G11-Cy05, K2G11-Cy06 and H6K3(H)-Cy10 due to the disulfide linkage formation, and thus cannot be competitively bound by the high-affinity IL2R.

Table 25. Biological activity of IL-2/anti-IL-2 antibody fusion proteins comprising the K2G11 antibody and disulfide linkage determined by cell proliferation assay in CTLL-2 and NK-92 cells

| Sample ID | Activity in Promoting CTLL-2 Cell Proliferation (EC50, nM) | Activity in Promoting NK-92 Cell Proliferation (EC50, nM) |
|---|---|---|
| hIL-2 | 0.0959 | 0.01552 |
| hIL2-DM ($\Delta$3, 125L) | 0.1556 | 0.02585 |
| K2G11: hIL2-DM ($\Delta$3, 125L) | 0.0642 | 0.01276 |
| K2G11-Cy04 | 9.174 | 0.04227 |
| K2G11-Cy05 | 11.23 | 0.04152 |
| K2G11-Cy06 | 5.773 | 0.05182 |
| H6K3(H)-Cy10 | 11.68 | 0.04628 |
| H6K3(H)-Cy08 | 0.4255 | 0.01554 |
| K2G11 | - | - |
| (hIL-2 is used as the control.) | | |

*Example 7: Binding Activity of hIL-2/anti-IL-2 Antibody Fusion Protein Comprising K2G11 to hIL2R$\alpha$, hIL2R$\alpha/\beta$ and hIL2R$\alpha/\beta/\gamma$ Analyzed by ForteBio*

The binding activity of the hIL-2/anti-IL-2 antibody fusion protein comprising K2G11 and hIL2-DM ($\Delta$3, 125L) to IL2R$\alpha$, IL2R$\alpha/\beta$ and IL2R$\alpha/\beta/\gamma$ was analyzed using a molecular interaction analyzer (ForteBio, Model Qke). The specific method was described as follows: biotin-CD25 (Aero) was diluted in 1×PBS to 5 $\mu$g/mL and added to a 96-well black-wall plate at 200 $\mu$L per well; hIL2R$\beta$ (Aero) was diluted to 5 $\mu$g/mL in 1×PBS and also added to the 96-well black-wall plate at 200 $\mu$L per well. The testing samples hIL2-DM ($\Delta$3, C125L) and K2G11-Cy05 were diluted to 100 nM using 1×PBS, 5 $\mu$g/mL hIL2R$\beta$ (Aero) and 5 $\mu$g/mL hII,2Ry (Aero), respectively, and then added to the 96-well black-wall plate at 200 $\mu$L per well. The 96-well black-wall plate containing the testing samples and a SA sensor was uploaded onto the analyzer, and a program was inputted. The SA sensor was firstly immersed into the well with biotin-CD25 (Aero) and then with hIL2R$\beta$ (Aero) till saturation. After that, the sensor was immersed in the testing sample well and the binding signal was recorded in real-time. At the end of each association-dissociation cycle, the sensor was regenerated by immersing in 500 mM phosphate buffer and then started with the next cycle of detection.

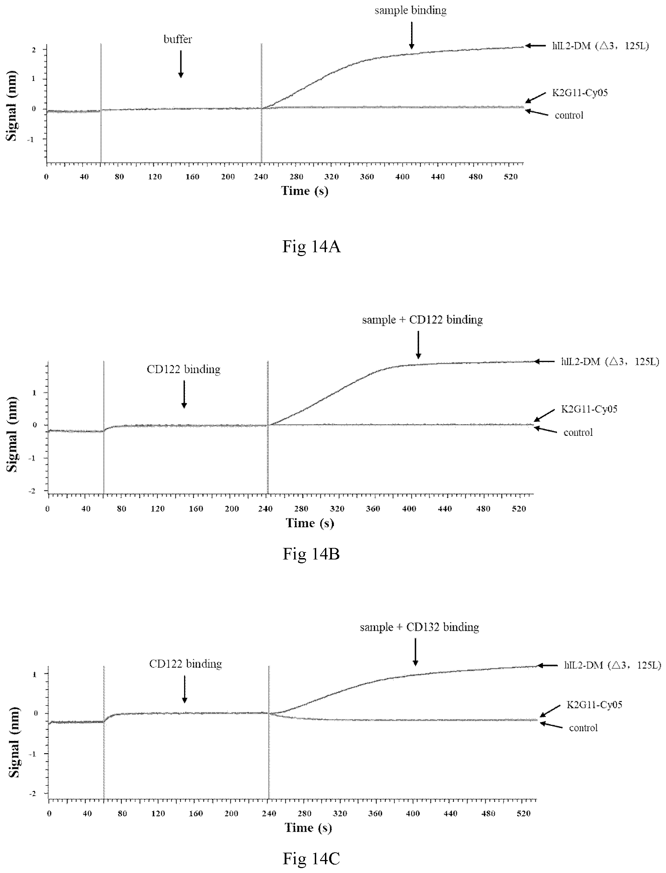

As shown in Figure 14, results demonstrate that the hIL-2/anti-IL-2 antibody fusion protein K2G11-Cy05 comprising K2G11 and hIL2-DM ($\Delta$3, 125L) does not bind to hIL2R$\alpha$ (Figure 14A), and also cannot bind to any complex comprising the hIL2R$\alpha$ subunit due to the steric hindrance, including the hIL2R$\alpha/\beta$ complex (Figure 14B) and the hIL2R$\alpha/\beta/\gamma$ complex (Figure 14C).

*Example 8: Binding Activity of the hIL-2/anti-IL-2 Antibody Fusion Protein K2G11-Cy05 to hCD25 in Comparison to NARA1leukin as Measured by ELISA*

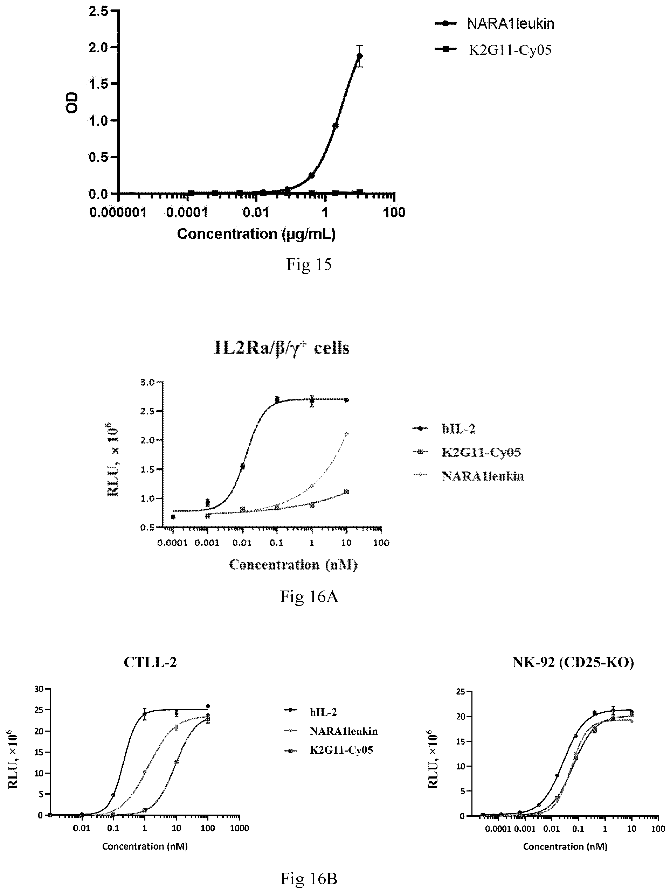

The binding activity of hIL-2/anti-IL-2 antibody fusion protein K2G11-Cy05 (as described in Example 6) and NARAlleukin protein (generated according to the method described in the patent application WO2017122130) to CD25 was evaluated according to the method described in Section 4.2.1 of Example 4, wherein hCD25 (Aero) was used to coat the plate and the HRP anti-human IgG Fc (H+L) (Jackson Immunoresearch) was used as the secondary antibody.
As shown in Figure 15, NARAlleukin exhibits a binding activity to hCD25 as the concentration increases, whereas the hIL-2/anti-IL-2 antibody fusion protein K2G11-Cy05 of the present application shows no binding activity to hCD25 in the concentration range being tested, indicating that the hIL-2/anti-IL-2 antibody fusion protein of the present application contains a more stable interaction between the anti-IL-2 antibody component and the hIL-2 mutein component due to the covalent disulfide linkage constructed in the fusion protein, which can maximally prevent the IL-2 component of the fusion protein from being competitively bound by hCD25.

*Example 9: Biological Activity of hIL-2/anti-IL-2 Antibody Fusion Protein of the Present Application Determined by Reporter Gene Assay and Cell Proliferation Assay*

9.1 Cell-based Reporter Gene Assay

The *in vitro* activity of the hIL-2/anti-IL-2 antibody fusion protein K2G11-Cy05 as described in Example 6 in activating the IL-2-induced downstream signaling pathway in cells expressing a high level of CD25 was evaluated by a bioluminescent cell-based reporter assay, wherein the NARAlleukin protein was used as a control. The specific method was as follows: IL-2 bioassay cells (Promega) were collected by centrifugation and then resuspended to $8 \times 10^5$ cells/mL in culture medium. Cells were seeded in a 96-well white-wall plate and incubated overnight at 37°C/5% CO2. K2G11-Cy05 and NARAlleukin were serially diluted in culture medium, and wild-type hIL-2 was used as the positive control. The diluted samples were added to the 96-well white-wall plate and incubated at 37°C/5% $CO_2$ for 6 hours. The 96-well plate was equilibrated to room temperature for 30 minutes and then detected using the One-Lite Luciferase detection reagent (Vazyme, Nanjing). The chemiluminescence signals were read by a multimode microplate reader.
As shown in Figure 16A, in cells expressing a high level of CD25, NARAlleukin at high concentrations exhibits activities of inducing the IL-2 downstream signaling activation, whereas the hIL-2/anti-IL-2 antibody fusion protein K2G11-Cy05 of the present application shows no detectable activation of the signaling pathway in the concentration range being tested.

9.2 Cell Proliferation Assay

The biological activity of the hIL2/anti-IL2 antibody fusion protein K2G11-Cy05 and NARAlleukin was evaluated by cell proliferation assay with CTLL-2 cells and CD25 knockout NK-92 (CD25-KO) cells according to the method described in Section 1.3 of Example 1. As shown in Figure 16B and Table 26, in CTLL-2 cells which express a high level of CD25, K2G11-Cy05 exhibits a significantly attenuated activity in inducing cell proliferation as compared to the hIL-2 control, which is also significantly lower than that of NARAlleukin. However, in CD25 knock-outNK-92 (CD25-KO) cells, K2G11-Cy05 and NARAlleukin show no significant difference in activity of inducing cell proliferation and their potency is comparable to hIL-2.
These results further demonstrate that the disulfide linkage described in the present application can further stabilize the structure of the hIL-2/anti-IL-2 antibody fusion protein, more effectively preventing the IL-2 component of the fusion protein from being competitively bound by hCD25.

Table 26. Biological activity of hIL-2/anti-IL-2 antibody fusion proteins K2G11-Cy05 and NARAlleukin determined by cell proliferation assay in CTLL-2 and NK-92 cells

| Sample ID | Activity in Promoting CTLL-2 Cell Proliferation (EC50, nM) | Activity in Promoting NK-92 (CD25-KO) Cell Proliferation (EC50, nM) |
|---|---|---|
| hIL-2 | 0.210 | 0.027 |
| NARA1leukin | 1.318 | 0.054 |
| K2G11-Cy05 | 9.170 | 0.065 |

*Example 10: Evaluation of Thermal Stability of Anti-IL-2 Antibody Comprising pI and TM Mutations in the Antibody Heavy Chain Constant Region and hIL-2/anti-IL-2 Antibody Fusion Protein Constructed with the Said Antibody*

The thermal stability of anti-IL-2 antibody comprising the pI and TM mutations in the antibody heavy chain constant region and the hIL-2/anti-IL-2 antibody fusion protein constructed with this antibody was evaluated using NanoTemper Prometheus Panta to determine the effect of pI and TM mutations introduced in the heavy chain constant region on the stability of the anti-IL-2 antibody as well as the hIL-2/anti-IL-2 antibody fusion protein comprising this antibody and the hIL-2 mutein (hIL2-DM ($\Delta$3, C125L)) with a disulfide linkage constructed inside of the molecule. The anti-IL-2 antibody K2G11-pITM containing pI and TM mutations in its heavy chain constant region was generated according to the method described in Example 5. The humanized anti-IL-2 antibody K2G11 without mutations in its heavy chain constant region was used as the control, which was described in Example 3. The hIL-2/anti-IL-2 antibody fusion proteins including K2G11-Cy04, K2G11-Cy05 and K2G11-Cy06 with pI and TM mutations in the heavy chain constant region of their antibody component were prepared according to the method described in Example 6. The specific detection method was as follows: each sample was diluted in 1×PBS to 1 mg/mL and centrifuged at 4°C for 15 minutes. The supernatant was collected in a capillary detection tube and loaded onto the detection platform. Each sample was tested in duplicate. A detection program was set up to run with a temperature range of 25°C to 95°C, and data were recorded. After the program was completed, obtained data were analyzed using the relevant software.

As shown in Table 27, although the $T_{m1}$ value (65.94°C) of K2G11-pITM is lower than that of the control antibody K2G11 (70.44°C), the $T_{onset}$ and $T_{turbility}$ values of K2G11-pITM (approximately 60°C and 80°C, respectively) are comparable to those of the control antibody K2G11, which are within the normal range for an antibody. Therefore, introduction of pI and TM mutations in the heavy chain constant region has no significant effect on the stability of the anti-IL-2 antibody of the present application.

Results of thermostability of the hIL-2/anti-IL-2 antibody fusion protein are shown in Table 28. The $T_{m1}$ value of the hIL-2/anti-IL-2 antibody fusion proteins K2G11-Cy04, K2G11-Cy05 and K2G11-Cy06 containing pI and TM mutations in the heavy chain constant region of their antibody component is about 65°C, which is similar to that of the humanized anti-IL-2 antibody K2G11-pITM, representing the denaturation temperature of CH2 domain of heavy chain constant region of the antibody component. The $T_{m2}$ value which reflects the stability of the binding complex of the Fab region of antibody component and the hIL-2 mutein (hIL2-DM ($\Delta$3, C125L)) component of hIL-2/anti-IL-2 antibody fusion proteins is above 80°C for all three testing samples, and their $T_{turbility}$ values also show a significant improvement over K2G11-pITM, indicating that the cysteine residue substitution introduced in the humanized anti-IL-2 antibody component and the hIL-2 mutein component can not only form a stable disulfide linkage between them, but also significantly improve the structural stability of the hIL-2/anti-IL-2 antibody fusion protein.

Table 27. Thermostability of K2G11 and K2G11-pITM (K2G11 with pI and TM mutations in its heavy chain constant region)

| Sample ID | $T_{m1}$ (°C) | | $T_{m2}$ (°C) | | $T_{onset}$(°C) | | $T_{turbility}$ (°C) | |
|---|---|---|---|---|---|---|---|---|
| | f | s | f | s | f | s | f | s |
| K2G11 | 70.44 | 003 | 78.54 | 0.06 | 63.84 | 0.29 | 78.27 | 0.15 |
| K2G11-pITM | 65.94 | 0.03 | 79.25 | 0.05 | 58.54 | 0.44 | 79.42 | 0.00 |
| (f = mean; s = standard deviation.) | | | | | | | | |

Table 28. Thermostability of hIL-2/anti-IL-2 antibody fusion protein with pI and TM mutations in the heavy chain constant region of its antibody component

| Sample ID | $T_{m1}$ (°C) | | $T_{m2}$ (°C) | | $T_{m3}$ (°C) | | $T_{onset}$ (°C) | | $T_{turbility}$ (°C) | |
|---|---|---|---|---|---|---|---|---|---|---|
| | f | s | f | s | f | s | f | s | f | s |
| K2G11-Cy04 | 65.82 | 0.05 | 81.90 | 0.12 | 89.75 | 0.07 | 58.55 | 0.15 | 88.97 | 0.29 |
| K2G11-Cy05 | 65.81 | / | 82.29 | / | 88.82 | / | 59.48 | / | 86.90 | / |

(continued)

| Sample ID | T$_{m1}$ (°C) | | T$_{m2}$ (°C) | | T$_{m3}$ (°C) | | T$_{onset}$ (°C) | | T$_{turbility}$ (°C) | |
|---|---|---|---|---|---|---|---|---|---|---|
| | f | s | f | s | f | s | f | s | f | s |
| K2G11-Cy06 | 65.86 | 0.09 | 83.56 | 0.15 | 87.92 | 0.04 | 57.29 | 0.14 | 84.89 | 0.19 |
| (f = mean; s = standard deviation.) | | | | | | | | | | |

*Example 11: Determination of Binding Kinetics of HumanizedAnti-IL-2 Antibody Comprising pI and TM mutations in the Heavy Chain Constant Region, and hIL-2/anti-IL-2 Antibody Fusion Protein Constructed therefrom, to Fc Receptors and FcRn*

The binding kinetics of the humanized anti-IL-2 antibody K2G11-pITM containing pI and TM mutations in the heavy chain constant region, the hIL-2/anti-IL-2 antibody fusion protein K2G11-Cy05 constructed therefrom, and the control antibody K2G11 to FcγRI, FcγRIIa, FcγRIIb, FcγRIIIa-158F, FcγRIIIa-158V, FcRn, C1q and FcRn were determined according to the method described in Section 1.2 of Example 1.

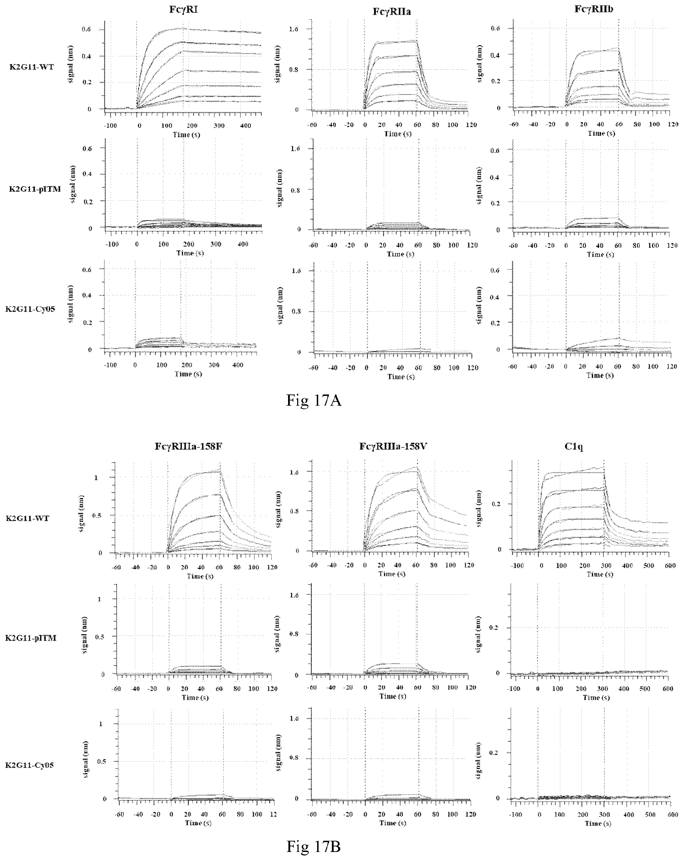

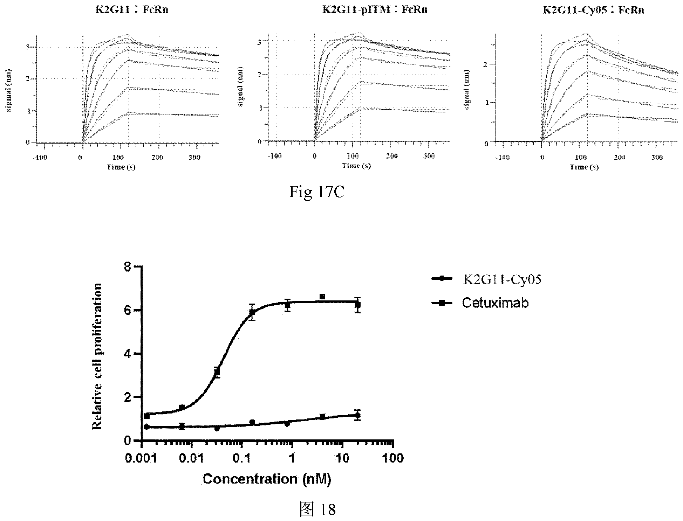

As shown in Figure 17, compared to the control antibody K2G11, both K2G11-pITM and K2G 11-Cy05 have essentially no binding activity to Fc receptors (including FcγRI, FcγRIIa, FcγRIIb, FcγRIIIa-158F and FcγRIIIa-158V) and C1q (Figures 17A and 17B), and there is no significant difference in the binding affinity to FcRn (Figure 17C). These results indicated that introduction of TM mutation in the heavy chain constant region can reduce the binding activity of the antibody Fc region to the Fc receptors, thereby attenuating the antibody effector function mediated by the Fc region of the antibody and/or the antibody fusion protein (including ADCC and CDC effects). In the meantime, the pITM mutations do not affect the binding activity to FcRn.

*Example 12: Determination of ADCC Activity of hIL-2/anti-hIL-2 Antibody Fusion Protein by Gene Reporter Assay*

To further verify if introduction of TM mutation in the heavy chain constant region of the antibody component could attenuate the Fc-mediated antibody effector function, the ADCC activity of K2G11-Cy05 containing TM mutation in the heavy chain constant region of the antibody component as prepared in Example 6 was determined by a gene reporter assay, wherein an Jurkat cell line (Promega) expressing FcγRIIIa-158V and firefly luciferase under the control of NFAT response elements was used as effector cells and a NK-92 cell line expressing IL2R was used as target cells, and the anti-EGFR antibody cetuximab and A431 cells were used as the positive control group. The specific method was as follows: NK-92 cells and A431 cells were collected by centrifugation and resuspended in ADCC assay buffer (RPMI-1640 containing 0.5% FBS), and then added to a 96-well white-wall plate at $1.5 \times 10^4$ cells per well. The testing samples were serially diluted in ADCC assay buffer and then added to the 96-well plate at 25 μL/well. The effector cells (Jurkat cells) were collected by centrifugation, resuspended in ADCC assay buffer, and then added to the 96-well plate at $1.5 \times 10^5$ cells per well. The plate was incubated at 37°C/5% $CO_2$ overnight. After the incubation, the 96-well plate was equilibrated to room temperature, the One-Lite Luciferase reagent (Vazyme, Nanjing) was added to the plate and chemiluminescence signals were read using a multimode microplate reader.

As shown in Figure 18, compared to the positive control group (cetuximab + A431 cells), K2G11-Cy05 does not induce luciferase expression significantly in the effector cells even at the highest concentration tested, confirming that introduction of TM mutation in the heavy chain constant region of the antibody component can significantly reduce the binding affinity of the hIL-2/anti-IL-2 antibody fusion protein with Fc receptors, thereby significantly reducing the ADCC activity mediated by Fc of the hIL-2/anti-IL-2 antibody fusion protein.

*Example 13: Evaluation of the Stability of hIL-2/anti-IL-2 Antibody Fusion Proteins in Cynomolgus Monkey Serum and Healthy Human Serum*

The serum stability of the hIL-2/anti-IL-2 antibody fusion proteins (including K2G11-Cy04 and K2G11-Cy05) prepared as described in Example 6 was evaluated after incubation in the cynomolgus monkey serum and human serum derived from healthy subjects.

13.1 Biological activity of K2G11-Cy04 and K2G11-Cy05 after incubation in cynomolgus monkey serum and human serum determined by NK-92 cell proliferation assay

The specific method was as follows: the hIL-2/anti-IL-2 antibody fusion proteins K2G11-Cy04 and K2G11-Cy05 were each spiked in serum samples obtained from two cynomolgus monkeys (one male and one female, named M6 and M7) or two healthy human subjects (named H2 and H3) to a final concentration of 100 μg/mL, and mixed well and then aliquoted to centrifuge tubes. The cynomolgus monkey serum-treated samples were labeled as M6-Cy04, M7-Cy04, M6-Cy05 and M7-Cy05, and the human serum-treated samples were labeled as H2-Cy04, H3-Cy04, H2-Cy05 and H3-Cy05, respectively. The samples were incubated at 37°C for a different period of time, and the time of starting the incubation was designated as Day 0 (0 hour). Samples were taken at different time-points and stored at -80°C until analysis. The sampling time-points for the cynomolgus monkey serum-treated samples were Day 3 (72 hours), Day 7 (168 hours) and Day 14 (336 hours); the sampling time-points for the human serum-treated samples were Day 7 (168 hours), Day 14 (336 hours) and Day 21 (504 hours). After all serum-treated samples were collected, biological activity of each sample was analyzed by cell proliferation assay in NK-92 cells according to the method described in Example 1, wherein the hIL-2/anti-IL-2 antibody fusion proteins K2G11-Cy04 and K2G11-Cy05 without serum treatment and hIL-2 were used as the controls.

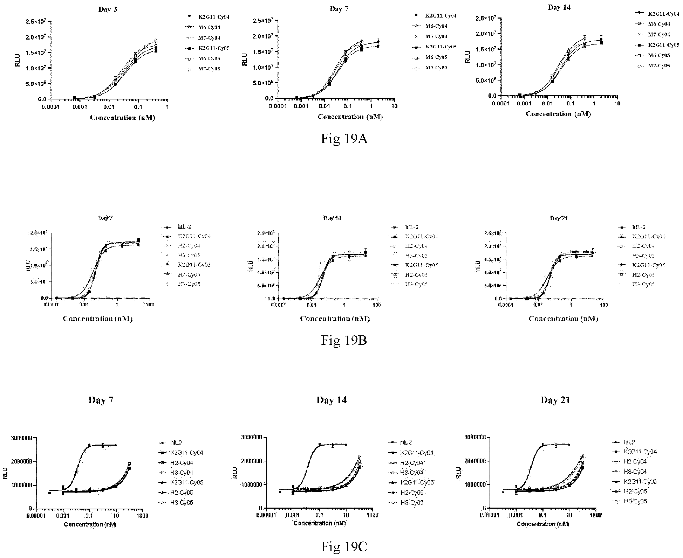

As shown in Table 29 and 30, and Figure 19A and 19B, compared to the samples without serum treatment, the hIL-2/anti-IL-2 antibody fusion proteins K2G11-Cy04 and K2G11-Cy05 retain full biological activity after the treatment at 37°C in cynomolgus monkey serum for up to 14 days (Figure 19A), or in human serum for up to 21 days (Figure 19B), indicating that the hIL-2/anti-IL-2 antibody fusion protein of the present application is stable in serum.

Table 29. Stability of hIL-2/anti-IL-2 antibody fusion proteins in cynomolgus monkey serum

| Time-point for Incubation | EC50 (nM) | | | | | |
|---|---|---|---|---|---|---|
| | M6-Cy04 | M7-Cy04 | M6-Cy05 | M7-Cy05 | K2G11-Cy04 | K2G11-Cy05 |
| Day 3 | 0.02452 | 0.03409 | 0.03991 | 0.03235 | 0.03450 | 0.03437 |
| Day 7 | 0.03132 | 0.03609 | 0.02518 | 0.03246 | 0.03795 | 0.03653 |
| Day 14 | 0.03234 | 0.03441 | 0.02422 | 0.02731 | 0.03795 | 0.03653 |

Table 30. Stability of hIL-2/anti-IL-2 antibody fusion proteins in healthy human serum

| Time-point for Incubation | EC50 (nM) | | | | | | |
|---|---|---|---|---|---|---|---|
| | H2-Cy04 | H3-Cy04 | H2-Cy05 | H3-Cy05 | K2G11-Cy04 | K2G11-Cy05 | hIL-2 |
| Day 7 | 0.04715 | 0.03973 | 0.04523 | 0.04897 | 0.04821 | 0.05252 | 0.03448 |
| Day 14 | 0.04616 | 0.02624 | 0.05170 | 0.04103 | 0.04821 | 0.05252 | 0.03448 |
| Day 21 | 0.04731 | 0.04436 | 0.05538 | 0.04546 | 0.04821 | 0.05252 | 0.03448 |

13.2 Biological activity of K2G11-Cy04 and K2G11-Cy05 after incubation in healthy human serum determined by cell-based gene reporter assay

The activity of human serum-treated samples H2-Cy04, H3-Cy04, H2-Cy05 and H3-Cy05 in activating the IL-2 downstream signaling pathway in cells expressing a high level of CD25 was evaluated according to the method described in Example 9.

As shown in Figure 19C, there is no significant difference in the activation of IL-2 downstream signaling pathway in cells expressing a high level of CD25 between treated and untreated samples of the hIL-2/anti-IL-2 antibody fusion proteins K2G11-Cy04 and K2G11-Cy05 after incubation with human serum at 37°C for up to 21 days or 7 days. These results suggest that, after incubated with human serum for 21 days or 7 days, the structural stability of the binding interface between the IL-2 component and the anti-IL-2 antibody component of the fusion proteins K2G11-Cy04 and K2G11-Cy05 was not affected, and the IL-2 component was completely blocked from binding by the high-affinity IL2R. Therefore, the hIL-2/anti-IL-2 antibody fusion protein of the present application has a good serum stability.

*Example 14: Detection of Disulfide Linkage in hIL-2/anti-IL-2 Antibody Fusion Proteins of the Present Application*

To verify whether the engineered disulfide linkage is formed correctly between the humanized anti-IL-2 antibody K2G11 component and the hIL-2 mutein component in the hIL-2/anti-IL-2 antibody fusion protein (including K2G11-Cy04 and K2G11-Cy05) prepared as described in Example 6, the disulfide linkage was analyzed by liquid chromatography-mass spectrometry (LC-MS). The specific method was as follows.

Detection of the disulfide linkage in K2G11-Cy04: The sample was denatured with guanidine hydrochloride, alkylated with N-ethylmaleimide (NEM), and then digested with Trypsin (Promega, V5113) after solution exchange and desalination. One aliquot of the Trypsin-digested sample was reduced by DTT, and TFA (Thermo, 28904) was then added to terminate the reaction; and another aliquot of the digested sample was mixed with TFA directly to terminate the reaction. The processed samples were analyzed by LC-MS, and obtained peptide mapping data were analyzed using UNIFI software to identify the disulfide linkage.

Detection of the disulfide linkage in K2G11-Cy05: The sample was denatured with guanidine hydrochloride, alkylated with N-ethylmaleimide (NEM), and then digested with Trypsin after solution exchange and desalination. After trypsin digestion, the samples were divided into two aliquots, one aliquot was further digested by Chymotrypsin (Promega, V106A), and the other aliquot was further digested by GluC (Promega, V165A). After all the digestions were completed, TFA was added directly for termination. The processed samples were analyzed by LC-MS, and obtained peptide mapping data were analyzed using UNIFI software to identify the disulfide linkage.

As shown in Table 31 and Table 32, all absorption peaks of the peptide fragments derived from the enzyme digestion of K2G11-Cy04 and K2G11-Cy05 match with their theoretical fragments, confirming that the engineered disulfide linkage is formed correctly in the hIL-2/anti-IL-2 antibody fusion protein described in the present application by introducing cysteine substitutions in the hIL-2 mutein component and the humanized anti-IL-2 antibody K2G11 component at the antigen-antibody binding interface.

Table 31. Detection of disulfide linkage in K2G11-Cy04

| Detected Peptide Fragment | Site of Disulfide Linkage in Peptide Fragment | Protease Treatment |
|---|---|---|
| SS1 | LC:C23 - LC:C88 | Trypsin |
| SS2<br>SS3 | LC:C52 - HC:C101<br>HC:C55 - HC:C102 | Trypsin |
| SS4 | LC:C134-LC:C194 | Trypsin |
| SS5 | LC:C214 - HC:C376 | trypsin |
| SS6 | HC:C172 - HC:C246 | Trypsin |
| SS7 | HC:C300 - HC:C356 | Trypsin |
| SS8<br>SS9 | HC:C382 - HC:C382<br>HC:C385 - HC:C385 | Trypsin |
| SS10 | HC:C417 - HC:C477 | Trypsin |
| SS11 | HC:C533 - HC:C581 | Trypsin |
| (The underlined site refers to the newly formed disulfide linkage.) | | |

Table 32. Detection of disulfide linkage in K2G1 1-Cy05

| Detected Peptide Fragment | Site of Disulfide Linkage in Peptide Fragment | Protease Treatment |
|---|---|---|
| SS1 | LC:C23 - LC:C88 | Trypsin + Chymotrypsin |
| SS2 | LC:C67 - HC:C99 | Trypsin + Chymotrypsin |
| SS3 | HC:C55 - HC:C102 | Trypsin + Glu-C |
| SS4 | LC:C134 - LC:C194 | Trypsin + Chymotrypsin |
| SS5 | LC:C214 - HC:C376 | Trypsin + Chymotrypsin |
| SS6 | HC:C172 - HC:C246 | Trypsin + Chymotrypsin |
| SS7 | HC:C300 - HC:C356 | Trypsin + Chymotrypsin |
| SS8<br>SS9 | HC:C382 - HC:C382<br>HC:C385 - HC:C385 | Trypsin + Chymotrypsin |
| SS10 | HC:C417 - HC:C477 | Trypsin + Chymotrypsin |

(continued)

| Detected Peptide Fragment | Site of Disulfide Linkage in Peptide Fragment | Protease Treatment |
|---|---|---|
| SS11 | HC:C533 - HC:C581 | Trypsin + Glu-C |
| (The underlined site refers to the newly formed disulfide linkage.) | | |

*Example 15: Pharmacokinetic Evaluation of AntiIL-2 Antibody with pI and TM mutations in its Heavy Chain Constant Region*

After introducing the pI mutation in the heavy chain constant region, the calculated pI value of anti-IL-2 antibody K2G11 is reduced from 7.56 to 6.54. To verify whether the reduced pI would lead to a prolonged plasma/serum half-life *in vivo,* the pharmacokinetics of the anti-IL-2 antibody K2G11 without the pI mutation and the anti-IL-2 antibody K2G11-pITM with pI and TM mutations in the heavy chain constant region were evaluated using BALB/c mice. The specific procedures were as follows: K2G11 or K2G11-pITM was injected into BALB/c mice via tail vein at a single dose of 10 mg/kg and 1 mg/kg, respectively. Blood samples were collected from the mice at 0.25, 1, 3, 8, 24, 48, 96, 168, 240, 336, 408 and 504 hours following the administration. Plasma samples were collected after centrifugation at 4°C and stored at - 80°C until analysis. The drug concentration in mouse plasma samples was determined by ELISA. The ELISA was performed as follows: a goat anti-human IgG Fc fragment antibody (Jackson Immunoresearch) was diluted in PBS to 1 $\mu$g/mL and added to a 96-well ELISA plate at 50 $\mu$L/well. The plate was coating overnight at 4°C. After washed with PBS, the plate was blocked with 1% BSA at room temperature for 1 hour. The testing samples were diluted in 1% BSA. In the meantime, mouse blank plasma was diluted in 1% BSA at a dilution factor same as the diluted testing samples, and this diluted mouse blank plasma was used as dilution buffer to dilute the K2G11 and K2G11-pITM standard samples to 1 $\mu$g/mL which was subsequently further diluted in a 3-fold serial dilution to prepare standard curve samples. The diluted testing samples and the standard curve samples were added to the ELISA plate at 50 $\mu$L per well and incubated at room temperature for 2 hours. After the plate was washed with PBST (PBS, pH7.4; 0.1% Tween-20), HRP-labeled goat anti-human IgG F(ab)$_2$ secondary antibody (Jackson Immunoresearch) diluted at a ratio of 1: 10,000 was added to each well at 50 $\mu$L and incubated at room temperature for 1 hour. After washed with PBST, TMB chromogenic solution was added to each well of the plate and incubated in dark at room temperature for 3-10 minutes. The reaction was stopped by adding 2 M HCl to each well. The OD values at 450 nm (OD450) and 570 nm (OD570) of each well were read using a multimode microplate reader (Varioskan, Thermo). OD450 was subtracted by OD570 to obtain the OD(450/570) value of each well. The standard curve was generated and the standard curve sample recovery rate was calculated using the Graphpad Prism 9 software, wherein the theoretical concentration of the standard curve was served as the abscissa and the OD(450/570) value was served as the ordinate. The recovery rate deviation = [(the measured concentration - the theoretical concentration)/the theoretical concentration] $\times$ 100%. The acceptance criteria for recovery rate deviation are $\pm$10% and used to determine the range of quantitation. If the OD(450/570) value of the testing sample is within the range of quantitation of the standard curve, the drug concentration of the testing sample is then extrapolated from the corresponding standard curve; if the value exceeds the range of quantitation, the dilution factor of the testing sample needs to be adjusted and the sample is retested by ELISA. The drug concentration of the testing sample was extrapolated from the standard curve according to its OD(450-570) value and back-calculated with the dilution factor, and then used to plot the drug concentration-time curve. Pharmacokinetic analysis was performed using the PKSolver 2.0 software in Microsoft EXCEL, and a non-compartmental model for intravenous administration was used for the pharmacokinetic parameter estimation.

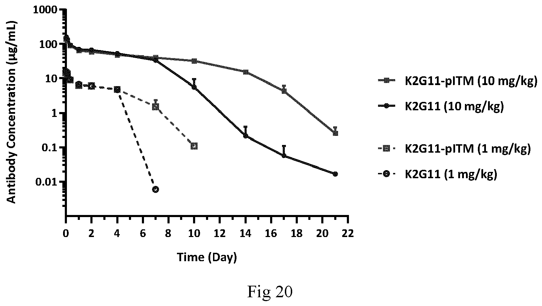

The drug concentration-time curves are shown in Figure 20. After introducing the pI mutation in the heavy chain constant region, the anti-IL-2 antibody K2G11-pITM exhibits a significantly improved pharmacokinetic property as compared to the K2G11 antibody without the pI mutation in the heavy chain constant region. At the dose of 1 mg/kg, the elimination half-life ($t_{1/2}$) and the area under the drug-time curve (AUC) of K2G11-pITM are approximately 2.5 and 1.3 times higher than K2G11. At the dose of 10 mg/kg, the half-life and AUC of K2G11-pITM are approximately 3.3 and 1.4 times higher than K2G11. These results demonstrate that the *in vivo* plasma half-life of the anti-IL-2 antibody K2G11-pITM in mice is significantly prolonged and the AUC is also significantly increased after introducing the pI mutation in the heavy chain constant region and resulting in a reduced pI.

*Example 16: In Vivo Pharmacodynamic Evaluation of hIL-2/anti-hIL-2 Antibody Fusion Protein*

16.1 *In Vivo* Pharmacodynamic Evaluation of hIL-2/anti-hIL-2 Antibody Fusion Protein in Cynomolgus Monkeys

CD8$^+$ T cell, NK cell and Treg cell counts and the changes in the proliferative marker Ki-67 expression in the peripheral blood of cynomolgus monkeys treated with the hIL-2/anti-hIL-2 antibody fusion protein K2G11-Cy05 prepared as described in Example 6 were evaluated. The specific methods were described below.

Dosing and Sampling: Two cynomolgus monkeys, one female and one male, were used. Each monkey was given three escalating doses (10, 100 and 300 μg/kg) of K2G11-Cy05 via intravenous injection once in a 7-day interval. Peripheral blood samples were collected before dosing and at 24 h, 72 h, and 168 h after the dosing. An additional peripheral blood sample was collected at 336 h with the high dose (300 μg/kg). Peripheral blood samples were analyzed by flow cytometry on the day of sampling.

Flow Cytometry: To each FACS tube, 5 μL of Human TruStain FcX reagent (Biolegend) was added, and then 100 μL of whole blood containing anticoagulant was added and incubated at room temperature for 10 minutes. Flow cytometry staining antibodies, BV510-labeled anti-human CD3 (BD), FITC-labeled anti-human CD4 (Biolegend), APC-labeled anti-human CD25 (Biolegend), AF700-labeled anti-human CD8 (Biolegend) and PE-Cy7-labeled anti-human CD16 (Biolegend), were mixed and added to the tube, mixed well and stained on ice for 60 minutes. 1×RBC Lysis Buffer (Biolegend) was added to the tube and incubated with the blood cells for 10-30 minutes in the dark at room temperature after gentle mixing. Cells were collected by centrifugation and washed once with pre-cooled FACS buffer (PBS buffer containing 1% BSA). True-Nuclear 1×Fix solution (Biolegend) was added to each tube and incubated at room temperature for 60 minutes after gentle mixing. True-Nuclear 1×Perm buffer was added to each tube and then centrifuged, followed by removing the supernatant; this step was repeated once. Cells in each tube were resuspended with True-Nuclear 1×Perm buffer, and flow cytometry antibodies, PE anti-human Foxp3 antibody (Biolegend) and eFluor 450-labeled anti-human Ki-67 antibody (Thermo), were added to the tube and incubated at room temperature for 40 minutes in the dark. The cells in each tube were washed with FACS buffer, resuspended in FACS buffer, and then analyzed by a flow cytometer (Agilent, NovoCyte 3005).

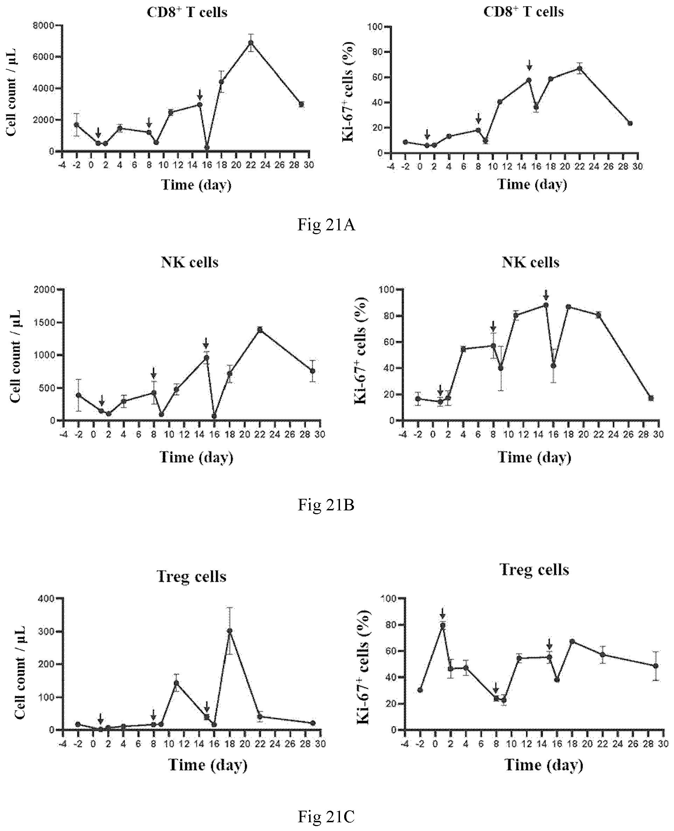

As shown in Figure 21, K2G11-Cy05 can significantly activate and promote the expansion of CD8$^+$ T cells (Figure 21A) and NK cells (Figure 21B) in the peripheral blood of cynomolgus monkeys, whereas the Treg cell counts only show a transient and minor increase at the medium and high doses, and the Ki-67 expression remains basically unchanged (Figure 21C). This Treg response may be provoked by the feedback regulation of the endogenous IL-2. Therefore, K2G11-Cy05 can selectively act on the IL2Rβ/γ-expressing CD8$^+$ T and NK cells in cynomolgus monkeys, promoting their proliferation, but cannot significantly activate the IL2Rα/β/γ-expressing Treg cells. During the entire period of study, there was no significant bodyweight change in animals, and no serious adverse effects were observed.

16.2 *In Vivo* Pharmacodynamic Evaluation of hIL-2/anti-hIL-2 Antibody Fusion Protein in C57BL/6 Mice

In the study, cell counts of memory CD8$^+$ T cells, NK cells, Treg cells, conventional CD4$^+$ T cells in the spleen of C57BL/6 mice treated with the hIL-2/anti-hIL-2 antibody fusion protein K2G11-Cy05 prepared as described in Example 6 were analyzed, the ratio of memory CD8$^+$ T cells to Treg cells and the ratio of NK cells to Treg cells were calculated, and the bodyweight changes in each treated mouse were measured. The specific methods were as follows.

Randomization and Dose Administration: C57BL/6 mice were randomized into 8 groups (at 5 mice per group), including a negative control group: PBS, a positive control group: hIL-2 (75 μg/kg), three experimental control groups: hIL-2-DM (Δ3, C125L) : K2G11-pITM immunocomplex formed by mixing hIL-2-DM (Δ3, C125L) and the anti-IL-2 antibody K2G11-pITM at a molar ratio of 2 : 1, where the dose levels of hIL-2-DM (Δ3, C125L) were 1.5 μg/kg, 5 μg/kg and 15 μg/kg, respectively, and three experimental groups: K2G11-Cy05 (10 μg/kg, 30 μg/kg and 100 μg/kg). The PBS group and the hIL-2 group were administered intravenously via tail vein once daily for a total of 4 doses. The experimental control groups (hIL-2-DM (Δ3, C125L) : K2G11-pITM immunocomplex) and the experimental groups (K2G11-Cy05) were administered intravenously via tail vein every two days for a total of 2 doses. The bodyweight of mice in each group was measured every other day. Mice in all groups were euthanized on the next day of the last injection, and the mouse splenocytes were collected and analyzed by flow cytometry.

Flow Cytometry: Mouse spleens were weighed, rinsed with ice-cold PBS, and placed onto a 200-mesh (75 μm) cell stainer (15-1070-ZX, Bilogix) and grinded to prepare the single splenocyte suspension. Splenocytes were collected by centrifugation, and 1×RBC Lysis Buffer (Biolegend) was added to each tube. After gentle shaking, the cells were incubated at room temperature for 5 minutes. Ice-cold PBS was added to stop the RBC lysis and cells were collected by centrifugation at 4°C and washed once with ice-cold PBS. Cells were resuspended in ice-cold PBS and dispended in a 96-well U-bottom deep-well plate for testing. All wells were tested in duplicate. Zombie Yellow (Biolegend) diluted 1:500 in ice-cold PBS was added to each well and incubated at room temperature for 15 minutes in the dark to stain dead cells. FACS buffer (PBS, pH7.4; 1% BSA; 2mM EDTA) was added to each well to stop the staining, and cells were collected by centrifugation. A Fc receptor blocking reagent was prepared, and cells were resuspended in 40 μL of FACS buffer containing 2 μL Mouse TruStain FcX reagent (Biolegend) and incubated on ice for 10 minutes. The flow cytometry staining antibodies, FITC-labeled anti-mouse CD3 (Biolegend), BV650-labeled anti-mouse CD4 (Biolegend), AF700-labeled anti-mouse CD8a (Biolegend), APC-labeled anti-mouse NK1.1 (Biolegend), PE-labeled anti-mouse CD25 (Biolegend) and BV510-labeled anti-mouse CD44 (Biolegend), were mixed and added to each well with 2 μL of each

antibody. Cells were incubated with the antibody mixture on ice for 40 minutes, and then washed once with FACS buffer and incubated with True-Nuclear 1×Fix at room temperature with shaking in the dark for 60 minutes. True-Nuclear 1×Perm buffer was added to each well and the supernatant was removed after centrifugation; this step was repeated once. Cells were stained with 2 μL of BV421-labeled anti-mouse Foxp3 antibody (Biolegend) prepared in 50 μL True-Nuclear 1×Perm buffer at room temperature for 60 minutes. Cells were washed twice with True-Nuclear 1×Perm buffer and resuspended in FACS buffer, and then analyzed by flow cytometry (Agilent NovoCyte 3005). Data were statistically analyzed by GraphPad Prism 9 software, differences between the experimental groups were analyzed by one-way Analysis of Variance (ANOVA), followed with Dunnett's test to analyze the differences between each experimental group and the PBS control group. If the $p$ value was less than 0.05, the difference was considered statistically significant.

As shown in Figure 22, compared to the PBS control, the Treg cell counts in the hIL-2 group is significantly increased ($p$ <0.0001) (Figure 22A), and the memory CD8+ T (CD8+ CD44 high) cell counts (Figure 22A) and NK cell counts (Figure 22A) also increase to some extent, but the increases are not statistically significant, indicating that high-dose hIL-2 preferentially stimulates and promotes the expansion of Treg cells *in vivo*. In the K2G11-Cy05 dose groups, the absolute cell counts of memory CD8+ T cells (Figure 22A) and NK cells (Figure 22A) are significantly increased in a dose-dependent manner as compared to the PBS control group and the hIL-2 group ($p$ <0.0001); although the Treg cell counts (Figure 22A) also shows a slight increase, it is not statistically different from the PBS control group, and the change is not dose-dependent, and it is lower than that of the hIL-2 group. The cell count ratio of memory CD8+ T cells to Treg cells (Figure 22B) and NK cells to Treg cells (Figure 22B) can reach up to about 100 and 40, respectively. These results confirm that K2G1 1-Cy05 is able to selectively activate and promote the proliferation of memory CD8+ T cells and NK cells *in vivo*. Compared to the hIL-2 group, the hIL-2-DM (Δ3,C125L) : K2G11-pITM immunocomplex induced only a modest activation of NK cells ($p$ <0.05) at the high dose (15 μg/kg), and there was no significant difference in the absolute cell count of memory CD8+ T and Treg cells; there is no statistical difference in the absolute cell counts of memory CD8+ T, NK and Treg cells in the other two dose groups as compared to the hIL-2 group. Although the corresponding IL-2 level of each dose group of hIL-2-DM (Δ3,C125L) : K2G11-pITM immunocomplex was equivalent to the dose groups of K2G11-Cy05, the effect of K2G11-Cy05 in inducing expansion of memory CD8+ T and NK cells is significantly stronger than the immunocomplex. In addition, there is no significant change in the conventional CD4+ T cell counts in all dose groups (Figure 22C). During the entire study period, there is no unscheduled animal death and no clinical adverse effects were observed, and there is no significant bodyweight change in mice (Figure 22D). These results suggest that K2G11-Cy05 is well-tolerated at dose levels used in the study.

16.3 Evaluation of Antitumor Activity of hIL-2/anti-hIL-2 Antibody Fusion Protein in Mouse CT26 Tumor Model

Balb/C mice were implanted subcutaneously with $5\times10^5$ CT26 colon carcinoma cells, and the day when the mean tumor volume reached approximately 100 mm³ was designated as Day 1. Tumor-bearing mice were randomized into 5 groups (8 mice per group), including the PBS control group, the NARA1leukin (0.3 mg/kg) control group, and three experimental groups of K2G11-Cy05 (0.03 mg/kg, 0.1 mg/kg and 0.3 mg/kg). The tumor-bearing mice in each group were dosed via intravenous injection through tail vein on Day 1, Day 4, Day 8 and Day 11. The tumor volume of tumor-bearing mice was measured on the day of injection, and the tumor volume was calculated using the following formula:

$$\text{Tumor Volume} = (\text{length}) \times (\text{width})^{2} \times 0.5.$$

As shown in Figure 23, K2G11-Cy05 significantly inhibited tumor growth in a dose-dependent manner. The mean tumor volume of the 0.3 mg/kg K2G11-Cy05 group was significantly smaller than that of the PBS control group. In addition, the antitumor activity of K2G11-Cy05 was higher than NARA1leukin at the same dose level. These results suggest that the hIL-2/hIL-2 antibody fusion protein of the present application has a potent antitumor activity by selectively activating and promoting the proliferation of memory CD8+ T and NK cells *in vivo*.

*Example 17: Construction of Stable hIL-2/anti-hIL-2 Antibody Fusion Proteins Comprising the other Anti-hIL-2 Antibody and hIL-2 with a Disulfide Linkage*

17.1 Construction of hIL-2/NARA1 Antibody Fusion Protein and hIL-2/TCB2 Antibody Fusion Protein

The hIL-2/NARA1 antibody fusion protein and the hIL-2/TCB2 antibody fusion protein were constructed according to the method described in Example 4. Briefly, hIL-2 was fused to the N-terminus of NARA1 or TCB2 antibody light chain through a linker of 4 tandem G4S sequences; the linker length can ensure the stability of internal structure of the constructed fusion protein and avoid interfering with the binding of hIL-2 to hIL-2Rβ. Next, the amino acid pairs in the antigen-antibody binding interface of hIL-2/NARA1 or hIL-2/TCB2 antigen-antibody complex with a spatial distance of 5 angstroms or less were identified, and these selected amino acid pairs were mutated to cysteine residues to form disulfide linkage and therefore further stabilize the interaction between the hIL-2 component and the NARA1 or TCB2 antibody component. The structure of constructed hIL-2/hIL-2 antibody fusion protein is schematically illustrated in Figure 4. The amino acid mutations and their sites for disulfide linkage formation between hIL-2 and the antibody NARA1 and TCB2 are shown in Table 33 and Table 34, respectively.

Table 33. Sites of amino acid mutations for disulfide linkage formation between hIL-2 and NARA1

| Sample ID | Position of Amino Acid Pair | Amino Acid Position in hIL2 | Amino Acid Position in NARA1 | Position of Introduced Cysteine Mutation in NARA1 |
|---|---|---|---|---|
| NARA1-Cy01 | IL:111-H:54 | T 111 | S 54 | HCDR2 |
| NARA1-Cy02 | IL:73-H:97 | A 73 | G 97 | HCDR3 |
| NARA1-Cy03 | IL:34-H:100B | P 34 | A 100B | HCDR3 |
| NARA1-Cy04 | IL:75-L:28 | S 75 | D 28 | LCDR1 |
| NARA1-Cy05 | IL:77-L:28 | N 77 | D 28 | LCDR1 |
| NARA1-Cy06 | IL:34-L:91 | P 34 | S 91 | LCDR3 |
| NARA1-Cy07 | IL:34-L:92 | P 34 | N 92 | LCDR3 |
| NARA1-Cy08 | IL:37-L:94 | T 37 | D 94 | LCDR3 |
| NARA1-Cy09 | IL:68-H:31 | E 68 | N 31 | HCDR1 |
| NARA1-Cy10 | IL:41-H:51 | T 41 | I 51 | HCDR2 |
| NARA1-Cy11 | IL:42-H:56 | F 42 | G 56 | HCDR2 |
| (IL represents IL-2; L represents antibody light chain; H represents antibody heavy chain; the number after IL or L/H represents the amino acid position for mutation.) | | | | |

Table 34. Sites of amino acid mutations for disulfide linkage formation between hIL-2 and TCB2

| Sample ID | Position of Amino Acid Pair | Amino Acid Position in hIL2 | Amino Acid Position in TCB2 | Position of Introduced Cysteine Mutation in TCB2 |
|---|---|---|---|---|
| TCB2-Cy01 | IL:68-H:97 | E 68 | A 97 | HCDR3 |
| TCB2-Cy02 | IL:42-H:99 | F 42 | R 99 | HCDR3 |
| TCB2-Cy03 | IL:68-H:100 | E 68 | G 100 | HCDR3 |
| TCB2-Cy04 | IL:38-H:100A | R 38 | F 100A | HCDR3 |
| TCB2-Cy05 | IL:109-H:54 | D 109 | D 54 | HCDR2 |
| TCB2-Cy06 | IL:34-L:27 | P 34 | T 27 | LCDR1 |
| TCB2-Cy07 | IL:34-L:92 | P 34 | D 92 | LCDR3 |

(continued)

| Sample ID | Position of Amino Acid Pair | Amino Acid Position in hIL2 | Amino Acid Position in TCB2 | Position of Introduced Cysteine Mutation in TCB2 |
|---|---|---|---|---|
| TCB2-Cy08 | IL:34-L:93 | P 34 | N 93 | LCDR3 |
| TCB2-Cy09 | IL:36-L:93 | L 36 | N 93 | LCDR3 |
| TCB2-Cy10 | IL:37-L:93 | T 37 | N 93 | LCDR3 |
| TCB2-Cy11 | IL:37-L:94 | T 37 | L 94 | LCDR3 |

(IL represents IL-2; L represents antibody light chain; H represents antibody heavy chain; the number after IL or L/H represents the amino acid position for mutation.)

The constructs shown in Tables 33 and Table 34 were constructed, expressed and purified according to the method described in Section 2.1 of Example 2, and obtained protein samples were analyzed by SEC-HPLC. Results showed that the following hIL-2/anti-IL-2 antibody fusion proteins were expressed mainly as monomer and selected for further evaluation: NARA1-Cy01, NARA1-Cy04, NARA1-Cy05, NARA1-Cy08, and NARA1-Cy09; or TCB2-Cy01, TCB2-Cy02, TCB2-Cy07, TCB2-Cy08, TCB2-Cy09, and TCB2-Cy11.

17.2 Binding Activity of hIL-2/anti-IL-2 Antibody Fusion Protein Comprising NARA1 or TCB2 to hIL2Rα (hCD25) and hIL2Rβ (hCD122) Determined by ELISA

The binding activity of hIL-2/anti-IL-2 antibody fusion protein comprising NARA1 or TCB2 to hCD25 or hCD122 was analyzed by ELISA according to the method described in Section 2.1.1 of Example 2, wherein the antigens were replaced by hCD25 and hCD122, respectively, and hIL2-DM-Fc was used as the control.

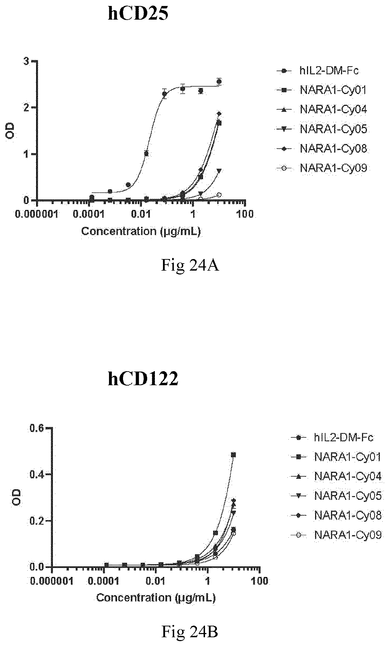

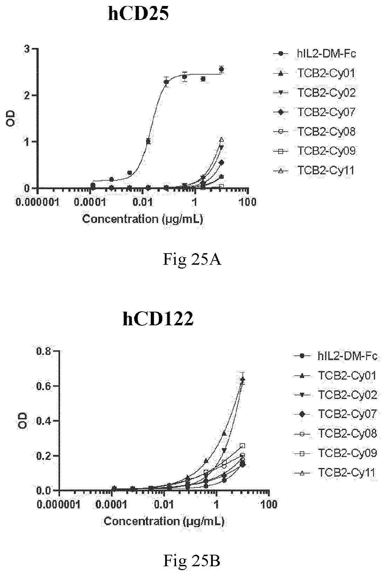

As shown in Figure 24 and Figure 25, compared to the hIL2-DM-Fc control, the hIL-2/anti-IL-2 antibody fusion proteins comprising NARA1 or TCB2 antibody exhibited significantly attenuated binding activities to hCD25 (Figure 24A and Figure 25A), whereas their binding activities to hCD122 were augmented (Figure 24B and Figure 25B). These results indicate that the hIL-2/anti-IL-2 antibody fusion protein comprising NARA1 or TCB2 antibody can retain or enhance the binding of hIL-2 to hCD122 and inhibit the binding of hIL-2 to hCD25, suggesting that the disulfide linkage introduced in the antigen-antibody binding interface of hIL-2/NARA1 and hIL-2/TCB2 antibody fusion proteins can enable the α subunit binding site of the hIL-2 component to be embedded inside of the antibody fusion protein and thus cannot be dissociated or competitively bound by hCD25.

17.3 Biological Activity of hIL-2/anti-IL-2 Antibody Fusion Proteins Comprising NARA1 or TCB2 Antibody

The biological activity of hIL-2/anti-IL-2 antibody fusion proteins comprising NARA1 or TCB2 antibody was evaluated by cell proliferation assay with CTLL-2 and NK-92 cells according to the method described in Section 1.3 of Example 1, wherein wild-type hIL-2 was used as the control.

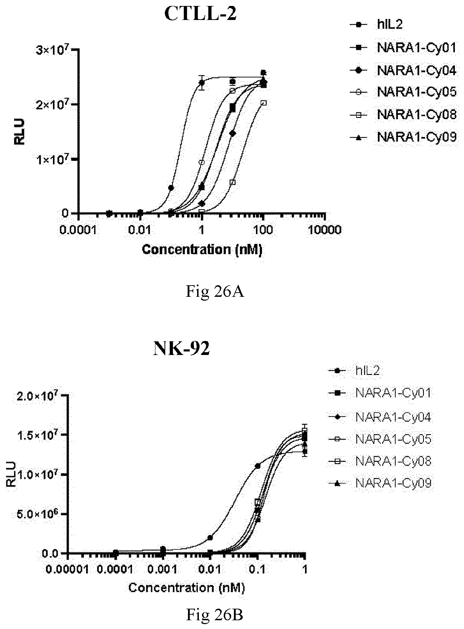

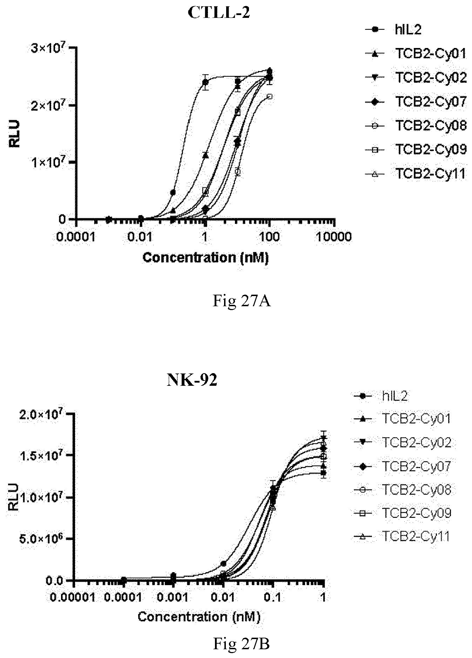

Results show that, compared to the hIL-2 control, hIL-2/NARA1 antibody fusion proteins (Figure 26A and Table 35) and hIL-2/TCB2 antibody fusion proteins (Figure 27A and Table 36) exhibit a different degree of reduction in their proliferative activity (EC50 value) in CTLL-2 cells which express a high level of CD25. In NK-92 cells which express a low level of CD25, these fusion proteins show a proliferative activity similar to the hIL-2 control (Figure 26B and Table 35, Figure 27B and Table 36).

Table 35. Biological activity of hIL-2/NARA 1 fusion proteins comprising NARA1 antibody determined by cell proliferation assay in CTLL-2 and NK-92 cells

| Sample ID | Proliferative Activity in CTLL-2 Cells (EC50, nM) | Proliferative Activity in NK-92 Cells (EC50, nM) |
|---|---|---|
| hIL-2 | 0.2101 | 0.03322 |
| NARA1-Cy01 | 2.915 | 0.1279 |
| NARA1-Cy04 | 7.464 | 0.1314 |
| NARA1-Cy05 | 1.340 | 0.1383 |

(continued)

| Sample ID | Proliferative Activity in CTLL-2 Cells (EC50, nM) | Proliferative Activity in NK-92 Cells (EC50, nM) |
|---|---|---|
| NARA1-Cy08 | 21.31 | 0.1201 |
| NARA1-Cy09 | 3.359 | 0.1463 |

Table 36. Biological activity of hIL-2/TCB2 fusion proteins comprising TCB2 antibody determined by cell proliferation assay in CTLL-2 and NK-92 cells

| Sample ID | Proliferative Activity in CTLL-2 Cells (EC50, nM) | Proliferative Activity in NK-92 Cells (EC50, nM) |
|---|---|---|
| hIL-2 | 0.2101 | 0.03322 |
| TCB2-Cy01 | 1.338 | 0.05139 |
| TCB2-Cy02 | 10.73 | 0.08893 |
| TCB2-Cy07 | 9278 | 0.07600 |
| TCB2-Cy08 | 13.06 | 0.05778 |
| TCB2-Cy09 | 3.763 | 0.07453 |
| TCB2-Cy11 | 3.766 | 0.09515 |

In summary, the disulfide linkage introduced in the binding interface of hIL-2 and the anti-IL-2 antibody specifically recognizing the IL2Rα binding site of IL-2 (such as NARA1 and TCB2) can stabilize the structure of complex formed by hIL-2 and the anti-IL-2 antibody, and prevent the hIL-2 component from binding to IL2Rα, while the activity of the hIL-2 component binding to hIL2Rβ/γ is not affected.

Although the present application has been described in terms of one, or two, or more embodiments, it should be understood that the present application is not limited to these embodiments and that the Description of the application is intended to cover all the substitutions, modifications and variations within the spirit and wide scope of the appended claims. All the references cited in the present application are incorporated into the present application by reference in their entirety.

**Claims**

1. A human interleukin-2 (hIL-2) mutein, wherein the hIL-2 mutein comprises at least one of the following properties: (1) the hIL-2 mutein comprises at least one amino acid mutation relative to the wild-type hIL-2 as set forth in SEQ ID NO: 1; (2) the hIL-2 mutein acquires the ability to bind to an anti-IL-2 antibody or an antigen-binding fragment thereof relative to the wild-type hIL-2 as set forth in SEQ ID NO: 1; and (3) the amino acid mutation has no obvious effect on biological activity of hIL-2;

   preferably, the hIL-2 mutein maintains the biological activity of the wild-type hIL-2; the anti-IL-2 antibody or antigen-binding fragment thereof is capable of competing with the IL-2 receptor alpha subunit (IL2Rα) for binding to the hIL-2 mutein ; the binding of the hIL-2 mutein to the anti-IL-2 antibody or antigen-binding fragment thereof can block the hIL-2 mutein from binding to the IL2Rα;
   or preferably, the hIL-2 mutein has 90% or more amino acid sequence identity, more preferably 93% or more amino acid sequence identity to the wild-type hIL-2 as set forth in SEQ ID NO: 1;
   or preferably, the hIL-2 mutein comprises one or more amino acid mutations relative to the wild-type hIL-2 as set forth in SEQ ID NO: 1.

2. The hIL-2 mutein of claim 1, wherein the amino acid mutation of the hIL-2 mutein is located in the binding interface between the hIL-2 mutein and the anti-IL-2 antibody or the antigen-binding fragment thereof, and preferably, the hIL-2 mutein comprises one or more amino acid mutations at positions 64, 90 and/or 104 relative to the amino acid sequence of the wild-type hIL-2 as set forth in SEQ ID NO: 1.

3. The hIL-2 mutein of claim 1 or 2, wherein, relative to the amino acid sequence of the wild-type hIL-2 as set forth in SEQ ID NO: 1, the hIL-2 mutein comprises at least one of the following mutations: (1) K is mutated to G, S, T, C, Y, N or Q at the position 64 corresponding to the wild-type hIL-2 as set forth in SEQ ID NO: 1; (2) N is mutated to R, K or H at the position 90 corresponding to the wild-type hIL-2 as set forth in SEQ ID NO: 1; or (3) M is mutated to E or D at the position 104 corresponding to the wild-type hIL-2 as set forth in SEQ ID NO: 1.

4. The hIL-2 mutein of any one of claims 1-3, wherein the hIL-2 mutein comprises one or more of the following amino acid mutations relative to the amino acid sequence of the wild-type hIL-2 as set forth in SEQ ID NO: 1: (1) an amino acid mutation K64G at the position 64 corresponding to the amino acid sequence of the wild-type hIL-2 as set forth in SEQ ID NO: 1; (2) an amino acid mutation N90R at the position 90 corresponding to the amino acid sequence of the wild-type hIL-2 as set forth in SEQ ID NO: 1; and (3) an amino acid mutation M104E at the position 104 corresponding to the amino acid sequence of the wild-type hIL-2 as set forth in SEQ ID NO: 1;

preferably, the hIL-2 mutein has amino acid mutations of K64G and/or N90R corresponding to the amino acid sequence of the wild-type hIL-2 as set forth in SEQ ID NO: 1;
or preferably, the hIL-2 mutein has amino acid mutations of K64G, N90R and M104E corresponding to the amino acid sequence of the wild-type hIL-2 as set forth in SEQ ID NO: 1;
more preferably, the hIL-2 mutein has amino acid mutations of K64G and N90R corresponding to the amino acid sequence of the wild-type hIL-2 as set forth in SEQ ID NO: 1.

5. The hIL-2 mutein of any one of claims 1-4, wherein the amino acid mutation of the hIL-2 mutein is located outside of the binding interface of the hIL-2 mutein and the anti-IL-2 antibody or antigen-binding fragment thereof;

preferably, the hIL-2 mutein comprises a deletion of the first three or five amino acids at the N-terminus of the amino acid sequence relative to the wild-type hIL-2 as set forth in SEQ ID NO: 1, or comprises an amino acid mutation of the third amino acid residue at the N-terminus of the amino acid sequence relative to the wild-type hIL-2 as set forth in SEQ ID NO: 1, and/or has an amino acid mutation at position 125 relative to the amino acid sequence of the wild-type hIL-2 as set forth in SEQ ID NO: 1;
or preferably, the hIL-2 mutein comprises amino acid mutations, wherein the amino acid mutations comprise a deletion of the first three N-terminal amino acids of the amino acid sequence of the wild-type hIL-2 as set forth in SEQ ID NO: 1, and/or amino acid mutation C125A, C125L, C125S, C125Q or C125V at the position 125 corresponding to the amino acid sequence of the wild-type hIL-2 as set forth in SEQ ID NO: 1.

6. An anti-IL-2 antibody or an antigen-binding fragment thereof, wherein the anti-IL-2 antibody or antigen-binding fragment thereof comprises at least one of the following properties: (1) the anti-IL-2 antibody or the antigen-binding fragment thereof is capable of competing with IL2R$\alpha$ for binding to the hIL-2 mutein of any one of claims 1-5 and competitively blocking the hIL-2 mutein of any one of claims 1-5 from binding with IL2R$\alpha$ or an IL-2 receptor complex comprising the IL2R$\alpha$ subunit; (2) the binding affinity value (KD) of the anti-IL-2 antibody or the antigen-binding fragment thereof to the hIL-2 mutein of any one of claims 1-5 is $< 5 \times 10^{-8}$ M, or preferably $< 1 \times 10^{-8}$ M, $< 5 \times 10^{-9}$ M, or $< 1 \times 10^{-9}$ M, or $< 5 \times 10^{-10}$ M, or more preferably $< 6 \times 10^{-10}$ M; and/or (3) the dissociation rate constant ($k_{dis}$ or $k_{off}$) of the anti-IL-2 antibody or the antigen-binding fragment thereof to the hIL-2 mutein of any one of claims 1-5 is $< 5 \times 10^{-3}$ s$^{-1}$, $< 1 \times 10^{-3}$ s$^{-1}$, $< 5 \times 10^{-4}$ s$^{-1}$, or $< 3 \times 10^{-4}$ S$^{-1}$.

7. The anti-IL-2 antibody or the antigen-binding fragment thereof of claim 6, wherein the anti-IL-2 antibody is selected from the group consisting of an anti-mouse IL-2 (mIL-2) antibody and a humanized antibody thereof, and further, the anti-mIL-2 antibody is selected from an anti-mIL-2 antibody that specifically recognizes the binding site of mIL-2 to mouse IL2R$\alpha$ (mIL2Ra), wherein the anti-mIL-2 antibody can cross-react with the hIL-2 mutein of any one of claims 1-5;
or preferably, the anti-IL-2 antibody or the antigen-binding fragment thereof comprises the following HCDR1, HCDR2 and HCDR3 of the heavy chain variable region, and LCDR1, LCDR2 and LCDR3 of the light chain variable region: (1) HCDR1 having an amino acid sequence of X1YYX2H (wherein X1=A or D; X2=L or M); (2) HCDR2 having an amino acid sequence of RIDPZ1DDSTKYAENFKX3 (wherein Z1=E or D; X3=N, S or G); (3) HCDR3 having an amino acid sequence of Z2TFZ3YZ4RELRWFAY (wherein Z2=V, I, R or T; Z3=Y, L, H or W; Z4=S, T, A, R, N, D, Q, E, H, I, L, K, F, P, W, Y or V), or preferably, Z2=V or I, Z3=Y or L, Z4=S or T; (4) LCDR1 having an amino acid sequence of QASQZ5IZ6NYLS (wherein Z5=D, Y, N, H, I, L or F; Z6=G, S, A, R, N, Q, I, L, T or D), or preferably, Z5=D or Y, Z6=G or S; (5) LCDR2 having an amino acid sequence of Z7AZ8SLAX4 (wherein Z7=S, Q, R, T, W or Y; Z8=T or G; X4=D, S or T), or preferably, Z7=S or Q, Z8=T, X4=S; and (6) LCDR3 having an amino acid sequence of LQHYSTPYT (as set forth in SEQ ID NO: 12).

8. The anti-IL-2 antibody or the antigen-binding fragment thereof of claim 6 or 7, wherein the anti-IL-2 antibody or antigen-binding fragment thereof comprises: HCDR1 as set forth in SEQ ID NOs: 7, 13 or 14; HCDR2 as set forth in SEQ ID NOs: 8, 15, 16 or 19; HCDR3 as set forth in SEQ ID NOs: 9, 20, 21, 22, 23, 24, 25, 29 or 34; and LCDR1 as set forth in SEQ ID NOs: 10, 26, 27, 30, 31, 35, 36, 37, 39, 40, 41, 42, 43 or 44; LCDR2 as set forth in SEQ ID NOs: 11, 17, 18, 28, 32 or 33; LCDR3 as set forth in SEQ ID NOs: 12 or 38.

9. The anti-IL-2 antibody or the antigen-binding fragment thereof of any one of claims 6-8, wherein the anti-IL-2 antibody or the antigen-binding fragment thereof comprises the following HCDR1, HCDR2 and HCDR3 of the heavy chain variable region, and LCDR1, LCDR2 and LCDR3 of the light chain variable region: (1) HCDR1 having an amino acid sequence as set forth in SEQ ID NO: 7, HCDR2 having an amino acid sequence as set forth in SEQ ID NO: 8, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NO: 11, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (2) HCDR1 having an amino acid sequence as set forth in SEQ ID NOs: 13 or 14, HCDR2 having an amino acid sequence as set forth in SEQ ID NO: 8, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NO: 11, and LCDR3 having an amino acid sequence as set forth in SEQ ID NO: 12, or amino acid sequences which are at least 85%, 86%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (3) HCDR1 having an amino acid sequence as set forth in SEQ ID NO: 7, HCDR2 having an amino acid sequence as set forth in SEQ ID NOs: 15 or 16, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NO: 11, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (4) HCDR1 having an amino acid sequence as set forth in SEQ ID NO: 7, HCDR2 having an amino acid sequence as set forth in SEQ ID NO: 8, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NOs: 17 or 18, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (5) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NO: 17, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively.

10. The anti-IL-2 antibody or the antigen-binding fragment thereof of any one of claims 6-9, wherein the anti-IL-2 antibody or the antigen-binding fragment thereof comprises the following HCDR1, HCDR2 and HCDR3 of the heavy chain variable region, and LCDR1, LCDR2 and LCDR3 of the light chain variable region: (1) HCDR1 having an amino acid sequence of X1YYX2H (wherein X1=A; X2=M); (2) HCDR2 having an amino acid sequence of RIDPZIDDSTKYAENFKX3 (wherein Z1=E or D ; X3=G); (3) HCDR3 having an amino acid sequence of Z2TFZ3YZ4RELRWFAY (wherein Z2=V, I, R or T; Z3=Y, L, H or W; Z4=S, T, A, R, N, D, Q , E, H, I, L, K, F, P, W, Y or V), or preferably Z2=V or I; Z3=Y or L; Z4=S or T; (4) LCDR1 having an amino acid sequence of QASQZ5IZ6NYLS (wherein Z5=D, Y, N, H, I, L or F; Z6=G, S, A, R, N, Q, I, L, T or D), or preferably Z5=D or Y; Z6=G or S; (5) LCDR2 having an amino acid sequence of Z7AZ8SLAX4 (wherein Z7=S, Q, R, T, W or Y; Z8=T or G; X4=D, S or T), or preferably Z7=S or Q; Z8=T; X4=S; and (6) LCDR3 having an amino acid sequence of LQHYSTPYT (as set forth in SEQ ID NO: 12).

11. The anti-IL-2 antibody or the antigen-binding fragment thereof of claim 10, wherein the anti-IL-2 antibody or the antigen-binding fragment thereof comprises the following HCDR1, HCDR2 and HCDR3 of the heavy chain variable region, and LCDR1, LCDR2 and LCDR3 of the light chain variable region: (1) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 9, LCDR1 having an amino acid sequence as set forth in SEQ ID NOs: 26 or 27, LCDR2 having an amino acid sequence as set forth in SEQ ID NOs: 17 or 28, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (2) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NOs: 20, 21, 22, 23, 24 or 25, LCDR1 having an amino acid sequence as set forth in SEQ ID NOs: 26 or 27, LCDR2 having an amino acid sequence as set forth in SEQ ID NOs: 17 or 28, and LCDR3 having an amino acid as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (3) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NOs: 9, 20, 23, 24 or 25, LCDR1 having an amino acid sequence as set forth in SEQ ID NOs: 26 or 27, LCDR2 having an amino acid sequence as set forth in SEQ ID NOs: 17 or 28, and LCDR3 having an amino acid as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively;

or preferably, the anti-IL-2 antibody or the antigen-binding fragment thereof comprises the following HCDR1, HCDR2 and HCDR3 of the heavy chain variable region, and LCDR1, LCDR2 and LCDR3 of the light chain variable region, comprising: (1) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NOs: 24 or 25, LCDR1 having an amino acid sequence as set forth in SEQ ID NOs: 26 or 27, LCDR2 having an amino acid sequence as set forth in SEQ ID NOs: 17 or 28, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (2) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NOs: 24 or 25, LCDR1 having an amino acid sequence as set forth in SEQ ID NOs: 26 or 27, LCDR2 having an amino acid sequence as set forth in SEQ ID NOs: 17 or 28, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively;
or more preferably, the anti-IL-2 antibody or the antigen-binding fragment thereof comprises the following HCDR1, HCDR2, HCDR3, LCDR1, LCDR2, and LCDR3, comprising: HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NO: 24, LCDR1 having an amino acid sequence as set forth in SEQ ID NO: 26, LCDR2 having an amino acid sequence as set forth in SEQ ID NO: 17, and LCDR3 amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively.

12. The anti-IL-2 antibody or the antigen-binding fragment thereof of any one of claims 6-11, wherein the anti-IL-2 antibody or the antigen-binding fragment thereof comprises: heavy chain variable region VH having at least 85% identity to a sequence as set forth in SEQ ID NOs: 5, 45, 47, 49, 51, 52, 53, 54, 55, 56, 57, 58, 63, 65, 66, 67, 68, 69, 70, 71, 72, 73, 74, 75, 86 or 112; and light chain variable region VL having at least 85% identity to a sequence as set forth in SEQ ID NOs: 6, 46, 48, 50, 59, 60, 61, 62, 64, 76, 77, 78, 79, 80, 81, 82, 83, 84, 85, 87, 88, 89, 90, 91, 92, 93, 104, 105, 106, 107, 108, 109, 110 or 111.

13. The anti-IL-2 antibody or the antigen-binding fragment thereof of any one of claims 6-12, wherein the anti-IL-2 antibody or the antigen-binding fragment thereof comprises the following heavy chain variable region having the HCDR1, HCDR2 and HCDR3, and/or the following light chain variable region having the LCDR1, LCDR2 and LCDR3: (1) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NOs: 45, 47 or 49 and/or the VL as set forth in SEQ ID NOs: 46, 48 or 50, respectively; or preferably, amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NO: 49 and/or the VL as set forth in SEQ ID NOs: 48 or 50, respectively; or (2) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NOs: 54, 55, 56, 57 or 58, and/or the VL as set forth in SEQ ID NO: 48, respectively; or (3) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NO: 49, and/or the VL as set forth in SEQ ID NOs: 59, 60 or 62, respectively; or (4) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NO: 63, and/or the VL as set forth in SEQ ID NO: 64, respectively;

alternatively, the anti-IL-2 antibody or the antigen-binding fragment thereof comprises the following heavy chain variable region having the HCDR1, HCDR2, and HCDR3, and/or the following light chain variable region having the LCDR1, LCDR2, and LCDR3: amino acid sequences which are at least 85%, 86%, 87%, 88%, 89%, 90%, 91%, 92%, 93%, 94%, 95%, 96%, 97%, 98%, 99% or 100% identical to the VH as set forth in any of SEQ ID NOs: 63 and 65-75, and/or the VL as set forth in any of SEQ ID NOs: 76-79, respectively;

alternatively, the anti-IL-2 antibody or the antigen-binding fragment thereof comprises the following heavy chain variable region having the HCDR1, HCDR2 and HCDR3, and/or the following light chain variable region having the LCDR1, LCDR2 and LCDR3: (1) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NO:71, and/or the VL as set forth in SEQ ID NO:76, respectively; or (2) amino acid sequences which are at least 85%, 86%, 87%, 88%, 89%, 90%, 91%, 92%, 93% 94%, 95%, 96%, 97%, 98%, 99% or 100% identical to the VH as set forth in SEQ ID NO:72, and/or the VL as set forth in SEQ ID NO:77, respectively; or (3) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID Nos: 72 or 74, and/or the VL as set forth in SEQ ID Nos: 76 or 79, respectively; or (4) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NOs: 74 or 75, and/or the VL as set forth in SEQ ID NOs: 77 or 78, respectively.

14. An hIL-2/anti-IL-2 antibody fusion protein, comprising: (1) the wild-type hIL-2 as set forth in SEQ ID NO: 1, or a mutein thereof; (2) an anti-IL-2 antibody or an antigen-binding fragment thereof; and (3) disulfide linkage(s) comprised at the antigen-antibody binding interface of hIL-2 or a mutein thereof and the anti-IL-2 antibody or the antigen-binding fragment thereof;

wherein the anti-IL-2 antibody or the antigen-binding fragment thereof can specifically bind to hIL-2 or a mutein thereof, and competitively block the hIL-2 or a mutein thereof from binding with IL2Rα or an IL-2 receptor complex comprising IL2Rα (i.e., IL2Rα/β or IL2Rα/β/γ); and

the disulfide linkage(s) is/are formed by introducing at least one cysteine mutation in the hIL-2 or a mutein thereof at the antigen-antibody binding interface and at least one cysteine mutation in the anti-IL-2 antibody or the antigen-binding fragment thereof at the antigen-antibody binding interface; and

the hIL-2/anti-IL-2 antibody fusion protein is unable to bind to IL2Rα, or the αβ heterodimer (i.e., IL2Rα/β), or the αβγ heterotrimer (i.e., IL2Rα/β/γ), but retains binding and functional activity to the dimeric receptor consisting of IL2Rβ and γ (i.e., IL2Rβ/γ).

15. The hIL-2/anti-IL-2 antibody fusion protein of claim 14, wherein the binding affinity (KD) of the anti-IL-2 antibody or the antigen-binding fragment thereof to the hIL-2 mutein is $< 5 \times 10^{-8}$ M, or preferably, $< 1 \times 10^{-8}$ M, $< 5 \times 10^{-9}$ M, or $< 1 \times 10^{-9}$ M, or $< 5 \times 10^{-10}$ M, or more preferably, $< 6 \times 10^{-10}$ M; and/or the dissociation rate constant ($k_{dis}$ or $k_{off}$) of the anti-IL-2 antibody or the antigen-binding fragment thereof to the hIL-2 mutein is $< 5 \times 10^{-3}$ s$^{-1}$, $< 1 \times 10^{-3}$ S$^{-1}$, $< 5 \times 10^{-4}$ s$^{-1}$, or $< 3 \times 10^{-4}$ s$^{-1}$.

16. The hIL-2/anti-IL-2 antibody fusion protein of claim 14 or 15, wherein the antigen-antibody binding interface of the hIL-2 or a mutein thereof and the anti-IL-2 antibody or the antigen-binding fragment thereof encompasses the binding interface of hIL-2 or a mutein thereof to IL2Rα, or the said antigen-antibody binding interface partially overlaps with the binding interface of hIL-2 or a mutein thereof to IL2Rα;

alternatively, the antigen-antibody binding interface of the hIL-2 or a mutein thereof and the anti-IL-2 antibody or the antigen-binding fragment thereof is located in AB loop, B helix, C helix, BC loop and/or CD loop, or preferably AB loop, B helix, C helix and CD loop of the hIL-2 or a mutein thereof; the IL2Rα binding interface of the hIL-2 or a mutein thereof is located in AB loop and B helix of the hIL-2 or a mutein thereof;

alternatively, the antigen-antibody binding interface of the hIL-2 or a mutein thereof and the anti-IL-2 antibody or the antigen-binding fragment thereof is located at the position 30-45 or their adjacent amino acid residues, the position 57-77 or their adjacent amino acid residues, and/or the position 90-111 or their adjacent amino acid residues of the hIL-2 or a mutein thereof.

**17.** The hIL-2/anti-IL-2 antibody fusion protein according to any one of claims 14-16, wherein amino acid mutations of the hIL-2 mutein are located in the binding interface of the hIL-2 mutein and the anti-IL-2 antibody or the antigen-binding fragment thereof, or preferably, the hIL-2 mutein comprises one or more amino acid mutations at the position 64, 90 and/or 104 relative to the amino acid sequence of the wild-type hIL-2 as set forth in SEQ ID NO: 1.

**18.** The hIL-2 mutein according to any one of claims 14-17, wherein relative to the amino acid sequence of the wild-type hIL-2 as set forth in SEQ ID NO: 1, the hIL-2 mutein comprises at least one of the following mutations: (1) K is mutated to G, S, T, C, Y, N or Q at the position 64 corresponding to the wild-type hIL-2 as set forth in SEQ ID NO: 1; (2) N is mutated to R, K or H at the position 90 corresponding to the wild-type hIL-2 as set forth in SEQ ID NO: 1; or (3) M is mutated to E or D at the position 104 corresponding to the wild-type hIL-2 as set forth in SEQ ID NO: 1.

**19.** The hIL-2/anti-IL-2 antibody fusion protein according to any one of claims 14-18, wherein the hIL-2 mutein comprises one or more of the following amino acid mutations relative to the amino acid sequence of the wild-type hIL-2 as set forth in SEQ ID NO: 1: (1) an amino acid mutation K64G at the position 64 corresponding to the amino acid sequence of the wild-type hIL-2 as set forth in SEQ ID NO: 1; (2) an amino acid mutation N90R at the position 96 corresponding to the amino acid sequence of the wild-type hIL-2 as set forth in SEQ ID NO: 1; and (3) an amino acid mutation M104E at the position 104 corresponding to the amino acid sequence of the wild-type hIL-2 as set forth in SEQ ID NO: 1;

preferably, the hIL-2 mutein has amino acid mutations K64G and/or N90R corresponding to the amino acid sequence of the wild-type hIL-2 as set forth in SEQ ID NO: 1; or preferably, the hIL-2 mutein has amino acid mutations K64G, N90R and M104E (as set forth in SEQ ID NO: 3) corresponding to the amino acid sequence of the wild-type hIL-2 as set forth in SEQ ID NO: 1;

or more preferably, the hIL-2 mutein has amino acid mutations K64G and N90R (as set forth in SEQ ID NO: 2) corresponding to the amino acid sequence of the wild-type hIL-2 as set forth in SEQ ID NO: 1.

**20.** The hIL-2/anti-IL-2 antibody fusion protein of any one of claims 14-19, wherein the anti-IL-2 antibody or the antigen-binding fragment thereof is selected from the group consisting of an anti-mouse IL-2 (mIL-2) antibody and a humanized antibody thereof; further, the anti-mIL-2 antibody is selected from an anti-mIL-2 antibody that specifically recognizes the binding site of mIL-2 to mouse IL2Rα (mIL2Rα ), and the anti-mIL-2 antibody can cross-react with the hIL-2 mutein; the anti-IL-2 antibody can also be the other humanized anti-IL-2 antibody which is capable of specifically recognizing the II,2Rα-binding site of hIL-2, or an antigen-binding fragment thereof, or preferably the other humanized anti-IL-2 antibody is selected from NARA1 and TCB2;

preferably, the anti-IL-2 antibody or the antigen-binding fragment thereof comprises the following HCDR1, HCDR2 and HCDR3 of the heavy chain variable region, and LCDR1, LCDR2 and LCDR3 of the light chain variable region: (1) HCDR1 having an amino acid sequence of X1YYX2H (wherein X1=A or D; X2=L or M); (2) HCDR2 having an amino acid sequence of RIDPZ1DDSTKYAENFKX3 (wherein Z1=E or D; X3=N, S or G); (3) HCDR3 having an amino acid sequence of Z2TFZ3YZ4RELRWFAY (wherein Z2=V, I, R or T; Z3=Y, L, H or W; Z4=S, T, A, R, N, D, Q, E, H, I, L, K, F, P, W, Y or V), or preferably Z2=V or I; Z3=Y or L; Z4=S or T; (4) LCDR1 having an amino acid sequence of QASQZ5IZ6NYLS (wherein Z5=D, Y, N, H, I, L or F; Z6=G, S, A, R, N, Q, I, L, T or D), or preferably, Z5=D or Y; Z6=G or S; (5) LCDR2 having an amino acid sequence of Z7AZ8SLAX4 (wherein Z7=S, Q, R, T, W or Y; Z8=T or G; X4=D, S or T), preferably, Z7=S or Q; Z8=T; X4=S; and (6) LCDR3 having an amino acid sequence of LQHYSTPYT (as set forth in SEQ ID NO: 12).

**21.** The hIL-2/anti-IL-2 antibody fusion protein of any one of claims 14-20 , wherein the anti-IL-2 antibody or the antigen-binding fragment thereof comprises: HCDR1 as set forth in SEQ ID NOs: 7, 13 or 14; HCDR2 as set forth in SEQ ID NOs: 8, 15, 16 or 19; HCDR3 as set forth in SEQ ID NOs: 9, 20, 21, 22, 23, 24, 25, 29 or 34; LCDR1 as set forth in SEQ ID NOs: 10, 26, 27, 30, 31, 35, 36, 37, 39, 40, 41, 42, 43 or 44; LCDR2 as set forth in 11, 17, 18, 28, 32 or 33; and LCDR3 as set forth in SEQ ID NOs: 12 or 38.

**22.** The hIL-2/anti-IL-2 antibody fusion protein of claim 20 or 21, wherein the anti-IL-2 antibody or the antigen-binding fragment thereof comprises the following HCDR1, HCDR2, and HCDR3 of the heavy chain variable region, and LCDR1, LCDR2 and LCDR3 of the light chain variable region: (1) HCDR1 having an amino acid sequence as set forth in SEQ ID NO: 7, HCDR2 having an amino acid sequence as set forth in SEQ ID NO: 8, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NO: 11, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (2) HCDR1 having an amino acid sequence as set forth in SEQ ID NOs: 13 or 14, HCDR2 having an amino acid sequence as set forth in SEQ ID NO: 8, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NO: 11, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (3) HCDR1 having an amino acid sequence as set forth in SEQ ID NO: 7, HCDR2 having an amino acid sequence as set forth in SEQ ID NOs: 15 or 16, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NO: 11, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (4) HCDR1 having an amino acid sequence as set forth in SEQ ID NO: 7, HCDR2 having an amino acid sequence as set forth in SEQ ID NO: 8, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NOs: 17 or 18, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (5) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NO: 17, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively;
preferably, the anti-IL-2 antibody or the antigen-binding fragment thereof in the hIL-2/anti-IL-2 antibody fusion protein comprises the following HCDR1, HCDR2, HCDR3, LCDR1, LCDR2, and LCDR3: HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NO: 17, and LCDR3 having an amino acid as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively.

23. The hIL-2/anti-IL-2 antibody fusion protein of any one of claims 20-22, wherein the anti-IL-2 antibody or the antigen-binding fragment thereof comprises the following HCDR1, HCDR2 and HCDR3 of the heavy chain variable region, and LCDR1, LCDR2 and LCDR3 of the light chain variable region: (1) HCDR1 having an amino acid sequence of X1YYX2H (wherein X1=A; X2=M); (2) HCDR2 having an amino acid sequence of RIDPZ1DDSTKYAENFKX3 (wherein Z1=E or D; X3=G); (3) HCDR3 having an amino acid sequence of Z2TFZ3YZ4RELRWFAY (wherein Z2=V, I, R or T; Z3=Y, L, H or W; Z4=S, T, A, R, N, D, Q, E, H, I, L, K, F, P, W, Y or V); preferably, Z2=V or I; Z3=Y or L; Z4=S or T; (4) LCDR1 having an amino acid sequence of QASQZ5IZ6NYLS (wherein Z5=D, Y, N, H, I, L or F; Z6=G, S, A, R, N, Q, I, L, T or D); preferably, Z5=D or Y; Z6=G or S; (5) LCDR2 having an amino acid sequence of Z7AZ8SLAX4 (wherein Z7=S, Q, R, T, W or Y; Z8=T or G; X4=D, S or T); preferably, Z7=S or Q; Z8=T; X4=S; and (6) LCDR3 having an amino acid sequence of LQHYSTPYT;

preferably, the anti-IL-2 antibody or the antigen-binding fragment thereof comprises the following HCDR1, HCDR2 and HCDR3 of the heavy chain variable region, and the following LCDR1, LCDR2 and LCDR3 of the light chain variable region: (1) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 9, LCDR1 having an amino acid sequence as set forth in SEQ ID NOs: 26 or 27, LCDR2 having an amino acid sequence as set forth in SEQ ID NOs: 17 or 28, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (2) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NOs: 20, 21, 22, 23, 24 or 25, LCDR1 having an amino acid sequence as set forth in SEQ ID NOs: 26 or 27, LCDR2 having an amino acid sequence as set forth in SEQ ID NOs: 17 or 28, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (3) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NOs: 9, 20, 23, 24 or 25, LCDR1 amino acid sequence as set forth in SEQ ID NOs: 26 or 27, LCDR2 having an amino acid sequence as set forth in SEQ ID NOs: 17 or 28, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively;

alternatively, the anti-IL-2 antibody or the antigen-binding fragment thereof comprises the following HCDR1, HCDR2 and HCDR3 of the heavy chain variable region, and LCDR1, LCDR2 and LCDR3 of the light chain variable region: (1) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NOs: 24 or 25, LCDR1 having an amino acid sequence as set forth in SEQ ID NOs: 26 or 27, LCDR2 having an amino acid sequence as set forth in SEQ ID NOs: 17 or 28, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively; or (2) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NOs: 24 or 25, LCDR1 having an amino acid sequence as set forth in SEQ ID NOs: 26 or 27, LCDR2 having an amino acid sequence as set forth in SEQ ID NOs: 17 or 28, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively;

preferably, the anti-IL-2 antibody or the antigen-binding fragment thereof comprises the following HCDR1, HCDR2, HCDR3, LCDR1, LCDR2, and LCDR3: HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NO: 24, LCDR1 having an amino acid sequence as set forth in SEQ ID NO: 26, LCDR2 having an amino acid sequence as set forth in SEQ ID NO: 17, and LCDR3 having an amino acid sequence as set forth in SEQ ID NO: 12, or amino acid sequences which are at least 85%, 86%, 87%, 88%, 89%, 90%, 91%, 92%, 93%, 94%, 95%, 96%, 97%, 98%, 99% or 100% identical to the said HCDR1, HCDR2, HCDR3, LCDR1, LCDR2, and LCDR3, respectively.

24. The hIL-2/anti-IL-2 antibody fusion protein of any one of claims 14-23, wherein the anti-IL-2 antibody or the antigen-binding fragment thereof comprises: heavy chain variable region VH having at least 85% identity to a sequence as set forth in SEQ ID NOs: 5, 45, 47, 49, 51, 52, 53, 54, 55, 56, 57, 58, 63, 65, 66, 67, 68, 69, 70, 71, 72, 73, 74, 75, 86 or 112; and light chain variable region VL having at least 85% identity to a sequence as set forth in SEQ ID NOs: 6, 46, 48, 50, 59, 60, 61, 62, 64, 76, 77, 78, 79, 80, 81, 82, 83, 84, 85, 87, 88, 89, 90, 91, 92, 93, 104, 105, 106, 107, 108, 109, 110 or 111.

25. The hIL-2/anti-IL-2 antibody fusion protein of any one of claims 14-24, wherein the anti-IL-2 antibody or the antigen-binding fragment thereof comprises the following heavy chain variable region having the HCDR1, HCDR2 and HCDR3, and the following light chain variable region having the LCDR1, LCDR2 and LCDR3: (1) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NOs: 45, 47 or 49, and/or the VL as set forth in SEQ ID NOs: 46, 48 or 50, respectively; preferably, amino acid sequences which are at least 85%, 86%, 87%, 88%, 89%, 90%, 91%, 92%, 93%, 94%, 95%, 96%, 97%, 98%, 99% or 100% identical to the VH amino acid sequence as set forth in SEQ ID NO: 49, and/or the VL as set forth in SEQ ID NOs: 48 or 50, respectively; or (2) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NOs: 54, 55, 56, 57 or 58, and/or the VL as set forth in SEQ ID NO: 48, respectively; or (3) amino acid sequences which are at least 85%, 86%, 87%, 88%, 89%, 90%, 91%, 92%, 93%, 94%, 95%, 96%, 97%, 98%, 99% or 100% identical to the VH amino acid sequence as set forth in SEQ ID NO: 49, and/or the VL as set forth in SEQ ID NOs: 59, 60 or 62, respectively; or (4) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NO: 63, and/or the VL as set forth in SEQ ID NO: 64, respectively;

alternatively, the anti-IL-2 antibody or the antigen-binding fragment thereof comprises the following heavy chain variable region having the HCDR1, HCDR2, and HCDR3, and/or the following light chain variable region having the LCDR1, LCDR2, and LCDR3: amino acid sequences which are at least 85%, 86%, 87%, 88%, 89%, 90%, 91%, 92%, 93%, 94%, 95%, 96%, 97%, 98%, 99% or 100% identical to the VH as set forth in any of SEQ ID NOs: 63 and 65-75, and/or the VL as set forth in any of SEQ ID NOs: 76-79, respectively;

alternatively, the anti-IL-2 antibody or the antigen-binding fragment thereof comprises the following heavy chain variable region having the HCDR1, HCDR2 and HCDR3, and/or the following light chain variable region having the LCDR1, LCDR2 and LCDR3: (1) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NO:71, and/or the VL as set forth in SEQ ID NO:76, respectively; or (2) amino acid sequences which are at least 85%, 86%, 87%, 88%, 89%, 90%, 91%, 92%, 93% 94%, 95%, 96%, 97%, 98%, 99% or 100% identical to the VH as set forth in SEQ ID NO:72, and/or the VL as set forth in SEQ ID NO:77, respectively; or (3) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID Nos: 72 or 74, and/or the VL as set forth in SEQ ID Nos: 76 or 79, respectively; or (4) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NOs: 74 or 75, and/or the VL as set forth in SEQ ID NOs: 77 or 78, respectively.

26. The hIL-2/anti-IL-2 antibody fusion protein of any one of claims 14-25 , wherein the antigen-antibody binding interface of the hIL-2 mutein and the anti-IL-2 antibody or the antigen-binding fragment thereof of the IL-2/anti-IL-2 antibody fusion protein comprises disulfide linkage(s), and the said antigen-antibody binding interface overlaps with the binding interface of the hIL-2 mutein to IL2Rα, and further, the antigen-antibody binding interface of the hIL-2 mutein and the anti-IL-2 antibody or the antigen-binding fragment thereof is located in AB loop, B helix, C helix, BC loop and CD loop of the hIL-2 mutein, preferably located at the position 41-45 or their adjacent amino acid residues, the position 57-68 or their adjacent amino acid residues, and/or the position 90-107 or their adjacent amino acid residues of the hIL-2 mutein; the disulfide linkage(s) is/are formed by at least one cysteine residue introduced in the anti-IL-2 antibody or the antigen-binding fragment thereof at the antigen-antibody binding interface and at least one cysteine residue introduced in the hIL-2 mutein at the antigen-antibody binding interface;

preferably, the antigen-antibody binding interface of the hIL-2 mutein and the anti-IL-2 antibody or the antigen-binding fragment thereof of the hIL-2/anti-IL-2 antibody fusion protein comprises two or more introduced cysteine residues to form the said disulfide linkage.

27. The hIL-2/anti-IL-2 antibody fusion protein of claim 26, wherein the hIL-2 mutein has one or more amino acid mutations at the positions 42, 60, 61, 64, 65, 102, and 104 corresponding to the amino acid sequence of the wild-type hIL-2 as set forth in SEQ ID NO: 1, to introduce a first cysteine residue.

28. The hIL-2/anti-IL-2 antibody fusion protein of claim 26, wherein the anti-IL-2 antibody or the antigen-binding fragment thereof comprises: (1) HCDR1 having an amino acid sequence of X1YYX2H (wherein X1=A; X2=M); (2) HCDR2 having an amino acid sequence of RIDPZ1DDSTKYAENFKX3 (wherein Z1=E or D; X3=G); (3) HCDR3 having an amino acid sequence of Z2TFZ3YZ4RELRWFAY (wherein Z2=V or I; Z3=Y or L; Z4=S or T); (4) LCDR1 having an amino acid sequence of QASQZ5IZ6NYLS (wherein Z5=D or Y; Z6=G or S); (5) LCDR2 having an amino acid sequence of Z7AZ8SLAX4 (wherein Z7=S or Q; Z8=T; X4=S), and (6) LCDR3 having an amino acid sequence of LQHYSTPYT (as set forth in SEQ ID NO: 12), and the anti-IL-2 antibody or the antigen-binding fragment thereof has one or more amino acid mutations at the position 3 of HCDR3, at the positions 7, 8 and/or 9 of LCDR1, at the positions 1 and 3 of LCDR2, or at position 4 of LCDR3, to introduce a second cysteine residue.

29. The hIL-2/anti-IL-2 antibody fusion protein of claim 26, wherein the anti-IL-2 antibody or the antigen-binding fragment thereof comprises the following heavy chain variable region having the HCDR1, HCDR2 and HCDR3, and/or the following light chain variable region having the LCDR1, LCDR2 and LCDR3: (1) amino acid sequences which are at least 85%, 86%, 87%, 88%, 89%, 90%, 91%, 92%, 93%, 94%, 95%, 96%, 97%, 98%, 99% or 100% identical to the VH as set forth in SEQ ID NO: 63, and/or the VL as set forth in SEQ ID NO: 64, respectively; or (2) amino acid sequences which are at least 85%, 86%, 87%, 88%, 89%, 90%, 91%, 92%, 93%, 94%, 95%, 96%, 97%, 98%, 99% or 100% identical to the VH as set forth in any of SEQ ID NOs: 63 and 65-75, and/or the VL as set forth in any of SEQ ID NOs: 76-79, respectively; and the anti-IL-2 antibody or the antigen-binding fragment thereof has one or more amino acid mutations in the framework region of the light chain variable region to introduce a second cysteine residue, and the amino acid mutation is located at the positions 67 and/or 68 of the said light chain variable region.

30. The hIL-2/anti-IL-2 antibody fusion protein of any one of claims 26-29, wherein the first cysteine residue is introduced into the hIL-2 mutein at the antigen-antibody binding interface of the hIL-2/anti-IL-2 antibody fusion protein by amino acid mutation, and the second cysteine is introduced into the humanized anti-IL-2 antibody or the antigen-binding fragment thereof at the antigen-antibody binding interface by amino acid mutation, and preferably, the amino acid mutation is amino acid substitution, insertion or addition, and the site for introducing the first cysteine residue in the hIL-2 mutein and the site for introducing the second cysteine in the humanized anti-IL-2 antibody or the antigen-binding fragment thereof can be paired correctly, and the correctly paired sites are located at the antigen-antibody binding interface of the hIL-2 mutein and the humanized anti-IL-2 antibody or the antigen-binding fragment thereof, and the spatial distance between paired amino acid residues is less than 10 angstroms, and preferably, the spatial distance is less than 5 angstroms.

31. The hIL-2/anti-IL-2 antibody fusion protein of any one of claims 26-30, wherein the pair of the first cysteine residue and the second cysteine residue for forming disulfide linkage at the antigen-antibody binding interface of the hIL-2 mutein and the humanized antibody or the antigen-binding fragment thereof of the hIL-2/anti-IL-2 antibody fusion protein is selected from one or more of the following paired amino acid mutations: (1) the first cysteine residue is introduced by a mutation F42C at the position 42, a mutation of K64C at the position 64, or a mutation of P65C at the position 65 of the hIL-2 mutein corresponding to the amino acid sequence of the wild-type hIL-2 as set forth in SEQ ID NO: 1; and the second cysteine residue is introduced by a mutation of F3C at the position 3 of HCDR3 having the amino acid sequence of Z2TFZ3YZ4RELRWFAY (wherein Z2=V or I; Z3=Y or L; Z4=S or T) in the humanized anti-IL-2 antibody or the antigen-binding fragment thereof; and/or (2) the first cysteine residue is introduced by a mutation of E60C at the position 60 of the hIL-2 mutein corresponding to the amino acid sequence of the wild-type hIL-2 as set forth in SEQ ID NO: 1; and the second cysteine residue is introduced by a mutation of G7C or S7C at the position 7 of LCDR1 having the amino acid sequence of QASQZ5IZ6NYLS (wherein Z5=D or Y; Z6=G or S) in the humanized anti-IL-2 antibody or the antigen-binding fragment thereof; and/or (3) the first cysteine residue is introduced by a mutation of E61C at the position 61 of the hIL-2 mutein corresponding to the amino acid sequence of the wild-type hIL-2 as set forth in SEQ ID NO: 1; and the second cysteine residue is introduced by a mutation of G7C or S7C at the position 7 of LCDR1 having the amino acid sequence of QASQZSIZ6NYLS (wherein Z5=D or Y; Z6=G or S) in the humanized anti-IL-2 antibody or the antigen-binding fragment thereof, or the second cysteine residue is introduced by a mutation of N8C at the position 8 of LCDR1 having the amino acid sequence of QASQZ5IZ6NYLS (wherein Z5=D or Y; Z6=G or S) in the humanized anti-IL-2 antibody or the antigen-binding fragment thereof, or the second cysteine residue is introduced by a mutation of S1C or Q1C at the position 1 of LCDR2 having the amino acid sequence of Z7AZ8SLAX4 (wherein Z7=S or Q; Z8=T; X4=S) in the humanized anti-IL-2 antibody or the antigen-binding fragment thereof; and/or (4) the first cysteine residue is introduced by a mutation of M104C at the position 104 of the hIL-2 mutein corresponding to the amino acid sequence of the wild-type hIL-2 as set forth in SEQ ID NO: 1; and the second cysteine residue is introduced by a mutation of T3C at the position 3 of LCDR2 having the amino acid sequence of Z7AZ8SLAX4 (wherein Z7=S or Q; Z8=T; X4=S) in the humanized anti-IL-2 antibody or the antigen-binding fragment thereof; and/or (5) the first cysteine residue is introduced by a mutation of T102C at the position 102 of the hIL-2 mutein corresponding to the amino acid sequence of the wild-type hIL-2 as set forth in SEQ ID NO: 1; and the second cysteine residue is introduced by a mutation S67C at the position 67 of the light chain variable region of the humanized anti-IL-2 antibody or the antigen-binding fragment thereof, or the second cysteine residue is introduced by a mutation of G68C at the position 68 of the light chain variable region of the humanized anti-IL-2 antibody or the antigen-binding fragment thereof; and/or (6) the first cysteine residue is introduced by a mutation of K64C at the position 64 of the hIL-2 mutein corresponding to the amino acid sequence of the wild-type hIL-2 as set forth in SEQ ID NO: 1; and the second cysteine residue is introduced by a mutation of Y9C at the position 9 of LCDR1 having the amino acid sequence of QASQZ5IZ6NYLS (wherein Z5=D or Y; Z6=G or S) in the humanized anti-IL-2 antibody or the antigen-binding fragment thereof, or the second cysteine residue is introduced by a mutation of Y4C at the position 4 of LCDR3 having the amino acid sequence LQHYSTPYT (SEQ ID NO: 12) of the humanized anti-IL-2 antibody or the antigen-binding fragment thereof; alternatively, the pair of the first cysteine residue and the second cysteine residue for forming the disulfide linkage at the antigen-antibody binding interface of the hIL-2 mutein and the humanized antibody or the antigen-binding fragment thereof in the hIL-2/anti-IL-2 antibody fusion protein is selected from one or more of the following paired amino acid mutations: (1) the first cysteine residue is introduced by a mutation of M104C at the position 104 of the hIL-2 mutein corresponding to the amino acid sequence of the wild-type hIL-2 as set forth in SEQ ID NO: 1, and the second cysteine residue is introduced by a mutation of T3C at the position 3 of LCDR2 in the humanized anti-IL-2 antibody or the antigen-binding fragment thereof, wherein the humanized anti-IL-2 antibody or the antigen-binding fragment thereof comprises HCDR1 having the amino acid sequence as set forth in SEQ ID NO: 14, HCDR2 having the amino acid sequence as set forth in SEQ ID NO: 19, HCDR3 having the amino acid sequence as set forth in SEQ ID NO: 24, LCDR1 having the amino acid sequence as set forth in SEQ ID NO: 26, LCDR2 having the amino acid sequence as set forth in SEQ ID NO: 17, and LCDR3 having the amino acid sequence as set forth in SEQ ID NO: 12; and/or (2) the first cysteine residue is introduced by a mutation of T102C at the position 102 of the hIL-2 mutein corresponding to the amino acid sequence of the wild-type hIL-2 as set forth in SEQ ID NO: 1, and the second cysteine residue is introduced by a mutation of S67C at the position 67 of the light chain variable region of the humanized anti-IL-2 antibody or the antigen-binding fragment thereof, wherein the humanized anti-IL-2 antibody or the binding fragment thereof comprises an amino acid sequence which is 85%, 86%, 87%, 88%, 89%, 90%, 91%, 92%, 93%, 94%, 95%, 96%, 97%, 98%, 99% or 100% identical to the VL as set forth in SEQ ID NO: 77; or the second cysteine residue is introduced by a mutation of G68C at the position 68 of the light chain variable region of the humanized anti-IL-2 antibody or the antigen-binding fragment thereof, wherein the humanized anti-IL-2 antibody or the binding fragment thereof comprises an amino acid sequence which is 85%, 86%, 87%, 88%, 89%, 90%, 91%, 92%, 93%, 94%, 95%, 96%, 97%, 98%, 99% or 100% identical to the VL as set forth in SEQ ID NO: 77; and/or (3) the first cysteine residue is introduced by a mutation of F42C at the position 42, a mutation of K64C at the position 64, or a mutation of P65C at the position 65 of the hIL-2 mutein corresponding to the amino acid sequence of the wild-type hIL-2 as set forth in SEQ ID NO: 1, and the second cysteine residue is introduced by a mutation of F3C at the position 3 of HCDR3 having the amino acid sequence of Z2TFZ3YZ4RELRWFAY (wherein Z2=V or I; Z3=Y or L; Z4=S or T) in the humanized anti-IL-2 antibody or the antigen-binding fragment thereof, wherein the humanized anti-IL-2 antibody or the antigen-binding fragment thereof comprises HCDR1 having the amino acid sequence as set forth in SEQ ID NO: 14, HCDR2 having the amino acid sequence as set forth in SEQ ID NO: 15, HCDR3 having the amino acid sequence as set forth in SEQ ID NO: 9, LCDR1 having the amino acid sequence as set forth in SEQ ID NO: 10, LCDR2 having the amino acid sequence as set forth in SEQ ID NO: 17, and LCDR3 having the amino acid sequence as set forth in SEQ ID NO: 12.

32. The hIL-2/anti-IL-2 antibody fusion protein of any one of claims 14-20, wherein disulfide linkage(s) is/are introduced at the binding interface of an antigen-antibody complex formed by the wild-type hIL-2 and the other humanized anti-IL-2 antibody or antigen-binding fragment thereof which specifically recognizes the IL2Rα-binding site of hIL-2, and the antigen-antibody complex is selected from the hIL-2 : NARA1 antigen-antibody complex and the hIL-2 : TCB2 antigen-antibody complex;

    wherein the NARA1 antibody or the antigen-binding fragment thereof comprises HCDR1 having the amino acid sequence as set forth in SEQ ID NO: 142, HCDR2 having the amino acid sequence as set forth in SEQ ID NO: 143, HCDR3 having the amino acid sequence as set forth in SEQ ID NO: 144, LCDR1 having the amino acid sequence as set forth in SEQ ID NO: 145, LCDR2 having the amino acid sequence as set forth in SEQ ID NO: 146, and LCDR3 having the amino acid sequence as set forth in SEQ ID NO: 147;
    the TCB2 antibody or the antigen-binding fragment thereof comprises HCDR1 having the amino acid sequence as set forth in SEQ ID NO: 170, HCDR2 having the amino acid sequence as set forth in SEQ ID NO: 171, HCDR3 having the amino acid sequence as set forth in SEQ ID NO: 172, LCDR1 having the amino acid sequence as set forth in SEQ ID NO: 173, LCDR2 having the amino acid sequence as set forth in SEQ ID NO: 174, and LCDR3 having the amino acid sequence as set forth in SEQ ID NO: 175.

33. The IL-2/anti-IL-2 antibody fusion protein of claim 32, wherein the binding interface of the hIL-2 : NARA1 antigen-antibody complex encompasses the binding interface between hIL-2 and IL2Ra, and the binding interface of the hIL-2 : NARA1 antigen-antibody complex is located in AB loop, B helix and BC loop of the hIL-2, and further, the binding interface of the hIL-2 : NARA1 antigen-antibody complex is located at the positions 30-43 or their adjacent amino acid residues and the positions 71-77 or their adjacent amino acid residues in the hIL-2.

34. The hIL-2/anti-IL-2 antibody fusion protein of claim 32 or 33, wherein the binding interface of the hIL-2 : NARA1 antigen-antibody complex comprises two or more cysteine residues so as to form the disulfide linkage, wherein the hIL-2 has one or more amino acid mutations at the position 34, 37, 41, 42, 68, 73, 75, 77 and 111 relative to the amino acid sequence of the wild-type hIL-2 as set forth in SEQ ID NO: 1, to introduce the first cysteine residue; the NARA1 antibody or antigen-binding fragment thereof has one or more amino acid mutations at specific positions in the CDR and/or FR regions, to introduce the second cysteine residue, and the amino acid mutations include those located at the position 1 of HCDR1, and/or at the position 2, 6 and/or 8 of HCDR2, and/or at the position 3 and/or 8 of HCDR3, and/or at the position 9 of LCDR1, and/or at the position 3, 4, and/or 6 of LCDR3 in the NARA1 antibody or the antigen-binding fragment thereof.

35. The hIL-2/anti-IL-2 antibody fusion protein of any one of claims 32-34, wherein the pair of the first cysteine residue and the second cysteine residue for forming the disulfide linkage at the binding interface of the hIL-2 : NARA1 antigen-antibody complex is selected from one or more of the following paired amino acid mutations: (1) the first cysteine residue is introduced by a mutation of P34C at the position 34 of the hIL-2, and the second cysteine residue is introduced by a mutation of A8C at the position 8 in HCDR3 of the NARA1 antibody or the antigen-binding fragment thereof, or the second cysteine residue is introduced by a mutation of S3C or N4C at the position 3 or 4 inLCDR3 of the NARA1 antibody or the antigen-binding fragment thereof; and/or (2) the first cysteine residue is introduced by a mutation of T37C at the position 37 of the hIL-2, and the second cysteine residue is introduced by a mutation of D6C at the position 6 in LCDR3 of the NARA1 antibody or antigen-binding fragment thereof; and/or (3) the first cysteine residue is a mutation of T41C at the position 41 of the hIL-2, and the second cysteine residue is introduced by a mutation of I2C at position 2 in HCDR2 of the NARA1 antibody or the antigen-binding fragment thereof; and/or (4) the first cysteine residue is introduced by a mutation F42C at the position 42 of the hIL-2, and the second cysteine residue is introduced by a mutation G8C at the position 8 in HCDR2 of the NARA1 antibody or antigen-binding fragment thereof; and/or (5) the first cysteine residue is introduced by a mutation E68C at the position 68 of the hIL-2, and the second cysteine residue is introduced by a mutation of N1C at the position 1 in HCDR1 of the NARA1 or antigen-binding fragment thereof; and/or (6) the first cysteine residue is introduced by a mutation of A73C at the position 73 of the hIL-2, and the second cysteine residue is introduced by a mutation of G3C at the position 3 in HCDR3 of the NARA1 or antigen-binding fragment thereof; and/or (7) the first cysteine residue is introduced by a mutation of S75C or N77C at the position 75 or 77 of the hIL-2, and the second cysteine residue is introduced by amino acid mutation D9C at the position 9 in LCDR1 of the NARA1 or antigen-binding fragment thereof; and/or (8) the first cysteine residue is introduced by a mutation T111C at the position 111 of the hIL-2, and the second cysteine residue is introduced by a mutation of S6C at the position 6 in HCDR2 of the NARA1 antibody or antigen-binding fragment thereof;

preferably, the pair of the first cysteine residue and the second cysteine residue for forming the disulfide linkage at the binding interface of the hIL-2 : NARA1 antigen-antibody complex is selected from one or more of the following paired amino acid mutations: (1) the first cysteine residue is introduced by a mutation of T37C at the position 37 of the hIL-2, and the second cysteine residue is introduced by a mutation of D6C at the position 6 in LCDR3 of the NARA1 antibody or antigen-binding fragment thereof; or (2) the first cysteine residue is introduced by a mutation of E68C at the position 68 of the hIL-2, and the second cysteine residue is introduced by a mutation of N1C at the position 1 in HCDR1 of the NARA1 antibody or antigen-binding fragment thereof; or (3) the first cysteine residue is introduced by a mutation of S75C or N77C at the position 75 or 77 of the hIL-2, and the second cysteine residue is introduced by a mutation of D9C at the position 9 in LCDR1 of the NARA1 antibody or antigen-binding fragment thereof; and/or (4) the first cysteine residue is introduced by a mutation of T111C at the position 111 of the hIL-2, and the second cysteine residue is introduced by a mutation of S6C at the position 6 in HCDR2 of the NARA1 antibody or antigen-binding fragment thereof.

36. The hIL-2/anti-IL-2 antibody fusion protein of claim 32, wherein the binding interface of the hIL-2 : TCB2 antigen-antibody complex encompasses the binding interface between hIL-2 and IL2Rα, and the binding interface of the hIL-2 : TCB2 antigen-antibody complex is located in AB loop, B helix and CD loop of the hIL-2, and further, the binding interface of the hIL-2 : TCB2 antigen-antibody complex is located at positions 34-45 or their adjacent amino acid residues, positions 62-76 or their adjacent amino acid residues, and the position 111 or the adjacent amino acid residues in the hIL-2.

37. The hIL-2/anti-IL-2 antibody fusion protein of claim 32 or 36, wherein the binding interface of the hIL-2 : TCB2 antigen-antibody complex comprises two or more cysteine residues so as to form the disulfide linkage(s), wherein the hIL-2 has on or more amino acid mutations at positions 34, 36, 37, 42, 68 and 109 relative to the amino acid sequence of the wild-type hIL-2 as set forth in SEQ ID NO: 1, to introduce the first cysteine residue; the TCB2 antibody or antigen-binding fragment thereof has one or more amino acid mutations at specific positions in the CDR and/or FR regions, to introduce a second cysteine residue, and the amino acid mutations include those located at the position 6 in HCDR2, and/or at positions 3, 5, 6 and/or 7 in HCDR3, and/or at the position 4 in LCDR1, and/or at positions 4, 5, and/or 6 in LCDR3 of the TCB2 antibody or antigen-binding fragment thereof.

38. The hIL-2/anti-IL-2 antibody fusion protein according to any one of claims 32, 36 and 37, wherein the pair of the first cysteine residue and the second cysteine residue for forming the disulfide linkage at the binding interface of the hIL-2 : TCB2 antigen-antibody complex is selected from one or more of the following paired amino acid mutations: (1) the first cysteine residue is introduced by a mutation of P34C at the position 34 of the hIL-2, and the second cysteine residue is introduced by a mutation of T4C at the position 4 in LCDR1 of the TCB2 or antigen-binding fragment thereof, or the second cysteine is introduced by a mutation of D4C or N5C at the position 4 or 5 in LCDR3 of the TCB2 antibody or antigen-binding fragment thereof; and/or (2) the first cysteine residue is introduced by a mutation L36C at the position 36 of the hIL-2, and the second cysteine residue is introduced by a mutation of N5C at the position 5 in LCDR3 of the TCB2 antibody or antigen-binding fragment thereof; and/or (3) the first cysteine residue is introduced by a mutation of T37C at the position 37 of the hIL-2, and the second cysteine residue is introduced by a mutation of N5C or L6C at the position 5 or 6 in LCDR3 of the TCB2 antibody or antigen-binding fragment thereof; and/or (4) the first cysteine residue is introduced by a mutation R38C at the position 38 of the hIL-2, and the second cysteine residue is introduced by a mutation of F7C at the position 7 in HCDR3 of the TCB2 antibody or antigen-binding fragment thereof; and/or (5) the first cysteine residue is introduced by a mutation of F42C at the position 42 of the hIL-2, and the second cysteine residue is introduced by a mutation of R5C at the position 5 in HCDR3 of the TCB2 antibody or antigen-binding fragment thereof; and/or (6) the first cysteine residue is introduced by a mutation of E68C at the position 68 of the hIL-2, and the second cysteine residue is introduced by a mutation of A3C or G6C at the position 3 or 6 in HCDR3 of the TCB2 antibody or antigen-binding fragment thereof; and/or (7) the first cysteine residue is introduced by a mutation of D109C at the position 109 of the hIL-2, and the second cysteine residue is introduced by a mutation D6C at the position 6 in HCDR2 in the TCB2 antibody or antigen-binding fragment thereof;

preferably, the pair of the first cysteine residue and the second cysteine residue for forming the disulfide linkage at the binding interface of the hIL-2 : TCB2 antigen-antibody complex is selected from one or more of the following paired amino acid mutations: (1) the first cysteine residue is introduced by a mutation of P34C at the position 34 of the hIL-2, and the second cysteine is introduced by a mutation of D4C or N5C at the position 4 or 5 in LCDR3 of the TCB2 antibody or antigen-binding fragment thereof; or (2) the first cysteine residue is introduced by a mutation of L36C at the position 36 of the hIL-2, and the second cysteine residue is introduced by a mutation of N5C at the position 5 in LCDR3 of the TCB2 antibody or antigen-binding fragment thereof; or (3) the first cysteine residue is introduced by a mutation of T37C at the position 37 of the hIL-2, and the second cysteine residue is introduced by a mutation of L6C at the position 6 in LCDR3 of the TCB2 antibody or antigen-binding fragment thereof; or (4) the first cysteine residue is introduced by a mutation of F42C at the position 42 of the hIL-2, and the second cysteine residue is introduced by a mutation of R5C at the position 5 in HCDR3 of the TCB2 antibody or antigen-binding fragment thereof; and/or (5) the first cysteine residue is introduced by a mutation of E68C at the position 68 of the hIL-2, and the second cysteine residue is introduced by a mutation of A3C at the position 3 in HCDR3 of the TCB2 antibody or antigen-binding fragment thereof.

39. The hIL-2/anti-IL-2 antibody fusion protein of any one of claims 14-31, wherein the hIL-2/anti-IL-2 antibody fusion protein comprises HCDR1, HCDR2, HCDR3 of the heavy chain variable region and LCDR1, LCDR2, LCDR3 of the light chain variable region, and the HCDR3, LCDR1, LCDR2 and/or LCDR3 comprise one or more cysteine residue mutations, the HCDR1, HCDR2, HCDR3 of the heavy chain variable region and LCDR1, LCDR2, LCDR3 of the light chain variable region comprising: (1) HCDR1 having the amino acid sequence of $X1YYX2H$ (wherein X1=A; X2=M, as set forth in SEQ ID NO: 14), (2) HCDR2 having the amino acid sequence of $RIDPZ1DDSTKYAENFKX3$ (wherein Z1=E or D; X3=G), (3) HCDR3 having the amino acid sequence of $Z2TX_{II}Z3YZ4RELRWFAY$ (wherein Z2=V or I; $X_{II}$=F or C; Z3=Y or L; Z4=S or T), (4) LCDR1 having the amino acid sequence of $QASQZ5IZ6X_IX_{IV}LS$ (wherein Z5=D or Y; Z6=G, S or C; $X_I$=N or C; $X_{IV}$=Y or C), (5) LCDR2 having the amino acid sequence of $Z7AZ8SLAX4$ (wherein Z7=S, Q or C; Z8=T or C; X4=S), and (6) LCDR3 having the amino acid sequence of $LQHX_{III}STPYT$ (wherein $X_{III}$=Y or C).

40. The hIL-2/anti-IL-2 antibody fusion protein of claim 39, wherein the hIL-2/anti-IL-2 antibody fusion protein comprises HCDR1, HCDR2, HCDR3 of the heavy chain variable region and LCDR1, LCDR2, LCDR3 of the light chain variable region, and the HCDR3, LCDR1, LCDR2 and/or LCDR3 comprise one or more cysteine residue mutations, the HCDR1, HCDR2, HCDR3 of the heavy chain variable region and LCDR1, LCDR2, LCDR3 of the light chain variable region comprising: (1) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 9, LCDR1 having an amino acid sequence as set forth in SEQ ID NOs: 30 or 31, LCDR2 having an amino acid sequence as set forth in SEQ ID NO: 17, and LCDR3 having an amino acid sequence as set forth in SEQ ID NO: 12; and/or (2) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 9, LCDR1 having an amino acid sequence as set forth in SEQ ID NO: 10, LCDR2 having an amino acid sequence as set forth in SEQ ID NOs: 17, 32 or 33, and LCDR3 having an amino acid sequence as set forth in SEQ ID NO: 12; and/or (3) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 29, LCDR1 having an amino acid sequence as set forth in SEQ ID NO: 10, LCDR2 having an amino acid sequence as set forth in SEQ ID NO: 17, and LCDR3 having an amino acid sequence as set forth in SEQ ID NO: 12; and/or (4) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NO: 24, LCDR1 having an amino acid sequence as set forth in SEQ ID NOs: 35 or 36, LCDR2 having an amino acid sequence as set forth in SEQ ID NO: 17, and LCDR3 having an amino acid sequence as set forth in SEQ ID NO: 12; and/or (5) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NO: 24, LCDR1 having an amino acid sequence as set forth in SEQ ID NO: 26, LCDR2 having an amino acid sequence as set forth in SEQ ID NOs: 17, 32 or 33, and LCDR3 having an amino acid sequence as set forth in SEQ ID NO: 12; and/or (6) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NO: 34, LCDR1 having an amino acid sequence as set forth in SEQ ID NO: 26, LCDR2 having an amino acid sequence as set forth in SEQ ID NO: 17, and LCDR3 having an amino acid sequence as set forth in SEQ ID NO: 12; and/or (7) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NO: 24, LCDR1 having an amino acid sequence as set forth in SEQ ID NO: 26, LCDR2 having an amino acid sequence as set forth in SEQ ID NO: 17, and LCDR3 having an amino acid sequence as set forth in SEQ ID NO: 38; and/or (8) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NO: 24, LCDR1 having an amino acid sequence as set forth in SEQ ID NO: 37, LCDR2 having an amino acid sequence as set forth in SEQ ID NO: 17, and LCDR3 having an amino acid sequence as set forth in SEQ ID NO: 12;

preferably, the hIL-2/anti-IL-2 antibody fusion protein comprises the following HCDR1, HCDR2, HCDR3 of the heavy chain variable region and LCDR1, LCDR2, LCDR3 of the light chain variable region: (1) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 29, LCDR1 having an amino acid sequence as set forth in SEQ ID NO: 10, LCDR2 having an amino acid sequence as set forth in SEQ ID NO: 17, and LCDR3 having an amino acid sequence as set forth in SEQ ID NO: 12; and/or (2) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NO: 24, LCDR1 having an amino acid sequence as set forth in SEQ ID NO: 26, LCDR2 having an amino acid sequence as set forth in SEQ ID NOs: 17 or 33, and LCDR3 having an amino acid sequence as set forth in SEQ ID NO: 12.

41. The hIL-2/anti-IL-2 antibody fusion protein of any one of claims 14-31, 39 or 40, wherein the hIL-2/anti-IL-2 antibody fusion protein can also have an amino acid mutation at the position 1 of the light chain variable region or at the position 4 of LCDR1 in the anti-IL-2 antibody, wherein the amino acid mutation comprises D1E, Q4D, Q4E, Q4S, Q4G, Q4K, or Q4R, preferably, the amino acid mutation comprising D1E, Q4D, Q4E, Q4S, or Q4G.

42. The hIL-2/anti-IL-2 antibody fusion protein of any one of claims 14-20 and 32-35, wherein the hIL-2/anti-IL-2 antibody fusion protein comprises HCDR1, HCDR2, HCDR3 of the heavy chain variable region and LCDR1, LCDR2, LCDR3 of the light chain variable region, and the HCDR1, HCDR2, HCDR3, LCDR1, and/or LCDR3 comprises one or more cysteine mutations, the HCDR1, HCDR2, HCDR3 of the heavy chain variable region and LCDR1, LCDR2, LCDR3 of the light chain variable region comprising: (1) HCDR1 having an amino acid sequence as set forth in SEQ ID NOs: 142 or 148; (2) HCDR2 having an amino acid sequence as set forth in SEQ ID NOs: 143, 149, 150 or 151; (3) HCDR3 having an amino acid sequence as set forth in SEQ ID NOs: 144, 152 or 153; (4) LCDR1 having an amino acid sequence as set forth in SEQ ID NOs: 145 or 154; (5) LCDR2 having an amino acid sequence as set forth in SEQ ID NO: 146; (6) LCDR3 having an amino acid sequence as set forth in SEQ ID NOs: 147, 155, 156 or 157;

further, the HCDR1, HCDR2, HCDR3 of the heavy chain variable region and the LCDR1, LCDR2, and LCDR3 of the light chain variable region comprise: (1) HCDR1 having an amino acid sequence as set forth in SEQ ID NO: 142, HCDR2 having an amino acid sequence as set forth in SEQ ID NO: 149, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147; and/or (2) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NOs: 152 or 153, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147; and/or (3) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 154, LCDR2 having an amino acid sequence as set forth in SEQ ID NO: 146, and LCDR3 having an amino acid sequence as set forth in SEQ ID NO: 147; and/or (4) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NOs: 155, 156 or 157;

and/or (5) HCDR1 having an amino acid sequence as set forth in SEQ ID NO: 148, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147; and/or (6) HCDR1 having an amino acid sequence as set forth in SEQ ID NO: 142, HCDR2 having an amino acid sequence as the set forth in SEQ ID NOs: 150 or 151, HCDR3 having an amino acid sequence as the set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147;

preferably, the hIL-2/anti-IL-2 antibody fusion protein comprises the following HCDR1, HCDR2, HCDR3 of the heavy chain variable region and LCDR1, LCDR2, LCDR3 of the light chain variable region: (1) HCDR1 having an amino acid sequence as set forth in SEQ ID NO: 142, HCDR2 having an amino acid sequence as set forth in SEQ ID NO: 149, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147; and/or (2) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 154, LCDR2 having an amino acid sequence as set forth in SEQ ID NO: 146, and LCDR3 having an amino acid sequence as set forth in SEQ ID NO: 147; and/or (3) HCDR1 having an amino acid sequence as set forth in SEQ ID NO: 142, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 157; and/or (4) HCDR1 having an amino acid sequence as set forth in SEQ ID NO: 148, HCDR2 having an amino acid sequence as set forth in SEQ ID NO: 143, HCDR3 having an amino acid sequence as set forth in SEQ ID NO: 144, LCDR1 having an amino acid sequence as set forth in SEQ ID NO: 145, LCDR2 having an amino acid sequence as set forth in SEQ ID NO: 146, and LCDR3 having an amino acid sequence as set forth in SEQ ID NO: 147.

43. The hIL-2/anti-IL-2 antibody fusion protein of any one of claims 14-20, 32, 36-38, wherein the hIL-2/anti-IL-2 antibody fusion protein comprises HCDR1, HCDR2, HCDR3 of the heavy chain variable region and LCDR1, LCDR2, LCDR3 of the light chain variable region, and the HCDR2, HCDR3, LCDR1, and/or LCDR3 comprising one or more cysteine mutations, the HCDR1, HCDR2, HCDR3 of the heavy chain variable region and the LCDR1, LCDR2, LCDR3 of the light chain variable region comprising: (1) HCDR1 having an amino acid sequence as set forth in SEQ ID NO: 170; (2) HCDR2 having an amino acid sequence as set forth in SEQ ID NOs: 171 or 176; (3) HCDR3 having an amino acid sequence as set forth in SEQ ID NOs: 172, 177, 178, 179 or 180; (4) LCDR1 having an amino acid sequence as set forth in SEQ ID NOs: 173 or 181; (5) LCDR2 having an amino acid sequence as set forth in SEQ ID NO: 174; (6) LCDR3 having an amino acid sequence as set forth in SEQ ID NOs: 175, 182, 183 or 184;

further, the HCDR1, HCDR2, HCDR3 of the heavy chain variable region and the LCDR1, LCDR2, and LCDR3 of the light chain variable region comprise: (1) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NOs: 177, 178, 179 or 180, LCDR1 having an amino acid sequence as set forth in SEQ ID NO: 173, LCDR2 having an amino acid sequence as SEQ ID NO: 174, and LCDR3 having an amino acid sequence as set forth in SEQ ID NO: 175; and/or (2) HCDR1 having an amino acid sequence as set forth in SEQ ID NO: 170, HCDR2 having an amino acid sequence as set forth in SEQ ID NO: 176, HCDR3 having an amino acid sequence as set forth in SEQ ID NO: 172, LCDR1 having an amino acid sequence as set forth in SEQ ID NO: 173, LCDR2 having an amino acid sequence as set forth in SEQ ID NO: 174, and LCDR3 having an amino acid sequence as set forth in SEQ ID NO: 175; and/or (3) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NO: 172, LCDR1 having an amino acid sequence as set forth in SEQ ID NO: 181, LCDR2 having an amino acid sequence as set forth in SEQ ID NO: 174, and LCDR3 having an amino acid sequence as set forth in SEQ ID NO: 175; and/or (4) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NO: 172, LCDR1 having an amino acid sequence as set forth in SEQ ID NO: 173, LCDR2 having an amino acid sequence as set forth in SEQ ID NO: 174, and LCDR3 having an amino acid sequence as set forth in SEQ ID NOs: 182, 183 or 184; preferably, the hIL-2/anti-IL-2 antibody fusion protein comprises the following HCDR1, HCDR2, HCDR3 of the heavy chain variable region and LCDR1, LCDR2, LCDR3 of the light chain variable region: (1) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NOs: 177 or 178, LCDR1 having an amino acid sequence as set forth in SEQ ID NO: 173, LCDR2 having an amino acid sequence as set forth in SEQ ID NO: 174, and LCDR3 having an amino acid sequence as set forth in SEQ ID NO: 175; and/or (2) HCDR1 having an amino acid sequence as set forth in SEQ ID NO: 170, HCDR2 having an amino acid sequence as set forth in SEQ ID NO: 171, HCDR3 having an amino acid sequence as set forth in SEQ ID NO: 172, LCDR1 having an amino acid sequence as set forth in SEQ ID NO: 173, LCDR2 having an amino acid sequence as set forth in SEQ ID NO: 174, and LCDR3 having an amino acid sequence as set forth in SEQ ID NOs: 182, 183 or 184.

44. The hIL-2/anti-IL-2 antibody fusion protein of any one of claims 14-31, 39, 40, wherein the hIL-2/anti-IL-2 antibody fusion protein comprises a heavy chain variable region having the HCDR1, HCDR2 and HCDR3 and/or a light chain variable region having the LCDR1, LCDR2 and LCDR3, the heavy chain variable region comprising an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NOs: 63, 86, 74 or 112, respectively, and/or the light chain variable region comprising an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NOs: 64, 77, 80, 81, 82, 83, 84, 85, 87, 88, 89, 90, 91, 92, 93, 104, 105, 106, 107, 108, 109, 110 or 111, respectively;

preferably, the heavy chain variable region comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NOs: 86 or 74, respectively, and/or the light chain variable region comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NOs: 64, 87, 88, 89, 90, 91, 107, 108 or 109, respectively;
alternatively, the hIL-2/anti-IL-2 antibody fusion protein comprises a heavy chain variable region and a light chain variable region, and the heavy chain variable region and light chain variable region comprise: (1) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 86, and the light chain variable region as set forth in SEQ ID NOs: 64, 87, 88, 89, 90 or 91, respectively; or (2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 74, and the light chain variable region as set forth in SEQ ID NOs: 107, 108 or 109, respectively.

45. The hIL-2/anti-IL-2 antibody fusion protein of any one of claims 14-20, 32-35 and 42, wherein the hIL-2/anti-IL-2 antibody fusion protein comprises a heavy chain variable region having the HCDR1, HCDR2 and HCDR3 and/or a light chain variable region having the LCDR1, LCDR2 and LCDR3, and the heavy chain variable region comprises an amino acid sequence which is at least 85%, 86%, 87%, 88%, 89%, 90%, 91%, 92%, 93%, 94%, 95%, 96%, 97%, 98%, 99% or 100 % identical to a sequence as set forth in SEQ ID NOs: 140, 158, 159, 160, 161, 162 or 163, respectively, and/or the light chain variable region comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NOs: 141, 164, 165, 166 or 167, respectively;

further, the hIL-2/anti-IL-2 antibody fusion protein comprises a heavy chain variable region and a light chain variable region, and the heavy chain variable region and the light chain variable region comprise: (1) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NOs: 158, 159, 160, 161, 162 or 163, and the light chain variable region as set forth in SEQ ID NO: 141, respectively; or (2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 140, and the light chain variable region as set forth in SEQ ID NOs: 164, 165, 166 or 167, respectively;
preferably, the hIL-2/anti-IL-2 antibody fusion protein comprises the following heavy chain variable region and light chain variable region: (1) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID Nos: 158 or 161, and the light chain variable region as set forth in SEQ ID NO: 141, respectively; or (2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 140, and the light chain variable region as set forth in SEQ ID NOs: 164 or 167, respectively.

46. The hIL-2/anti-IL-2 antibody fusion protein of any one of claims 14-20, 32, 36-38 and 43, wherein the hIL-2/anti-IL-2 antibody fusion protein comprises a heavy chain variable region having HCDR1, HCDR2 and HCDR3 and/or a light chain variable region having the LCDR1, LCDR2 and LCDR3, and the heavy chain variable regions comprises an amino acid sequence which is at least 85%, 86%, 87%, 88%, 89%, 90%, 91%, 92%, 93%, 94%, 95%, 96%, 97%, 98%, 99% or 100 % identical to a sequence as set forth in SEQ ID NOs: 168, 185, 186, 187, 188 or 189, respectively, and/or the light chain variable region comprises an amino acid sequence which is at least 85%, 86%, 87%, 88%, 89%, 90%, 91%, 92%, 93%, 94%, 95%, 96%, 97%, 98%, 99% or 100 % identical to a sequence as set forth in SEQ ID NOs: 169, 190, 191, 192 or 193, respectively;

further, the hIL-2/anti-IL-2 antibody fusion protein comprises the following heavy chain variable region and light chain variable region: (1) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NOs: 185, 186, 187, 188 or 189, and the light chain variable region as set forth in SEQ ID NO: 169, respectively; or (2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 168, and the light chain variable region as set forth in SEQ ID NOs: 190, 191, 192 or 193, respectively; preferably, the hIL-2/anti-IL-2 antibody fusion protein comprises the following heavy chain variable region and light chain variable region: (1) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NOs: 185 or 186, and the light chain variable region as set forth in SEQ ID NO: 169, respectively; or (2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 168, and the light chain variable region as set forth in SEQ ID NOs: 191, 192 or 193, respectively.

47. The hIL-2/anti-IL-2 antibody fusion protein according to any one of claims 14-46, wherein the anti-IL-2 antibody or the antigen-binding fragment thereof can be linked to hIL-2 or a mutein thereof by a linker, and the linker is selected from $(G4S)_4$, $(G4S)_3$, $(GAF)_2$, and preferably, the linker is $(G4S)_4$.

48. The hIL-2/anti-IL-2 antibody fusion protein of any one of claims 14-47, wherein the hIL-2 or a mutein thereof further comprises the following one or more amino acid mutations: (1) a deletion of the first three or five amino acids at the N-terminus corresponding to the amino acid sequence of the wild-type hIL-2 as set forth in SEQ ID NO: 1; or (2) an amino acid mutation at the third amino acid residue at the N-terminus corresponding to the amino acid sequence of the wild-type hIL-2 as set forth in SEQ ID NO: 1, and/or (3) an amino acid mutation C125A, C125L, C125S, C125Q, or C125V at the position 125 corresponding to the amino acid sequence of the wild-type hIL-2 as set forth in SEQ ID NO: 1;
preferably, the hIL-2 or a mutein thereof comprises the following amino acid mutations: a deletion of the first three N-terminal amino acids, and/or an amino acid mutation of C125A, C125L, C125S, C125Q, or C125V at the position 125 corresponding to the amino acid sequence of the wild-type hIL-2 as set forth in SEQ ID NO: 1.

49. The hIL-2/anti-IL-2 antibody fusion protein of any one of claims 14-48, wherein the hIL-2/anti-IL-2 antibody fusion protein comprises a first polypeptide, and the amino acid sequence of the first polypeptide is at least 85%, 86%, 87%, 88%, 89%, 90%, 91%, 92%, 93%, 94%, 95%, 96%, 97%, 98%, 99% or 100% identical to SEQ ID NOs: 95, 96, 97, 98, 99, 100, 101, 102, 103, 113, 114, 115, 116, 117, 118, 119, 120, 133, 134, 135, 136, 137, 138, 206, 207, 208, 209, 210, 211, 212, 213, 214, 215, 216, 217, 218, 219, 220, 221, 222, 223, 224, 225, or 226.

50. The hIL-2/anti-IL-2 antibody fusion protein of any one of claims 14-49, wherein the IL-2/anti-IL-2 antibody fusion protein comprises a first polypeptide and a second polypeptide, wherein the first polypeptide comprises hIL-2 or a mutein thereof having a cysteine mutation which is operatively linked to one variable region of the anti-IL-2 antibody or antigen-binding fragment thereof, and the second polypeptide comprises the other variable region of the anti-IL-2 antibody or antigen-binding fragment thereof;
further, the first polypeptide comprises the hIL-2 or a mutein thereof having a cysteine mutation, a linker and one variable region of the anti-IL-2 antibody or antigen-binding fragment thereof, and another cysteine mutation can be placed in one variable region of the anti-IL-2 antibody or antigen-binding fragment thereof in the first polypeptide and/or in the other variable region of the anti-IL-2 antibody or antigen-binding fragment thereof in the second polypeptide, whereby a correctly paired and stable disulfide linkage can be formed with the cysteine mutation in the hIL-2 or a mutein thereof, and the said disulfide linkage is located at the antigen-antibody binding interface of the said hIL-2 or a mutein thereof and the said anti-IL-2 antibody or antigen-binding fragment thereof.

51. The hIL-2/anti-IL-2 antibody fusion protein of claim 49 or 50, wherein the first polypeptide comprises the hIL-2 mutein with a cysteine residue mutation, a linker and a heavy chain variable region of the anti-IL-2 antibody or antigen-binding fragment thereof, and the second polypeptide comprises a light chain variable region of the anti-IL-2 antibody or antigen-binding fragment thereof, wherein the first polypeptide comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 95-103, 113-120, or 133-138, and/or the second polypeptide comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 64, 77, 80-85, 87-93, or 104-111.

52. The hIL-2/anti-IL-2 antibody fusion protein of claim 51, wherein the hIL-2/anti-IL-2 antibody fusion protein comprises the following first and second polypeptides: (1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 95, and the second polypeptide as set forth in SEQ ID NO: 80, respectively; or (2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 96, and the second polypeptide as set forth in SEQ ID NO: 81, respectively; or (3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 97, and the second polypeptide as set forth in SEQ ID NO: 82, respectively; or (4) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 98, and the second polypeptide as set forth in SEQ ID NO: 83, respectively; or (5) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 99, and the second polypeptide as set forth in SEQ ID NO: 84, respectively; or (6) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 100, and the second polypeptide as set forth in SEQ ID NO: 85, respectively; or (7) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in any of SEQ ID NOs: 101, 102, 103 and 133-138, and the second polypeptide as set forth in SEQ ID NO: 64, respectively; or (8) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 102, and the second polypeptide as set forth in any of SEQ ID NOs: 87-93, respectively; or (9) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 113, and the second polypeptide as set forth in SEQ ID NOs: 104, 105 or 106, respectively; or (10) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 114, and the second polypeptide as set forth in SEQ ID NO: 107, respectively; or (11) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 115, and the second polypeptide as set forth in SEQ ID NO: 108 or 109, respectively; or (12) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NOs: 116, 117 or 119, and the second polypeptide as set forth in SEQ ID NO: 77, respectively; or (13) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 118, and the second polypeptide as set forth in SEQ ID NO: 110, respectively; or (14) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 120, and the second polypeptide as set forth in SEQ ID NO: 104, respectively; or (15) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 118, and the second polypeptide as set forth in SEQ ID NO: 111, respectively; preferably, the hIL-2/anti-IL-2 antibody fusion protein comprises the following first and second polypeptides: (1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in any of SEQ ID NOs: 101, 102, 103 and 133-138, and the second polypeptide as set forth in SEQ ID NO: 64, respectively; or (2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 102, and the second polypeptide as set forth in any of SEQ ID NOs: 87-91, respectively; or (3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 114,

and the second polypeptide as set forth in SEQ ID NO: 107, respectively; or (4) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 115, and the second polypeptide as set forth in SEQ ID NO: 108 or 109, respectively.

53. The hIL-2/anti-IL-2 antibody fusion protein of claim 49 or 50, wherein the first polypeptide comprises a hIL-2 mutein with a cysteine mutation, a linker and a light chain variable region of the anti-IL-2 antibody or antigen-binding fragment thereof, and the second polypeptide comprises a heavy chain variable region of the anti-IL-2 antibody or antigen-binding fragment thereof, wherein the first polypeptide comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 206-226, and/or the second polypeptide comprises an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 140, 158-163, 168 or 185-189.

54. The hIL-2/anti-IL-2 antibody fusion protein of claim 53, wherein the hIL-2/anti-IL-2 antibody fusion protein comprises the following first and second polypeptides: (1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 206, and the second polypeptide as set forth in SEQ ID NO: 158, respectively; or (2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 207, and the second polypeptide as set forth in SEQ ID NO: 159, respectively; or (3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 208, and the second polypeptide as set forth in SEQ ID NO: 160, respectively; or (4) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in any of SEQ ID NOs: 209-213, and the second polypeptide as set forth in SEQ ID NO: 140, respectively; or (5) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 214, and the second polypeptide as set forth in SEQ ID NO: 161 respectively; or (6) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 215, and the second polypeptide as set forth in SEQ ID NO: 162, respectively; or (7) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 216, and the second polypeptide as set forth in SEQ ID NO: 163, respectively; or (8) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 217, and the second polypeptide as set forth in SEQ ID NO: 185 or 187, respectively; or (9) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 218, and the second polypeptide as set forth in SEQ ID NO: 186, respectively; or (10) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 219, and the second polypeptide as set forth in SEQ ID NO: 188, respectively; or (11) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 220, and the second polypeptide as set forth in SEQ ID NO: 189, respectively; or (12) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in any of SEQ ID Nos: 221-226, and the second polypeptide as set forth in SEQ ID NO: 168, respectively;

preferably, the hIL-2/anti-IL-2 antibody fusion protein comprises the following first and second polypeptides: (1) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 206, and the second polypeptide as set forth in SEQ ID NO: 158, respectively; or (2) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NOs: 209, 210 or 213, and the second polypeptide as set forth in SEQ ID NO: 140, respectively; or (3) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 214, and the second polypeptide as set forth in SEQ ID NO: 161, respectively; or (4) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NO: 217, and the second polypeptide as set forth in SEQ ID NO: 185, respectively; or (5) amino acid sequences which are at least 85%, 86%, 87%, 88%, 89%, 90%, 91%, 92%, 93%, 94%, 95%, 96%, 97%, 98%, 99%

or 100% identical to the first polypeptide as set forth in SEQ ID NO: 218, and the second polypeptide as set forth in SEQ ID NO: 186, respectively; or (6) amino acid sequences which are at least 85%, 86%, 87%, 88%, 89%, 90%, 91%, 92%, 93%, 94%, 95%, 96%, 97%, 98%, 99% or 100% identical to the first polypeptide as set forth in SEQ ID NOs: 222, 223, 224 or 226, and the second polypeptide as set forth in SEQ ID NO: 168, respectively.

55. An hIL-2/anti-IL-2 antibody fusion protein, comprising: (1) the hIL-2 mutein of any one of claims 1-5; (2) the anti-IL-2 antibody or antigen-binding fragment thereof of any one of claims 6-13; and (3) the antigen-antibody binding interface of the hIL-2 mutein and the anti-IL-2 antibody or antigen-binding fragment thereof comprises disulfide linkage(s);

wherein, the anti-IL-2 antibody or antigen-binding fragment thereof can compete with IL2Rα for binding of the hIL-2 mutein, and can block the hIL-2 mutein from binding with IL2Rα or an IL-2 receptor complex comprising the IL2Rα subunit (i.e., IL2Rα/β or IL2Rα/β/γ);
wherein the antigen-antibody binding interface partially overlaps with the binding interface of the hIL-2 mutein to IL2Rα, the disulfide linkage is formed by at least one cysteine residue introduced in the hIL-2 mutein at the antigen-antibody binding interface and at least one cysteine residue introduced in the anti-IL-2 antibody or antigen-binding fragment thereof at the antigen-antibody binding interface; and
the hIL-2/anti-IL-2 antibody fusion protein is unable to bind to the α subunit, or the α/β heterodimer, or the α/β/γ heterotrimer of IL2R but retains binding and functional activity to the heterodimeric receptor consisting of IL2Rβand γc (i.e., IL2Rβ/γ).

56. The IL-2/anti-IL-2 antibody fusion protein of claim 55, wherein the antigen-antibody binding interface of the hIL-2 mutein and the anti-IL-2 antibody or antigen-binding fragment thereof is located in AB loop, B helix, C helix, BC loop and/or CD loop of the hIL-2 mutein, preferably AB loop, B helix, C helix and CD loop of the hIL-2 mutein; the IL2Rα binding interface of the hIL-2 mutein is located in AB loop and B helix of the hIL-2 mutein.

57. The hIL-2/anti-IL-2 antibody fusion protein of claim 55 or 56, wherein the antigen-antibody binding interface of the hIL-2 mutein and the anti-IL-2 antibody or antigen-binding fragment thereof is located at positions 30-45 or their adjacent amino acid residues, positions 57-77 or their adjacent amino acid residues, and/or positions 90-111 or their adjacent amino acid residues of the hIL-2 mutein; preferably, the antigen-antibody binding interface of the hIL-2 mutein and the anti-IL-2 antibody or antigen-binding fragment thereof is located at positions 41-45 or their adjacent amino acid residues, positions 57-68 or their adjacent amino acid residues, and/or positions 90-107 or their adjacent amino acid residues of the hIL-2 mutein.

58. The hIL-2/anti-IL-2 antibody fusion protein of any one of claims 55-57, wherein the antigen-antibody binding interface of the hIL-2 mutein and the anti-IL-2 antibody or antigen-binding fragment thereof in the hIL-2/anti-IL-2 antibody fusion protein comprises two or more disulfide linkages formed by introducing the following cysteine residues: (1) the hIL-2 mutein has one or more amino acid mutations at positions 42, 64, 65, 102, and 104 corresponding to the amino acid sequence of the wild-type hIL-2 as set forth in SEQ ID NO: 1, to introduce a first cysteine residue; and (2) the anti-IL-2 antibody or antigen-binding fragment thereof of any one of claims 6-13 has one or two amino acid mutations at the position 3 in HCDR3 and at the position 3 in LCDR2, to introduce a second cysteine residue; or, the anti-IL-2 antibody or antigen-binding fragment thereof of claim 13 has one or two amino acid mutations in the framework region of the light chain variable region, to introduce a second cysteine residue, wherein the amino acid mutation is located at positions 67 and/or 68 of the said light chain variable region; the site for introducing the first cysteine residue in the hIL-2 mutein and the site for introducing the second cysteine in the humanized anti-IL-2 antibody or antigen-binding fragment thereof can be paired correctly, and the correctly paired sites are located in the antigen-antibody binding interface of the hIL-2 mutein and the humanized anti-IL-2 antibody or antigen-binding fragment thereof, and the spatial distance between the paired amino acid residues is less than 10 angstroms, preferably, less than 5 angstroms.

59. The hIL-2/anti-IL-2 antibody fusion protein of any one of claims 55-58, wherein the pairing of the first cysteine residue and the second cysteine residue to form disulfide linkage at the antigen-antibody binding interface between the hIL-2 mutein and the anti-IL-2 antibody or antigen-binding fragment thereof in the IL-2/anti-IL-2 antibody fusion protein is selected from one or more of the following paired amino acid mutations: (1) the first cysteine residue is introduced by an amino acid mutation M104C at position 104 in the hIL-2 mutein corresponding to the amino acid sequence of the wild-type hIL-2 as set forth in SEQ ID NO: 1, and the second cysteine residue is introduced by amino acid mutation T3C at position 3 of LCDR2 in the anti-IL-2 antibody or antigen-binding fragment thereof, wherein the anti-IL-2 antibody or antigen-binding fragment thereof comprises HCDR1 having an amino acid sequence as set forth in SEQ ID NO: 14, HCDR2 having an amino acid sequence as set forth in SEQ ID NO: 19, HCDR3 having amino acid sequence as set forth in SEQ ID NO: 24, LCDR1 having an amino acid sequence as set forth in SEQ ID NO: 26, LCDR2 having an amino acid sequence as set forth in SEQ ID NO: 17, and LCDR3 having an amino acid sequence as set forth in SEQ ID NO: 12; and/or (2) the first cysteine residue is introduced by an amino acid mutation T102C at position 102 in the hIL-2 mutein corresponding to the amino acid sequence of the wild-type hIL-2 as set forth in SEQ ID NO: 1, and the second cysteine residue is introduced by amino acid mutation S67C at position 67 of the light chain variable region in the humanized anti-IL-2 antibody or antigen-binding fragment thereof, wherein the anti-IL-2 antibody or binding fragment thereof comprises an amino acid sequence which is 85%, 86%, 87%, 88%, 89%, 90%, 91%, 92%, 93%, 94%, 95%, 96%, 97%, 98%, 99% or 100% identical to the VL as set forth in SEQ ID NO: 77, or the second cysteine residue is introduced by amino acid mutation G68C at position 68 of the light chain variable region in the anti-IL-2 antibody or antigen-binding fragment thereof, wherein the anti-IL-2 antibody or binding fragment thereof comprises an amino acid sequence which is 85%, 86%, 87%, 88%, 89%, 90%, 91%, 92%, 93%, 94%, 95%, 96%, 97%, 98%, 99% or 100% identical to the VL as set forth in SEQ ID NO: 77; and/or (3) the first cysteine residue is introduced by an amino acid mutation F42C at position 42 amino acid residue, or K64C at position 64 amino acid residue, or P65C at position 65 in the hIL-2 mutein corresponding to the amino acid sequence of the wild-type hIL-2 as set forth in SEQ ID NO: 1, and the second cysteine residue is introduced by amino acid mutation F3C at position 3 of HCDR3 in the anti-IL-2 antibody or antigen-binding fragment thereof, wherein the anti-IL-2 antibody or antigen-binding fragment thereof comprises HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 9, LCDR1 having amino acid sequence as set forth in any of SEQ ID NOs: 39-42, LCDR2 having an amino acid sequence as set forth in SEQ ID NO: 17, and LCDR3 having an amino acid sequence as set forth in SEQ ID NO: 12.

60. The hIL-2/anti-IL-2 antibody fusion protein of any one of claims 55-59, wherein the hIL-2/anti-IL-2 antibody fusion protein comprises the heavy chain variable region having HCDR1, HCDR2, HCDR3 and the light chain variable region having LCDR1, LCDR2, LCDR3, the HCDR3 and/or LCDR2 comprising a cysteine mutation, the said HCDR1, HCDR2, HCDR3 of the heavy chain variable region and the said LCDR1, LCDR2, LCDR3 of the light chain variable region comprising: (1) HCDR1 having an amino acid sequence as set forth in SEQ ID NO: 14, HCDR2 having an amino acid sequence as set forth in SEQ ID NO: 15, HCDR3 having an amino acid sequence as set forth in SEQ ID NO: 29, LCDR1 having an amino acid sequence as set forth in any of SEQ ID NOs: 10 or 39-42, LCDR2 having an amino acid sequence as set forth in SEQ ID NO: 2, and LCDR3 having an amino acid sequence as set forth in SEQ ID NO: 12; or (2) HCDR1 having an amino acid sequence as set forth in SEQ ID NO: 14, HCDR2 having an amino acid sequence as set forth in SEQ ID NO: 19, HCDR3 having an amino acid sequence as set forth in SEQ ID NO: 24, LCDR1 having an amino acid sequence as set forth in SEQ ID NO: 26, LCDR2 having an amino acid sequence as set forth in SEQ ID NOs: 17 or 33, and LCDR3 having an amino acid sequence as set forth in SEQ ID NO: 12.

61. The hIL-2/anti-IL-2 antibody fusion protein of any one of claims 55-60, wherein the hIL-2/anti-IL-2 antibody fusion protein comprises a heavy chain variable region having the HCDR1, HCDR2 and HCDR3, and/or a light chain variable region having the LCDR1, LCDR2 and LCDR3; the heavy chain variable region comprising an amino acid sequence which is at least 85%, 86%, 87%, 88%, 89%, 90%, 91%, 92%, 93%, 94%, 95%, 96%, 97%, 98%, 99% or 100% identical to a sequence as set forth in SEQ ID NOs: 86 or 74, respectively; and/or the light chain variable region comprising an amino acid sequence which is at least 85%, 86%, 87%, 88%, 89%, 90%, 91%, 92%, 93%, 94%, 95%, 96%, 97%, 98%, 99% or 100% identical to a sequence as set forth in any of SEQ ID NOs: 64, 87-91, 107, 108 or 109, respectively;
further, the heavy chain variable region and the light chain variable region comprise: (1) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 86, and the light chain variable region as set forth in any of SEQ ID NOs: 64 or 87-91, respectively; or (2) amino acid sequences which are at least 85%, 86%, 87%, 88%, 89%, 90%, 91%, 92%, 93%, 94%, 95%, 96%, 97%, 98%, 99% or 100% identical to the heavy chain variable region as set forth in SEQ ID NO: 74, and the light chain variable region as set forth in SEQ ID NOs: 107, 108 or 109, respectively.

62. The hIL-2/anti-IL-2 antibody fusion protein of any one of claims 14-61, wherein the hIL-2/anti-IL-2 antibody fusion protein also comprises a constant region comprising a heavy chain constant region and a light chain constant region of an antibody, wherein the heavy chain constant region includes native and mutant forms of the Fc region of a human IgG heavy chain constant region, and further includes a truncated form of polypeptide containing a hinge region that facilitates the dimerization, and the preferred Fc region is derived from human IgGs, including IgG1, IgG2, IgG3 and IgG4;

and further, the Fc region comprises an engineered human IgG1 Fc region, comprising the following one or more amino acid substitution mutations: (1) L234F/L235E/P331S (EU numbering system) which has reduce binding activities of the Fc region to FcγRs and C1q; and/or (2) N203D/K274Q/Q419E (EU numbering system) which has prolong the plasma/serum half-life of the IL-2/anti-IL-2 fusion protein.

63. A nucleic acid encoding the hIL-2 mutein of any one of claims 1-5, the anti-IL-2 antibody or antigen-binding fragment thereof of any one of claims 6-13, or the nucleic acid encoding the hIL-2/anti-IL-2 antibody fusion protein of any one of claims 14-62.

64. An expression vector capable of expressing the nucleic acid of claim 63.

65. A host cell comprising the nucleic acid of claim 63 or the expression vector of claim 64.

66. A method of preparing the hIL-2 mutein of any one of claims 1-5, the anti-IL-2 antibody or antigen-binding fragment thereof of any one of claims 6-13, or the hIL-2/anti-IL-2 antibody fusion protein of any one of claims 14-62 by using the host cell of claim 65, comprising: (1) expressing the hIL-2 mutein, the anti-IL-2 antibody or antigen-binding fragment thereof, or the hIL-2/anti-IL-2 antibody fusion protein in the host cell; and (2) isolating the hIL-2 mutein, the anti-IL-2 antibody or antigen-binding fragment thereof, or the hIL-2/anti-IL-2 antibody fusion protein from the host cell or cell culture.

67. A bispecific molecule, comprising the anti-IL-2 antibody or antigen-binding fragment thereof of any one of claims 6-13, or the hIL-2/anti-IL-2 antibody fusion protein of any one of claims 14-62, wherein one of the two arms of the bispecific antibody comprise the anti-IL-2 antibody or antigen-binding fragment thereof, or the hIL-2/anti-IL-2 antibody fusion protein, while the other arm comprises an antibody specific for an antigen other than hIL-2, preferably a tumor-associated antigen or an immune checkpoint protein antigen, or comprises an antibody or an antigen-binding fragment thereof specifically binding to an antigen associated with immune effector cells, wherein the said tumor-associated antigen may be selected from HER2, EGFR, VEGF, VEGFR, CD19, CD20, CD30, CD33, CD52, BCMA, SLAMF7, MUC1, MUC16, EphB2, E-selectin, EpCam, CEA, PSMA, PSA, ERBb3, c-MET, ILT3, ILT7, 5T4, GPC-3, DLL3, etc., and the immune checkpoint protein antigen can be selected from PD-1, PD-L1, PD-L2, TIM3, CTLA-4, LAG-3, CEACAM-1, CEACAM-5, VISTA, BTLA, TIGIT, LAIR1, CD40, CD40L, OX40, CD160, 2B4, or TGFR.

68. An immunoconjugate, a chimeric antigen receptor, an engineered T cell receptor, or an oncolytic virus, comprising the anti-IL-2 antibody or antigen-binding fragment thereof of any one of claims 6-13, or the hIL-2/anti-IL-2 antibody fusion protein of any one of claims 14-62.

69. A pharmaceutical composition comprising the anti-IL-2 antibody or antigen-binding fragment thereof of any one of claims 6-13, the hIL-2/anti-IL-2 antibody fusion protein of any one of claims 14-62, the bispecific molecule of claim 67, or the immunoconjugate, the chimeric antigen receptor, the engineered T cell receptor, or the oncolytic virus of claim 68, and a pharmaceutically acceptable carrier.

70. A kit set, comprising the pharmaceutical composition of claim 69, and optionally another anticancer agent and/or immunomodulatory agent, wherein the anticancer agent comprises a microtubule disrupting agent, an antimetabolite, a topoisomerase inhibitor, a DNA intercalator, an alkylating agent, a hormone therapeutic agent, a kinase inhibitor (e.g., a tyrosine kinase inhibitor, including an EGFR inhibitor, a HER2 inhibitor, a HER3 inhibitor, an IGFR inhibitor and a Met inhibitor), a receptor antagonist, a tumor cell apoptosis activator (including an IAP inhibitor, a Bc12 inhibitor, a MC11 inhibitor, a TRAIL inhibitor, a CHK inhibitor), or any anti-angiogenic drug; the immunomodulatory agent comprises a PD-1, PD-L1, PD-L2, TIM3, CTLA-4, LAG-3, CEACAM-1, CEACAM-5, VISTA, BTLA, TIGIT, LAIR1, CD160, 2B4, or TGFR inhibitor.

71. A kit, comprising an effective amount of the anti-IL-2 antibody or antigen-binding fragment thereof of any one of claims 6-13, or the hIL-2/anti-IL-2 antibody fusion protein of any one of claims 14-62, or the pharmaceutical composition of claim 69.

72. Use of the anti-IL-2 antibody or antigen-binding fragment thereof of any one of claims 6-13, or the hIL-2/anti-IL-2 antibody fusion protein of any one of claims 14-62 in the manufacture of a pharmaceutical composition or the preparation for the treatment of diseases, including proliferative disease, infectious disease, and immunodeficiency disease, wherein the proliferative disease includes cancer and other cell proliferative disorders.

73. The use of claim 72, wherein the cancer includes melanoma, lung cancer, colorectal cancer, prostate cancer, breast cancer, ovarian cancer, cervical cancer, kidney cancer, liver cancer, brain cancer, esophageal cancer, gallbladder cancer, pancreatic cancer, gastric cancer, thyroid cancer, bladder cancer and lymphoma; the immunodeficiency disease includes HIV positive, immunosuppressive disease, chronic infection.

74. A method of treating a disease, condition or disorder by boosting host immune response, wherein the method comprises administering to a subject a therapeutically effective amount of the anti-IL-2 antibody or antigen-binding fragment thereof of claims 6-13, or the hIL-2/anti-IL-2 antibody fusion protein of claims 14-62, or the pharmaceutical composition of claim 69 , or the kit set of claim 70, or the kit of claim 71, to specifically activate effector cells expressing the IL2Rβ/γ(e.g., CD8$^+$ T cells and NK cells), thereby improving disease status.

75. A method to stimulate the immune system, wherein the method comprises administering to a subject a therapeutically effective amount of the anti-IL-2 antibody or antigen-binding fragment thereof of claim 6-13, or the hIL-2/anti-IL-2 antibody fusion protein of claim 14-62, or the pharmaceutical composition of claim 69, or the kit set of claim 70, or the kit of claim 71, to stimulate the immune system, wherein the immune system stimulation includes a general increase in immunity, an increase in T cell function, an increase in B cell function, a recovery of lymphocyte function, an increase in IL2Rβγ expression, an increase in T cell responsiveness, an increase in NK cell activity or lymphokine-activated killer cell activity, an increase in the ratio of CD8$^+$ T cells to CD4$^+$ T cells in the subject.

76. A method of treating, alleviating or preventing a disease condition in which stimulation of the host immune system is beneficial, wherein the method comprises administering to a subject a therapeutically effective amount of the anti-IL-2 antibody or antigen-binding fragment thereof of claims 6-13, or the hIL-2/anti-IL-2 antibody fusion protein of claim 14-62, or the pharmaceutical composition of claim 69, or the kit set of claim 70, or the kit of claim 71, to improve a disease condition by enhancing cellular immune responses in the subject, wherein the disease condition includes insufficient immune response or immunodeficiency in the host; and further the disease condition is a proliferative disease, an infectious disease, or an immunodeficiency disease, wherein the proliferative disease includes cancer and other cell proliferative disorders.

77. The method of claim 76, wherein the cancer is selected from: squamous cell carcinoma (e.g., epithelial squamous cell carcinoma), lung cancer (including small cell lung cancer, non-small cell lung cancer, adenocarcinoma of the lung, and squamous cell carcinoma of the lung), peritoneal cancer, hepatocellular carcinoma, gastric cancer (including gastrointestinal cancer and gastrointestinal stromal cancer), pancreatic cancer, glioblastoma, cervical cancer, ovarian cancer, liver cancer, bladder cancer, urethra cancer, liver tumor, breast cancer, colon cancer, rectal cancer, colorectal cancer, endometrial or uterine cancer, salivary gland cancer, kidney cancer, prostate cancer, vulvar cancer, thyroid cancer, anal cancer, penile cancer, melanoma, superficial spreading melanoma, lentigo maligna melanoma, acral melanoma, nodular melanoma, multiple myeloma and B-cell lymphoma, Non-Hodgkin Lymphoma, chronic lymphocytic leukemia (CLL), acute lymphoblastic leukemia (ALL), hairy cell leukemia, chronic myeloblastic leukemia, and post-transplant lymphoproliferative disorder (PTLD), as well as abnormal vascular proliferation associated with phakomatoses, edema (e.g., associated with brain tumor) and Meigs syndrome, brain tumor and brain cancer, and head and neck cancer.

78. The method of claim 76, further comprising administering an immune checkpoint inhibitor, an antigen-specific immunotherapeutic agent, a chemotherapeutic agent, an epigenetic modifier, a cytokine, a growth factor, an inhibitor, an antibody targeting tumor antigen, a tumor vaccine, and/or other cancer therapy;

    wherein the immune checkpoint includes PD-1, PD-L1, PD-L2, TIM3, CTLA-4, LAG-3, CEACAM-1, CEACAM-5, VISTA, BTLA, TIGIT, LAIR1, CD160, 2B4, or TGFR;
    the antigen-specific immunotherapeutic agent includes chimeric antigen receptor T cell (CAR-T cell), chimeric antigen receptor NK cell (CAR-NK cell), and/or tumor infiltrating lymphocyte (TIL);
    the other cancer therapy includes surgery, chemotherapy, cytotoxic agent, photodynamic therapy, immunomodulation or radiation therapy.

79. The method of claim 76, wherein the immunodeficiency disease includes HIV positive, immunosuppressive disease, chronic infection.

**CTLL-2**

Fig 1A

**NK-92**

Fig 1B

Fig 2

Fig 3A

Fig 3B

Fig 4A

Fig 4B

Fig 5A

Fig 5B

Fig 6A

Fig 6B

Fig 7

Fig 8A

Fig 8B

Fig 9A

Fig 9B

**CTLL-2**

Fig 10A

**NK-92**

Fig 10B

**CTLL-2**

Fig 11A

**NK-92**

Fig 11B

Fig 12A

Fig 12B

Fig 13A

Fig 13B

Fig 14A

Fig 14B

Fig 14C

Fig 15

Fig 16A

Fig 16B

Fig 17A

Fig 17B

K2G11：FcRn　　　　　K2G11-pITM：FcRn　　　　　K2G11-Cy05：FcRn

Fig 17C

图 18

Fig 19A

Fig 19B

Fig 19C

Fig 20

Fig 21A

Fig 21B

Fig 21C

Fig 22A

Fig 22B

Fig 22C

## Body weight

Fig 22D

Fig 23

**hCD25**

Fig 24A

**hCD122**

Fig 24B

**hCD25**

Fig 25A

**hCD122**

Fig 25B

## CTLL-2

Fig 26A

## NK-92

Fig 26B

**CTLL-2**

Fig 27A

**NK-92**

Fig 27B

## INTERNATIONAL SEARCH REPORT

| International application No. |
|---|
| **PCT/CN2022/109997** |

**A. CLASSIFICATION OF SUBJECT MATTER**

C07K 19/00(2006.01)i; C07K 14/55(2006.01)i; C07K 1/107(2006.01)i; A61K 38/20(2006.01)i; A61K 47/68(2017.01)i; A61P 35/00(2006.01)i; A61P 37/02(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

VEN, CNABS, CNTXT, EPTXT, USTXT, WOTXT, ISI Web of Knowledge, CNKI, NCBI GENBANK: 徕特康 (苏州) 生物制药有限公司, 白细胞介素-2, 白介素2, IL-2抗体, 抗体, 融合蛋白, 人源化抗体, Latticon (Suzhou) Biopharmaceuticals Co., Ltd., IL-2, fusion, Interleukin-2, Anti IL-2 mAb antibody, complex, K64G, N90R, M104E, Humanized, IL-2Rβγ, IL-2Rα, SEQ ID NOs: 7-38

**C. DOCUMENTS CONSIDERED TO BE RELEVANT**

| Category* | Citation of document, with indication, where appropriate, of the relevant passages | Relevant to claim No. |
|---|---|---|
| X | ROJAS, G. et al. "Deciphering the molecular bases of the biological effects of antibodies against Interleukin-2: A versatile platform for fine epitope mapping" *IMMUNOBIOLOGY*, Vol. 218, No. 1, 31 December 2013 (2013-12-31), abstract, page 107, left-hand column, paragraph 3, figure 1, table 1, page 109, table 2, page 110, table 3, and page 111, paragraph 2 | 1-5 |
| Y | ROJAS, G. et al. "Deciphering the molecular bases of the biological effects of antibodies against Interleukin-2: A versatile platform for fine epitope mapping" *IMMUNOBIOLOGY*, Vol. 218, No. 1, 31 December 2013 (2013-12-31), abstract, page 107, left-hand column, paragraph 3, figure 1, table 1, page 109, table 2, page 110, table 3, and page 111, paragraph 2 | 15-54, 56-79 |
| X | CN 109071648 A (PFIZER INC. et al.) 21 December 2018 (2018-12-21) description, paragraphs 9 and 16 | 6-13 |
| Y | CN 109071648 A (PFIZER INC. et al.) 21 December 2018 (2018-12-21) description, paragraphs 9 and 16 | 15-54, 56-79 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **12 October 2022** | **26 October 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2022/109997** |

### C.    DOCUMENTS CONSIDERED TO BE RELEVANT

| Category* | Citation of document, with indication, where appropriate, of the relevant passages | Relevant to claim No. |
|---|---|---|
| Y | TOMALA, J. et al. "Chimera of IL-2 Linked to Light Chain of anti-IL-2 mAb Mimics IL-2/ anti-IL-2 mAb Complexes Both Structurally and Functionally" *ACS CHEMICAL BIOLOGY*, Vol. 8, No. 5, 18 February 2013 (2013-02-18), abstract, and page 872, figure 1 | 14-79 |
| Y | LETOURNEAU, S. et al. "IL-2/anti-IL-2 antibody complexes show strong biological activity by avoiding interaction with IL-2 receptor α subunit CD25." *PNAS*, Vol. 107, No. 5, 02 February 2010 (2010-02-02), abstract, and page 2171, right-hand column, paragraph 1 | 14-79 |
| A | KRIEG, C. et al. "Improved IL-2 immunotherapy by selective stimulation of IL-2 receptors on lymphocytes and endothelial cells" *PNAS*, Vol. 107, No. 26, 29 June 2010 (2010-06-29), entire document | 1-79 |
| A | US 20170081382 A1 (AMGEN INC.) 23 March 2017 (2017-03-23) entire document | 1-79 |
| A | CN 101166823 A (CHIRON CORP.) 23 April 2008 (2008-04-23) entire document | 1-79 |
| A | CN 102199218 A (INSTITUTE OF BASIC MEDICAL SCIENCES, THE ACADEMY OF MILITARY MEDICAL SCIENCES) 28 September 2011 (2011-09-28) entire document | 1-79 |
| A | CN 110256583 A (INSTITUTE OF BIOPHYSICS, CHINESE ACADEMY OF SCIENCES) 20 September 2019 (2019-09-20) entire document | 1-79 |

Form PCT/ISA/210 (second sheet) (January 2015)

**INTERNATIONAL SEARCH REPORT**

| International application No. |
| --- |
| **PCT/CN2022/109997** |

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    [1]  The actually submitted sequence table is an XML file in Standard ST.26.

Form PCT/ISA/210 (continuation of first sheet) (January 2015)

**INTERNATIONAL SEARCH REPORT**

| International application No. |
| --- |
| **PCT/CN2022/109997** |

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **74-79**
   because they relate to subject matter not required to be searched by this Authority, namely:

   [1]   Claims 74-79 relate to a disease treatment method, and do not comply with PCT Rule 39.1(iv). The present report is formed on the basis that the claims are amended to relate to a pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/109997**

| Patent document cited in search report | | | Publication date (day/month/year) | Patent family member(s) | | | Publication date (day/month/year) |
|---|---|---|---|---|---|---|---|
| CN | 109071648 | A | 21 December 2018 | JP | 2018537962 | A | 27 December 2018 |
| | | | | US | 2019106488 | A1 | 11 April 2019 |
| | | | | KR | 20180064541 | A | 14 June 2018 |
| | | | | CA | 2946113 | A1 | 23 April 2017 |
| | | | | TW | 201722989 | A | 01 July 2017 |
| | | | | AU | 2016340989 | A1 | 26 April 2018 |
| | | | | WO | 2017070561 | A1 | 27 April 2017 |
| | | | | US | 2017114130 | A1 | 27 April 2017 |
| | | | | US | 2021061902 | A1 | 04 March 2021 |
| | | | | EP | 3365369 | A1 | 29 August 2018 |
| US | 20170081382 | A1 | 23 March 2017 | CA | 2906708 | A1 | 25 September 2014 |
| | | | | WO | 2014153063 | A1 | 25 September 2014 |
| | | | | NZ | 712066 | A | 28 May 2021 |
| | | | | CN | 105358570 | A | 24 February 2016 |
| | | | | DK | 2970423 | T3 | 08 July 2019 |
| | | | | ME | 03437 | B | 20 January 2020 |
| | | | | TR | 201910802 | T4 | 21 August 2019 |
| | | | | PL | 2970423 | T3 | 31 October 2019 |
| | | | | JP | 2016518823 | A | 30 June 2016 |
| | | | | CA | 3149348 | A1 | 25 September 2014 |
| | | | | CL | 2015002686 | A1 | 15 April 2016 |
| | | | | AU | 2014236281 | A1 | 24 September 2015 |
| | | | | UY | 35454 | A | 30 September 2014 |
| | | | | PE | 20151763 | A1 | 10 December 2015 |
| | | | | EA | 201591731 | A1 | 29 April 2016 |
| | | | | KR | 20150127185 | A | 16 November 2015 |
| | | | | DK | 2970441 | T3 | 03 June 2019 |
| | | | | PL | 2970441 | T3 | 30 September 2019 |
| | | | | CN | 105143253 | A | 09 December 2015 |
| | | | | US | 2018237489 | A1 | 23 August 2018 |
| | | | | CA | 2905141 | A1 | 25 September 2014 |
| | | | | CL | 2015002669 | A1 | 09 September 2016 |
| | | | | TW | 202115106 | A | 16 April 2021 |
| | | | | HK | 1220695 | A1 | 12 May 2017 |
| | | | | MX | 2015012890 | A | 03 December 2015 |
| | | | | KR | 20220101009 | A | 18 July 2022 |
| | | | | SI | 2970423 | T1 | 30 August 2019 |
| | | | | US | 2018319859 | A1 | 08 November 2018 |
| | | | | JO | 3796 | B1 | 31 January 2021 |
| | | | | RS | 58791 | B1 | 31 July 2019 |
| | | | | US | 2021094997 | A1 | 01 April 2021 |
| | | | | TW | 202005979 | A | 01 February 2020 |
| | | | | MA | 38477 | A1 | 29 September 2017 |
| | | | | TW | 201522366 | A | 16 June 2015 |
| | | | | JP | 2016514161 | A | 19 May 2016 |
| | | | | KR | 20150130342 | A | 23 November 2015 |
| | | | | EA | 201591766 | A1 | 29 February 2016 |
| | | | | US | 2014286898 | A1 | 25 September 2014 |
| | | | | PT | 2970441 | T | 11 June 2019 |
| | | | | SI | 2970441 | T1 | 31 July 2019 |

Form PCT/ISA/210 (patent family annex) (January 2015)

**INTERNATIONAL SEARCH REPORT**
Information on patent family members

International application No.

**PCT/CN2022/109997**

| Patent document cited in search report | | | Publication date (day/month/year) | Patent family member(s) | | | Publication date (day/month/year) |
|---|---|---|---|---|---|---|---|
| | | | | LT | 2970441 | T | 10 June 2019 |
| | | | | JP | 2019058182 | A | 18 April 2019 |
| | | | | TN | 2015000416 | A1 | 03 January 2017 |
| | | | | WO | 2014153111 | A2 | 25 September 2014 |
| | | | | AP | 2015008737 | A0 | 30 September 2015 |
| | | | | PT | 2970423 | T | 23 July 2019 |
| | | | | SG | 11201507420 U | A | 29 October 2015 |
| | | | | BR | 112015022440 | A2 | 24 October 2017 |
| | | | | LT | 2970423 | T | 27 May 2019 |
| | | | | US | 2017137485 | A1 | 18 May 2017 |
| CN | 101166823 | A | 23 April 2008 | CN | 101426916 | A | 06 May 2009 |
| | | | | CN | 1930300 | A | 14 March 2007 |
| CN | 102199218 | A | 28 September 2011 | None | | | |
| CN | 110256583 | A | 20 September 2019 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 20080034473 A, Gillies
- WO 9404678 A
- WO 9425591 A
- US 6005079 A
- EP 404097 A
- WO 9311161 A
- US 4816567 A
- US 5202238 A
- US 5204244 A
- US 5225539 A
- US 5530101 A
- US 5585089 A
- US 5693762 A
- US 6180370 B
- WO 2016005950 A
- WO 2018217058 A
- US 5624821 A
- US 6194551 B
- US 7087600 B
- US 6989452 B
- US 7129261 B
- WO 02096910 A
- WO 07038658 A
- WO 07051081 A

- WO 07059404 A
- WO 08083312 A
- WO 08103693 A
- US 20060024317 A
- US 20060004081 A
- US 20060247295 A
- US 5399163 A
- US 5383851 A
- US 5312335 A
- US 5064413 A
- US 4941880 A
- US 4790824 A
- US 4596556 A
- US 4487603 A
- US 4486194 A
- US 4447233 A
- US 4447224 A
- US 4439196 A
- US 4475196 A
- US 4522811 A
- US 5374548 A
- US 5416016 A
- US 5399331 A
- WO 2017122130 A

**Non-patent literature cited in the description**

- **SMITH.** *Science,* 1988, vol. 240, 1169-1176
- **BAZAN.** *Science,* 1992, vol. 257, 410-413
- **KREIG.** *PNAS,* 2010, vol. 107, 11906-11911
- **WANG.** *Science,* 2005, vol. 310, 1159-1163
- **BOYMAN.** *Nat Rev Immunol,* 2012, vol. 12, 180-190
- **ARENAS-RAMIREZ N.** *Trends Immunol,* 2015, vol. 36, 763-777
- **FONTENOT.** *Nat Immunol,* 2005, vol. 6, 1142-1151
- **JOSEFOWICZ.** *Annu Rev Immunol,* 2012, vol. 30, 531-564
- **MALEK ; BAYER.** *Nat Rev Immunol,* 2004, vol. 4, 665-674
- **ZHU.** *Annual Review of Immunology,* 2010, vol. 28, 445-489
- **LIAO.** *Nat Immunol,* 2008, vol. 9, 1288-1296
- **LIAO.** *Nat Immunol,* 2011, vol. 12, 551-559
- **CHENG.** *Immunol Rev,* 2011, vol. 241, 63-76
- **LIAO.** *Immunity,* 2013, vol. 38, 13-25

- **WALDMANN.** *Nat Rev Immuno,* 2006, vol. 6, 595-601
- **MALEK.** *Annu Rev Immunol,* 2008, vol. 26, 453-479
- **ATKINS.** *J Clin Oncol,* 1999, vol. 17, 2105-2116
- **FISHER.** *Cancer J Sci Am,* 2000, vol. 6 (1), S55-S57
- **KLAPPER.** *Cancer,* 2008, vol. 113, 293-301
- **KRIEG.** *Proc Nat Acad Sci USA,* 2010, vol. 107, 11906-11911
- **D'CRUZ.** *Nature Immunol,* 2005, vol. 6, 1152-1159
- **MALOY.** *Nature Immunol,* 2005, vol. 97, 189-192
- **FACCIABENE.** *Cancer Res,* 2012, vol. 72, 2162-2171
- **LEVIN.** *Nature,* 2012, vol. 484, 529-533
- **DEBORAH H.** *Clin Cancer Res,* 2016, vol. 22, 680-690

- **JANKU.** *European Journal of Cancer,* 2020
- **BONI.** *Journal of Clinical Oncology,* 2021, vol. 39 (15), 2513
- **KAMIMURA.** *J Immunol,* 2006, vol. 177, 306-314
- **BOYMAN.** *Science,* 2006, vol. 311, 1924-1927
- **LEE.** *Oncoimmunology,* 2020, vol. 9, e1681869
- **HARRIS KE.** *Scientific Reports,* 2021, vol. 11, 1-15
- **MARGOLIN.** *Clin Cancer Res,* 2007, vol. 13, 3312-3319
- **SILVA.** *Nature,* 2019, vol. 565, 186-191
- **HUSTON JS et al.** *Proc Natl Acad Sci USA,* 1988, vol. 85, 5879-5883
- **RIECHMANN L ; MUYLDERMANS S.** *J Immunol Methods,* 1999, vol. 231, 25-38
- **MUYLDERMANS S.** *J Biotechnol,* 2001, vol. 74, 277-302
- **HAMERS-CASTERMAN C et al.** *Nature,* 1993, vol. 363, 446-448
- **NGUYEN VK et al.** Heavy-chain antibodies in Camelidae: a case of evolutionary innovation. *Immunogenetics,* 2002, vol. 54, 39-47
- **NGUYEN VK et al.** *Immunology,* 2003, vol. 109, 93-101
- **KOCH-NOLTE F et al.** *FASEB J,* 2007, vol. 21, 3490-3498
- **HOLLIGER P et al.** *Proc Natl Acad Sci USA,* 1993, vol. 90, 6444-6448
- **KUBY.** Immunology. WH Freeman & Co, 2000
- **KABAT et al.** Sequences of Proteins of Immunological Interest. U.S. Department of Health and Human Services, 1991
- **JOHNSON et al.** *Nucleic Acids Res,* 2001, vol. 29, 205-206
- **CHOTHIA ; LESK.** *J Mol Biol,* 1987, vol. 196, 901-917
- **CHOTHIA et al.** *Nature,* 1989, vol. 342, 877-883
- **CHOTHIA et al.** *J Mol Biol,* 1992, vol. 227, 799-817
- **AL-LAZIKANI et al.** *J Mol Biol,* 1997, vol. 273, 927-748
- **LEFRANC MP et al.** *IMGT, The International ImmunoGenetics Database, Nucleic Acids Research,* 1999, vol. 27, 209-212
- **MACCALLUM RM et al.** *J Mol Biol,* 1996, vol. 262, 732-745
- **RUIZ et al.** *Nucleic Acids Res,* 2000, vol. 28, 219-221
- **LEFRANC MP.** *Nucleic Acids Res,* 2001, vol. 29, 207-209
- **LEFRANC MP.** *The Immunologist,* 1999, vol. 7, 132-136
- **LEFRANC MP et al.** *Dev Comp Immunol,* 2003, vol. 27, 55-77
- **MACCALLUM et al.** *J Mol Biol,* 1996, vol. 262, 732-745
- **MARTIN et al.** *Proc Natl Acad Sci USA,* 1989, vol. 86, 9268-9272
- **MARTIN et al.** *Methods Enzymol,* 1991, vol. 203, 121-153
- **REES et al.** Protein Structure Prediction. Oxford University Press, 1996, 141-172
- **KABAT et al.** NIH Publication No. 91-3242. National Technical Information Service, 1991
- **MORRISON SL et al.** *Proc Natl Acad Sci USA,* 1984, vol. 81, 6851-6855
- **ALTSCHUL SF et al.** *J Mol Biol,* 1990, vol. 215, 403-410
- **STEPHEN F et al.** *Nucleic Acids Res,* 1997, vol. 25, 3389-3402
- **HIGGINS DG et al.** *Methods in Enzymology,* 1996, vol. 266, 383-402
- **LARKIN MA et al.** *Bioinformatics,* 2007, vol. 23, 2947-2948
- **RIECHMANN et al.** *Nature,* 1998, vol. 332, 323-327
- **JONES et al.** *Nature,* 1986, vol. 321, 522-525
- **QUEEN et al.** *Proc Natl Acad Sci USA,* 1989, vol. 86, 10029-10033
- **KABAT et al.** *Ibid,* 1991
- **TOMLINSON et al.** *J Mol Biol,* 1992, vol. 227, 776-798
- **COX et al.** *Eur J Immunol,* 1994, vol. 24, 827-836
- **RAGHAVAN et al.** *Annu Rev Cell Dev Biol,* 1996, vol. 12, 181-220
- **GHETIE et al.** *Annu Rev Immunol,* 2000, vol. 18, 739-766
- **RAVETCH et al.** *Annu Rev Immunol,* 2001, vol. 19, 275-290
- **WARD et al.** *Ther Immunol,* 1995, vol. 2, 77-94
- **OGANESYAN V et al.** *Acta Crystallogr D Biol Crystallogr,* 2008, vol. 64, 700-704
- **JEFFERIS et al.** *Immunol Lett,* 2002, vol. 82, 57-65
- **BOSWELL CA et al.** *Bioconjug Chem,* 2010, vol. 21, 2153-2163
- **LI et al.** *MAbs,* 2014, vol. 6, 1255-1264
- **SAIKI et al.** *Science,* 1988, vol. 239, 487-491
- Recombinant DNA Methodology. Academic Press, Inc, 1989, 189-196
- PCR Protocols: A Guide to Methods and Applications. Academic Press, Inc, 1990
- **SAMBROOK et al.** Molecular Cloning: A Laboratory Manual. Cold Spring Harbor Laboratory, 1989
- **GRAHAM et al.** *J Gen Virol,* 1977, vol. 36, 59-74
- **URLAUB et al.** *Proc Natl Acad Sci USA,* 1980, vol. 77, 4216-4220
- **MATHER.** *Biol Reprod,* 1980, vol. 23, 243-251

- **MATHER et al.** *Ann NY Acad Sci,* 1982, vol. 383, 44-68
- **CAO ; SURESH.** *Bioconjugate Chemistry,* 1998, vol. 9, 635-644
- **VAN SPRIEL et al.** *Immunology Today,* 2000, vol. 21, 391-397
- Remington: The Science and Practice of Pharmacy. Lippincott Williams & Wilkins, 2003
- Sustained and Controlled Release Drug Delivery Systems. Marcel Dekker, Inc, 1978
- **RANADE VV.** *J Clin Pharmacol,* 1989, vol. 29, 685-694
- **UMEZAWA et al.** *Biochem Biophys Res Commun,* 1988, vol. 153, 1038-1044
- **BLOEMAN et al.** *FEBS Lett,* 1995, vol. 357, 140-144
- **OWAIS M et al.** *Antimicrob Agents Chemother,* 1995, vol. 39, 180-184
- **BRISCOE et al.** *Am J Physiol,* 1995, vol. 268, L374-L380
- **SCHREIER et al.** *J Biol Chem,* 1994, vol. 269, 9090-9098
- **KEINANEN ; LAUKKANEN.** *FEBS Lett,* 1994, vol. 346, 123-126
- **KILLION ; FIDLER.** *Immunomethods,* 1994, vol. 4, 273-279